# EUROPEAN PATENT APPLICATION

(11) **EP 2 009 006 A1**
(43) Date of publication of application: **31.12.2008**
(21) Application number: 07739727.1
(22) Date of filing: 27.03.2007
(51) Int. Cl.: C07D 263/48, A61K 31/4525, A61K 31/4535, A61K 31/454, A61K 31/46, A61K 31/496, A61P 25/04, A61P 29/00, C07D 277/20, C07D 277/42, C07D 401/04, C07D 405/04, C07D 409/04, C07D 413/04, C07D 417/04, C07D 451/02

(54) **UREIDE DERIVATIVE AND USE THEREOF FOR MEDICAL PURPOSES**

(30) Priority: 27.03.2006 JP 2006086563
(71) Applicant: TORAY INDUSTRIES, INC., Tokyo 103-8666 (JP)
(72) Inventor: SUGAWARA, Yuji, Kamakura-shi, Kanagawa 2488555 (JP); IZUMIMOTO, Naoki, Kamakura-shi, Kanagawa 2488555 (JP); TSUTSUI, Hideyuki, Kamakura-shi, Kanagawa 2488555 (JP)
(74) Representative: Kador, Ulrich
(86) International application number: PCT/JP2007/056289
(87) International publication number: WO 2007/111323

(57) **Abstract**

This invention relates to a pharmaceutical comprising, as an active ingredient, a ureide derivative represented by formula: 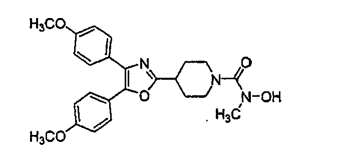 or a pharmaceutically acceptable salt thereof. The ureide derivative or a pharmaceutically acceptable salt thereof according to the present invention is useful for relieving pain and treating or preventing neuropathic pain.

## Description

### Technical Field

The present invention relates to an ureide derivative and pharmaceutical applications thereof.

### Background Art

The term "pain" refers to "an unpleasant feeling" or "an experience involving unpleasant emotions" that develops when tissue is or may be damaged, or "an unpleasant feeling" or "an experience involving unpleasant emotions" that is represented by a term indicating such damage.

Pain develops when tissue is damaged by a disease or external injuries and pain-producing substances are topically produced. Nociceptive pain, in particular, develops when a body is stimulated externally (e.g., occurrence of external injuries), or when internal tissue develops a lesion.

An example of another type of pain having a developmental mechanism different from that of nociceptive pain is neuropathic pain. Neuropathic pain results from a dysfunction that has developed in the central nervous system because of abnormality in peripheral tissue or peripheral nerve endings or damage to peripheral nerves. Further, neuropathic pain develops when the central nervous system is damaged.

A wide variety of alternatives are available for treatment of pain. Examples include nonsteroidal anti-inflammatory drugs (NSAIDs) or narcotic analgesics (e.g., opioids). Use of a nonsteroidal anti-inflammatory drug, however, causes adverse side effects, such as gastrointestinal injury or nephropathy, and use of a narcotic analgesic causes adverse side effects, such as constipation, drowsiness, nausea, or vomition.

In contrast, neuropathic pain is treated with the use of an anticonvulsant, such as gabapentin or pregabalin, and the frequency of development of central nervous system side effects, such as dizziness, nausea, or vomition is high. Accordingly, development of novel analgesics and therapeutic agents for neuropathic pain has been awaited.

Several reports have been made regarding analgesic effects that are expected for compounds having diaryl-substituted hetero 5-membered ring. In particular, JP Patent No. 3003148 discloses a compound similar to the compound of the present invention having a thiazole skeleton. Specifically, JP Patent No. 3003148 relates to a thiazole compound represented by the formula below, a method for producing the same, and a pharmaceutical composition comprising the same: 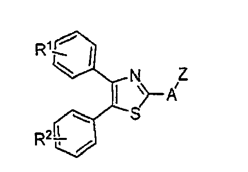 wherein R¹ and R² each independently represent a halogen atom, a lower alkyloxy group, or the like; A represents a single bond or the like; and Z represents a piperazinyl group, a piperidyl group, or the like.

Also, JP Patent Publication (kohyo) No. 2006-514095 A discloses a compound similar to the compound of the present invention having a pyrazole skeleton. More particularly, JP Patent Publication (kohyo) No. 2006-514095 A relates to a pyrazole derivative represented by the formula below and pharmaceutical applications thereof: 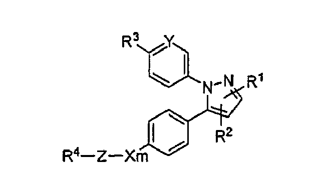 wherein R¹ represents a hydrogen atom or a lower alkyl group; R² represents a lower alkyl group or the like; R³ represents a lower alkoxy group or the like; R⁴ represents a hydroxyl group or the like; X represents O, S, or the like; Y represents CH or N; Z represents a lower alkylene or lower alkenylene group; and m is 0 or 1.

Further, JP Patent Publication (kohyo) No. 2006-517535 A discloses a compound similar to the compound of the present invention having an oxazole skeleton. More particularly, JP Patent Publication (kohyo) No. 2006-517535 A relates to an oxazole derivative represented by the formula below and pharmaceutical applications thereof: 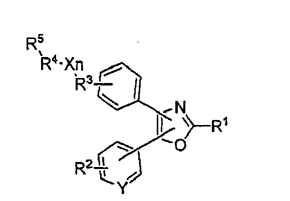 wherein R¹ represents a hydrogen atom, a lower alkyl group, or the like; R² represents a lower alkyl group or the like; R³ represents a lower alkylene group, a lower alkenylene group, or a covalent bond; R⁴ represents a lower alkylene group, a lower alkenylene group, or a covalent bond; R⁵ represents a hydrogen atom, a lower alkyl group, or the like; X represents O, S, or the like; Y represents CH or N; and n is 0 or 1.

Further, JP Patent Publication (kohyo) No. 2004-517121 A discloses a compound similar to the compound of the present invention having an imidazole skeleton. More particularly, JP Patent Publication (kohyo) No. 2004-517121 A relates to an imidazole derivative represented by the formula below and pharmaceutical applications thereof: 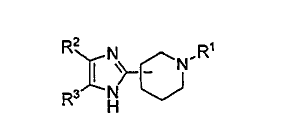 wherein R¹ represents a hydrogen atom or -X-Y-R⁴; R² represents an alkyl group, an aryl group, or the like; R³ represents an optionally-substituted aryl group, or the like; R⁴ represents an alkyl group or the like; and X-Y represents a single bond, -CO-, -CONH-, or the like.

However, none of these patent documents describe the ureide derivative of the present invention, and none of such documents suggest the efficacy of the compound of the present invention as a pharmaceutical.

### Disclosure of the Invention

### Object to Be Attained by the Invention

The present invention provides an excellent compound that is useful for treating or preventing various types of pain or neuropathic pain and pharmaceutical applications thereof.

### Means for Attaining the Object

The present inventors have conducted concentrated studies in order to attain the above object. As a result, they discovered a ureide derivative exhibiting the effects of ameliorating various types of pain or neuropathic pain, thereby completing the present invention.

Specifically, the present invention is summarized as follows.
(1) An ureide derivative represented by general formula (I): 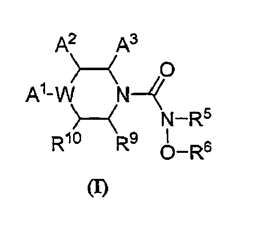 wherein either A¹ or A² represents a hydrogen atom, and the other represents formula (IIa), (IIb), or (IIc); 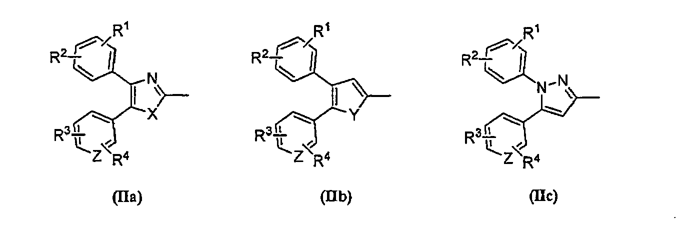 wherein A³ represents a hydrogen atom, a halogen atom, an alkyl group having 1 to 6 carbon atoms, a cyano group, or a hydroxyl group; X and Y each independently represent O, S, or NR⁷; Z represents CH or N, W represents CR⁸ or N; R¹, R², R³, and R⁴ each independently represent a hydrogen atom, a halogen atom, an alkyl group having 1 to 6 carbon atoms, a haloalkyl group having 1 to 3 carbon atoms, an alkoxy group having 1 to 4 carbon atoms, a hydroxyl group, or a 2-hydroxyethoxy group; R⁵ represents a hydrogen atom, an alkyl group having 1 to 6 carbon atoms, or -(CH₂)ₘCO₂R¹¹, wherein m is an integer between 0 and 3; R⁶ represents a hydrogen atom, an alkyl group having 1 to 6 carbon atoms, an aralkyl group having 7 to 12 carbon atoms, an acyl group having 2 to 8 carbon atoms, or -(CH₂)ₙCO₂R¹², wherein n is an integer between 0 and 3; R⁷ represents a hydrogen atom, an alkyl group having 1 to 6 carbon atoms, an acyl group having 2 to 8 carbon atoms, or -(CH₂)₁CO₂R¹³, wherein 1 is an integer between 0 and 3; R⁸ represents a hydrogen atom, an alkyl group having 1 to 6 carbon atoms, an alkenyl group having 2 to 6 carbon atoms, a cyano group, a phenyl group, a carbamoyl group, an amino group, -(CH₂)ₖOR¹⁴, wherein k is an integer between 0 and 3, or -(CH₂)ⱼCO₂R¹⁵, wherein j is 0 or 1; R⁹ and R¹⁰ each independently represent a hydrogen atom, a halogen atom, an alkyl group having 1 to 6 carbon atoms, a cyano group, or a hydroxyl group, A³ and R⁹ may together form -(CH₂)₂-; R¹¹ and R¹³ each independently represent a hydrogen atom or an alkyl group having 1 to 6 carbon atoms; R¹² and R¹⁵ each independently represent a hydrogen atom, an alkyl group having 1 to 6 carbon atoms, or an aralkyl group having 7 to 12 carbon atoms; and R¹⁴ represents a hydrogen atom, an alkyl group having 1 to 6 carbon atoms, or an acyl group having 2 to 8 carbon atoms, or a pharmaceutically acceptable salt thereof.
(2) The ureide derivative according to (1), wherein A¹ represents formula (IIa), (IIb), or (IIc) and A² represents a hydrogen atom, or a pharmaceutically acceptable salt thereof.
(3) The ureide derivative according to (1) or (2), wherein A¹ represents formula (IId), (IIe), or (IIf): 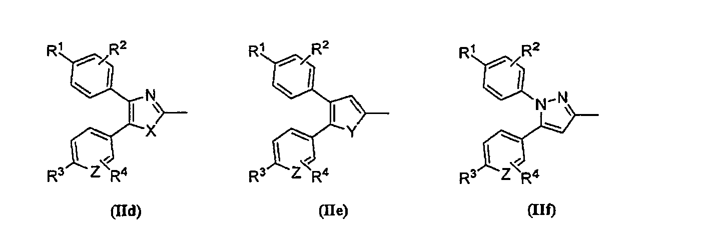 wherein X, Y, Z, R¹, R², R³, and R⁴ are as defined above, or a pharmaceutically acceptable salt thereof.
(4) The ureide derivative according to any one of (1) to (3), wherein A³ represents a hydrogen atom, a fluorine atom, a chlorine atom, a methyl group, an ethyl group, a cyano group, or a hydroxyl group, or a pharmaceutically acceptable salt thereof.
(5) The ureide derivative according to any one of (1) to (4), wherein X represents O or S and Y represents O, or a pharmaceutically acceptable salt thereof.
(6) The ureide derivative according to any one of (1) to (5), wherein W represents CR⁸ wherein R⁸ is as defined above, or a pharmaceutically acceptable salt thereof.
(7) The ureide derivative according to any one of (1) to (6), wherein R¹, R², R³, and R⁴ each independently represent a hydrogen atom, a fluorine atom, a chlorine atom, a methyl group, an ethyl group, an isopropyl group, a tert-butyl group, a difluoromethyl group, a trifluoromethyl group, a methoxy group, an ethoxy group, an isopropyloxy group, a hydroxyl group, or a 2-hydroxyethoxy group, or a pharmaceutically acceptable salt thereof.
(8) The ureide derivative according to any one of (1) to (7), wherein R⁵ represents a hydrogen atom, a methyl group, an ethyl group, an isopropyl group, a cyclohexyl group, a tert-butyl group, -CH₂CO₂CH₃, -CH₂CO₂CH₂CH₃, or -CH₂CO₂H, or a pharmaceutically acceptable salt thereof.
(9) The ureide derivative according to any one of (1) to (8), wherein R⁶ represents a hydrogen atom, a methyl group, an ethyl group, an isopropyl group, a tert-butyl group, a benzyl group, an acetyl group, a benzoyl group, -CO₂CH₃, -CH₂CO₂CH₃, -CH₂CO₂CH₂CH₃, or -CH₂CO₂H, or a pharmaceutically acceptable salt thereof.
(10) The ureide derivative according to any one of (1) to (9), wherein R⁸ represents a hydrogen atom, a methyl group, an ethyl group, a propyl group, 2-propenyl group, a cyano group, a phenyl group, a carbamoyl group, an amino group, a hydroxyl group, a hydroxymethyl group, an acetoxymethyl group, -CO₂CH₃, -CO₂CH₂CH₃, -CO₂CH₂Ph, -CO₂H, -CH₂CO₂CH₃, -CH₂CO₂CH₂CH₃, or -CH₂CO₂H, or a pharmaceutically acceptable salt thereof.
(11) The ureide derivative according to any one of (1) to (10), wherein R⁹ and R¹⁰ each independently represent a hydrogen atom, a fluorine atom, a chlorine atom, a methyl group, an ethyl group, a cyano group, or a hydroxyl group, R⁹ and A³ may together form - (CH₂)₂-, or a pharmaceutically acceptable salt thereof.
(12) The ureide derivative according to any one of (1) to (10), wherein all of R⁹, R¹⁰, and A³ represent a hydrogen atom, or a pharmaceutically acceptable salt thereof.
(13) A pharmaceutical comprising the ureide derivative according to any one of (1) to (12) or a pharmaceutically acceptable salt thereof.
(14) An analgesic comprising the ureide derivative according to any one of (1) to (12) or a pharmaceutically acceptable salt thereof.
(15) A therapeutic or preventive agent for neuropathic pain comprising the ureide derivative according to any one of (1) to (12) or a pharmaceutically acceptable salt thereof.
(16) A method of relieving pain comprising administering the ureide derivative according to any one of (1) to (12) or a pharmaceutically acceptable salt thereof.
(17) A method of treating or preventing neuropathic pain comprising administering the ureide derivative according to any one of (1) to (12) or a pharmaceutically acceptable salt thereof.
(18) Use of the ureide derivative according to any one of (1) to (12) or a pharmaceutically acceptable salt thereof for the manufacture of an analgesic.
(19) Use of the ureide derivative according to any one of (1) to (12) or a pharmaceutically acceptable salt thereof for the manufacture of a therapeutic or preventive agent for neuropathic pain.

### Effects of the Invention

The compound of the present invention exhibits excellent analgesic effects and effects of treating or preventing neuropathic pain. Thus, such compound is useful as a novel analgesic and therapeutic or preventive agent for neuropathic pain.

### Best Modes for Carrying out the Invention

In general formulae (I) and (IIa) to (IIf) of the present invention, an alkyl group having 1 to 6 carbon atoms represented by A³ R¹, R², R³, R⁴, R⁵, R⁶, R⁷, R⁸, R⁹, R¹⁰, R¹¹, R¹², R¹³, R¹⁴, or R¹⁵ is a linear, branched, or cyclic alkyl group having 1 to 6 carbon atoms, such as a methyl group, an ethyl group, an n-propyl group, an isopropyl group, a cyclopropyl group, a cyclopropylmethyl group, an n-butyl group, a sec-butyl group, a tert-butyl group, a hexyl group, or a cyclohexyl group.

An aralkyl group having 7 to 12 carbon atoms represented by R⁶, R¹², or R¹⁵ is, for example, a benzyl group, a phenethyl group, a naphthylmethyl group, or a naphthylethyl group.

An acyl group having 2 to 8 carbon atoms represented by R⁶, R⁷, or R¹⁴ is an aliphatic or aromatic acyl group having 2 to 8 carbon atoms, such as an acetyl group, a propionyl group, a butanoyl group, an isobutanoyl group, a pivaloyl group, a benzoyl group, an o-toluoyl group, an m-toluoyl group, a p-toluoyl group, an o-methoxybenzoyl group, a m-methoxybenzoyl group, or a p-methoxybenzoyl group.

A halogen atom represented by A³, R¹, R², R³, R⁴, R⁹, or R¹⁰ is a fluorine atom, a chlorine atom, a bromine atom, or an iodine atom.

A haloalkyl group having 1 to 3 carbon atoms represented by R¹, R², R³, or R⁴ is a group in which all or part of the hydrogen atom of the linear alkyl group having 1 to 3 carbon atoms has been substituted with the above-described halogen atom. Examples thereof include a monochloromethyl group, a monofluoromethyl group, a difluoromethyl group, a trifluoromethyl group, a trichloromethyl group, or a pentafluoroethyl group.

An alkoxy group having 1 to 4 carbon atoms represented by R¹, R², R³, or R⁴ is a linear, branched, or cyclic alkyl-oxy group having 1 to 4 carbon atoms, such as a methoxy group, an ethoxy group, an n-propyloxy group, an isopropyloxy group, a cyclopropyloxy group, or a tert-butoxy group.

An alkenyl group having 2 to 6 carbon atoms represented by R⁸ is a linear, branched, or cyclic alkenyl group having 2 to 6 carbon atoms, such as a vinyl group, a 1-propenyl group, 2-propenyl group (an allyl group), an isopropenyl group, a 1-butenyl group, a 2-butenyl group, a 3-butenyl group, a 1-methyl-1-propenyl group, a 2-methyl-2-butenyl group, a 1-cyclopentenyl group, a 2-cyclopentenyl group, a 1-cyclohexenyl group, a 2-cyclohexenyl group, or a 3-cyclohexenyl group.

In general formula (I), either one of A¹ and A² represents a hydrogen atom and the other group represents formula (IIa), (IIb), or (IIc). Preferably, A¹ represents formula (IIa), (IIb), or (IIc), and A² represents a hydrogen atom. More preferably, A¹ represents formula (IIa) or (IIc), and A² represents a hydrogen atom. Further preferably, A¹ represents formula (IId) or (IIf), and A² represents a hydrogen atom.

In general formula (I), A³ preferably represents a hydrogen atom, a fluorine atom, a chlorine atom, a methyl group, an ethyl group, a cyano group, or a hydroxyl group, more preferably represents a hydrogen atom or a methyl group, and further preferably represents a hydrogen atom. Also, A³ and R⁹ may together form -(CH₂)₂-.

In general formulae (IIa) and (IId), X represents O, S, or NR⁷. As an analgesic, X preferably represents O or S, and further preferably represents O. As a therapeutic or preventive agent for neuropathic pain, X preferably represents O or S, with S being further preferable.

In general formulae (IIb) and (IIe), Y represents O, S, or NR⁷, preferably represents O or S, and further preferably represents O.

In general formula (I), W represents CR⁸ or N, and preferably represents CR⁸.

In general formulae (IIa) to (IIf), R¹, R², R³, and R⁴ each independently and preferably represent a hydrogen atom, a fluorine atom, a chlorine atom, a methyl group, an ethyl group, an isopropyl group, a tert-butyl group, a difluoromethyl group, a trifluoromethyl group, a methoxy group, an ethoxy group, an isopropyloxy group, a hydroxyl group, or a 2-hydroxyethoxy group, more preferably a hydrogen atom, a fluorine atom, a chlorine atom, a methyl group, an ethyl group, a trifluoromethyl group, a methoxy group, an ethoxy group, or a hydroxyl group. Further preferably, R¹ and R³ each independently represent a hydrogen atom, a fluorine atom, a chlorine atom, a methyl group, or a methoxy group. R² and R⁴ each independently and further preferably represent a hydrogen atom, a fluorine atom, a methyl group, or a methoxy group, and most preferably represent a hydrogen atom or a methoxy group.

In general formula (I), R⁵ preferably represents a hydrogen atom, a methyl group, an ethyl group, an isopropyl group, a cyclohexyl group, a tert-butyl group, -CH₂CO₂CH₃, - CH₂CO₂CH₂CH₃, or -CH₂CO₂H, more preferably represents a methyl group, an ethyl group, or an isopropyl group, and further preferably represents a methyl group.

In general formula (I), R⁶ preferably represents a hydrogen atom, a methyl group, an ethyl group, an isopropyl group, a tert-butyl group, a benzyl group, an acetyl group, a benzoyl group, -CO₂CH₃, -CH₂CO₂CH₃, -CH₂CO₂CH₂CH₃, or -CH₂CO₂H, more preferably represents a hydrogen atom or a methyl group, and further preferably represents a hydrogen atom.

In general formulae (IIa), (IIb), (IId), and (IIe), when X and Y each independently represent NR⁷, R⁷ preferably represents a hydrogen atom, a methyl group, an ethyl group, an isopropyl group, an n-hexyl group, an acetyl group, -CH₂CO₂CH₃, -CH₂CO₂CH₂CH₃, or - CH₂CO₂H, and more preferably represents a hydrogen atom, -CH₂CO₂CH₃, -CH₂CO₂CH₂CH₃, or -CH₂CO₂H.

In general formula (I), when W represents CR⁸, R⁸ preferably represents a hydrogen atom, a methyl group, an ethyl group, a propyl group, a 2-propenyl group, a cyano group, a phenyl group, a carbamoyl group, an amino group, a hydroxyl group, a hydroxymethyl group, an acetoxymethyl group, -CO₂CH₃, -CO₂CH₂CH₃, -CO₂CH₂Ph, -CO₂H, -CH₂CO₂CH₃, -CH₂CO₂CH₂CH₃, or -CH₂CO₂H, more preferably represents a hydrogen atom, a methyl group, an ethyl group, a cyano group, a carbamoyl group, a hydroxyl group, a hydroxymethyl group, an acetoxymethyl group, -CO₂CH₃, -CO₂CH₂CH₃, -CO₂H, -CH₂CO₂CH₃, - CH₂CO₂CH₂CH₃, or -CH₂CO₂H, and further preferably represents a hydrogen atom, a methyl group, an ethyl group, a cyano group, a carbamoyl group, a hydroxyl group, -CO₂CH₃, or - CO₂CH₂CH₃.

In general formula (I), R⁹ and R¹⁰ each independently and preferably represent a hydrogen atom, a fluorine atom, a chlorine atom, a methyl group, an ethyl group, a cyano group, or a hydroxyl group, more preferably represents a hydrogen atom or a methyl group, and further preferably represents a hydrogen atom. Also, A³ and R⁹ may together form -(CH₂)₂-.

Specific examples of preferable compounds represented by general formula (I) of the present invention are provided in Table 1, although the present invention is not limited thereto.

**Table 1**

| No | Structural formula | No | Structural formula | No | Structural formula |
|---|---|---|---|---|---|
| 1 | 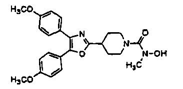 | 2 | 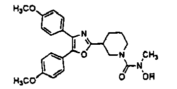 | 3 | 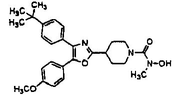 |
| 4 | 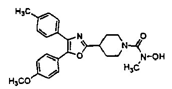 | 5 | 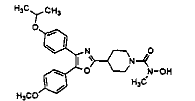 | 6 | 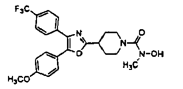 |
| 7 | 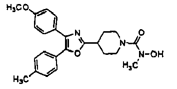 | 8 | 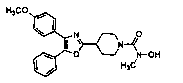 | 9 | 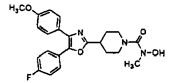 |
| 10 | 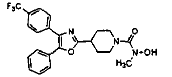 | 11 | 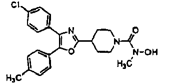 | 12 | 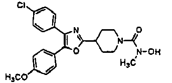 |
| 13 | 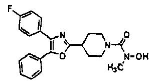 | 14 | 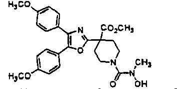 | 15 | 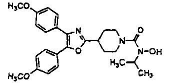 |
| 16 | 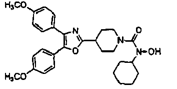 | 17 | 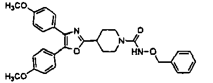 | 18 | 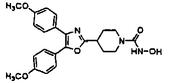 |
| 19 | 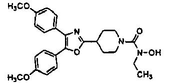 | 20 | 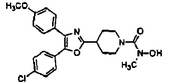 | 21 | 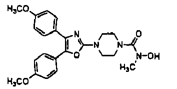 |
| 22 | 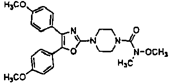 | 23 | 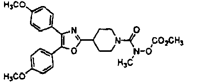 | 24 | 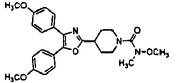 |
| 25 | 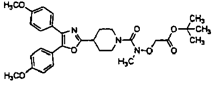 | 26 | 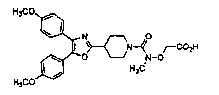 | 27 | 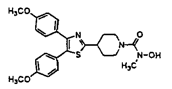 |
| 28 | 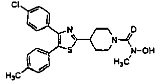 | 29 | 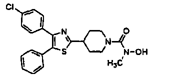 | 30 | 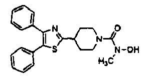 |
| 31 | 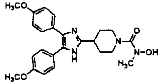 | 32 | 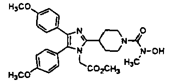 | 33 | 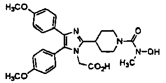 |
| 34 | 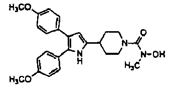 | 35 | 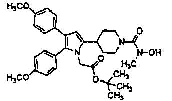 | 36 | 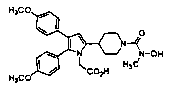 |
| 37 | 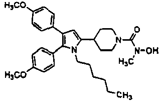 | 38 | 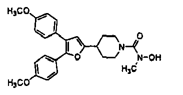 | 39 | 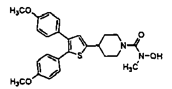 |
| 40 | 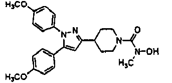 | 41 | 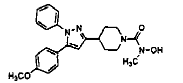 | 42 | 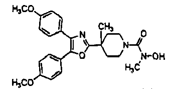 |
| 43 | 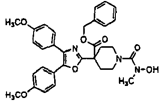 | 44 | 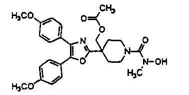 | 45 | 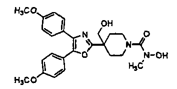 |
| 46 | 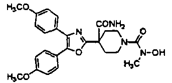 | 47 | 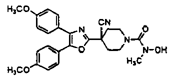 | 48 | 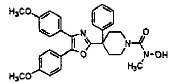 |
| 49 | 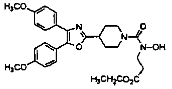 | 50 | 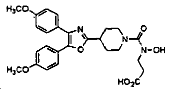 | 51 | 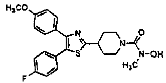 |
| 52 | 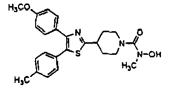 | 53 | 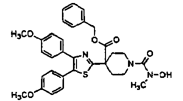 | 54 | 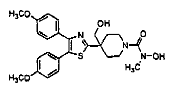 |
| 55 | 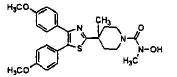 | 56 | 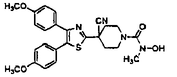 | 57 | 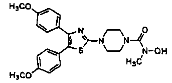 |
| 58 | 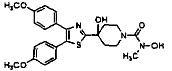 | 59 | 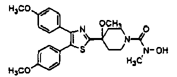 | 60 | 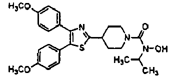 |
| 61 | 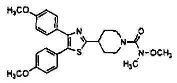 | 62 | 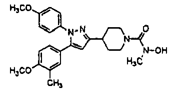 | 63 | 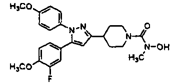 |
| 64 | 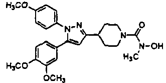 | 65 | 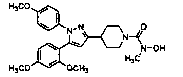 | 66 | 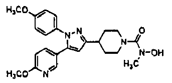 |
| 67 | 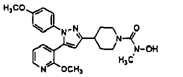 | 68 | 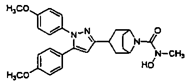 | 69 | 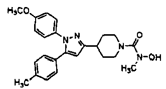 |
| 70 | 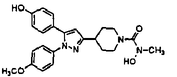 | 71 | 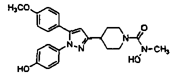 | 72 | 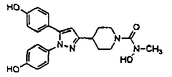 |
| 73 | 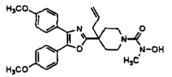 | 74 | 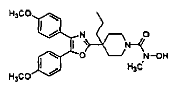 | 75 | 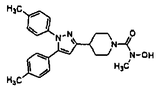 |
| 76 | 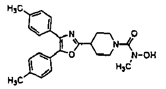 | 77 | 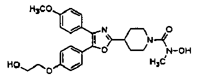 | 78 | 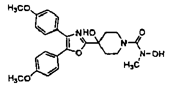 |
| 79 | 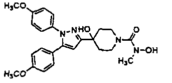 | 80 | 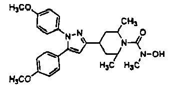 | 81 | 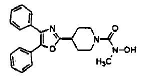 |
| 82 | 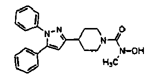 | 83 | 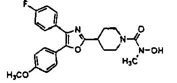 | 84 | 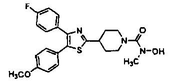 |
| 85 | 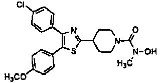 | 86 | 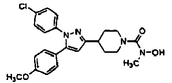 | 87 | 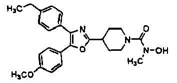 |
| 88 | 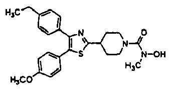 | 89 | 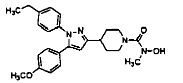 | 90 | 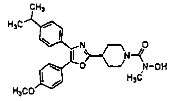 |
| 91 | 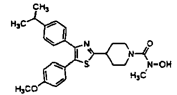 | 92 | 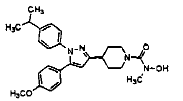 | 93 | 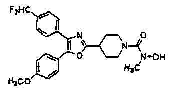 |
| 94 | 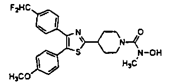 | 95 | 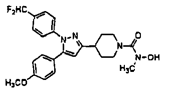 | 96 | 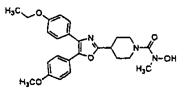 |
| 97 | 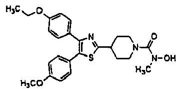 | 98 | 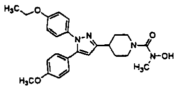 | 99 | 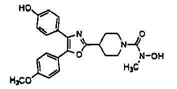 |
| 100 | 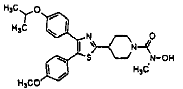 | 101 | 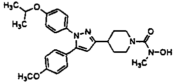 | 102 | 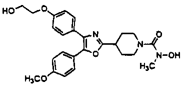 |
| 103 | 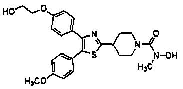 | 104 | 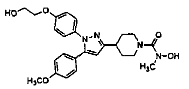 | 105 | 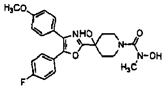 |
| 106 | 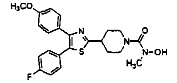 | 107 | 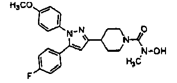 | 108 | 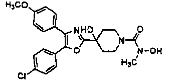 |
| 109 | 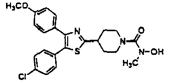 | 110 | 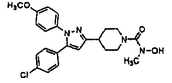 | 111 | 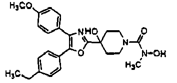 |
| 112 | 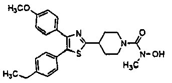 | 113 | 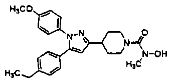 | 114 | 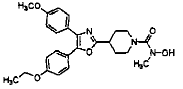 |
| 115 | 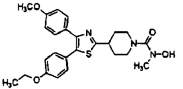 | 116 | 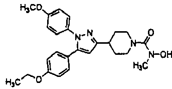 | 117 | 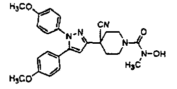 |
| 118 | 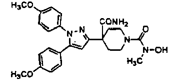 | 119 | 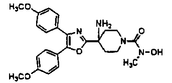 | 120 | 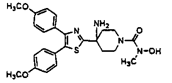 |
| 121 | 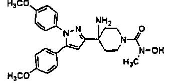 | 122 | 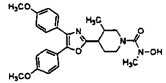 | 123 | 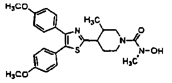 |
| 124 | 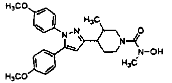 | 125 | 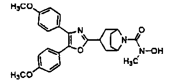 | 126 | 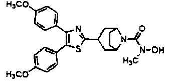 |
| 127 | 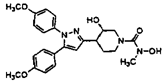 | 128 | 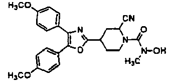 | 129 | 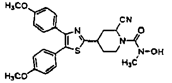 |
| 130 | 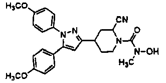 | 131 | 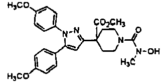 | 132 | 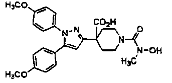 |
| 133 | 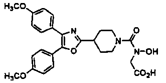 | 134 | 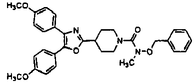 | 135 | 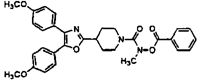 |
| 136 | 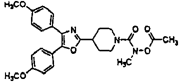 | 137 | 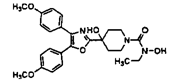 | 138 | 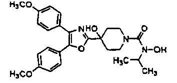 |
| 139 | 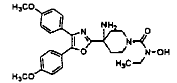 | 140 | 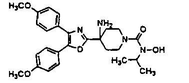 | 141 | 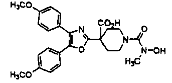 |
| 142 | 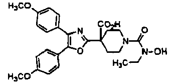 | 143 | 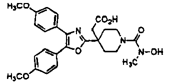 | 144 | 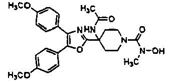 |
| 145 | 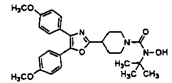 | 146 | 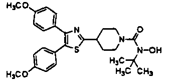 | 147 | 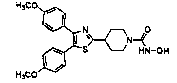 |
| 148 | 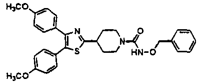 | 149 | 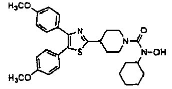 | 150 | 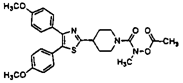 |
| 151 | 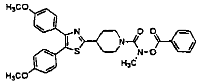 | 152 | 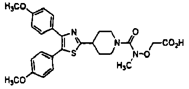 | 153 | 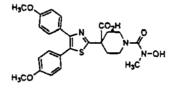 |
| 154 | 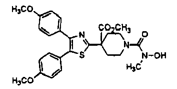 | 155 | 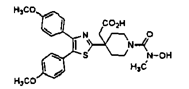 | 156 | 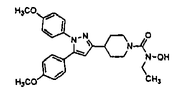 |
| 157 | 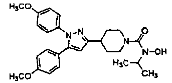 | 158 | 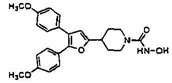 | 159 | 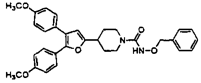 |
| 160 | 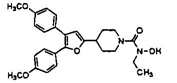 | 170 | 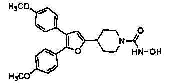 | 171 | 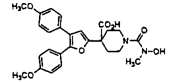 |
| 172 | 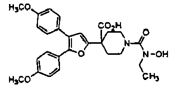 | 173 | 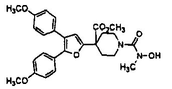 | 174 | 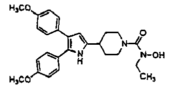 |
| 175 | 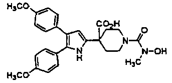 | 176 | 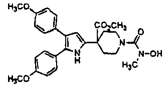 | 177 | 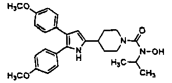 |
| 178 | 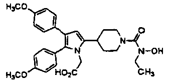 | 179 | 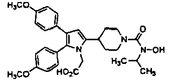 | 180 | 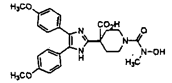 |
| 181 | 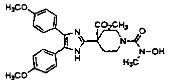 | 182 | 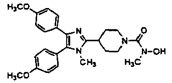 | 183 | 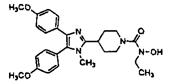 |
| 184 | 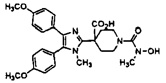 | 185 | 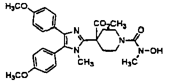 | 186 | 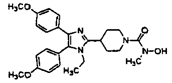 |
| 187 | 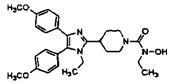 | 188 | 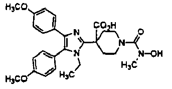 | 189 | 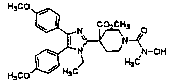 |
| 190 | 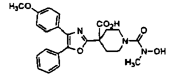 | 191 | 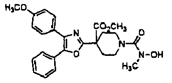 | 192 | 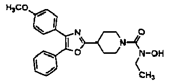 |
| 193 | 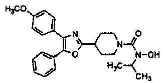 | 194 | 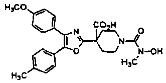 | 195 | 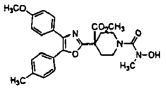 |
| 196 | 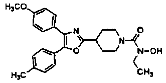 | 197 | 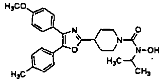 | 198 | 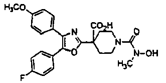 |
| 199 | 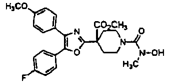 | 200 | 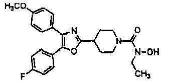 | 201 | 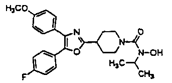 |
| 202 | 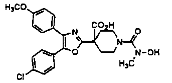 | 203 | 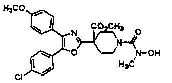 | 204 | 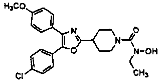 |
| 205 | 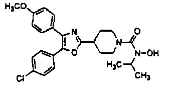 | 206 | 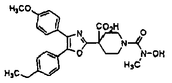 | 207 | 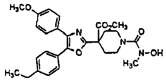 |
| 208 | 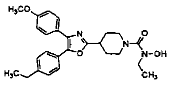 | 209 | 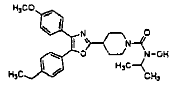 | 210 | 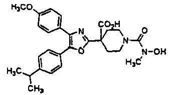 |
| 211 | 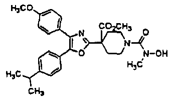 | 212 | 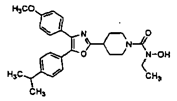 | 213 | 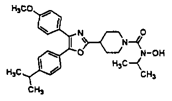 |
| 214 | 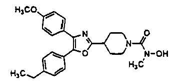 | 215 | 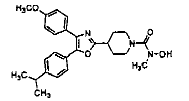 | 216 | 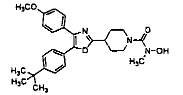 |
| 217 | 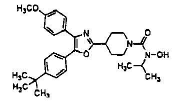 | 218 | 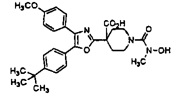 | 219 | 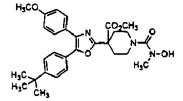 |
| 220 | 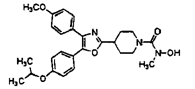 | 221 | 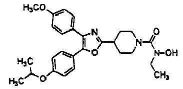 | 222 | 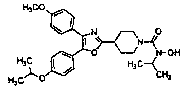 |
| 223 | 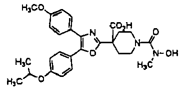 | 224 | 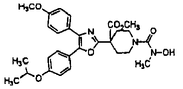 | 225 | 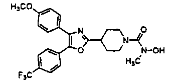 |
| 226 | 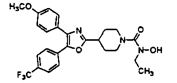 | 227 | 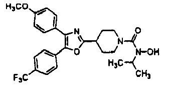 | 228 | 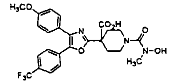 |
| 229 | 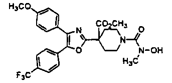 | 230 | 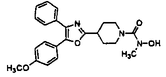 | 231 | 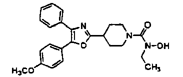 |
| 232 | 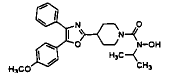 | 233 | 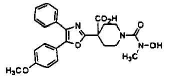 | 234 | 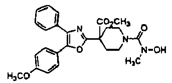 |
| 235 | 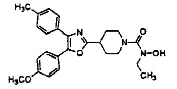 | 236 | 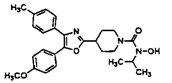 | 237 | 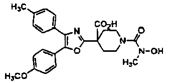 |
| 238 | 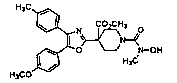 | 239 | 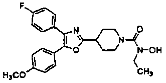 | 240 | 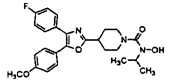 |
| 241 | 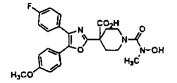 | 242 | 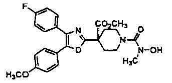 | 243 | 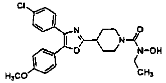 |
| 244 | 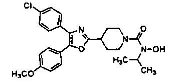 | 245 | 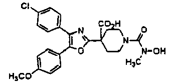 | 246 | 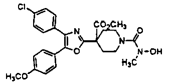 |
| 247 | 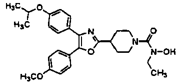 | 248 | 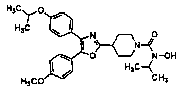 | 249 | 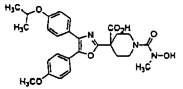 |
| 250 | 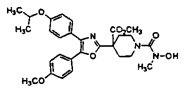 | 251 | 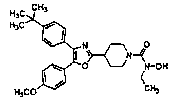 | 252 | 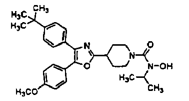 |
| 253 | 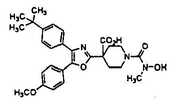 | 254 | 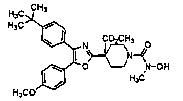 | 255 | 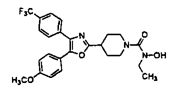 |
| 256 | 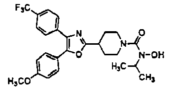 | 257 | 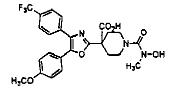 | 258 | 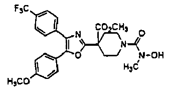 |
| 259 | 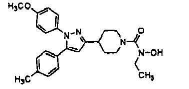 | 260 | 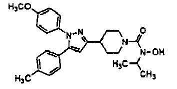 | 261 | 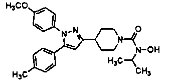 |
| 262 | 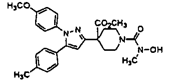 | 263 | 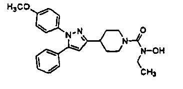 | 264 | 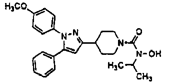 |
| 265 | 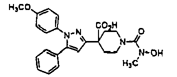 | 266 | 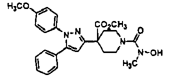 | 267 | 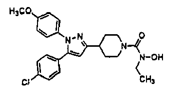 |
| 268 | 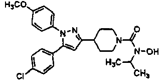 | 269 | 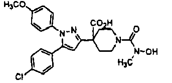 | 270 | 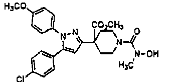 |
| 271 | 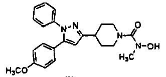 | 272 | 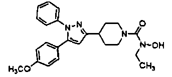 | 273 | 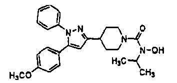 |
| 274 | 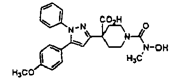 | 275 | 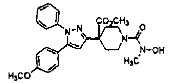 | 276 | 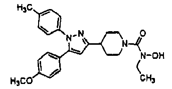 |
| 277 | 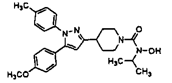 | 278 | 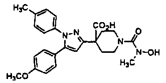 | 279 | 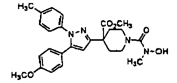 |
| 280 | 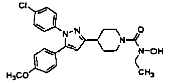 | 281 | 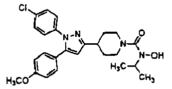 | 282 | 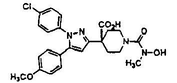 |
| 283 | 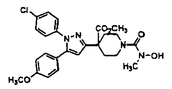 | 284 | 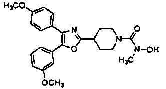 | 285 | 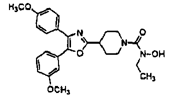 |
| 286 | 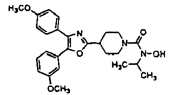 | 287 | 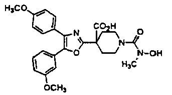 | 288 | 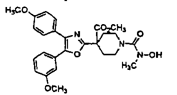 |
| 289 | 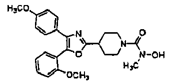 | 290 | 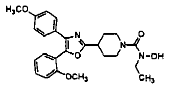 | 291 | 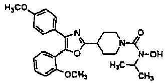 |
| 292 | 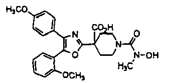 | 293 | 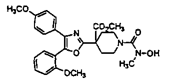 | 294 | 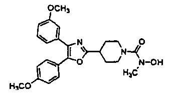 |
| 295 | 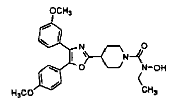 | 296 | 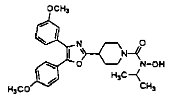 | 297 | 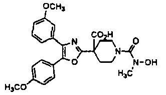 |
| 298 | 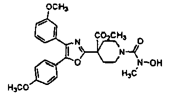 | 299 | 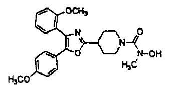 | 300 | 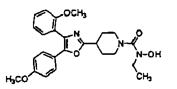 |
| 301 | 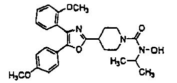 | 302 | 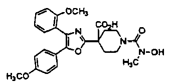 | 303 | 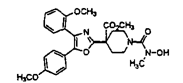 |
| 304 | 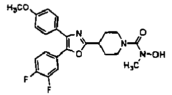 | 305 | 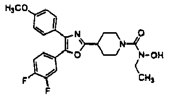 | 306 | 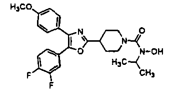 |
| 307 | 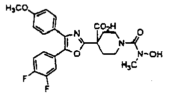 | 308 | 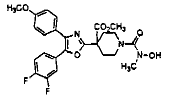 | 309 | 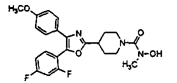 |
| 310 | 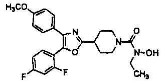 | 311 | 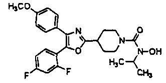 | 312 | 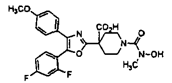 |
| 313 | 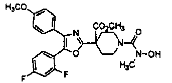 | 314 | 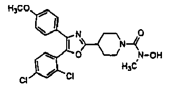 | 315 | 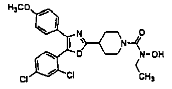 |
| 316 | 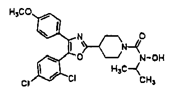 | 317 | 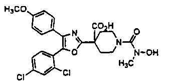 | 318 | 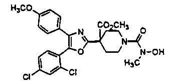 |
| 319 | 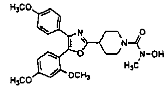 | 320 | 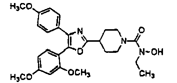 | 321 | 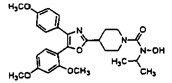 |
| 322 | 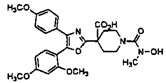 | 323 | 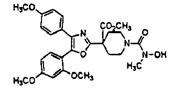 | 324 | 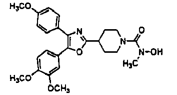 |
| 325 | 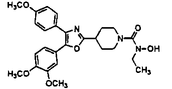 | 326 | 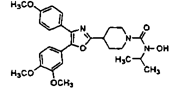 | 327 | 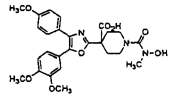 |
| 328 | 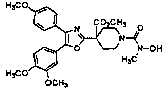 | 329 | 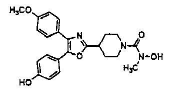 | 330 | 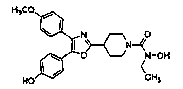 |
| 331 | 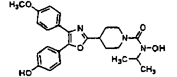 | 332 | 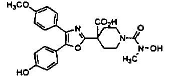 | 333 | 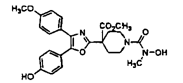 |
| 334 | 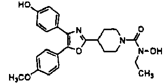 | 335 | 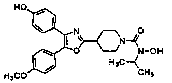 | 336 | 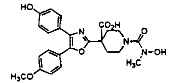 |
| 337 | 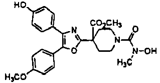 | 338 | 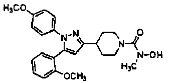 | 339 | 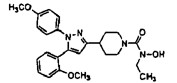 |
| 340 | 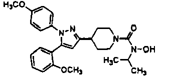 | 341 | 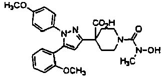 | 342 | 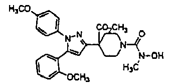 |
| 343 | 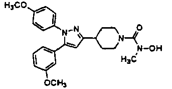 | 344 | 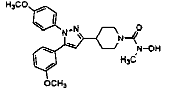 | 345 | 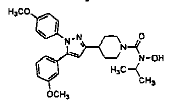 |
| 346 | 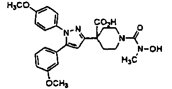 | 347 | 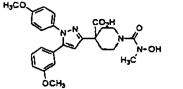 | 348 | 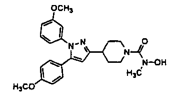 |
| 349 | 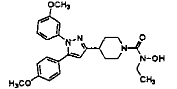 | 350 | 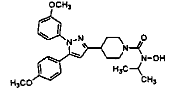 | 351 | 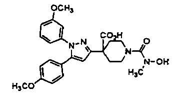 |
| 352 | 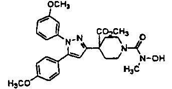 | 353 | 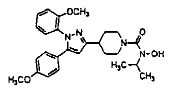 | 354 | 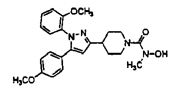 |
| 355 | 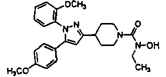 | 356 | 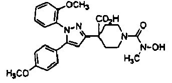 | 357 | 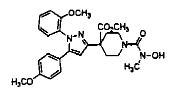 |
| 358 | 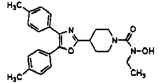 | 359 | 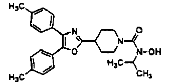 | 360 | 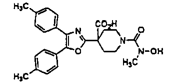 |
| 361 | 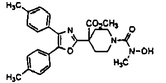 | 362 | 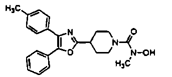 | 363 | 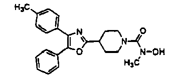 |
| 364 | 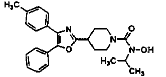 | 365 | 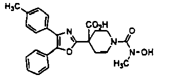 | 366 | 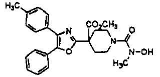 |
| 367 | 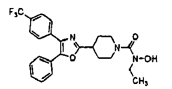 | 368 | 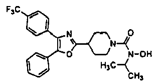 | 369 | 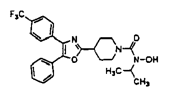 |
| 370 | 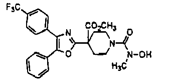 | 371 | 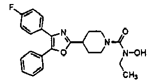 | 372 | 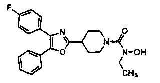 |
| 373 | 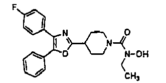 | 374 | 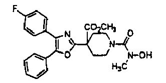 | 375 | 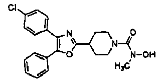 |
| 376 | 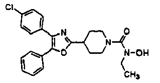 | 377 | 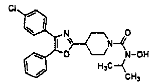 | 378 | 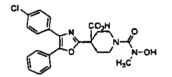 |
| 379 | 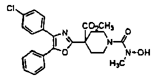 | 380 | 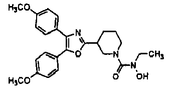 | 381 | 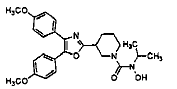 |
| 382 | 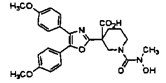 | 383 | 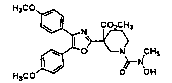 | 384 | 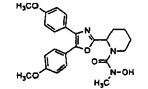 |
| 385 | 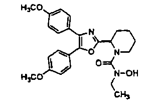 | 386 | 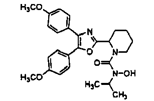 | 387 | 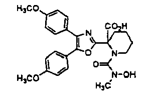 |
| 388 | 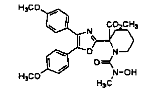 | 389 | 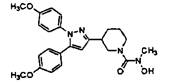 | 390 | 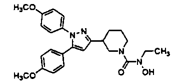 |
| 391 | 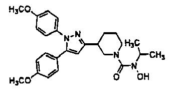 | 392 | 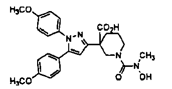 | 393 | 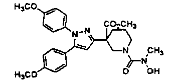 |
| 394 | 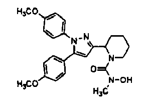 | 395 | 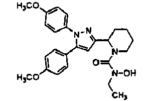 | 396 | 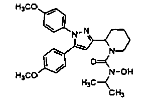 |
| 397 | 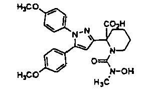 | 398 | 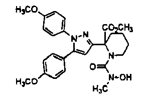 | 399 | 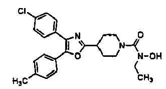 |
| 400 | 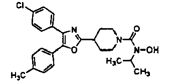 | 401 | 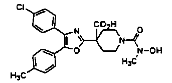 | 402 | 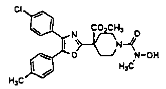 |
| 403 | 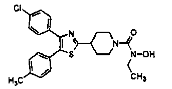 | 404 | 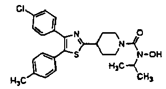 | 405 | 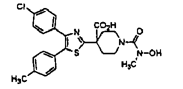 |
| 406 | 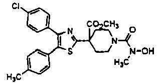 | 407 | 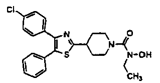 | 408 | 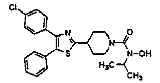 |
| 409 | 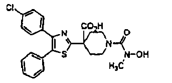 | 410 | 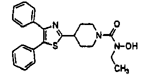 | 412 | 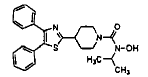 |
| 413 | 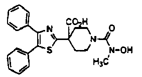 | 414 | 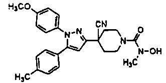 | 415 | 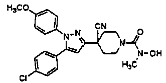 |

When the compound of the present invention contains asymmetric carbon atoms, either enantiomer thereof and a mixture of both enantiomers thereof are within the scope of the present invention.

When the compound of the present invention contains stereoisomer, either stereoisomer thereof and a mixture of both stereoisomer thereof are within the scope of the present invention.

Examples of pharmaceutically acceptable salts according to the present invention include: inorganic acid salts, such as hydrochloride, sulfate, phosphate, and hydrobromate; organic acid salts, such as oxalate, malonate, citrate, fumarate, lactate, malate, succinate, tartrate, acetate, trifluoroacetate, maleate, gluconate, benzoate, ascorbate, methanesulfonate, p-toluenesulfonate, or cinnamate; inorganic basic salts, such as sodium salt, potassium salt, calcium salt, magnesium salt, and ammonium salt; and organic basic salts, such as methylamine salt, diethylamine salt, trimethylamine salt, triethylamine salt, pyridinium salt, triethanolamine salt, ethylenediamine salt, and guanidine salts.

The compound of the present invention represented by formula (I) (hereafter it may be occasionally referred to as compound (I)) can be synthesized in accordance with, for example, the production method shown below.

### Production method 1 (Production method for compound (I) of the present invention)

Compound (I) of the present invention can be obtained by converting compound (III) into compound (IV) using triphosgene and allowing the resultant to react with compound (V) in the manner shown in the formula below, for example: 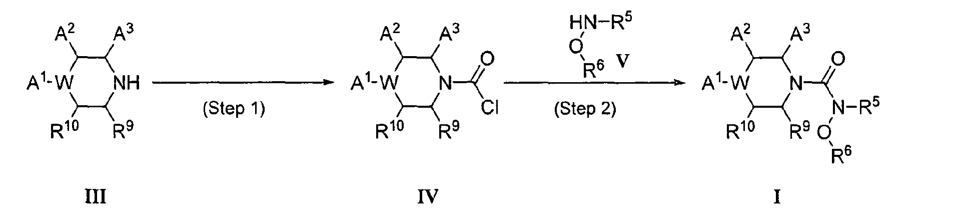 wherein A¹, A², A³, R⁵, R⁶, R⁹, and R¹⁰ are as defined above.

(Step 1) The reaction is generally carried out in a solvent, and a solvent that would not inhibit the reaction is adequately selected. Examples of such solvents that can be used include halogenated hydrocarbons, such as dichloromethane, chloroform, carbon tetrachloride, or 1,2-dichloroethane, and ethers, such as tetrahydrofuran, 1,2-dimethoxyethane, or 1,4-dioxane, and such solvents can be used independently or in combinations of two or more.

Triphosgene is used in amounts of 0.3 to 2 moles, and preferably 0.3 to 0.5 moles, relative to a mole of compound (III). In the reaction, adequate amounts of phosgene, diphosgene, or the like may be adequately used instead of triphosgene.

The reaction temperature is -78°C to 120°C and preferably -20°C to 50°C.

The reaction time varies depending on the reaction conditions, it is 5 minutes to 72 hours, and it is preferably 30 minutes to 48 hours.

(Step 2) A commercially available product can be used as hydrochloride or free base of compound (V).

The reaction is generally carried out in a solvent, and a solvent that would not inhibit the reaction is adequately selected. Examples of such solvents that can be used include halogenated hydrocarbons, such as dichloromethane, chloroform, carbon tetrachloride, or 1,2-dichloroethane, and ethers, such as tetrahydrofuran, 1,2-dimethoxyethane, or 1,4-dioxane, and such solvents can be used independently or in combinations of two or more.

Compound (V) is used in amounts of 0.5 to 5 moles, and preferably 0.9 to 3 moles, relative to a mole of compound (IV).

The reaction temperature is -78°C to 120°C and preferably -20°C to 100°C.

The reaction time varies depending on the reaction conditions, it is 5 minutes to 72 hours, and it is preferably 30 minutes to 48 hours.

### Production method 2 (Production method for compound (Ia) of the present invention)

Compound (Ia) in which R⁵ represents a hydrogen atom can be obtained by, for example, allowing chloroformic acid ester to react with compound (Va) to obtain compound (VI) and then allowing the resultant to react with compound (III), as shown in the following reaction formula: 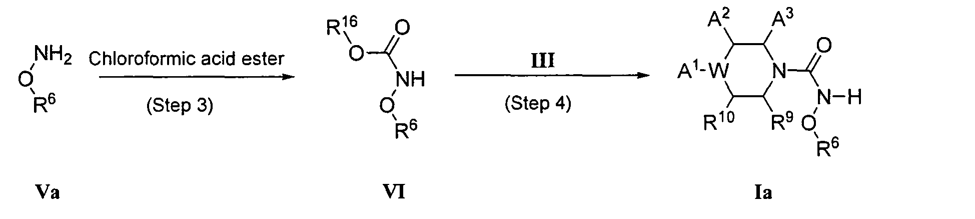 wherein A¹, A², A³, R⁶, R⁹, and R¹⁰ are as defined above; and R¹⁶, for example, represents a methyl group, an ethyl group, a 2-chloroethyl group, a phenyl group, or a 4-nitrophenyl group.

(Step 3) A commercially available product can be used as hydrochloride or free base of compound (Va).

The reaction is generally carried out in the presence of base in a solvent, and a solvent that would not inhibit the reaction is adequately selected. Examples of such base that can be used include organic bases, such as triethylamine, diisopropylethylamine, or pyridine, or inorganic bases, such as sodium bicarbonate, sodium carbonate, potassium carbonate, or cesium carbonate. Examples of solvents that can be used include halogenated hydrocarbons, such as dichloromethane, chloroform, carbon tetrachloride, or 1,2-dichloroethane, ethers, such as tetrahydrofuran, 1,2-dimethoxyethane, or 1,4-dioxane, and acetonitrile. These solvents can be used independently or in combinations of two or more.

Base is used in amounts of 0.5 to 5 moles, and preferably 0.9 to 3 moles, relative to a mole of compound (Va).

Examples of preferable chloroformic acid esters include methyl chloroformate, ethyl chloroformate, chloroethyl chloroformate, phenyl chloroformate, and 4-nitrophenyl chloroformate, with phenyl chloroformate or 4-nitrophenyl chloroformate being more preferable.

Chloroformic acid ester is used in amounts of 0.5 to 3 moles, and preferably 0.9 to 2 moles, relative to a mole of compound (Va).

The reaction temperature is -78°C to 200°C and preferably -20°C to 100°C.

The reaction time varies depending on the reaction conditions, it is 5 minutes to 72 hours, and it is preferably 30 minutes to 48 hours.

(Step 4) The reaction is generally carried out in the presence of base in a solvent, and a solvent that would not inhibit the reaction is adequately selected. Examples of such base that can be used include organic bases, such as triethylamine, diisopropylethylamine, or pyridine, and inorganic bases, such as sodium bicarbonate, sodium carbonate, potassium carbonate, or cesium carbonate. Examples of solvents that can be used include halogenated hydrocarbons, such as dichloromethane, chloroform, carbon tetrachloride, or 1,2-dichloroethane, ethers, such as tetrahydrofuran, 1,2-dimethoxyethane, or 1,4-dioxane, and acetonitrile. These solvents can be used independently or in combinations of two or more.

Intermediate (VI) resulting from this reaction is often unstable, and steps 3 and 4 are preferably carried out via a one-pot reaction without work-up procedure after step 3. In such a case, base is used in amounts of 0.5 to 5 moles, and preferably 0.9 to 3 moles, relative to a mole of compound (Va).

Compound (III) is used in amounts of 0.5 to 3 moles, and preferably 0.8 to 3 moles, relative to a mole of compound (Va).

The reaction temperature is -20°C to 200°C and preferably 0°C to 120°C.

The reaction time varies depending on the reaction conditions, it is 5 minutes to 72 hours, and it is preferably 30 minutes to 48 hours. Production method 3 (Production method for compound (Ic) of the present invention)

Compound (Ic) in which R⁶ is other than a hydrogen atom can be obtained via alkylation with alkyl halide or acylation with carboxylic acid or acyl halide of compound (Ib) in which R⁶ is a hydrogen atom, in accordance with a method obvious to those skilled in the art: 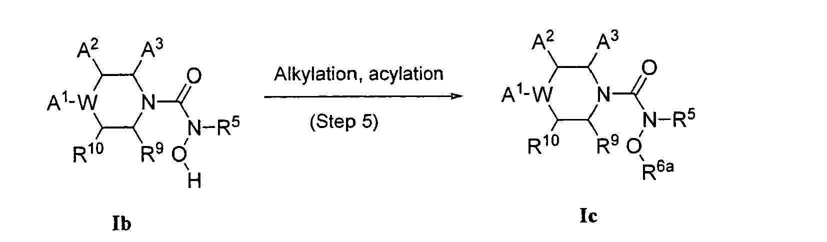 wherein A¹, A², A³, R⁵, R⁹, and R¹⁰ are as defined above; and R^{6a} is as defined above with regard to R⁶, except that in case of a hydrogen atom.

### Production method 4 (Production method for compound (Id) of the present invention)

Compound (Id) in which R⁵ is other than a hydrogen atom can be obtained by alkylation of compound (Ia) in which R⁵ is a hydrogen atom with alkyl halide in accordance with a method obvious to those skilled in the art. 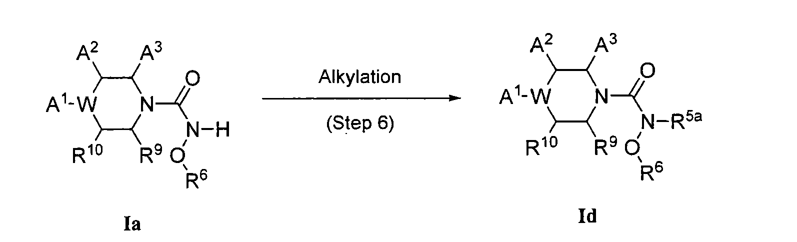 wherein A¹, A², A³, R⁶, R⁹, and R¹⁰ are as defined above, R^{5a} is as defined above with regard to R⁵, except that in cace of a hydrogen atom.

### Production method 5 (Production method for compound (If) of the present invention)

In the case of compound (I) comprising an ester structure in its molecules, i.e., compound (Ie), compound (If) can be obtained by hydrolysis of compound (Ie), in accordance with a method obvious to those skilled in the art: 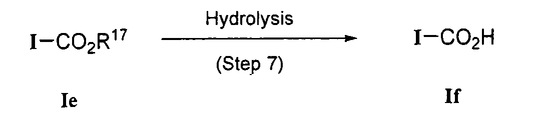 wherein "I" represents compound (I); and R¹⁷ represents an alkyl group, an aralkyl group, or the like.

### Production method 6 (Production method for compound (III) as an intermediate of the compound of the present invention)

Compound (III) can be synthesized by deprotecting a protective group P of compound (VII). A method of deprotection varies depending on a type of a protective group, and deprotection can be carried out in accordance with a method described in, for example,

### Protective Groups In Organic Synthesis (Wiley-Interscience):

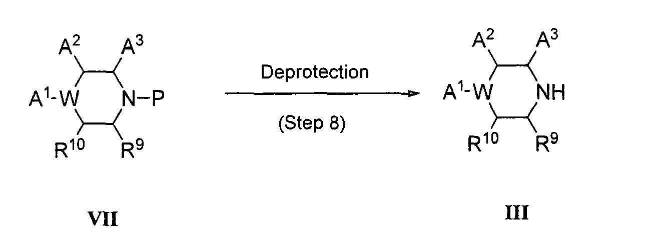

wherein A¹, A², A³, R⁹, and R¹⁰ are as defined above; and P represents a protective group.

### Production method 7 (Production method for compound (VIIa) as an intermediate of the compound of the present invention)

Compound (VIIa) can be obtained by, for example, allowing compound (VIII) to react with compound (IX) or by converting compound (VIII) into compound (XI), allowing compound (IXa) to react therewith to obtain compound (X), and then allowing the resultant to react in the presence of ammonium salt in a manner shown in the following formula: 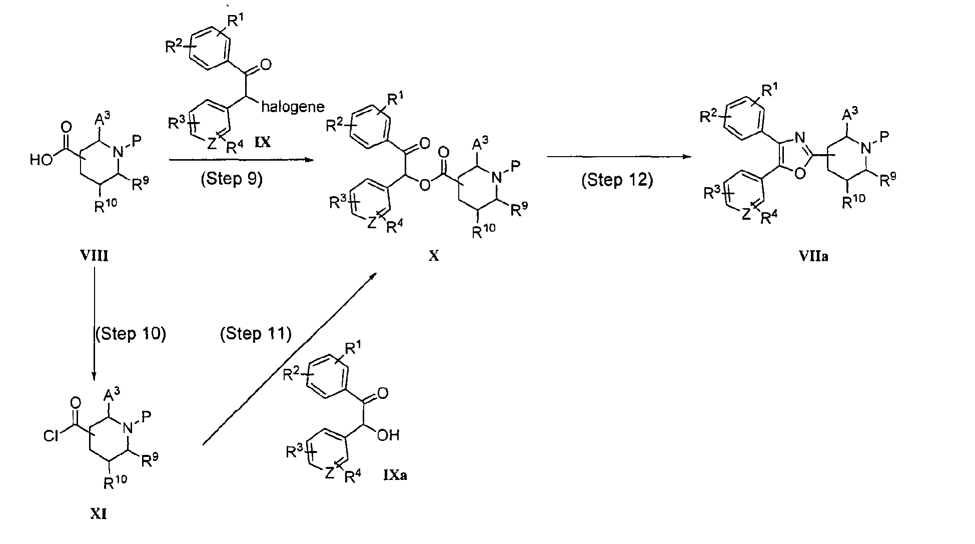 wherein A³, R¹ to R⁴, R⁹, R¹⁰, Z, and P are as defined above; and "halogen" represents a halogen atom.

(Step 9) A commercially available compound or a compound synthesized in accordance with the production method 20 below can be used as compound (IX).

The reaction is generally carried out in the presence of base. Examples of such base that can be used include alkali metal bicarbonates, such as sodium bicarbonate and potassium bicarbonate, alkali metal carbonates, such as potassium carbonate and cesium carbonate, amines, such as triethylamine, diisopropylethylamine, or pyridine, and sodium hydride.

The reaction is generally carried out in a solvent, and a solvent that would not inhibit the reaction is adequately selected. Examples of such solvents that can be used include halogenated hydrocarbons, such as dichloromethane, chloroform, carbon tetrachloride, or 1,2-dichloroethane, ethers, such as tetrahydrofuran, 1,2-dimethoxyethane, or 1,4-dioxane, acetone, ethyl methyl ketone, acetamide, N,N-dimethylformamide, acetonitrile, and water. These solvents can be used independently or in combinations of two or more.

Base is used in amounts of 0.5 to 5 moles, and preferably 0.8 to 2 moles, and compound (IX) is used in amounts of 0.5 to 3 moles, and preferably 0.8 to 1.5 moles, relative to a mole of compound (VIII).

The reaction temperature is -10°C to 150°C and preferably 0°C to 100°C.

The reaction time varies depending on the reaction conditions, it is 5 minutes to 100 hours, and it is preferably 30 minutes to 48 hours.

(Step 10) Compound (XI) can be synthesized from compound (VIII) in accordance with a method obvious to those skilled in the art involving the use of, for example, thionyl chloride or oxalyl chloride.

(Step 11) A commercially available compound can be used as compound (IXa).

The reaction is generally carried out in the presence of base. Examples of such base that can be used include alkali metal hydroxides, such as sodium hydroxide or potassium hydroxide and amines, such as triethylamine, diisopropylethylamine, or pyridine. Presence of 4-(dimethylamino)pyridine as a catalyst is also preferable.

The reaction is generally carried out in a solvent, and a solvent that would not inhibit the reaction is adequately selected. Examples of such solvents that can be used include halogenated hydrocarbons, such as dichloromethane, chloroform, carbon tetrachloride, or 1,2-dichloroethane, ethers, such as tetrahydrofuran, 1,2-dimethoxyethane or 1,4-dioxane, acetamide, N,N-dimethylformamide, and water. These solvents can be used independently or in combinations of two or more.

Base is used in amounts of 0.5 to 5 moles, and preferably 0.8 to 2 moles, and compound (IXa) is used in amounts of 0.5 to 3 moles, and preferably 0.8 to 1.5 moles, relative to a mole of compound (XI).

4-(Dimethylamino)pyridine is used in amounts of 0 to 2 moles, and preferably 0 to 1 mole, relative to a mole of compound (XI).

The reaction temperature is -10°C to 150°C and preferably 0°C to 100°C.

The reaction time varies depending on the reaction conditions, it is 5 minutes to 100 hours, and it is preferably 30 minutes to 48 hours.

(Step 12) The reaction involves the use of ammonium salt. The term "ammonium salt" used herein refers to, for example, ammonium acetate, ammonium formate, or ammonium carbonate.

The reaction is generally carried out in a solvent, and a solvent that would not inhibit the reaction can be adequately selected. Such solvent is preferably, for example, acetic acid or formic acid.

Ammonium salt is used in amounts of 1 to 20 moles, and preferably 2 to 15 moles, relative to a mole of compound (X).

The reaction temperature is 0°C to 200°C and preferably 50°C to 120°C.

The reaction time varies depending on the reaction conditions, it is 5 minutes to 100 hours, and it is preferably 30 minutes to 48 hours. Production method 8 (Synthesis method for compound (IIIa) as an intermediate of the compound of the present invention)

Compound (IIIa) can be synthesized from compound (IX) via a three-step process, as shown in the following formula: 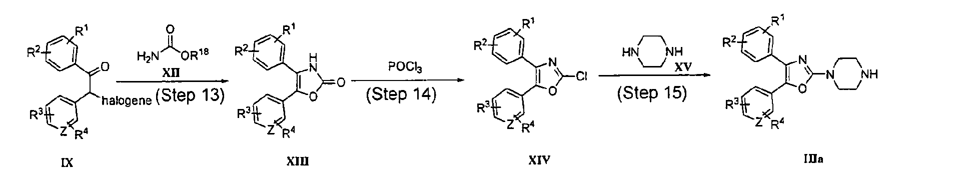 wherein R¹ to R⁴, Z, and halogen are as defined above; and R¹⁸ represents a methyl group or an ethyl group.

(Step 13) A commercially available compound can be used as compound (XII).

Compound (XIII) can be obtained by allowing compound (IX) to react with compound (XII) in accordance with the method described in, for example, Helv. Chim. Acta., 60,342, 1977.

(Step 14) Compound (XIV) can be obtained by heating compound (XIII) in phosphorus oxychloride in accordance with the method described in, for example, Chem. Ber 92, 1928, 1959.

(Step 15) A commercially available compound can be used as compound (XV).

The reaction is generally carried out in a solvent, and a solvent that would not inhibit the reaction is adequately selected. Examples of such solvents that can be used include ethers, such as tetrahydrofuran, 1,2-dimethoxyethane, or 1,4-dioxane, alcohols, such as methanol or ethanol, acetamide, N,N-dimethylformamide, acetonitrile, and water. These solvents can be used independently or in combinations of two or more.

Compound (XV) is used in amounts of 1 to 20 moles, and preferably 2 to 15 moles, relative to a mole of compound (XIV).

The reaction temperature is 0°C to 150°C and preferably 50°C to 120°C.

The reaction time varies depending on the reaction conditions, it is 5 minutes to 100 hours, and it is preferably 30 minutes to 48 hours. Production method 9 (Production method for compound (VIIb) as an intermediate of the compound of the present invention)

Compound (VIIb) can be synthesized by allowing compound (XX) synthesized from diethanolamine to react with compound (XVIII) synthesized from compound (IXa) in a manner shown in the following formula, for example: 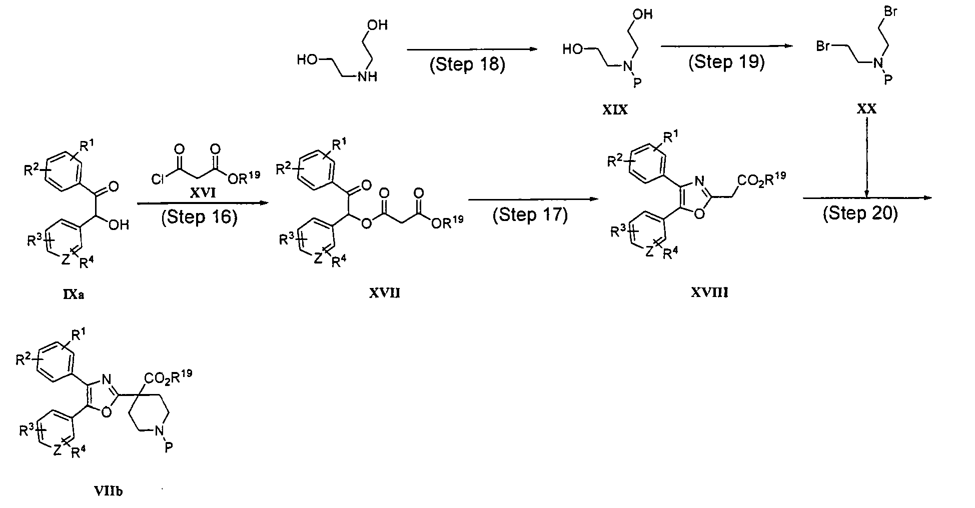 wherein R¹ to R⁴, Z, and P are as defined above; and R¹⁹ represents a methyl group, an ethyl group, or a benzyl group.

(Step 16) A commercially available compound can be used as compound (XVI).

The reaction is generally carried out in the presence of base. Examples of such base that can be used include alkali metal hydroxides, such as sodium hydroxide or potassium hydroxide, and amines, such as triethylamine, diisopropylethylamine, or pyridine. Presence of 4-(dimethylamino)pyridine as a catalyst is also preferable.

The reaction is generally carried out in a solvent, and a solvent that would not inhibit the reaction is adequately selected. Examples of such solvents that can be used include halogenated hydrocarbons, such as dichloromethane, chloroform, carbon tetrachloride, or 1,2-dichloroethane, ethers, such as tetrahydrofuran, 1,2-dimethoxyethane, or 1,4-dioxane, acetamide, N,N-dimethylformamide, and water. These solvents can be used independently or in combinations of two or more.

Base is used in amounts of 0.5 to 5 moles, and preferably 0.8 to 2 moles, and compound (XVI) is used in amounts of 0.5 to 3 moles, and preferably 0.8 to 2 moles, relative to a mole of compound (IXa).

4-(Dimethylamino)pyridine is used in amounts of 0 to 2 moles, and preferably 0 to 1 mole, relative to a mole of compound (XI).

The reaction temperature is -10°C to 150°C and preferably 0°C to 100°C.

The reaction time varies depending on the reaction conditions, it is 5 minutes to 100 hours, and it is preferably 30 minutes to 48 hours.

(Step 17) The reaction involves the use of ammonium salt. The term "ammonium salt" used herein refers to, for example, ammonium acetate, ammonium formate, or ammonium carbonate.

The reaction is generally carried out in a solvent, and a solvent that would not inhibit the reaction can be adequately selected. Such solvent is preferably, for example, acetic acid or formic acid.

Ammonium salt is used in amounts of 1 to 20 moles, and preferably 2 to 15 moles, relative to a mole of compound (XVII).

The reaction temperature is 50°C to 150°C and preferably 70°C to 110°C.

The reaction time varies depending on the reaction conditions, it is 5 minutes to 100 hours, and it is preferably 30 minutes to 48 hours.

(Step 18) The reaction is to introduce a protective group into diethanolamine. A method for introducing a protective group varies depending on a type of a protective group. For example, a method described in Protective Groups In Organic Synthesis (Wiley-Interscience) can be employed.

(Step 19) The reaction can be carried out using a brominating agent well-known in the art, such as phosphorus tribromide or triphenylphosphine-carbon tetrabromide.

The reaction is generally carried out in a solvent, and a solvent that would not inhibit the reaction is adequately selected. Examples of such solvents that can be used include ethers, such as tetrahydrofuran, 1,2-dimethoxyethane, or 1,4-dioxane, dichloromethane, and acetonitrile. These solvents can be used independently or in combinations of two or more.

A brominating agent is used in amounts of 1.5 to 4 moles, and preferably 1.8 to 3 moles, relative to a mole of compound (XIX).

The reaction temperature is -20°C to 200°C and preferably 0°C to 100°C.

The reaction time varies depending on the reaction conditions, it is 5 minutes to 72 hours, and it is preferably 30 minutes to 48 hours.

(Step 20) The reaction is generally carried out using sodium hydride in N,N-dimethylformamide.

Sodium hydride is used in amounts of 1.5 to 4 moles, and preferably 1.9 to 2.5 moles, and compound (XX) is used in amounts of 0.5 to 4 moles, and preferably 0.8 to 2 moles, relative to a mole of compound (XVIII).

The reaction temperature is -20°C to 150°C and preferably 0°C to 100°C.

The reaction time varies depending on the reaction conditions, it is 5 minutes to 72 hours, and it is preferably 30 minutes to 48 hours.

### Production method 10 (Production method for compounds (VIIc) and (VIId) as intermediates of the compound of the present invention)

Compound (VIIc) can be synthesized by converting compound (XXI) into compound (VIIIa), allowing the resultant to react with compound (IX) to obtain compound (Xa), and allowing the resultant to react in the presence of ammonium salt in accordance with the procedure shown in the following formula. Compound (VIId) can be synthesized by reducing compound (VIIc) with the aid of an adequate reducing agent: 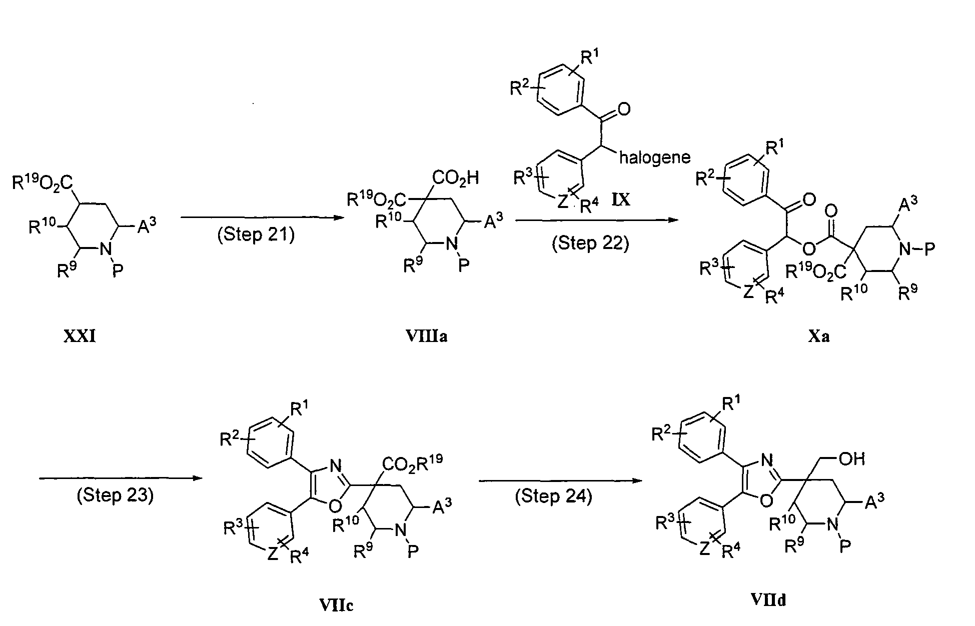 wherein A³, R¹ to R⁴, R⁹, R¹⁰, R¹⁹, Z, P, and halogen are as defined above.

(Step 21) A commercially available compound or a compound synthesized from compound (VIII) by a method well-known in the art can be used as compound (XXI).

The reaction is generally carried out in a solvent, with the use of potassium bis(trimethylsilyl)amide as base and introducing carbon dioxide gas or adding dry ice. A solvent that would not inhibit the reaction is adequately selected. Examples of such solvents that can be used include ethers, such as tetrahydrofuran, 1,4-dioxane, or ethylene glycol dimethyl ether, and aromatic hydrocarbons, such as benzene or toluene.

Base is used in amounts of 0.5 to 4 moles, and preferably 0.8 to 2 moles, relative to a mole of compound (XXI).

Carbon dioxide gas or dry ice is used 0.5 moles to a large excess thereof, and preferably 0.9 to a large excess thereof.

The reaction temperature is -78°C to 200°C and preferably -78°C to 50°C.

The reaction time varies depending on the reaction conditions, carbon dioxide gas is introduced or dry ice is added 5 minutes to 5 hours, and preferably 30 minutes to 2 hours after the addition of base, and the reaction is then continued for 5 minutes to 72 hours, and preferably 30 minutes to 48 hours.

(Step 22) The reaction can be carried out in accordance with the method described in Step 9 above.

(Step 23) The reaction can be carried out in accordance with the method described in Step 12 above.

(Step 24) The reaction is generally carried out in a solvent using an adequate reducing agent, and a solvent that would not inhibit the reaction is adequately selected. Examples of such solvents that can be used include halogenated hydrocarbons, such as dichloromethane or chloroform, ethers, such as tetrahydrofuran, 1,4-dioxane, or ethylene glycol dimethyl ether, alcohols, such as methanol or ethanol, and water. Such solvents can be used independently or in combinations of two or more.

Examples of a reducing agent that can be used include sodium borohydride, lithium borohydride, lithium aluminum hydride, and diisobutylaluminum hydride.

A reducing agent is used in amounts of 0.1 to 10 moles, and preferably 0.25 to 5 moles, relative to a mole of compound (VIIc).

The reaction temperature is -78°C to 200°C and preferably -20°C to 100°C.

The reaction time varies depending on the reaction conditions, it is generally 5 minutes to 72 hours, and it is preferably 30 minutes to 48 hours.

### Production method 11 (Production method for compounds (VIIIb) and (VIIIc) as intermediates of the compound of the present invention)

Compound (VIIIb) can be synthesized by converting compound (VIIIa) into compound (XXII) and then converting an ester portion into carboxylic acid in accordance with a procedure shown in the following formula, for example. Also, compound (VIIIc) can be synthesized by converting compound (XXII) into compound (XXIII) and then converting an ester portion into carboxylic acid: 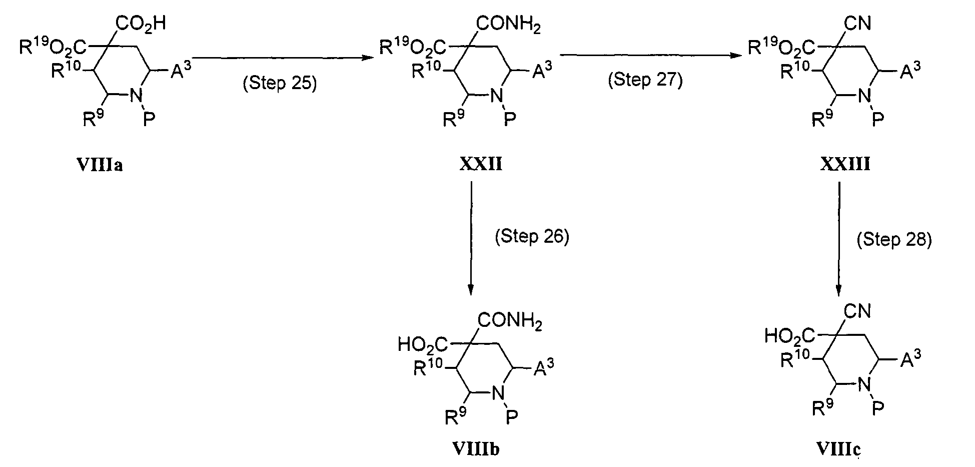 wherein A³, R⁹, R¹⁰, R¹⁹, and P are as defined above.

(Step 25) The reaction is generally carried out in a solvent by, for example, forming an acid anhydride mixture using chloroformic acid ester in the presence of base and then allowing the same to react with ammonia water. In general, a solvent that would not inhibit the reaction is adequately selected. Examples of such solvents that can be used include ethers, such as tetrahydrofuran, 1,4-dioxane, or ethylene glycol dimethyl ether, halogenated hydrocarbons, such as dichloromethane or chloroform, and N,N-dimethylformamide. These solvents can be used independently or in combinations of two or more.

Chloroformic acid ester used in the reaction is, for example, methyl chloroformate, ethyl chloroformate, isopropyl chloroformate, or sec-butyl chloroformate. Such substance is used in amounts of 0.5 to 4 moles, and preferably 0.9 to 2 moles, relative to a mole of compound (VIIIa).

Examples of base used in the reaction include inorganic bases, such as sodium bicarbonate, sodium carbonate, potassium carbonate, or cesium carbonate, and organic bases, such as triethylamine, diisopropylethylamine, or pyridine. Such substance is used in amounts of 0.5 to 5 moles, and preferably 0.9 to 2.5 moles, relative to a mole of compound (VIIIa).

The reaction is preferably carried out at -78°C to 200°C, and preferably at -20°C to 100°C, an acid anhydride mixture is prepared, and ammonia water is then added at -78 to 200°C, and preferably at -20°C to 100°C.

The reaction time varies depending on the reaction conditions, it takes 5 minutes to 48 hours, and preferably 30 minutes to 24 hours to prepare an acid anhydride mixture, ammonia water is added, and the reaction is then continued for 5 minutes to 72 hours, and preferably 30 minutes to 48 hours.

(Step 26) The reaction can be generally carried out via hydrolysis or deprotection well-known in the art, although the reaction conditions vary depending on a type of R¹⁹.

(Step 27) The reaction can be carried out by allowing commercially available (methoxycarbonylsulfamoyl)triethylammonium hydroxide inner salt to react in a solvent such as dichloromethane or tetrahydrofuran.

(Methoxycarbonylsulfamoyl)triethylammonium hydroxide inner salt is used in amounts of 0.5 to 10 moles, and preferably 0.9 to 5 moles, relative to a mole of compound (XXII).

The reaction temperature is -20°C to 200°C and preferably 0°C to 100°C.

The reaction time varies depending on the reaction conditions, it is 5 minutes to 72 hours, and it is preferably 30 minutes to 48 hours.

(Step 28) The reaction can be generally carried out via hydrolysis or deprotection well-known in the art, although the reaction conditions vary depending on a type of R¹⁹. Production method 12 (Production method for compound (VIIId) as an intermediate of the compound of the present invention)

Compound (VIIId) can be synthesized by allowing compound (XXI) to react with alkyl halide in the presence of base to obtain compound (XXIV) and hydrolyzing the product in accordance with a procedure shown in the following formula: 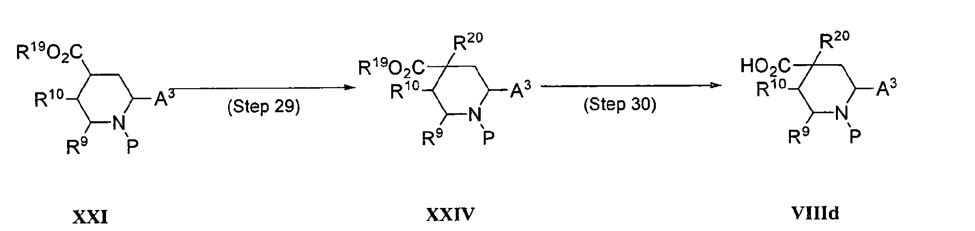 wherein A³, R⁹, R¹⁰, R¹⁹, and P are as defined above; and R²⁰ represents an alkyl group having 1 to 6 carbon atoms.

(Step 29) The reaction is generally carried out by allowing alkyl halide in an anhydrous solvent using potassium bis(trimethylsilyl)amide as base.

Examples of solvents that can be used include ethers, such as tetrahydrofuran, 1,4-dioxane, or ethylene glycol dimethyl ether, and aromatic hydrocarbons, such as benzene or toluene.

Base and alkyl halide are used in amounts of 0.5 to 4 moles, and preferably 0.8 to 2 moles, relative to a mole of compound (XXI).

The reaction temperature is -78°C to 200°C and preferably -78°C to 100°C.

The reaction time varies depending on the reaction conditions, alkyl halide is added 5 minutes to 5 hours, and preferably 30 minutes to 2 hours, after the addition of base, and the reaction is then continued for 5 minutes to 72 hours, and preferably 30 minutes to 48 hours.

(Step 30) The reaction can be generally carried out via hydrolysis or deprotection well-known in the art, although the reaction conditions vary depending on a type of R¹⁹. Production method 13 (Production method for compound (VIIe) as an intermediate of the compound of the present invention)

Compound (VIIe) can be synthesized by amidating compound (VIIIe) to obtain compound (XXV), subjecting the resultant to thioamidation to obtain compound (XXVI), and allowing the resultant to react with compound (IX) in accordance with the procedure shown in, for example, the following formula: 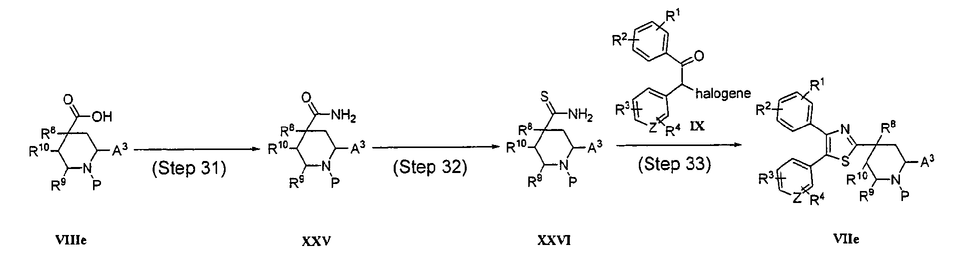 wherein A³, R¹ to R⁴, R⁸ to R¹⁰, Z, P, and halogen are as defined above.

(Step 31) A commercially available compound or compound (VIII), (VIIIa), (VIIIc), or (VIIId) described above can be used as compound (VIIIe). The reaction can be carried out in accordance with the method described in Step 25 above.

(Step 32) The reaction is generally carried out in a solvent with the use of a Lawesson's reagent or the like, and a solvent that would not inhibit the reaction is adequately selected. Examples of such solvents that can be used include saturated hydrocarbons, such as benzene or toluene, halogen solvents, such as dichloromethane or chloroform, and ethers, such as tetrahydrofuran or 1,4-dioxane. These solvents can be used independently or in combinations of two or more.

A Lawesson's reagent is used in amounts of 0.3 to 4 moles, and preferably 0.4 to 2 moles, relative to a mole of compound (XXV).

The reaction temperature is -20°C to 200°C and preferably 0°C to 120°C.

The reaction time varies depending on the reaction conditions, it is 5 minutes to 72 hours, and it is preferably 30 minutes to 48 hours.

(Step 33) A commercially available compound can be also used as compound (XXVI). The reaction is generally carried out in a solvent, and a solvent that would not inhibit the reaction is adequately selected. Such solvents are preferably, for example, alcohols, such as methanol or ethanol, ethers, such as tetrahydrofuran or 1,4-dioxane, and acetonitrile. These solvents can be used independently or in combinations of two or more.

Compound (IX) is used in amounts of 0.5 to 4 moles, and preferably 0.9 to 1.5 moles, relative to a mole of compound (XXVI).

The reaction temperature is -20°C to 200°C and preferably 0°C to 100°C.

The reaction time varies depending on the reaction conditions, and it is generally 5 minutes to 72 hours, and it is preferably 30 minutes to 48 hours.

### Production method 14 (Production method for compounds (VIIg) and (VIIh) as intermediates of the compound of the present invention)

Compound (VIIg) can be synthesized by allowing compound (VIIf), i.e., compound (VIIc) in which R⁸ is a hydrogen atom, to react with compound (XXX) in accordance with the procedure shown in, for example, the following formula. Compound (VIIh) can be synthesized by alkylating or acylating compound (VIIg): 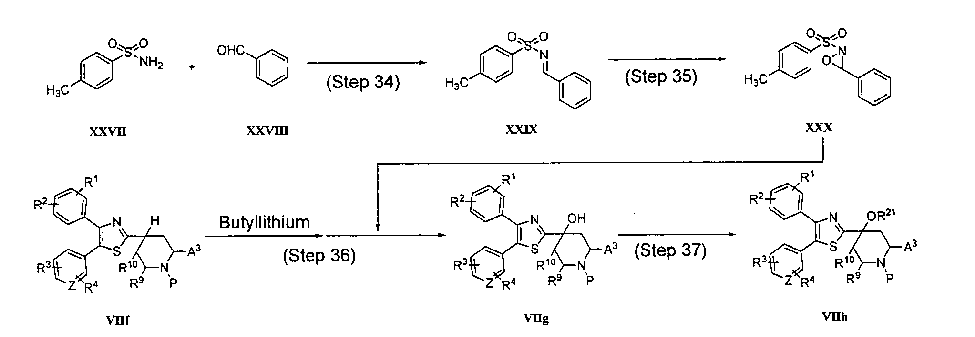 wherein A³, R¹ to R⁴, R⁹, R¹⁰, Z, and P are as defined above; and R²¹ represents an alkyl group having 1 to 6 carbon atoms or an acyl group having 2 to 8 carbon atoms.

(Steps 34 and 35) Commercially available compounds can be used as compound (XXVII) and compound (XXVIII). Compound (XXX) can be synthesized in accordance with the method described in, for example, Tetrahedron 1989, 45, 5703-5742, Davis, F. A.; Sheppard, A. C.

(Step 36) The reaction is generally carried out in an anhydrous solvent by allowing butyllithium to react with compound (VIIf) and then allowing compound (XXX) to react therewith. In general, a solvent that would not inhibit the reaction is adequately selected. Examples of such solvents that can be used include ethers, such as tetrahydrofuran, 1,4-dioxane, or ethylene glycol dimethyl ether, and hexamethylphosphoramide. These solvents can be used independently or in combinations of two or more.

Examples of butyllithium that can be used in the reaction include n-butyllithium, secbutyllithium, and tert-butyllithium.

Butyllithium and compound (XXX) are used in amounts of 0.5 to 5 moles, and preferably 0.8 to 3 moles, relative to a mole of compound (VIIf).

The reaction temperature is -78°C to 200°C and preferably -78°C to 100°C.

The reaction time varies depending on the reaction conditions. In general, butyllithium is added, the reaction is then performed for 5 minutes to 5 hours, and preferably 30 minutes to 3 hours, compound (XXX) is added, and the reaction is then continued for 5 minutes to 72 hours, and preferably 30 minutes to 48 hours.

(Step 37) The reaction is generally carried out via reaction with alkyl halide, acid halide, or acid anhydride in the presence of base in a solvent. In general, a solvent that would not inhibit the reaction is adequately selected. Examples of such solvents that can be used include ethers, such as tetrahydrofuran, 1,4-dioxane, or ethylene glycol dimethyl ether, acetone, acetonitrile, and N,N-dimethylformamide. These solvents can be used independently or in combinations of two or more.

Examples of base used in the reaction include inorganic bases, such as sodium carbonate, potassium carbonate, cesium carbonate, potassium tert-butoxide, or sodium hydride, and organic bases, such as triethylamine, diisopropylethylamine, or pyridine.

Base is used in amounts of 0.5 to 6 moles, and preferably 0.8 to 3 moles, and alkyl halide, acid halide, or acid anhydride is used in amounts of 0.5 to 5 moles, and preferably 0.8 to 2 moles, relative to a mole of compound (VIIg).

The reaction temperature is -78°C to 200°C and preferably -20°C to 100°C.

The reaction time varies depending on the reaction conditions, it is 5 minutes to 78 hours, and it is preferably 30 minutes to 48 hours.

### Production method 15 (Production method for compound (VIIi) as a synthetic intermediate of the compound of the present invention)

Compound (VIIi) can be synthesized by allowing compound (XXXI) to react with compound (XXXII) in the presence of base to obtain compound (XXXIII) and then allowing compound (XXXIV) to react therewith in accordance with the procedure shown in, for example, the following formula: 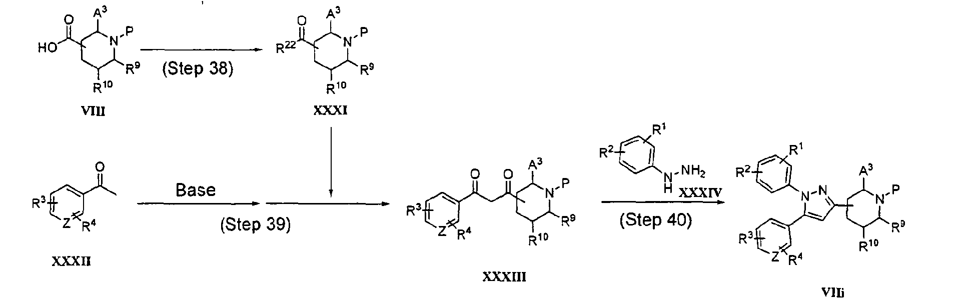 wherein A³, R¹ to R⁴, R⁹, R¹⁰, Z, and P are as defined above; and R²² represents a methyl group, an ethyl group, a chlorine atom, or an imidazole group.

(Step 38) Compound (VIII) can be converted into ester, acid chloride, or a carbonylimidazole compound (XXXI) in accordance with a method well-known in the art or other means.

(Step 39) A commercially available compound can be used as compound (XXXII). The reaction is generally carried out in a solvent, and a solvent that would not inhibit the reaction is adequately selected. Examples of such solvents that can be used include ethers, such as tetrahydrofuran, 1,4-dioxane, or ethylene glycol dimethyl ether, hydrocarbons, such as benzene or toluene, alcohols, such as methanol, ethanol, or tert-butanol, N,N-dimethylformamide, and dimethylformamide. These solvents can be used independently or in combinations of two or more.

Examples of base used in the reaction include lithium diisopropylamide, lithium bis(trimethylsilyl)amide, potassium bis(trimethylsilyl)amide, sodium bis(trimethylsilyl)amide, sodium hydride , sodium methoxide, sodium ethoxide, and potassium tert-butoxide.

Base is used in amounts of 0.5 to 3 moles, and preferably 0.8 to 2 moles, and compound (XXXI) is used in amounts of 0.2 to 2 moles, and preferably 0.3 to 1.2 moles, relative to a mole of compound (XXXII).

The reaction temperature is -78°C to 200°C and preferably -78°C to 100°C.

The reaction time varies depending on the reaction conditions. Base is added to compound (XXXII), the reaction is then performed for 5 minutes to 5 hours, and preferably 30 minutes to 3 hours, compound (XXXI) is added, and the reaction is then continued for 5 minutes to 72 hours, and preferably 30 minutes to 48 hours.

(Step 40) A commercially available compound can be used as hydrochloride or a free compound of compound (XXXIV). The reaction is generally carried out in a solvent, and a solvent that would not inhibit the reaction is adequately selected. Examples of such solvents that can be used include alcohols, such as methanol or ethanol, and acetic acid.

In case that compound (XXXIV) is hydrochloride, triethylamine may be added in amounts of 1 to 3 moles relative to a mole of compound (XXXIV) to perform the reaction.

Compound (XXXIV) is used in amounts of 0.5 to 5 moles, and preferably 0.8 to 2 moles, relative to a mole of compound (XXXIII).

The reaction temperature is 0°C to 200°C and preferably 50°C to 120°C.

The reaction time varies depending on the reaction conditions, it is 5 minutes to 72 hours, and it is preferably 30 minutes to 48 hours.

### Production method 16 (Other production method for compound (VIIi) as a synthetic intermediate of the compound of the present invention)

Compound (VIIi) can be synthesized in accordance with the procedure shown in, for example, the following formula. Compound (VIII) is reduced, then resultant is oxidized to obtain compound (XXXVI), allowing the resultant to react with compound (XXXIV), adding compound (XXXIX) synthesized from compound (XXXVII) thereto, and performing the reaction in the presence of oxygen: 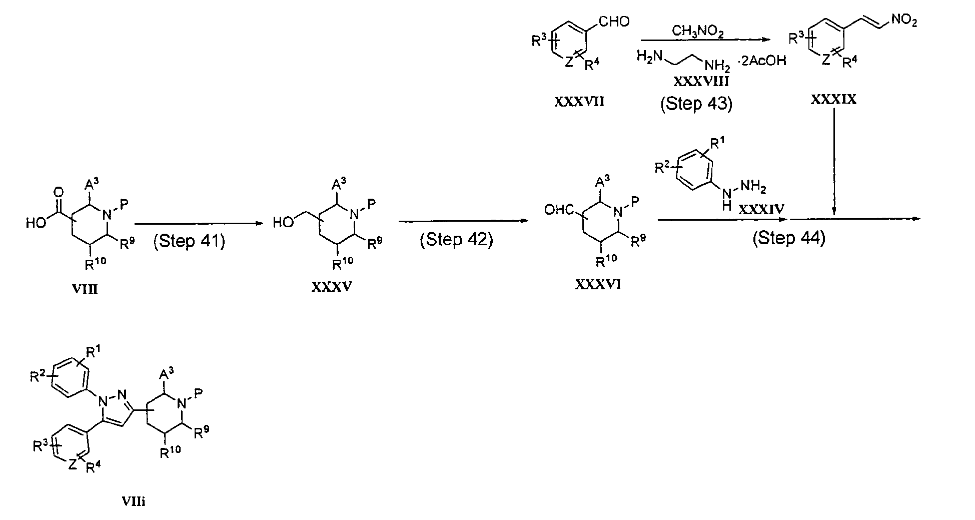 wherein A³, R¹ to R⁴, R⁹, R¹⁰, W, Z, and P are as defined above.

(Step 41) The reaction is generally carried out in an anhydrous solvent, and a solvent that would not inhibit the reaction is adequately selected. Examples of such solvents that can be used include ethers, such as tetrahydrofuran, 1,4-dioxane, or ethylene glycol dimethyl ether, and hydrocarbons, such as benzene or toluene. These solvents can be used independently or in combinations of two or more.

Examples of a reducing agent that can be used in the reaction include lithium aluminum hydride, diisobutylaluminum hydride, and a borane-tetrahydrofuran complex.

A reducing agent is used in amounts of 0.5 to 5 moles, and preferably 0.8 to 2 moles, relative to a mole of compound (VIII).

The reaction temperature is -78°C to 200°C and preferably -20°C to 100°C.

The reaction time varies depending on the reaction conditions, it is 5 minutes to 72 hours, and it is preferably 30 minutes to 48 hours.

(Step 42) The reaction is generally carried out in a solvent, and a solvent that would not inhibit the reaction is adequately selected. Examples of such solvents that can be used include trifluoroacetic acid, pyridine, ether, acetone, pentane, benzene, dichloromethane, and chloroform. These solvents can be used independently or in combinations of two or more.

Examples of an oxidizing agent that can be used in the reaction include manganese dioxide, sulfur trioxide-pyridine, activated dimethyl sulfoxide, and the Dess-Martin reagent.

An oxidizing agent is used in amounts of 0.5 to 3 moles, and preferably 0.8 to 2 moles, relative to a mole of compound (XXXV).

The reaction temperature varies depending on a type of an oxidizing agent, and it is - 78°C to 200°C, and more preferably -78°C to 100°C.

The reaction time varies depending on the reaction conditions, and it is 5 minutes to 72 hours, and preferably 30 minutes to 24 hours.

(Step 43) A commercially available compound can be used as compound (XXXVII). Compound (XXXVIII) can be obtained by adding acetic acid to a solution of ethylenediamine in diethyl ether and filtering precipitated powders.

The reaction can involve the use of alcohols, such as methanol or ethanol, as a solvent.

Nitromethane is used in amounts of 0.5 to 3 moles, and preferably 0.8 to 2 moles, and compound (XXXVIII) is used in amounts of 0.1 to 3 moles, and preferably 0.2 to 1.2 moles, relative to a mole of compound (XXXVII).

The reaction temperature is -20°C to 200°C and preferably 20°C to 100°C.

The reaction time varies depending on the reaction conditions, it is 5 minutes to 72 hours, and it is preferably 30 minutes to 48 hours.

(Step 44) A commercially available compound can be used as compound (XXXVI). The reaction is generally carried out in a solvent. For example, alcohols, such as methanol, ethanol, or isopropanol, are used.

Compound (XXXIV) is used in amounts of 0.5 to 5 moles, and preferably 0.8 to 1.5 moles, and compound (XXXIX) is used in amounts of 0.5 to 5 moles, and preferably 0.8 to 1.5 moles, relative to a mole of compound (XXXVI).

The reaction temperature is -20°C to 200°C and preferably 0°C to 100°C.

The reaction time varies depending on the reaction conditions, the duration of the reaction between compound (XXXVI) and compound (XXXIV) is 5 minutes to 5 hours, and preferably 30 minutes to 3 hours, and the reaction is continued for 5 minutes to 72 hours, and preferably 30 minutes to 48 hours after the addition of compound (XXXIX). Production method 17 (Production method for compound (VIIj) as an intermediate of the compound of the present invention)

Compound (VIIj) can be synthesized by allowing thiophosgene to react with compound (XL) to obtain compound (XLI) and then allowing compound (IX) to react therewith in accordance with the procedure shown in, for example, the following formula: 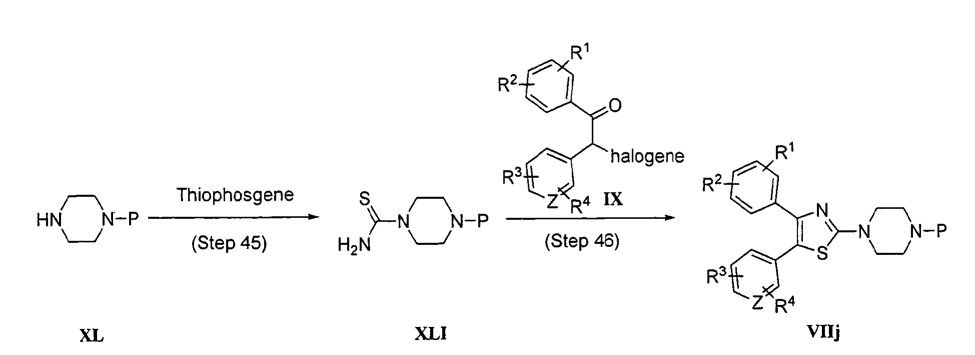 wherein R¹ to R⁴, Z, and P are as defined above.

(Step 45) A commercially available compound can be used as compound (XL). The reaction is generally carried out in a solvent, and a solvent that would not inhibit the reaction is adequately selected. Examples of such solvents that can be used include halogen solvents, such as dichloromethane or chloroform, and ethers, such as tetrahydrofuran or 1,4-dioxane. These solvents can be used independently or in combinations of two or more.

Thiophosgene is used in amounts of 0.5 to 5 moles, and preferably 0.8 to 2 moles, relative to a mole of compound (XL).

The reaction temperature is -78°C to 150°C and preferably -20°C to 50°C.

The reaction time varies depending on the reaction conditions, it is 5 minutes to 72 hours, and it is preferably 30 minutes to 48 hours.

(Step 46) The reaction can be carried out in accordance with the method described in Step 33 above.

### Production method 18 (Production method for compounds (VIIk), (VIIm), (VIIn), and (VIIo) as intermediates of the compound of the present invention)

Compounds (VIIk), (VIIm), (VIIn), and (VIIo) can be obtained in accordance with the procedure shown in, for example, the following formula. Compound (XLII) is allowed to react with butyllithium, and compound (XXXI) is then allowed to react therewith to obtain compound (XLIII). Subsequently, the resultant is allowed to react with compound (IXb) to obtain compound (XLIV), and the resultant is then subjected to hydrolysis and decarboxylation to obtain compound (XLV). Compound (XLV) is subjected to the reaction with the use of a sulfuration agent, such as a Lawesson's reagent to obtain compound (VIIk), subjected to the reaction with an acid such as sulfuric acid to obtain compound (VIIm) or subjected to the reaction with ammonium salt to obtain compound (VIIn). Compound (VIIn) may be subjected to the reaction with alkyl halide, acid chloride, or acid anhydride to obtain compound (VIIo): 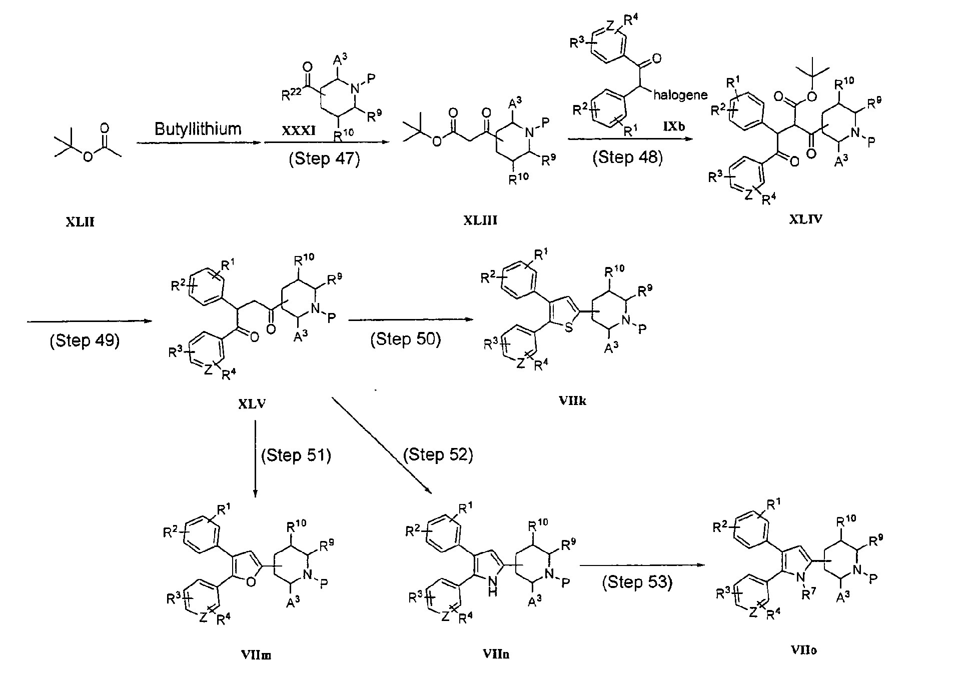 wherein A³, R¹ to R⁴, R⁷, R⁹, R¹⁰, R²², Z, P, and halogen are as defined above.

(Step 47) A commercially available compound can be used as compound (XVII). The reaction is generally carried out in an anhydrous solvent, and a solvent that would not inhibit the reaction is adequately selected. Examples of such solvents that can be used include tetrahydrofuran, 1,4-dioxane, ethylene glycol, dimethyl ether, and hexane. These solvents can be used independently or in combinations of two or more.

Examples of butyllithium used in the reaction include n-butyllithium, secbutyllithium, and tert-butyllithium.

Butyllithium is used in amounts of 0.5 to 5 moles, and preferably 0.8 to 2 moles, and compound (XXXI) is used in amounts of 0.2 to 5 moles, and preferably 0.3 to 2 moles, relative to a mole of compound (XLII).

The reaction temperature is -78°C to 100°C and preferably -78°C to 50°C.

The reaction time varies depending on the reaction conditions, the duration of the reaction between compound (XLII) and butyllithium is 5 minutes to 5 hours, and preferably 30 minutes to 2 hours, and the reaction is continued for 5 minutes to 72 hours, and preferably 30 minutes to 48 hours, after the addition of compound (XXXI).

(Step 48) The reaction is generally carried out in the presence of base in a solvent, and a solvent that would not inhibit the reaction is adequately selected. Examples of such solvents that can be used include ethers, such as tetrahydrofuran, 1,4-dioxane, or ethylene glycol dimethyl ether, and hydrocarbons, such as benzene or toluene. These solvents can be used independently or in combinations of two or more.

Examples of base used in the reaction include sodium carbonate, potassium carbonate, cesium carbonate, sodium hydride, and potassium tert-butoxide.

Base is used in amounts of 0.5 to 5 moles, and preferably 0.8 to 2 moles, and compound (IXb) is used in amounts of 0.5 to 5 moles, and preferably 0.8 to 2 moles, relative to a mole of compound (XLIII).

The reaction temperature is -20°C to 200°C and preferably 0°C to 100°C.

The reaction time varies depending on the reaction conditions, it is 5 minutes to 72 hours, and it is preferably 30 minutes to 48 hours.

(Step 49) The reaction is carried out in a solvent or in the absence of a solvent. Compound (XLIV) is subjected to hydrolysis in a dichloromethane or chloroform solvent, with the addition of trifluoroacetic acid. Alternatively, hydrolysis may be carried out in a methanol or ethanol solvent, with the addition of hydrochloric acid, with heating. The resulting carboxylic acid may be converted into compound (VIIm) due to advanced decarboxylation and dehydration depending on hydrolysis conditions. In general, carboxylic acid is decarboxylated by heating in the absence of a solvent or in dimethylformamide.

Trifluoroacetic acid or hydrochloric acid is used in amounts of 0.5 moles to a large excess thereof, and preferably 1 mole to a large excess thereof, relative to a mole of compound (XLIV).

Hydrolysis is carried out at a reaction temperature of 0°C to 200°C, and preferably 0°C to 70°C. Decarboxylation is carried out at 0°C to 200°C, and preferably 0°C to 180°C.

The reaction time varies depending on the reaction conditions, hydrolysis is carried out for 5 minutes to 48 hours, and preferably 30 minutes to 24 hours, and decarboxylation is carried out for 5 minutes to 72 hours, and preferably 30 minutes to 48 hours.

(Step 50) The reaction is generally carried out in a solvent with the use of a Lawesson's reagent or the like, and a solvent that would not inhibit the reaction is adequately selected. Examples of such solvents that can be used include saturated hydrocarbons, such as benzene or toluene, halogen solvents, such as dichloromethane or chloroform, and ethers, such as tetrahydrofuran or 1,4-dioxane. These solvents can be used independently or in combinations of two or more.

A Lawesson's reagent is used in amounts of 0.3 to 4 moles, and preferably 0.4 to 2 moles, relative to a mole of compound (XLV).

The reaction temperature is -20°C to 200°C and preferably 0°C to 120°C.

The reaction time varies depending on the reaction conditions, it is 5 minutes to 72 hours, and it is preferably 30 minutes to 48 hours.

(Step 51) The reaction proceeds by, for example, agitating compound (XLV) in concentrated sulfuric acid.

An excess amount of concentrated sulfuric acid is used as a solvent, relative to a mole of compound (XLV).

The reaction temperature is -20°C to 200°C and preferably 0°C to 100°C.

The reaction time is 5 minutes to 72 hours, and it is preferably 30 minutes to 48 hours.

(Step 52) Examples of ammonium salts used in the reaction include ammonium acetate, ammonium formate, and ammonium carbonate.

The reaction is generally carried out in a solvent, and a solvent that would not inhibit the reaction is adequately selected. Such solvent is preferably, for example, acetic acid or formic acid.

Ammonium salt is used in amounts of 1 to 20 moles, and preferably 2 to 15 moles, relative to a mole of compound (XLV).

The reaction temperature is 0°C to 200°C and preferably 50°C to 120°C.

The reaction time varies depending on the reaction conditions, it is 5 minutes to 100 hours, and it is preferably 30 minutes to 48 hours.

(Step 53) The reaction is generally carried out in the presence of base via reaction with alkyl halide, acid halide, or acid anhydride in a solvent. In general, a solvent that would not inhibit the reaction is adequately selected. Examples of such solvents that can be used include ethers, such as tetrahydrofuran, 1,4-dioxane, or ethylene glycol dimethyl ether, acetone, acetonitrile, and N,N-dimethylformamide. These solvents can be used independently or in combinations of two or more.

Examples of base used in the reaction include inorganic bases, such as sodium carbonate, potassium carbonate, cesium carbonate, potassium tert-butoxide, or sodium hydride, and organic bases, such as triethylamine, diisopropylethylamine, or pyridine.

Base and alkyl halide, acid halide, or acid anhydride are used in amounts of 0.5 to 5 moles, and preferably 0.8 to 2 moles, relative to a mole of compound (VIIn).

The reaction temperature is -78°C to 200°C and preferably -20°C to 100°C.

The reaction time varies depending on the reaction conditions, it is 5 minutes to 78 hours, and it is preferably 30 minutes to 48 hours.

### Production method 19 (Production method for compound (XXXVI) and compound (VIII) as intermediates of the compound of the present invention)

Compound (XXXVI) is synthesized by converting compound (XLVI) into compound (XLVIII) and then subjecting the resultant to acid hydrolysis in accordance with the procedure shown in, for example, the following formula. The resultant may further be subjected to oxidation to synthesize compound (VIII): 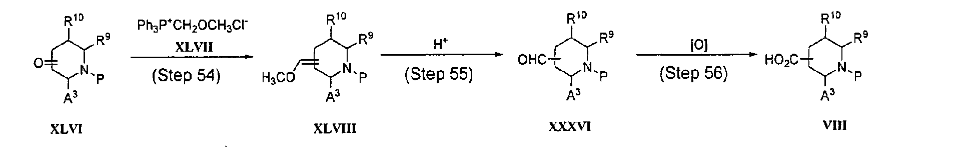 wherein A³, R⁹, R¹⁰, and P are as defined above.

(Step 54) Commercially available compounds can be used as compound (XLVI) and compound (XLVII). The reaction is generally carried out in a solvent, and a solvent that would not inhibit the reaction is adequately selected. Examples of such solvents that can be used include tetrahydrofuran, diethyl ether, tert-butanol, and dimethyl sulfoxide. These solvents can be used independently or in combinations of two or more.

The reaction is generally carried out in the presence of base. Examples of base that can be used include lithium diisopropylamide, potassium tert-butoxide, sodium hydride, phenyllithium, and tert-butyllithium.

Base is used in amounts of 0.5 to 3 moles, and preferably 0.8 to 2 moles, and compound (XLVII) is used in amounts of 0.5 to 3 moles, and preferably 0.8 to 2 moles, relative to a mole of compound (XLVI).

The reaction temperature is -78°C to 100°C and preferably -78°C to 50°C.

The reaction time varies depending on the reaction conditions, it is 5 minutes to 72 hours, and it is preferably 30 minutes to 48 hours.

(Step 55) The reaction is generally carried out in a solvent, and a solvent that would not inhibit the reaction is adequately selected. Examples of such solvents that can be used include ethers, such as tetrahydrofuran, 1,4-dioxane, or ethylene glycol dimethyl ether, and alcohols, such as methanol, ethanol, or tert-butanol, acetonitrile, and water. These solvents can be used independently or in combinations of two or more.

A concentration of an acid to be used is 0.1 M to 12 M. Examples thereof include 1 mole to an excess amount of inorganic acid, such as hydrochloric acid or sulfuric acid, and an organic acid, such as acetic acid, relative to a mole of compound (XLVIII).

The reaction temperature is -20°C to 200°C and preferably 0°C to 100°C.

The reaction time is 5 minutes to 48 hours, and preferably 30 minutes to 24 hours.

(Step 56) The reaction is generally carried out in a solvent, and a solvent that would not inhibit the reaction is adequately selected. Examples of such solvents that can be used include acetic acid, acetone, benzene, and water. Such solvent can be used independently or in combinations of two or more. Methods obvious to those skilled in the art, which involve the use of chromium (VI) oxide-acetate or the Jones reagent, or other methods can be employed. Production method 20 (Production method for compound (IX) as an intermediate of the compound of the present invention)

Compound (IX) can be synthesized by allowing compound (XLIX) to react with 1,1'-carbonyl imidazole, adding compound (LI) and sodium hydride thereto so as to obtain compound (LII), converting compound (LII) into compound (LIII) by the Krapcho method, and halogenating the resultant in accordance with the procedure shown in, for example, the following formula: 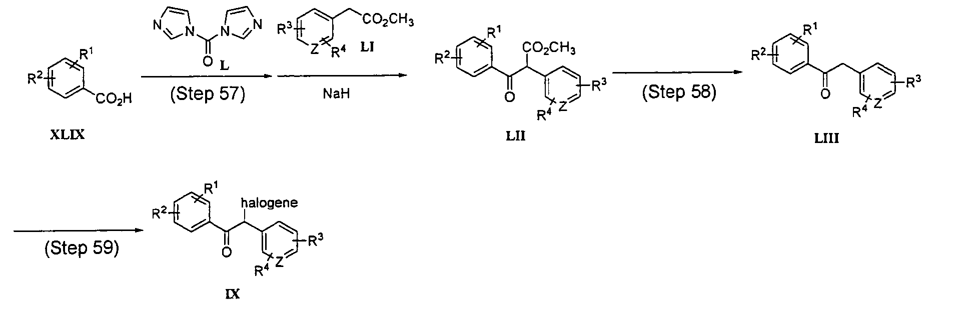 wherein R¹ to R⁴, Z, and halogen are as defined above.

(Step 57) Commercially available compounds can be used as compound (XLIX) and compound (LI). The reaction is generally carried out in a solvent, and a solvent that would not inhibit the reaction is adequately selected. Examples of such solvents that can be used include ethers, such as tetrahydrofuran, 1,4-dioxane, and N,N-dimethylformamide.

Compound (L), compound (LI), and sodium hydride are used in amounts of 0.5 to 4 moles, and preferably 0.9 to 2 moles, respectively, relative to a mole of compound (XLIX).

The reaction temperature is -20°C to 100°C and preferably 0°C to 50°C.

The reaction time varies depending on the reaction conditions, the duration of the reaction with compound (L) is for 5 minutes to 5 hours, and preferably 30 minutes to 2 hours, and that with compound (LI) is 5 minutes to 72 hours, and preferably 30 minutes to 48 hours.

In Step 57, compound (XLIXa) and compound (LIa) may be used instead of compound (XLIX) and compound (LI), and compound (LIIa) and compound (LIIIa) may be produced, which results in synthesis of compound (IXb) therethrough. Commercially available compounds can be used as compound (XLIXa) and compound (LIa): 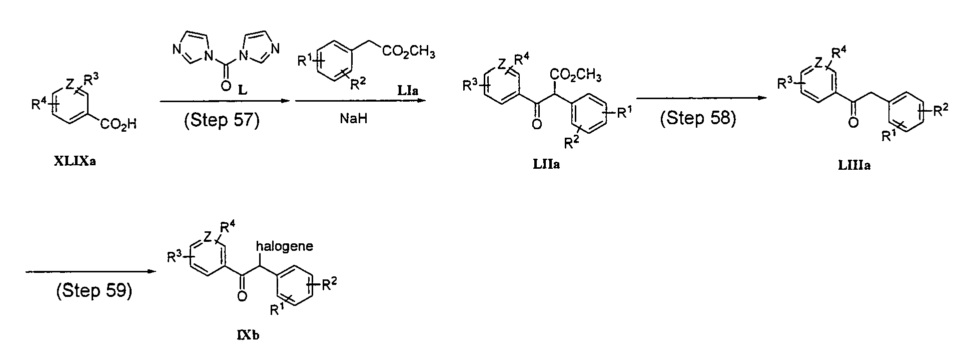 wherein R¹ to R⁴, Z, and halogen are as defined above.

(Step 58) The reaction is carried out in the presence of sodium chloride and water in N,N-dimethylformamide.

Sodium chloride is used in amounts of 0.5 to 5 moles, and preferably 0.9 to 2 moles, and water is used in amounts of 0.5 moles to an excess amount, and preferably 0.9 moles to an excess amount, relative to a mole of compound (LII).

The reaction temperature is 100°C to 200°C and preferably 130°C to 180°C.

The reaction time varies depending on the reaction conditions, it is 5 minutes to 72 hours, and it is preferably 30 minutes to 48 hours.

(Step 59) The reaction is generally carried out in a solvent, and a solvent that would not inhibit the reaction is adequately selected. Examples of such solvents that can be used include halogen solvents, such as dichloromethane or chloroform, and ethers, such as tetrahydrofuran or 1,4-dioxane. These solvents can be used independently or in combinations of two or more.

Examples of halogenating agents used in the reaction include chlorine, bromine, N-chlorosuccinimide, N-bromosuccinimide, copper (II) bromide, and pyridinium tribromide.

Halogenating agents are used in amounts of 0.5 to 2 moles, and preferably 0.8 to 1.2 moles, relative to a mole of compound (LIII).

The reaction temperature is -20°C to 200°C and preferably 0°C to 100°C.

The reaction time varies depending on the reaction conditions, it is 5 minutes to 72 hours, and it is preferably 30 minutes to 48 hours. Production method 21 (Production method for compounds (VIIp) and (VIIq) as intermediates of the compound of the present invention)

Compound (VIIp) can be synthesized by oxidizing compound (IXa) to obtain compound (LV) and allowing the resultant to react with compound (XXXVI) in the presence of ammonium salt, in accordance with the procedure shown in, for example, the following formula. Further, alkylation or acylation may be carried out by a method well-known in the art to synthesize compound (VIIq): 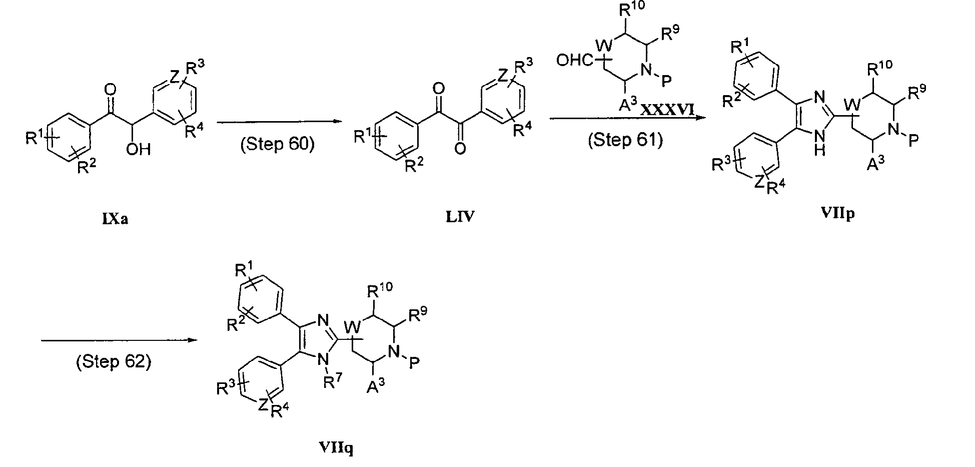 wherein A³, R¹ to R⁴, R⁷, R⁹, R¹⁰, Z, W, and P are as defined above.

(Step 60) The reaction can be carried out in accordance with the method described in Step 42 above.

(Step 61) Examples of ammonium salt used in the reaction include ammonium acetate, ammonium formate, or ammonium carbonate.

The reaction is generally carried out in a solvent, and a solvent that would not inhibit the reaction can be adequately selected. Such solvent is preferably acetic acid or formic acid, for example.

Ammonium salt is used in amounts of 1 to 20 moles, and preferably 2 to 15 moles, compound (XXXVI) is used in amounts of 0.5 to 5 moles, and preferably 0.8 to 1.5 moles, relative to a mole of compound (LV).

The reaction temperature is 0°C to 200°C and preferably 50°C to 120°C.

The reaction time varies depending on the reaction conditions, it is 5 minutes to 100 hours, and it is preferably 30 minutes to 48 hours.

(Step 62) The reaction can be carried out in accordance with the method described in Step 53 above.

### Production method 22 (Step of forming salt of the compound (I) of the present invention)

Compound (I) is subjected to salt formation generally via mixing with an acid or base in a solvent, and a solvent that would not inhibit the reaction is adequately selected. Examples of such solvents that can be used include alcohols, such as methanol, ethanol, or isopropanol, ethers, such as tetrahydrofuran, 1,4-dioxane, or ethylene glycol dimethyl ether, acetone, and ethyl acetate. These solvents can be used independently or in combinations of two or more.

Excellent analgesic effects or effects of treating or preventing neuropathic pain of the compound of the present invention can be evaluated using adequate animal models. Examples of adequate animal models of nociceptive pain include acetic acid-induced writhing model in mice, formalin test in mice or rats, and carrageenin-induced inflammation model in rats, although means of evaluation are not limited thereto. Examples of adequate animal models of neuropathic pain include partial sciatic nerve ligation model in mice or rats and spinal nerve ligation model in mice or rats, although means of evaluation are not limited thereto.

The compound of the present invention has excellent analgesic effects or effects of treating or preventing neuropathic pain. Accordingly, the compound can be used for a pharmaceutical, and it can be preferably used as an analgesic or therapeutic or preventive agent for neuropathic pain.

When the compound of the present invention is used for analgesics, such compound is preferably used for nociceptive pain. The term "nociceptive pain" used herein refers to, for example, pain caused by injury such as bone fracture or cut, and pain resulting from inflammatory diseases, such as postoperative pain, pain of sprains, pain of a bruise, joint pain, lumbar pain, muscle soreness, pain after tooth extraction, dental pain, appendicitis, chronic rheumatoid arthritis, rheumatic fever, osteoarthritis, ankylosing spondylarthritis, osteoarthritis of spine, cervicobrachial syndrome, periarthritis, cellulitis, acute otitis media, prostatitis, alveolar periostitis, or colpitis. In the present invention, deep seated pain or visceral pain (e.g., headache, abdominal pain, lumbar backache, pain resulting from chronic pelvic pain syndrome or heterotopic endometriosis, pain resulting from urinary tract stone disease or urethrolith, colic resulting from digestive lesion, pelvic pain, disease resulting from urinary system diseases, and the like) are classified as the nociceptive pain. When the compound of the present invention is used for analgesics, examples of more preferable target diseases include chronic rheumatoid arthritis, osteoarthritis, postoperative pain, joint pain, lumbar pain, muscle soreness, and dental pain.

The compound of the present invention is also used as a therapeutic or preventive agent for neuropathic pain. The term "neuropathic pain" refers to, for example, cancer pain, pain of herpes zoster, postherpetic neuralgia, trigeminal neuralgia, and pain resulting from diabetic neuritis.

An example of features of the compound of the present invention is the effects of inhibiting cyclooxygenase-1 (COX-1) of mammalian animals, such as mice, rats, hamsters, rabbits, cats, dogs, cows, sheep, mice, and humans. Analgesic effects are known as one of the COX-1-inhibiting effects, and such effects enable preferable use of the compound of the present invention for pharmaceuticals. When the compound of the present invention is administered to a human, particularly excellent analgesic effects or effects of treating or preventing neuropathic pain can be produced. Further, the compound of the present invention does not have the effects of inhibiting cyclooxygenase-2 (COX-2). Accordingly, pharmaceuticals comprising the compound of the present invention may bring about small side effects of cardiovascular disorders resulting from COX-2-inhibiting effects.

The compound of the present invention is not only used for analgesics or therapeutic or preventive agents for neuropathic pain as described above but also used for relieving pain, treating or preventing neuropathic pain, or preparing analgesics or therapeutic or preventive agents for neuropathic pain.

When the compound of the present invention is administered, the compound can be administered in that state. Alternatively, a pharmaceutically acceptable carrier may be mixed, and the resultant may be administered orally or parenterally.

Examples of oral dosage forms of a pharmaceutical preparation comprising the compound of the present invention include a tablet including a sugar-coated tablet and a film coated tablet, a pill, a granule, a powdered drug, a capsule including a soft capsule and a microcapsule, syrup, an emulsion, and a suspension. Examples of parenteral dosage forms include an injection, an impregnation agent, drop, and a suppository. Mixing of such pharmaceutical preparation with an adequate base, such as a butyric acid polymer, a glycolic acid polymer, a copolymer of butyric acid and glycolic acid, a mixture of a butyric acid polymer and a glycolic acid polymer, polyglycerol esters of fatty acids, or the like, to prepare a sustained release preparation is also effective.

A pharmaceutical preparation comprising the compound of the present invention can be prepared into the aforementioned dosage form via a known technique generally employed in the art. When such dosage form is prepared, an excipient, a binder, a lubricant, a disintegrator, a sweetening agent, a surfactant, a suspending agent, an emulsifier, or the like, which is generally used in the art, can be incorporated into the preparation, according to need.

When a tablet preparation comprising the compound of the present invention is prepared, an excipient, a binder, a disintegrator, a lubricant, or the like can be incorporated. When a pill or granule is prepared, an excipient, a binder, a disintegrator, or the like can be incorporated. When a powdered drug or capsule is prepared, for example, an excipient can be incorporated. Syrup can be prepared with the addition of a sweetening agent or the like. An emulsion or suspension can be prepared with the addition of a surfactant, a suspending agent, an emulsifier, or the like.

Examples of an excipient include lactose, glucose, starch, sucrose, microcrystalline cellulose, powdered glycyrrhiza, mannitol, sodium bicarbonate, calcium phosphate, and calcium sulfate.

Examples of a binder include starch paste, a gum Arabic solution, a gelatin liquid, a tragacanth liquid, a carboxymethylcellulose liquid, a sodium alginate liquid, and glycerin.

Examples of a disintegrator include starch and calcium carbonate.

Examples of a lubricant include magnesium stearate, stearic acid, calcium stearate, and purified talc.

Examples of a sweetening agent include glucose, fructose, invert sugar, sorbitol, xylitol, glycerine, and simple syrup.

Examples of a surfactant include sodium lauryl sulfate, polysorbate 80, sorbitan monofatty acid ester, and polyoxyl 40 stearate.

Examples of a suspending agent include gum Arabic, sodium alginate, carboxymethylcellulose sodium, methylcellulose, and bentonite.

Examples of an emulsifier include gum Arabic, tragacanth, gelatin, and polysorbate 80.

Further, when a pharmaceutical preparation comprising the compound of the present invention is prepared into the aforementioned dosage form, a coloring agent, a preservative, an aromatic, a flavoring agent, a stabilizer, a thickener, and the like, which are generally used in the art, can be added.

A daily dose of the pharmaceutical preparation varies depending on, for example, the conditions and the body weight of a patient, a type of the compound, and a route of administration. In the case of oral administration, for example, a daily dose for an adult (with a body weight of about 60 kg) is between 1 mg and 1,000 mg, and the pharmaceutical preparation can be administered once or two or three separate doses. In the case of parenteral administration, for example, administration is preferably in the form of an intravenous injection in an amount of 0.01 mg to 100 mg per kg of body weight, in general.

An adequate amount of the ureide derivative of the present invention can be mixed with other agents or the ureide derivative can be used in combination with other agents, for the purpose of complementing or enhancing the therapeutic or preventive effects or for the purpose of reducing a dose. An agent that can be used in combination with the ureide derivative of the present invention, i.e., compound (I) of the present invention or a salt thereof, can be used in combination with agents listed below.

Examples of an antitussive drug, an expectorant, and an antitussive/expectorant composition include dextromethorphan, benproperine, dimemorfan, clofedanol, ephedrine, huscode, fominoben, methylephedrine, acetylcysteine, ambroxol, carbocisteine, bromhexine, eprazinone, cherry bark (Pruni jamasakura bark) extract, codeine, dihydrocodeine, and tipepidine.

Examples of a bronchodilator include clenbuterol, cromoglycate, salbutamol, salmeterol, tulobuterol, theophylline, and procaterol.

Examples of a drug for peptic ulcer include azulene, aldioxa, irsogladine, ecabet, omeprazole, ornoprostil, cimetidine, sucralfate, sulpiride, cetraxate, and famotidine.

Examples of antibiotics include amoxicillin, azithromycin, erythromycin, clarithromycin, tetracycline, and doxycycline.

Examples of narcotic analgesics include opium alkaloid, ethylmorphine, oxycodone, morphine, cocaine, fentanyl, and pethidine.

### Brief Description of the Drawings

Fig. 1 shows the effects of the compounds of the present invention on partial sciatic nerve ligation model in mice (i.e., Examples 14, 27, and 40) (oral administration).
Fig. 2 shows the analgesic effects of the compounds of the present invention on formalin test in mice (i.e., Examples 27 and 40) (oral administration).

Each group indicates mean ± standard error. The number of examples is: groups to which solvents have been administered: n = 10; and the groups to which compositions of Examples 40 and 27 have been administered: n = 5.

#: p < 0.025: compared to the vehicle control (1-tailed Shirley-Williams test).

*: p < 0.025: compared to the vehicle control (1-tailed Williams test).

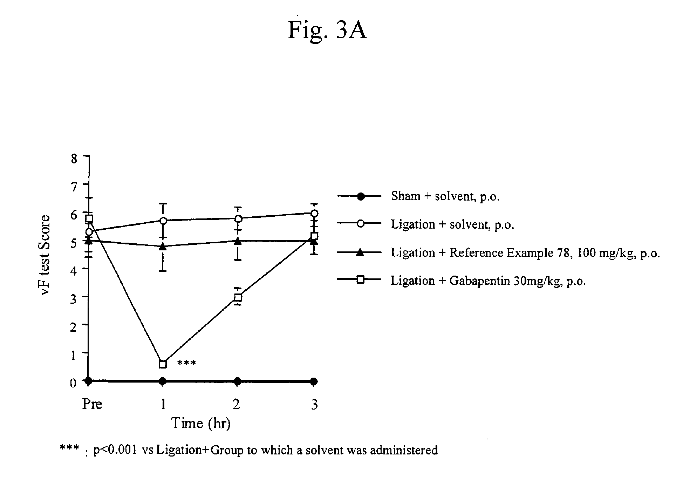

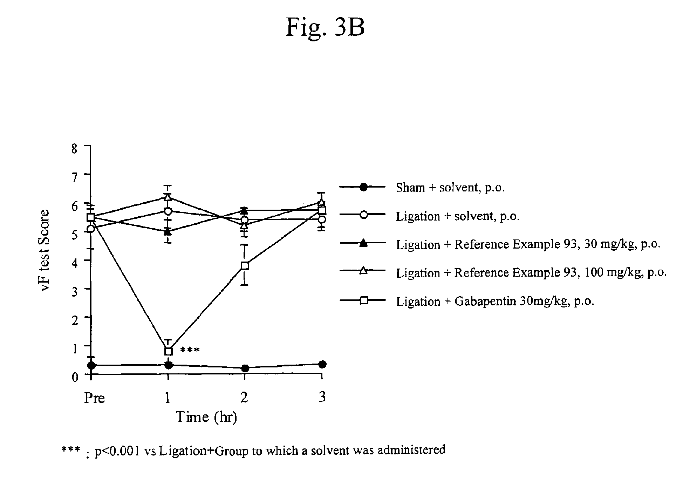

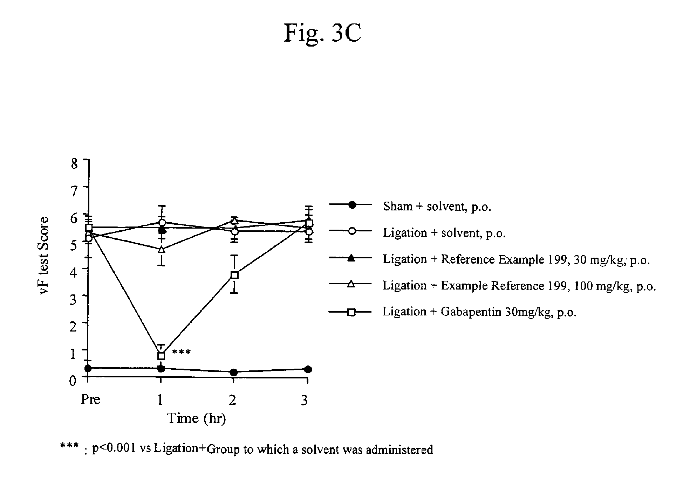

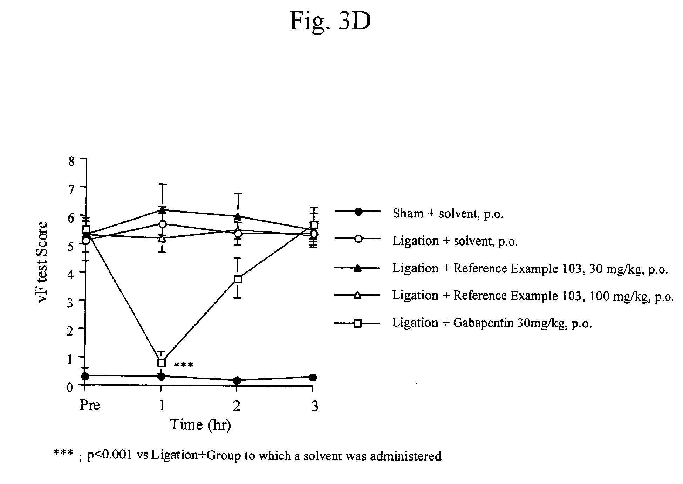

Fig 3 shows the results of evaluation of compounds having a structure similar to that of the compound of the present invention (a compound of Reference Example 78 (sulfate), a compound of Reference Example 93 (toluenesulfonate), a compound of Reference Example 199, and a compound of Reference Example 103 (sulfate)) on partial sciatic nerve ligation model in mice (oral administration), which are disclosed in JP Patent No. 3003148, JP Patent Publication (kohyo) No. 2006-514095 A, and JP Patent Publication (kohyo) No. 2006-517535 A.

This description includes part or all of the contents as disclosed in the description and/or drawings of Japanese Patent Application No. 2006-086563, which is a priority document of the present application.

### Examples

Hereafter, the present invention is described in detail with reference to Reference Examples and Examples, although the present invention is not limited thereto. In the sections regarding separation via column chromatography, solvents described in parentheses represent elution solvents, and percentages represent volume ratios. In the sections regarding NMR, solvents described in parentheses represent solvents that were used for measurement.

The 400 MHz NMR spectrum was measured using the nuclear magnetic resonance equipment (JNM-AL400, JEOL Ltd.). A chemical shift was represented in terms of δ (unit: ppm) using tetramethylsilane as a standard, and signals were represented by s (single line), d (double line), t (triple line), q (quadruple line), quint (quintuple line), sept (septuple line), m (multiple line), brs (broad single line), brd (broad double line), dd (double-double line), dt (double-triple line), ddd (double-double-double line), dq (double-quadruple line), and tt (triple-triple line). The IR spectrum was measured using FT/IR-410 (JASCO Corporation), and the ESI-MS spectrum was measured using Micromass ZQ2K (Waters). Commercially available solvents were used. Flash chromatography was carried out using YFLC W-prep2XY (Yamazen Corporation).

Synthesis of starting materials and intermediates for the compound of the present invention and comparative compounds of the compound of the present invention are described below as Reference Examples. Compounds used for synthesis of comparative compounds, which are not described in terms of synthesis methods, are commercially available compounds.

### Reference Example 1

### Methyl 3-(4-tert-butylphenyl)-2-(4-methoxyphenyl)-3-oxopropanoate

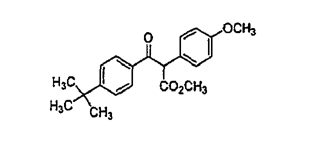

N,N'-carbonyldiimidazole (2.26 g, 13.9 mmol) was added to a solution of 4-tert-butylbenzoic acid (2.28 g, 12.8 mmol) in N,N-dimethylformamide (15 ml) at 0°C. The reaction solution was stirred at room temperature for 1 hour, methyl 4-methoxyphenylacetate (2.10 ml, 13.2 mmol) was added, and 55% sodium hydride (615 mg, 14.0 mmol) was then added in several batches at 0°C. The reaction solution was stirred at room temperature for 5 hours, distilled water was added to dilute the reaction solution, and the resultant was acidified with 6M hydrochloric acid. The mixed solution was extracted with ethyl acetate, washed with water, dried over magnesium sulfate, and then concentrated under a reduced pressure. The residue was purified by column chromatography (silica gel, n-hexane/ethyl acetate = 8/1) to give a title compound as a light yellow oil product (2.91 g, 8.54 mmol, 67%).

¹H-NMR (400 MHz, CDCl₃) δ: 1.30 (9H, s), 3.75 (3H, s), 3.78 (3H, s), 5.57 (1H, s), 6.88 (2H, d, J = 8.8 Hz), 7.32 (2H, d, J = 8.8 Hz), 7.43 (2H, d, J = 8.8 Hz), 7.89 (2H, d, J = 8.8 Hz).

### Reference Example 2

### Methyl 2-(4-methoxyphenyl)-3-oxo-3-(4-tolyl)propanoate

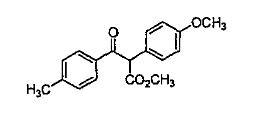

A title compound (1.60 g, 5.36 mmol, 42%) was obtained as a yellow oil product from 4-methylbenzoic acid (1.73 g, 12.8 mmol) and methyl 4-methoxyphenylacetate (2.10 ml, 13.2 mmol) in the same manner as in Reference Example 1.

¹H-NMR (400 MHz, CDCl₃) δ: 2.33 (3H, s), 3.90 (3H, s), 3.97 (3H, s), 5.27 (1H, s), 6.97 (2H, d, J = 8.8 Hz), 7.15 (2H, d, J = 8.5 Hz), 7.24 (2H, d, J = 8.5 Hz), 7.87 (2H, d, J = 8.8 Hz).

### Reference Example 3

### Methyl 3-(4-chlorophenyl)-2-(4-methoxyphenyl)-3-oxopropanoate

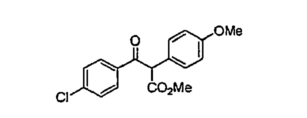

A title compound (2.49 g, 7.81 mmol, 61%) was obtained as a yellow oil product from 4-chlorobenzoic acid (2.00 g, 12.8 mmol) and methyl 4-methoxyphenylacetate (2.10 ml, 13.2 mmol) in the same manner as in Reference Example 1.

¹H-NMR (400 MHz, CDCl₃) δ: 3.76 (3H, s), 3.78 (3H, s), 5.50 (1H, s), 6.89 (2H, d, J = 8.5 Hz), 7.28 (2H, d, J = 8.8 Hz), 7.39 (2H, d, J = 8.8 Hz), 7.87 (2H, d, J = 8.5 Hz).

### Reference Example 4

### Methyl 3-(4-isopropyloxyphenyl)-2-(4-methoxyphenyl)-3-oxopropanoate

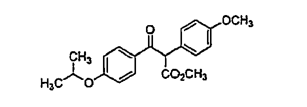

A title compound (2.18 g, 6.37 mmol, 50%) was obtained as a yellow oil product from 4-isopropyloxybenzoic acid (2.30 g, 12.8 mmol) and 4-methoxyphenylacetic acid (2.10 ml, 13.2 mmol) in the same manner as in Reference Example 1.

¹H-NMR (400 MHz, CDCl₃) δ: 1.34 (6H, d, J = 5.8 Hz), 3.75 (3H, s), 3.78 (3H, s), 4.61 (1H, sept, J = 5.8 Hz), 5.53 (1H, s), 6.84 (2H, d, J = 8.8 Hz), 6.89 (2H, d, J = 8.8 Hz), 7.31 (2H, d, J = 8.8 Hz), 7.90 (2H, d, J = 8.8 Hz).

### Reference Example 5

### Methyl 2-(4-methoxyphenyl)-3-oxo-3-(4-trifluoromethylphenyl)propanoate

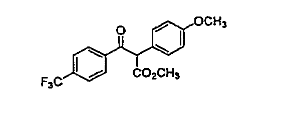

A title compound (3.61 g, 10.2 mmol, 79%) was obtained as a yellow oil product from 4-trifluoromethylbenzoic acid (2.43 g, 12.8 mmol) and 4-methoxyphenylacetic acid (2.10 ml, 13.2 mmol) in the same manner as in Reference Example 1.

¹H-NMR (400 MHz, CDCl₃) δ: 3.77 (3H, s), 3.78 (3H, s), 5.54 (1H, s), 6.89 (2H, d, J = 8.8 Hz), 7.29 (2H, d, J = 8.8 Hz), 7.68 (2H, d, J = 8.3 Hz), 8.03 (2H, d, J = 8.3 Hz). Reference Example 6 1-(4-tert-Butylphenyl)-2-(4-methoxyphenyl)ethanone

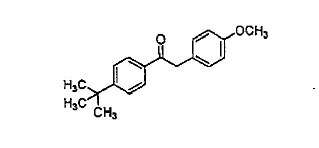

To a solution of methyl 3-(4-tert-butylphenyl)-2-(4-methoxyphenyl)-3-oxopropanoate (Reference Example 1) (2.91 g, 8.54 mmol) in dimethyl sulfoxide (2.5 ml) was added sodium chloride (659 mg, 11.3 mmol) and distilled water (200 µl, 11.1 mmol). The reaction solution was stirred at 160°C for 19 hours and then cooled to room temperature. The reaction solution was diluted with the addition of distilled water and then extracted with ethyl acetate. The organic layer was washed with water, dried over magnesium sulfate, and concentrated under a reduced pressure. The residue was recrystallized (ethyl acetate/n-hexane = 3/7) to give a title compound (1.28 g, 4.53 mmol, 53%) as a white crystal.

¹H-NMR (400 MHz, CDCl₃) δ: 1.33 (9H, s), 3.78 (3H, s), 4.20 (2H, s), 6.86 (2H, d, J = 8.8 Hz), 7.19 (2H, d, J = 8.8 Hz), 7.46 (2H, d, J = 8.8 Hz), 7.95 (2H, d, J = 8.8 Hz).

### Reference Example 7

### 2-(4-Methoxyphenyl)-1-(4-tolyl)ethanone

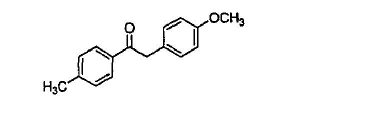

A title compound (704 mg, 2.93 mmol, 54%) was obtained as a light yellow powder from methyl 2-(4-methoxyphenyl)-3-oxo-3-(4-tolyl)propanoate (Reference Example 2) (1.60 g, 5.36 mmol) in the same manner as in Reference Example 6.

¹H-NMR (400 MHz, CDCl₃) δ: 2.40 (3H, s), 3.78 (3H, s), 4.20 (2H, s), 6.86 (2H, d, J = 9.0 Hz), 7.18 (2H, d, J = 8.5 Hz), 7.24 (2H, d, J = 8.5 Hz), 7.90 (2H, d, J = 9.0 Hz).

### Reference Example 8

### 1-(4-Chlorophenyl)-2-(4-methoxyphenyl)ethanone

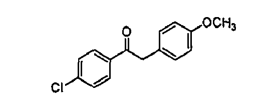

A title compound (739 mg, 2.83 mmol, 43%) was obtained as a white needle-like crystal from methyl 3-(4-chlorophenyl)-2-(4-methoxyphenyl)-3-oxopropanoate (Reference Example 3) (2.10 g, 6.59 mmol) in the same manner as in Reference Example 6.

¹H-NMR (400 MHz, CDCl₃) δ: 3.79 (3H, s), 4.19 (2H, s), 6.86 (2H, d, J = 8.8 Hz), 7.16 (2H, d, J = 8.5 Hz), 7.42 (2H, d, J = 8.8 Hz), 7.93 (2H, d, J = 8.5 Hz).

### Reference Example 9

### 1-(4-Isopropyloxyphenyl)-2-(4-methoxyphenyl)ethanone

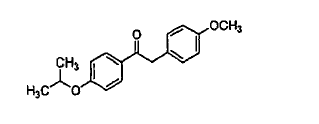

A title compound (1.03 g, 3.62 mmol, 57%) was obtained as a light yellow needle-like crystal from methyl 3-(4-isopropyloxyphenyl)-2-(4-methoxyphenyl)-3-oxopropanoate (Reference Example 4) (2.18 g, 6.37 mmol) in the same manner as in Reference Example 6.

¹H-NMR (400 MHz, CDCl₃) δ: 1.36 (6H, d, J = 5.8 Hz), 3.78 (3H, s), 4.16 (2H, s), 4.63 (1H, sept, J = 5.8 Hz), 6.85 (2H, d, J = 8.8 Hz), 6.89 (2H, d, J = 8.8 Hz), 7.18 (2H, d, J = 8.8 Hz), 7.96 (2H, d, J = 8.8 Hz).

### Reference Example 10

### 2-(4-Methoxyphenyl)-1-(4-trifluoromethylphenyl)ethanone

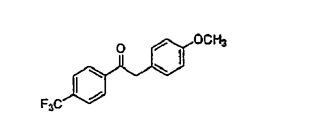

A title compound (1.30 g, 6.15 mmol, 60%) was obtained as a white crystal from methyl 2-(4-methoxyphenyl)-3-oxo-3-(4-trifluoromethylphenyl)propanoate (Reference Example 5) (3.61 g, 10.2 mmol) in the same manner as in Reference Example 6.

¹H-NMR (400 MHz, CDCl₃) δ: 3.79 (3H, s), 4.24 (2H, s), 6.87 (2H, d, J = 8.8 Hz), 7.17 (2H, d, J = 8.8 Hz), 7.71 (2H, d, J = 8.3 Hz), 8.09 (2H, d, J = 8.3 Hz).

### Reference Example 11

### 1-(4-Methoxyphenyl)-2-(4-tolyl)ethanone

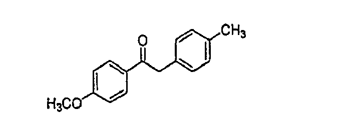

To a solution of 4-methylphenylacetic acid (1.00 g, 6.65 mmol), anisole (2.20 ml, 20.1 mmol), and 85% phosphoric acid (0.45 ml) in acetonitrile (25 ml), a solution of trifluoroacetic anhydride (3.75 ml, 27.0 mmol) in acetonitrile (10 ml) was added dropwise at 50°C. The mixture was stirred at the same temperature for 2 hours, cooled to room temperature, and then poured on ice. After the mixed solution was extracted with ethyl acetate, the organic layer was washed with saturated aqueous sodium bicarbonate, dried over magnesium sulfate, and concentrated under a reduced pressure. The residue was purified by column chromatography (silica gel, ethyl acetate/n-hexane = 5/1-4/1), and the resulting solid was recrystallized (ethyl acetate/n-hexane = 1/3) to give a title compound (645 mg, 2.68 mmol, 40%) as a white crystal.

¹H-NMR (400 MHz, CDCl₃) δ: 2.31 (3H, s). 3.86 (3H, s), 4.19 (2H, s), 6.91 (2H, d, J = 8.8 Hz), 7.12 (2H, d, J = 8.3 Hz), 7.15 (2H, d, J = 8.3 Hz), 7.99 (2H, d, J = 8.8 Hz).

### Reference Example 12

### 2-(4-Chlorophenyl)-1-(4-methoxyphenyl)ethanone

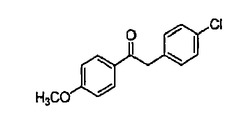

A title compound (958 mg, 3.67 mmol, 62%) was obtained as a yellow solid from 4-chlorophenylacetic acid (1.01 g, 5.86 mmol) in the same manner as in Reference Example 11.

¹H-NMR (400 MHz, CDCl₃) δ: 3.87 (3H, s), 4.20 (2H, s), 6.93 (2H, d, J = 9.0 Hz), 7.19 (2H, d, J = 8.5 Hz), 7.29 (2H, d, J = 8.5 Hz), 7.98 (2H, d, J = 9.0 Hz).

### Reference Example 13

### 2-(4-Fluorophenyl)-1-(4-methoxyphenyl)ethanone

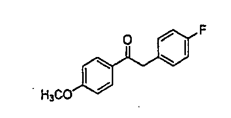

A title compound (964 mg, 3.94 mmol, 59%) was obtained as a white crystal from 4-fluorophenylacetic acid (1.02 g, 6.65 mmol) in the same manner as in Reference Example 11.

¹H-NMR (400 MHz, CDCl₃) δ: 3.87 (3H, s), 4.21 (2H, s), 6.94 (2H, d, J = 9.0 Hz), 7.02 (2H, t, J = 8.5Hz), 7.22 (2H, dd, J = 5.4, 8.5 Hz), 7.99 (2H, d, J = 9.0 Hz).

### Reference Example 14

### 1,2-Bis(4-methoxyphenyl)-2-bromoethanone

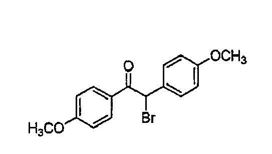

To a solution of 1,2-bis(4-methoxyphenyl)ethanone (2.56 g, 10.0 mmol) in dichloromethane (50 ml) was added pyridinium tribromide (3.84 g, 12.0 mmol) and the resultant was stirred at room temperature for 1 hour. An aqueous solution of 5% sodium thiosulfate was added to the reaction solution, the resultant was stirred, and the solution was then separated. The organic layer was dried over sodium sulfate and concentrated under a reduced pressure to give a title compound (3.37 g, 10.0 mmol, quant.) as a light brown solid.

¹H-NMR (400 MHz, CDCl₃) δ: 3.79 (3H, s), 3.86 (3H, s), 6.37 (1 H, s), 6.88 (2H, d, J = 8.8 Hz), 6.91 (2H, d, J = 8.8 Hz), 7.46 (2H, d, J = 8.8 Hz), 7.97 (2H, d, J = 8.8 Hz). IR (KBr, cm⁻¹): 3426, 2964, 2932, 1679, 1602, 1513, 1254, 1171, 1028, 845.

### Reference Example 15

### 2-Bromo-1-(4-tert-butylphenyl)-2-(4-methoxyphenyl)ethanone

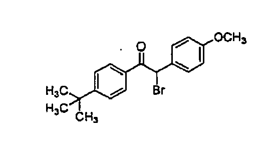

A title compound (451 mg, 1.25 mmol, quant.) was obtained as a light yellow oil product from 1-(4-tert-butylphenyl)-2-(4-methoxyphenyl)ethanone (Reference Example 6) (353 mg, 1.25 mmol) in the same manner as in Reference Example 14.

¹H-NMR (400 MHz, CDCl₃) δ: 1.32 (9H, s), 3.80 (3H, s), 6.39 (1H, s), 6.89 (2H, d, J = 8.8 Hz), 7.46 (2H, d, J = 8.8 Hz), 7.47 (2H, d, J = 8.8 Hz), 7.93 (2H, d, J = 8.8 Hz).

### Reference Example 16

### 2-Bromo-2-(4-methoxyphenyl)-1-(4-tolyl)ethanone

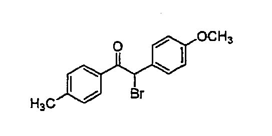

A title compound (919 mg, 2.88 mmol, quant.) was obtained as an orange solid from 2-(4-methoxyphenyl)-1-(4-tolyl)ethanone (Reference Example 7) (629 mg, 2.88 mmol) in the same manner as in Reference Example 14.

¹H-NMR (400 MHz, CDCl₃) δ: 2.39 (3H, s), 3.79 (3H, s), 6.38 (1H, s), 6.88 (2H, d, J = 8.8 Hz), 7.24 (2H, d, J = 8.3 Hz), 7.46 (2H, d, J = 8.8 Hz), 7.88 (2H, d, J = 8.3 Hz).

### Reference Example 17

### 2-Bromo-1-(4-chlorophenyl)-2-(4-methoxyphenyl)ethanone

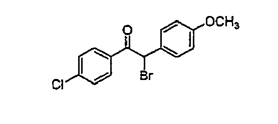

A title compound (944 mg, 2.78 mmol, quant.) was obtained as a yellow oil product from 1-(4-chlorophenyl)-2-(4-methoxyphenyl)ethanone (Reference Example 8) in the same manner as in Reference Example 14.

¹H-NMR (400 MHz, CDCl₃) δ: 3.80 (3H, s), 6.32 (1H, s), 6.89 (2H, d, J = 8.8 Hz), 7.41 (2H, d, J = 8.8 Hz), 7.44 (2H, d, J = 8.8 Hz), 7.92 (2H, d, J = 8.8 Hz).

### Reference Example 18

### 2-Bromo-1-(4-isopropyloxyphenyl)-2-(4-methoxyphenyl)ethanone

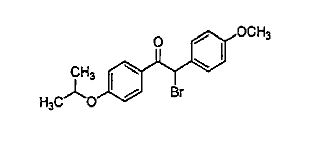

A title compound (454 mg, 1.25 mmol, quant.) was obtained as a light brown oil product from 1-(4-isopropyloxyphenyl)-2-(4-methoxyphenyl)ethanone (Reference Example 9) (355 mg, 1.25 mmol) in the same manner as in Reference Example 14.

¹H-NMR (400 MHz, CDCl₃) δ: 1.35 (6H, d, J = 6.4 Hz), 3.80 (3H, s), 4.63 (1H, sept, J = 6.4 Hz), 6.37 (1H, s), 6.87 (2H, d, J = 8.8 Hz), 6.88 (2H, d, J = 8.8 Hz), 7.47 (2H, d, J = 8.8 Hz), 7.95 (2H, d, J = 8.8 Hz).

### Reference Example 19

### 2-Bromo-2-(4-methoxyphenyl)-1-(4-trifluoromethylphenyl)ethanone

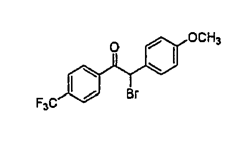

A title compound (466 mg, 1.25 mmol, quant.) was obtained as a light yellow solid from 2-(4-methoxyphenyl)-1-(4-trifluoromethylphenyl)ethanone (Reference Example 10) (368 mg, 1.25 mmol) in the same manner as in Reference Example 14.

¹H-NMR (400 MHz, CDCl₃) δ: 3.81 (3H, s), 6.34 (1H, s), 6.91 (2H, d, J = 8.8 Hz), 7.45 (2H, d, J = 8.8 Hz), 7.71 (2H, d, J = 8.8 Hz), 8.08 (2H, d, J = 8.8 Hz).

### Reference Example 20

### 2-Bromo-1-(4-methoxyphenyt)-2-(4-tolyl)ethanone

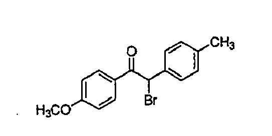

A title compound (849 mg, 2.66 mmol, quant.) was obtained as a yellow solid from 1-(4-methoxyphenyl)-2-(4-tolyl)ethanone (Reference Example 11) (640 mg, 2.66 mmol) in the same manner as in Reference Example 14.

¹H-NMR (400 MHz, CDCl₃) δ: 2.33 (3H, s), 3.85 (3H, s), 6.35 (1H, s), 6.90 (2H, d, J = 9.0 Hz), 7.17 (2H, d, J = 8.1 Hz), 7.41 (2H, d, J = 8.1 Hz), 7.97 (2H, d, J = 9.0 Hz).

### Reference Example 21

### 2-Bromo-2-(4-chlorophenyl)-1-(4-methoxyphenyl)ethanone

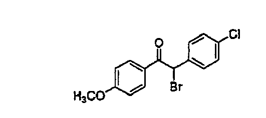

A title compound (1.24 g, 3.67 mmol, quant.) was obtained as a yellow solid from 2-(4-chlorophenyl)-1-(4-methoxyphenyl)ethanone (Reference Example 12) (958 mg, 3.67 mmol) in the same manner as in Reference Example 14.

¹H-NMR (400 MHz, CDCl₃) δ: 3.87 (3H, s), 6.29 (1H, s), 6.93 (2H, d, J = 9.0 Hz), 7.34 (2H, d, J = 8.5 Hz), 7.48 (2H, d, J = 8.5 Hz), 7.97 (2H, d, J = 9.0 Hz).

### Reference Example 22

### 2-Bromo-2-(4-fluorophenyl)-1-(4-methoxyphenyl)ethanone

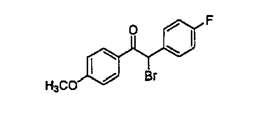

A title compound (492 mg, 1.52 mmol, 72%) was obtained as a white solid from 2-(4-fluorophenyl)-1-(4-methoxyphenyl)ethanone (Reference Example 13) (510 mg, 2.09 mmol) in the same manner as in Reference Example 14.

¹H-NMR (400 MHz, CDCl₃) δ: 3.87 (3H, s), 6.32 (1H, s), 6.93 (2H, d, J = 9.0 Hz), 7.06 (2H, t, J = 8.5 Hz), 7.53 (2H, dd, J = 5.1, 8.5 Hz), 7.98 (2H, d, J = 9.0 Hz).

### Reference Example 23

### 2-Bromo-1-(4-fluorophenyl)-2-phenylethanone

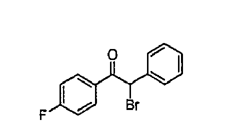

A title compound (721 mg, 2.46 mmol, quant.) was obtained as a light yellow solid from 1-(4-fluorophenyl)-2-phenylethanone (527 mg, 2.46 mmol) in the same manner as in

### Reference Example 14.

¹H-NMR (400 MHz, CDCl₃) δ: 6.32 (1H, s), 7.10-7.14 (2H, m), 7.33-7.40 (3H, m), 7.52 (2H, d, J = 8.0 Hz), 8.00-8.04 (2H, m).

### Reference Example 24

### 2-Bromo-2-phenyl-1-(4-trifluoromethylphenyl)ethanone

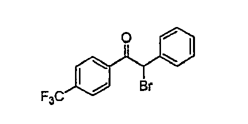

A title compound (662 mg, 1.93 mmol, quant.) was obtained as a light yellow solid from 2-phenyl-1-(4-trifluoromethylphenyl)ethanone (511 mg, 1.93 mmol) in the same manner as in Reference Example 14.

¹H-NMR (400 MHz, CDCl₃) δ: 6.33 (1H, s), 7.35-7.41 (3H, m), 7.52 (2H, d, J = 6.6 Hz), 7.72 (2H, d, J = 8.0 Hz), 8.09 (2H, d, J = 8.0 Hz).

### Reference Example 25

### 4,5-Bis(4-methoxyphenyl)oxazol-2-one

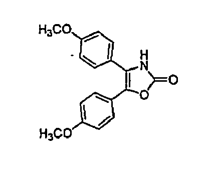

A mixture of p-anisoin (35.3 g, 0.130 mol), methyl carbamate (24.5 g, 0.327 mol), and pyridine (10 ml) was stirred at 150°C for 22 hours and then cooled to room temperature. Distilled water (100 ml) was added to the reaction mixture, and a solid was filtered, followed by rinsing with water. The resulting slurry was washed with ethanol, and a title compound (35.9 g, 0.121 mol, 93%) was obtained as a light brown solid.

¹H-NMR (400 MHz, DMSO-d₆) δ: 3.76 (3H, s), 3.79 (3H, s), 6.94 (2H, d, J = 8.8 Hz), 7.01 (2H, d, J = 8.8 Hz), 7.31 (2H, d, J = 8.8 Hz), 7.38 (2H, d, J = 8.8 Hz), 11.14 (1H, brs).

### Reference Example 26

### 2-Chloro-4,5-bis(4-methoxyphenyl)oxazole

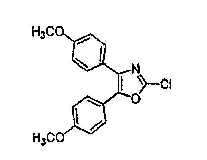

A mixture of 4,5-bis(4-methoxyphenyl)oxazol-2-one (Reference Example 25) (35.9 g, 0.121 mol) and phosphorus oxychloride (70 ml) was stirred at 90°C for 20 hours and then cooled to room temperature. The reaction mixture was poured on ice, and aqueous ammonia was added to neutralize the mixture, followed by extraction with dichloromethane. The organic layer was dried over sodium sulfate and concentrated. The residue was recrystallized (ethyl acetate/n-hexane = 1/1) to give a title compound (18.5 g, 0.0585 mol, 48%) as a light yellow crystal.

¹H-NMR (400 MHz, CDCl₃) δ: 3.83 (3H, s), 3.84 (3H, s), 6.89 (2H, d, J = 8.8 Hz), 6.99 (2H, d, J = 8.8 Hz), 7.49 (2H, d, J = 8.8 Hz), 7.54 (2H, d, J = 8.8 Hz).

### Reference Example 27

### 1-(Benzyloxycarbonyl)piperidine-4-carboxylic acid

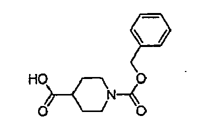

To an aqueous solution (i.e., 300 ml of distilled water) of isonipecotic acid (10.0 g, 77.4 mmol) and sodium bicarbonate (19.5 g, 232 mmol) was added benzyl chloroformate (11.5 ml, 80.9 mmol). The reaction solution was stirred at room temperature for 20 hours, and ethyl acetate was added thereto then the solution was separated. Concentrated hydrochloric acid was added to the aqueous layer to bring a pH level to 1, followed by extraction with ethyl acetate. The organic layer was dried over magnesium sulfate and concentrated under a reduced pressure to give a title compound (14.7 g, 55.8 mmol, 72%) as a colorless oil product.

¹H-NMR (400 MHz, CDCl₃) δ: 1.69 (2H, m), 1.93 (2H, m), 2.51 (1H, m), 2.96 (2H, m), 4.11 (2H, m), 5.13 (2H, s), 7.29-7.39 (5H, m).

### Reference Example 28

### 4-(1,2-Bis(4-methoxyphenyl)-2-oxoethyl) 1-benzyloxycarbonyl-4-piperidinecarboxylate

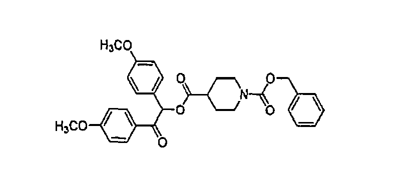

To a solution of 1-(benzyloxycarbonyl)piperidine-4-carboxylic acid (Reference Example 27) (14.7 g, 55.8 mmol) in dichloromethane (100 ml) was added oxalyl chloride (7.0 ml, 7.89 mmol) and N,N-dimethylformamide (0.1 ml) with stirring. The reaction solution was stirred at room temperature for 1.5 hours and concentrated under a reduced pressure to give 1-benzyloxycarbonylisonipecotic acid chloride.

To a solution of p-anisoin (15.2 g, 55.8 mmol) and triethylamine (11.5 ml, 84.0 mmol) in dichloromethane (100 ml), a solution of 1-benzyloxycarbonylisonipecotic acid chloride in dichloromethane (50 ml) was added dropwise at 0°C. The reaction solution was stirred at the same temperature for 15 hours and 1M hydrochloric acid was added to the solution, then the solution was separated. The organic layer was washed with saturated aqueous sodium bicarbonate, dried over sodium sulfate, and concentrated under a reduced pressure. The residue was purified by column chromatography (silica gel, n-hexane/ethyl acetate = 2/1) to give a title compound (23.2 g, 44.8 mmol, 80%) as a colorless to light yellow oil product.

¹H-NMR (400 MHz, CDCl₃) δ: 1.74 (2H, m), 1.91 (1H, m), 2.02 (1H, m), 2.65 (1H, m), 2.99 (2H, m), 3.77 (3H, s), 3.82 (3H, s), 4.06 (2H, m), 5.12 (2H, s), 6.79 (1H, s), 6.86 (2H, d, J = 8.5 Hz), 6.87 (2H, d, J = 8.5 Hz), 7.28-7.38 (7H, m), 7.90 (2H, d, J = 8.5 Hz).

### Reference Example 29

### 1-(Benzyloxycarbonyl)piperidine-3-carboxylic acid

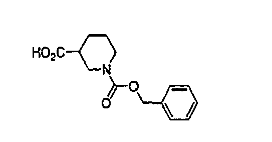

A title compound (1.28 g, 4.86 mmol, 97%) was obtained as a colorless oil product from nipecotic acid (646 mg, 5.00 mmol) in the same manner as in Reference Example 27.

¹H-NMR (400 MHz, CDCl₃) δ: 1.48-1.55 (1H, m), 1.64-1.75 (2H, m), 2.07-2.11 (1H, m), 2.50-2.55 (1H, m), 2.94 (1H, ddd, J = 13.6, 10.8, 2.8 Hz), 3.10-3.18 (1H, m), 3.95-3.99 (1H, m), 4.14-4.23 (1H, m), 5.11 (1H, d, J = 12.0 Hz), 5.16 (1H, d, J = 12.0 Hz), 7.30-7.41 (5H, m).

IR (neat, cm⁻¹): 2947, 1701, 1441, 1263, 1239, 1151.

### Reference Example 30

### 3-(1,2-Bis(4-methoxyphenyl)-2-oxoethyl) 1-benzyloxycarbonyl-3-piperidinecarboxylate

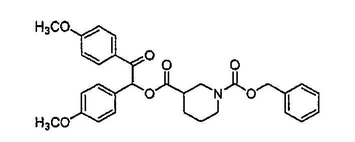

A title compound (1.09 g, 2.11 mmol, 55%) was obtained as a colorless oil product from 1-(benzyloxycarbonyl)-3-piperidinecarboxylic acid (Reference Example 29) (1.00 g, 3.80 mmol) in the same manner as in Reference Example 28.

¹H-NMR (400 MHz, CDCl₃) δ: 1.48-1.54 (1H, m), 1.68-1.80 (2H, m), 2.09-2.13 (1H, m), 2.23-2.26 (1H, m), 2.60-2.65 (1H, m), 2.84-2.94 (1H, m), 3.10-3.18 (1H, m), 3.77 (3H, s), 3.84 (3H, s), 3.97-4.03 (1H, m), 5.10, 5.13 (2H, s×2, diastereomers), 6.76, 6.77 (2H, s×2, diastereomers), 6.85-6.87 (4H, m), 7.28-7.36 (7H, m), 7.90 (2H, d, J = 8.8 Hz).

IR (KBr, cm⁻¹): 2939, 1733, 1690, 1514, 1262, 1237, 1171, 1029, 833.

### Reference Example 31

### (1-(4-Methoxyphenyl)-2-(4-tert-butylphenyl)-2-oxoethyl) 1-benzyloxycarbonyl-4-piperidinecarboxylate

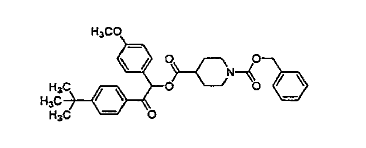

To a solution of 2-bromo-1-(4-tert-butylphenyl)-2-(4-methoxyphenyl)ethanone (Reference Example 15) (451 mg, 1.25 mmol) and 1-(benzyloxycarbonyl)piperidine-4-carboxylic acid (362 mg, 1.38 mmol) in N,N-dimethylformamide (5 ml) was added sodium bicarbonate (315 mg, 3.75 mmol). The reaction solution was stirred at room temperature for 16 hours, and an aqueous solution of saturated ammonium chloride was added, followed by extraction with ethyl acetate. The organic layer was dried over sodium sulfate and concentrated under a reduced pressure. The residue was purified by column chromatography (silica gel, n-hexane/ethyl acetate = 5/1) to give a title compound (575 mg, 1.06 mmol, 85%) as a white amorphous product.

¹H-NMR (400 MHz, CDCl₃) δ: 1.29 (9H, s), 1.71-1.80 (2H, m), 1.89-1.93 (1H, m), 2.01-2.20 (1H, m), 2.65 (1H, tt, J = 10.4, 4.0 Hz), 2.99-3.02 (2H, m), 3.78 (3H, s), 4.00-4.13 (2H, m), 5.12 (2H, s), 6.81 (2H, d, J = 8.8 Hz), 7.28-7.36 (5H, m), 7.37 (2H, d, J = 8.8 Hz), 7.40 (2H, d, J = 8.8 Hz), 7.85 (2H, d, J = 8.8 Hz).

IR (KBr, cm⁻¹): 2962, 1733, 1694, 1608, 1514, 1269, 1253, 1232, 1170, 1030, 829

ESI-MS: m/z = 544 (M+H⁺).

### Reference Example 32

### (1-(4-Methoxyphenyl)-2-(4-tolyl)-2-oxoethyl) 1-benzyloxycarbonyl-4-piperidinecarboxylate

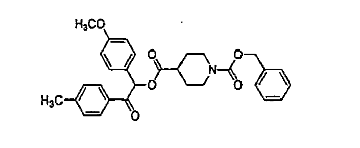

A title compound (1.12 g, 2.23 mmol, 77%) was obtained as a light yellow oil product from 1-(benzyloxycarbonyl)piperidine-4-carboxylic acid (Reference Example 27) (760 mg, 2.88 mmol) and 2-bromo-2-(4-methoxyphenyl)-1-(4-tolyl)ethanone (Reference Example 16) (934 mg, 2.88 mmol) in the same manner as in Reference Example 31.

¹H-NMR (400 MHz, CDCl₃) δ: 1.75 (2H, m), 1.90 (1H, m), 2.01 (1H, m), 2.35 (3H, s), 2.65 (1H, m), 3.00 (2H, m), 3.77 (3H, s), 4.07 (2H, m), 5.12 (2H, s), 6.80 (1H, s), 6.87 (2H, d, J = 8.8 Hz), 7.18 (2H, d, J = 8.8 Hz), 7.27-7.36 (7H, m), 7.81 (2H, d, J = 8.8 Hz).

### Reference Example 33

### (2-(4-Isopropyloxyphenyl)-1-(4-methoxyphenyl)-2-oxoethyl) 1-benzyloxycarbonyl-4-piperidinecarboxylate

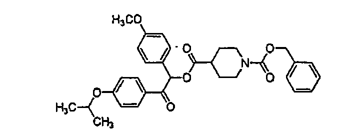

A title compound (547 mg, 1.00 mmol, 80%) was obtained as a white amorphous product from 1-(benzyloxycarbonyl)piperidine-4-carboxylic acid (Reference Example 27) and 2-bromo-1-(4-isopropyloxyphenyl)-2-(4-methoxyphenyl)ethanone (Reference Example 18) (454 mg, 1.25 mmol) in the same manner as in Reference Example 31.

¹H-NMR (400 MHz, CDCl₃) δ: 1.32 (6H, d, J = 6.0 Hz), 1.71-1.79 (2H, m), 1.89-1.94 (1H, m), 2.00-2.05 (1H, m), 2.65 (1H, tt, J = 10.0, 4.0 Hz), 2.99-3.01 (2H, m), 3.78 (3H, s), 4.02-4.12 (2H, m), 4.59 (1H, sept, J = 6.0 Hz), 5.12 (2H, s), 6.79 (1H, s), 6.82 (2H, d, J = 9.2 Hz), 6.88 (2H, d, J = 8.8 Hz), 7.29-7.38 (7H, m), 7.88 (2H, d, J = 8.8 Hz).

IR (KBr, cm⁻¹): 2977, 2934, 1733, 1690, 1599, 1513, 1426, 1235, 1170, 1029, 946, 830.
ESI-MS: m/z = 546 (M+H⁺).

### Reference Example 34

### (1-(4-Methoxyphenyl)-2-(4-trifluoromethylphenyl)-2-oxoethyl) 1-benzyloxycarbonyl-4-piperidinecarboxylate

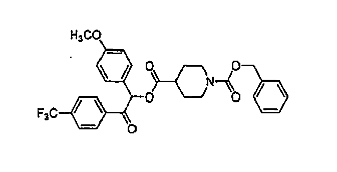

A title compound (590 mg, 1.06 mmol, 85%) was obtained as a white amorphous product from 1-(benzyloxycarbonyl)piperidine-4-carboxylic acid (Reference Example 27) and 2-bromo-2-(4-methoxyphenyl)-1-(4-trifluoromethylphenyl)ethanone (Reference Example 19) (466 mg, 1.25 mmol) in the same manner as in Reference Example 31.

¹H-NMR (400 MHz, CDCl₃) δ: 1.72-1.77 (2H, m), 1.91-1.92 (1H, m), 2.00-2.05 (1H, m), 2.63-2.70 (1H, m), 2.98-3.04 (2H, m), 3.79 (3H, s), 4.02-4.12 (2H, m), 5.13 (2H, s), 6.77 (1H, s), 6.90 (2H, d, J = 8.8 Hz), 7.29-7.38 (7H, m), 7.66 (2H, d, J = 8.8 Hz), 7.99 (2H, d, J = 8.8 Hz).

IR (KBr, cm⁻¹): 2957, 1734, 1702, 1610, 1514, 1433, 1324, 1229, 1170, 1129, 1067, 1029,829.

ESI-MS: m/z = 556 (M+H⁺).

### Reference Example 35

### (2-(4-Methoxyphenyl)-1-(4-tolyl)-2-oxoethyl) 1-benzyloxycarbonyl-4-piperidinecarboxylate

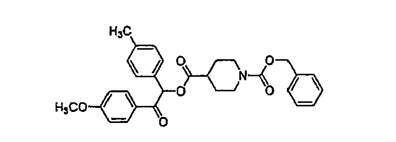

A title compound (604 mg, 1.20 mmol, 64%) was obtained as a colorless amorphous product from 1-(benzyloxycarbonyl)piperidine-4-carboxylic acid (Reference Example 27) (489 mg, 1.86 mmol) and 2-bromo-1-(4-methoxyphenyl)-2-(4-tolyl)ethanone (Reference Example 20) (619 mg, 1.93 mmol) in the same manner as in Reference Example 31.

¹H-NMR (400 MHz, CDCl₃) δ: 1.75 (2H, m), 1.90 (1H, m), 2.01 (1H, m), 2.32 (3H, s), 2.66 (1H, m), 2.99 (2H, m), 3.82 (3H, s), 4.07 (2H, m), 5.12 (2H, s), 6.80 (1H, s), 6.86 (2H, d, J = 8.8 Hz), 7.16 (2H, d, J = 8.1 Hz), 7.29-7.38 (7H, m), 7.90 (2H, d, J = 8.8 Hz).

### Reference Example 36

### (1-(4-Chlorophenyl)-2-(4-methoxyphenyl)-2-oxoethyl) 1-benzyloxycarbonyl-4-piperidinecarboxylate

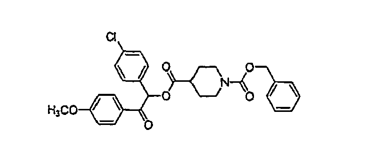

A title compound (685 mg, 1.31 mmol, 52%) was obtained as a yellow amorphous product from 1-(benzyloxycarbonyl)piperidine-4-carboxylic acid (Reference Example 27) (667 mg, 2.53 mmol) and 2-bromo-2-(4-chlorophenyl)-1-(4-methoxyphenyl)ethanone (Reference Example 21) (864 mg, 2.53 mmol) in the same manner as in Reference Example 31.

¹H-NMR (400 MHz, CDCl₃) δ: 1.74 (2H, m), 1.90 (1H, m), 2.00 (1H, m), 2.66 (1H, m), 3.00 (2H, m), 3.83 (3H, s), 4.08 (2H, m), 5.12 (2H, s), 6.80 (1H, s), 6.89 (2H, d, J = 9.0 Hz), 7.29-7.39 (9H, m), 7.89 (2H, d, J = 9.0 Hz).

### Reference Example 37

### (1-(4-Fluorophenyl)-2-(4-methoxyphenyl)-2-oxoethyl) 1-tert-butoxycarbonyl-4-piperidinecarboxylate

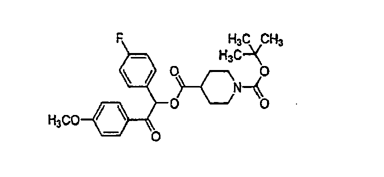

A title compound (370 mg, 0.784 mmol, 52%) was obtained as a white amorphous product from 2-bromo-2-(4-fluorophenyl)-1-(4-methoxyphenyl)ethanone (Reference Example 22) (490 mg, 1.51 mmol) and 1-tert-butoxycarbonylisonipecotic acid (364 mg, 1.59 mmol) in the same manner as in Reference Example 31.

¹H-NMR (400 MHz, CDCl₃) δ: 1.45 (9H, s), 1.71 (2H, m), 1.89 (1H, m), 2.00 (1H, m), 2.63 (1H, m), 2.88 (2H, m), 3.83 (3H, s), 4.01 (2H, m), 6.82 (1H, s), 6.88 (2H, d, J = 9.0 Hz), 7.05 (2H, t, J = 8.8 Hz), 7.43 (2H, dd, J = 5.1, 8.8 Hz), 7.90 (2H, d, J = 9.0 Hz).

### Reference Example 38

### (1-Phenyl-2-(4-trifluoromethylphenyl)-2-oxoethyl) 1-benzyloxycarbonyl-4-piperidinecarboxylate

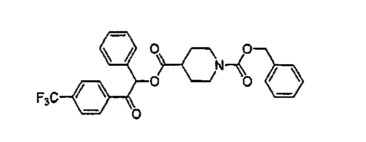

A title compound (242 mg, 0.460 mmol, 46%) was obtained as a white amorphous product from 1-(benzyloxycarbonyl)piperidine-4-carboxylic acid (Reference Example 27) (275 mg, 1.04 mmol) and 2-bromo-2-phenyl-1-(4-trifluoromethylphenyl)ethanone (Reference Example 24) (344 mg, 1.00 mmol) in the same manner as in Reference Example 31.

¹H-NMR (400 MHz, CDCl₃) δ: 1.75 (2H, m), 1.92 (1H, m), 2.03 (1H, m), 2.68 (1H, m), 3.01 (2H, m), 4.08 (2H, m), 5.13 (2H, s), 6.80 (1H, s), 7.29-7.44 (10H, m), 7.67 (2H, d, J = 8.3 Hz), 8.00 (2H, d, J = 8.3 Hz).

### Reference Example 39

### (2-(4-Chlorophenyl)-1-(4-methoxyphenyl)-2-oxoethyl) 1-acetyl-4-piperidinecarboxylate

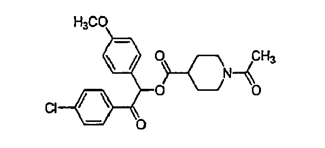

A title compound (970 mg, 2.25 mmol, 77%) was obtained as a white amorphous product from 2-bromo-1-(4-chlorophenyl)-2-(4-methoxyphenyl)ethanone (Reference Example 17) (988 mg, 2.91 mmol) and 1-acetylisonipecotic acid (550 mg, 3.21 mmol)) in the same manner as in Reference Example 31.

¹H-NMR (400 MHz, CDCl₃) δ: 1.77 (2H, m), 1.94 (1H, m), 2.05 (1H, m), 2.09 (3H, s), 2.72 (1H, m), 2.92 (1H, m), 3.18 (1H, m), 3.78 (3H, s), 3.79 (1H, m), 4.35 (1H, m), 6.75 (1H, s), 6.89 (2H, d, J = 8.8 Hz), 7.33 (2H, d, J = 8.8 Hz), 7.37 (2H, d, J = 8.5 Hz), 7.84 (2H, d, J = 8.5 Hz).

### Reference Example 40

### (2-(4-Chlorophenyl)-1-(4-tolyl)-2-oxoethyl) 1-acetyl-4-piperidinecarboxylate

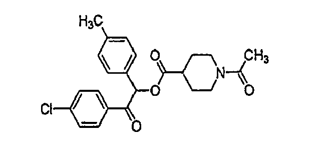

A title compound (396 mg, 0.956 mmol, 96%) was obtained as a white amorphous product from 2-bromo-1-(4-chlorophenyl)-2-(4-tolyl)-2-ethanone (324 mg, 1.00 mmol) and 1-acetylisonipecotic acid (256 mg, 1.50 mmol) in the same manner as in Reference Example 31.

¹H-NMR (400 MHz, CDCl₃) δ: 1.68-1.84 (2H, m), 1.90 (2H, m), 2.09 (3H, s), 2.33 (3H, s), 2.72 (1H, m), 2.92 (1H, m), 3.19 (1H, m), 3.79 (1H, m), 4.35 (1H, m), 6.76 (1H, s), 7.18 (2H, d, J = 8.3 Hz), 7.30 (2H, d, J = 8.3Hz), 7.36 (2H, d, J = 8.5 Hz), 7.84 (2H, d, J = 8.5 Hz).

### Reference Example 41

### (2-(4-Fluorophenyl)-1-phenyl-2-oxoethyl) 1-tert-butoxycarbonyl-4-piperidinecarboxylate

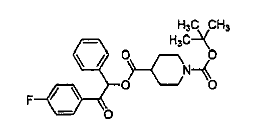

A title compound (246 mg, 0.557 mmol, 53%) was obtained as a colorless oil product from 2-bromo-1-(4-fluorophenyl)-2-phenylethanone (Reference Example 23) (304 mg, 1.04 mmol) and N-tert-butoxycarbonylisonipecotic acid (257 mg, 1.04 mmol) in the same manner as in Reference Example 31.

¹H-NMR (400 MHz, CDCl₃) δ: 1.45 (9H, s), 1.72 (2H, m), 1.90 (1H, m), 2.02 (1H, m), 2.64 (1H, m), 2.90 (2H, m), 4.01 (2H, m), 6.80 (1H, s), 7.08 (2H, t, J = 8.8 Hz), 7.36-7.45 (5H, m), 7.95 (2H, dd, J = 5.4, 8.8 Hz).

### Reference Example 42

### Methyl 1-(N-hydroxy-N-methylcarbamoyl)-4-piperidinecarboxylate

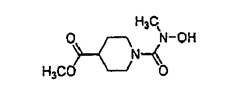

To a solution of triphosgene (3.47 g, 11.7 mmol) in dichloromethane (10 ml), methyl isonipecotate (5.03 g, 35.1 mmol) and triethylamine (4.80 ml, 35.1 mmol) were added at - 78°C. Thereafter, the reaction solution was stirred at room temperature for 3 hours and 1M hydrochloric acid was added, followed by extraction with ethyl acetate. The organic layer was dried over magnesium sulfate and it was then concentrated under a reduced pressure (4.58 g). The residue was dissolved in dichloromethane (20 ml) was added N-methylhydroxylamine hydrochloride (1.49 g, 17.8 mmol) and triethylamine (3.5 ml, 25.6 mmol), then the resultant was stirred at room temperature for 3 hours. 1M hydrochloric acid was added to the reaction solution then the solution was separated. The organic layer was dried over sodium sulfate and concentrated under a reduced pressure. The residue was purified by column chromatography (silica gel, n-hexane/ethyl acetate = 1/1) to give a title compound (2.04 g, 9.43 mmol, 27%) as a light yellow oil product.

¹H-NMR (400 MHz, CDCl₃) δ: 1.71 (2H, m), 1.95 (2H, m), 2.55 (1H, m), 2.97 (3H, s), 3.02 (2H, m), 3.70 (3H, s), 3.96 (2H, m), 6.77 (1H, brs).

### Reference Example 43

### 1-(N-hydroxy-N-methylcarbamoyl)-4-piperidinecarboxylic acid

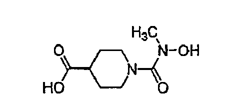

A mixed solution of methyl 1-(N-hydroxy-N-methylcarbamoyl)-4-piperidinecarboxylate (Reference Example 42) (206 mg, 1.02 mmol) and lithium hydroxide monohydrate (43 mg, 1.0 mmol) in tetrahydrofuran (1.0 ml) and distilled water (1.0 ml) was stirred at room temperature for 14 hours. The reaction solution was diluted with the addition of distilled water and then extracted with ethyl acetate. The organic layer was dried over magnesium sulfate and concentrated under a reduced pressure to give a title compound (143 mg, 0.707 mmol, 69%) as a light yellow oil product.

¹H-NMR (400 MHz, CDCl₃) δ: 1.73 (2H, m), 1.98 (2H, m), 2.59 (1H, m), 2.98 (3H, s), 3.04 (2H, m), 3.96 (2H, m).

### Reference Example 44

### (1-(4-Chlorophenyl)-2-(4-methoxyphenyl)-2-oxoethyl) 1-(N-hydroxy-N-methylcarbamoyl)-4-piperidinecarboxylate

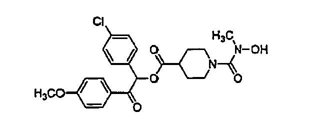

A title compound (97 mg, 0.21 mmol, 30%) was obtained as a white amorphous product from 1-(N-hydroxy-N-methylcarbamoyl)-4-piperidinecarboxylic acid (Reference Example 43) (140 mg, 0.692 mmol) and 2-bromo-2-(4-chlorophenyl)-1-(4-methoxyphenyl)ethanone (235 mg, 0.692 mmol) in the same manner as in Reference Example 31.

¹H-NMR (400 MHz, CDCl₃) δ: 1.81 (2H, m), 1.95 (1H, m), 2.05 (1H, m), 2.73 (1H, m), 2.96 (3H, s), 3.10 (2H, m), 3.83 (3H, s), 3.93 (2H, m), 6.74 (1H, brs), 6.74 (1H, s), 6.88 (2H, d, J = 8.8 Hz), 7.34 (2H, d, J = 8.5 Hz), 7.38 (2H, d, J = 8.5 Hz), 7.89 (2H, d, J = 8.8 Hz).

### Reference Example 45

### tert-Butyl 3-(1-benzyloxycarbonyl-4-piperidyl)-3-oxopropionate

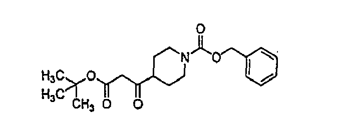

To a solution of 1-(benzyloxycarbonyl)piperidine-4-carboxylic acid (Reference Example 27) (2.63 g, 10.0 mmol) in dichloromethane (50 ml) was added oxalyl chloride (1.39 g, 11.0 mmol) and N,N-dimethylformamide (0.1 ml). The reaction solution was stirred at room temperature for 1.5 hours and then concentrated under a reduced pressure to give 1-benzyloxycarbonyl-4-piperidinecarboxylic acid chloride.

To a solution of diisopropylamine (4.34 ml, 31.0 mmol) in tetrahydrofuran (30 ml), a solution of n-butyllithium in n-hexane (12.3 ml, 30.0 mmol, 2.44 M in hexane) was added dropwise at -78°C. The reaction solution was stirred at the same temperature for 1 hour, and tert-butyl acetate (4.04 ml, 30.0 mmol) was then added dropwise thereto. The reaction solution was stirred for 1 hour, and a solution of 1-benzyloxycarbonyl-4-piperidinecarboxylic acid chloride in tetrahydrofuran (10 ml) was added at -78°C. The reaction solution was stirred at the same temperature for 1.5 hours, and an aqueous solution of saturated ammonium chloride was then added, followed by extraction with ethyl acetate. The organic layer was washed with saturated aqueous sodium bicarbonate, dried over sodium sulfate, and concentrated under a reduced pressure. The residue was purified by column chromatography (silica gel, n-hexane/ethyl acetate = 5/1) to give a title compound (1.90 g, 5.25 mmol, 53%) as a light yellow oil product.

¹H-NMR (400 MHz, CDCl₃) δ: 1.46 (9H, s), 1.55-1.63 (2H, m), 1.81-1.90 (2H, m), 2.64 (1H, tt, J = 11.2, 4.0 Hz), 2.86-2.92 (2H, m), 3.40 (2H, s), 4.12-4.23 (2H, m), 5.12 (2H, s), 7.30-7.36 (5H, m).

IR (KBr, cm⁻¹): 3419, 1745, 1690, 1428, 1367, 1321, 1269, 1226, 1153, 1130, 1060, 1017,945,841.

### Reference Example 46

### tert-Butyl 2-(1-benzyloxycarbonyl-4-piperidinecarbonyl)-3,4-bis(4-methoxyphenyl)-4-oxobutyrate

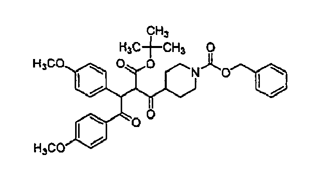

To a solution of 1,2-bis(4-methoxyphenyl)-2-bromoethanone (Reference Example 14) (1.69 g, 5.04 mmol) and tert-butyl 3-(1-benzyloxycarbonyl-4-piperidinecarbonyl)-3-oxopropionate (Reference Example 45) (1.82 g, 5.04 mmol) in N,N-dimethylformamide (20 ml) was added potassium carbonate (1.39 g, 10.1 mmol), the resultant was stirred at room temperature for 17 hours, and an aqueous solution of saturated ammonium chloride was then added, followed by extraction with ethyl acetate. The organic layer was dried over sodium sulfate and concentrated under a reduced pressure. The residue was purified by column chromatography (silica gel, n-hexane/ethyl acetate = 3/2) to give a title compound (2.47 g, 4.01 mmol, 80%) as a light yellow amorphous product.

¹H-NMR (400 MHz, CDCl₃, ca. 1:1 of diastereomeric mixture) δ: 1.22 (4.5H, s), 1.34 (4.5H, s), 1.40-1.80 (2H, m), 2.05-2.25 (2H, m), 2.64-2.93 (3H, m), 3.71 (1.5H, s), 3.73 (1.5H, s), 3.80 (3H, s), 4.03-4.15 (2H, m), 4.56 (0.5H, d, J = 11.2 Hz), 4.64 (0.5H, d, J = 11.6 Hz), 5.08 (0.5H, s), 5.12 (0.5H, s), 5.22 (0.5H, d, J = 11.6 Hz), 5.25 (0.5H, d, J = 11.2 Hz), 6.76-6.87 (4H, m), 7.16 (1H, d, J = 8.8 Hz), 7.21 (1H, d, J = 8.8 Hz), 7.30-7.35 (5H, m), 7.92 (1H, d, J = 8.8 Hz), 7.95 (1H, d, J = 8.8 Hz).

IR (KBr, cm⁻¹): 3439, 2934, 1704, 1600, 1511, 1443, 1368, 1264, 1172, 1029,831.

### Reference Example 47

### 1-Benzyloxycarbonyl-4-(3,4-bis(4-methoxyphenyl)-1,4-dioxobutyl)piperidine

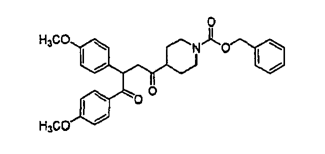

To a solution of tert-butyl 2-(1-benzyloxycarbonyl-4-piperidinecarbonyl)-3,4-bis(4-methoxyphenyl)-4-oxobutyrate (Reference Example 46) (4.13 g, 6.70 mmol) in dichloromethane (10 ml) was added trifluoroacetic acid (10 ml). The reaction solution was stirred at room temperature for 15 hours, and the solvent was removed by distillation under a reduced pressure. The residue was dissolved in dimethyl sulfoxide (15 ml) and then stirred at 140°C for 4.5 hours. After the reaction solution was cooled to room temperature, water was added, followed by extraction with ethyl acetate. The organic layer was dried over sodium sulfate, concentrated under a reduced pressure, and the residue was purified by column chromatography (silica gel, n-hexane/ethyl acetate = 5/1-3/1) to give a title compound (790 mg, 1.53 mmol, 23%) as a brown amorphous product.

¹H-NMR (400 MHz, CDCl₃) δ: 1.50-1.57 (2H, m), 1.80-1.90 (2H, m), 2.52-2.56 (1H, m), 2.69 (1H, dd, J = 17.6, 4.0 Hz), 2.84-2.90 (2H, m), 3.55 (1H, dd, J = 17.6, 10.0 Hz), 3.74 (3H, s), 3.81 (3H, s), 4.11-4.16 (2H, m), 5.04 (1H, dd, J = 10.0, 4.0 Hz), 5.11 (2H, s), 6.80 (2H, d, J = 8.8 Hz), 6.84 (2H, d, J = 8.8 Hz), 7.16 (2H, d, J = 8.8 Hz), 7.30-7.36 (5H, m), 7.93 (2H, d, J = 8.8 Hz).

IR (KBr, cm⁻¹): 2952, 2837, 1708, 1599, 1511, 1440, 1254, 1168, 1130, 1026, 835.

ESI-MS: m/z = 516 (M+H⁺).

### Reference Example 48

### 1-Benzyloxycarbonyl-4-(3-(4-methoxyphenyl)-1,3-dioxopropyl)piperidine

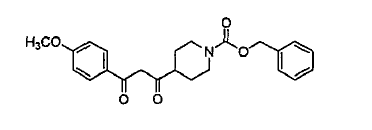

To a solution of diisopropylamine (2.65 ml, 18.9 mmol) in tetrahydrofuran (30 ml) was added dropwise a solution of n-butyllithium in n-hexane (11.6 ml, 18.3 mmol, 1.58 M in hexane) at -78°C. The reaction solution was stirred at the same temperature for 40 minutes, and a solution of 4-methoxyacetophenone (2.75 g, 18.3 mmol) in tetrahydrofuran (10 ml) was then added dropwise thereto. The reaction solution was stirred for 1 hour, and a solution of 1-benzyloxycarbonyl-4-piperidinecarboxylic acid chloride (1.72 g, 6.11 mmol) in tetrahydrofuran (10 ml) was added at -78°C. The reaction solution was stirred at the same temperature for 1 hour, and an aqueous solution of saturated ammonium chloride was added, followed by extraction with ethyl acetate. The organic layer was dried over sodium sulfate and then concentrated under a reduced pressure. The residue was purified by column chromatography (silica gel, n-hexane/ethyl acetate = 3/1) to give a title compound (1.84 g, 4.65 mmol, 76%) as a light yellow oil product.

¹H-NMR (400 MHz, CDCl₃) δ: 1.67-1.69 (2H, m), 1.89-1.93 (2H, m), 2.47 (1H, tt, J = 11.6, 3.6 Hz), 2.88 (2H, m), 3.86 (2H, s), 3.87 (3H, s), 4.28 (2H, m), 5.14 (2H, s), 6.11 (1H, s), 6.94 (2H, d, J = 8.8 Hz), 7.30-7.40 (5H, m), 7.86 (2H, d, J = 8.8 Hz).

IR (neat, cm⁻¹): 3463, 2938, 1736, 1698, 1602, 1512, 1435, 1256, 1174, 1030, 844.

ESI-MS: m/z = 396 (M+H⁺).

### Reference Example 49

### 1-Benzyloxycarbonyl-4-(4,5-bis(4-methoxyphenyl)oxazol-2-yl)piperidine

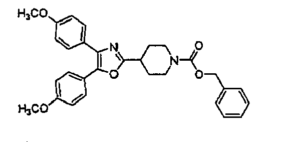

To a solution of 4-(1,2-bis(4-dimethoxyphenyl)-2-oxoethyl) 1-benzyloxycarbonyl-4-piperidinecarboxylate (Reference Example 28) (23.2 g, 44.8 mmol) in acetic acid (100 ml) was added ammonium acetate (20.4 g, 264 mmol), the reaction solution was stirred at 90°C for 17 hours, and the resultant was then cooled to room temperature. The reaction solution was concentrated under a reduced pressure, and the residue was dissolved in ethyl acetate, followed by washing with distilled water. The organic layer was neutralized with saturated aqueous sodium bicarbonate, dried over magnesium sulfate, and then concentrated under a reduced pressure. The residue was purified by column chromatography (silica gel, n-hexane/ethyl acetate = 4/1-3/1) to give a title compound (21.0 g, 41.9 mmol, 93%) as a yellow oil product.

¹H-NMR (400 MHz, CDCl₃) δ: 1.90 (2H, m), 2.11 (2H, m), 3.00-3.12 (3H, m), 3.83 (3H, s), 3.83 (3H, s), 4.20 (2H, m), 5.15 (2H, s), 6.88 (2H, d, J = 9.0 Hz), 6.89 (2H, d, J = 9.0 Hz), 7.30-7.37 (5H, m), 7.48 (2H, d, J = 9.0 Hz), 7.54 (2H, d, J = 9.0 Hz).

### Reference Example 50

### 1-Benzyloxycarbonyl-3-(4,5-bis(4-methoxyphenyl)oxazol-2-yl)piperidine

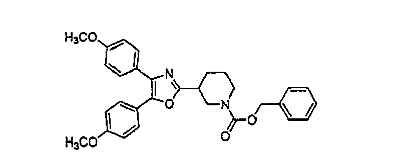

A title compound (940 mg, 1.89 mmol, 90%) was obtained as a white amorphous product from 3-(1,2-bis(4-dimethoxyphenyl)-2-oxoethyl) 1-benzyloxycarbonyl-3-piperidinecarboxylate (Reference Example 30) (1.09 g, 2.10 mmol) in the same manner as in

### Reference Example 49.

¹H-NMR (400 MHz, CDCl₃) δ: 1.58-1.61 (1H, m), 1.84-1.93 (2H, m), 2.25-2.28 (1H, m), 2.29-3.06 (2H, m), 3.25-3.35 (1H, m), 3.83 (6H, s), 4.08-4.15 (1H, m), 4.30-4.40 (1H, m), 5.15 (2H, s), 6.88 (2H, d, J = 8.8 Hz), 6.90 (2H, d, J = 8.8 Hz), 7.30-7.35 (5H, m), 7.48 (2H, d, J = 8.8 Hz), 7.54 (2H, d, J = 8.8 Hz).

IR (KBr, cm⁻¹): 2941, 1696, 1603, 1427, 1260, 1165, 1028, 835.

### Reference Example 51

### 1-Benzyloxycarbonyl-4-(4-(4-tert-butylphenyl)-5-(4-methoxyphenyl)oxazol-2-yl)piperidine

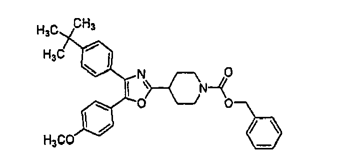

A title compound (499 mg, 0.95 mol, 95%) was obtained as a white amorphous product from (2-(4-tert-butylphenyl)-1-(4-methoxyphenyl)-2-oxoethyl) 1-benzyloxycarbonyl-4-piperidinecarboxylate (Reference Example 31) (543 mg, 1.00 mmol) in the same manner as in Reference Example 49.

¹H-NMR (400 MHz, CDCl₃) δ: 1.33 (9H, s), 1.87-1.95 (2H, m), 2.09-2.17 (2H, m), 3.02-3.09 (3H, m), 3.84 (3H, s), 4.16-4.25 (2H, m), 5.15 (2H, s), 6.90 (2H, d, J = 8.8 Hz), 7.29-7.38 (7H, m), 7.52 (2H, d, J = 8.8 Hz), 7.56 (2H, d, J = 8.8 Hz).

IR (KBr, cm⁻¹): 2958, 1701, 1577, 1519, 1498, 1431, 1252, 1221, 1176, 1120, 1029, 835.

ESI-MS: m/z = 525 (M+H⁺).

### Reference Example 52

### 1-Benzyloxycarbonyl-4-(5-(4-methoxyphenyl)-4-(4-tolyl)oxazol-2-yl)piperidine

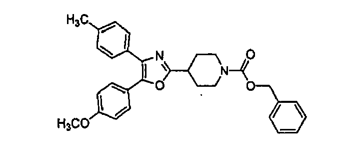

A title compound (1.04 g, 2.15 mmol, 96%) was obtained as a white amorphous product from (1-(4-methoxyphenyl)-2-(4-tolyl)-2-oxoethyl) 1-benzyloxycarbonyl-4-piperidinecarboxylate (Reference Example 32) (1.12 g, 2.23 mmol) in the same manner as in

### Reference Example 49.

¹H-NMR (400 MHz, CDCl₃) δ: 1.90 (2H, m), 2.11 (2H, m), 2.36 (3H, s), 2.96-3.10 (3H, m), 3.83 (3H, s), 4.21 (2H, m), 5.15 (2H, s), 6.88 (2H, d, J = 8.8 Hz), 7.16 (2H, d, J = 8.3 Hz), 7.29-7.37 (5H, m), 7.49 (2H, d, J = 8.8 Hz), 7.51 (2H, d, J = 8.3 Hz).

### Reference Example 53

### 1-Benzyloxycarbonyl-4-(4-(4-isopropyloxyphenyl)-5-(4-methoxyphenyl)oxazol-2-yl)piperidine

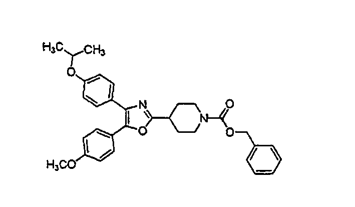

A title compound (447 mg, 0.85 mol, 90%) was obtained as a light yellow oil product from (2-(4-isopropyloxyphenyl)-1-(4-methoxyphenyl)-2-oxoethyl) 1-benzyloxycarbonyl-4-piperidinecarboxylate (Reference Example 33) (512 mg, 0.94 mmol) in the same manner as in Reference Example 49.

¹H-NMR (400 MHz, CDCl₃) δ: 1.35 (6H, d, J = 6.0 Hz), 1.87-1.95 (2H, m), 2.10-2.14 (2H, m), 3.02-3.08 (3H, m), 3.83 (3H, s), 4.17-4.26 (2H, m), 4.57 (1H, sept, J = 6.0 Hz), 5.15 (2H, s), 6.87 (2H, d, J = 8.8 Hz), 6.88 (2H, d, J = 8.8 Hz), 7.31-7.38 (5H, m), 7.49 (2H, d, J = 8.8 Hz), 7.52 (2H, d, J = 8.8 Hz).

IR (KBr, cm⁻¹): 2975, 1700, 1612, 1518, 1499, 1431, 1248, 1179, 1120, 1029, 953, 835.

ESI-MS: m/z = 527 (M+H⁺).

### Reference Example 54

### 1-Benzyloxycarbonyl-4-(5-(4-methoxyphenyl)-4-(4-trifluoromethylphenyl)oxazol-2-yl)piperidine

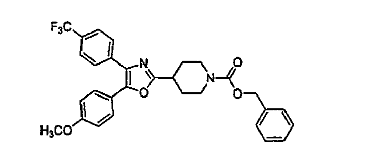

A title compound (325mg, 0.61 mol, 63%) was obtained as a white amorphous product from (1-(4-methoxyphenyl)-2-(4-trifluoromethylphenyl)-2-oxoethyl) 1-benzyloxycarbonyl-4-piperidinecarboxylate (Reference Example 34) (532 mg, 0.96 mmol) in the same manner as in Reference Example 49.

¹H-NMR (400 MHz, CDCl₃) δ: 1.87-1.95 (2H, m), 2.08-2.16 (2H, m), 3.05-3.10 (3H, m), 3.85 (3H, s), 4.18-4.27 (2H, m), 5.15 (2H, s), 6.92 (2H, d, J = 8.8 Hz), 7.31-7.38 (5H, m), 7.47 (2H, d, J = 8.8 Hz), 7.59 (2H, d, J = 8.8 Hz), 7.76 (2H, d, J = 8.8 Hz).

IR (KBr, cm⁻¹): 2933, 1702, 1620, 1502, 1326, 1255, 1121, 1070, 1028, 849, 833.

ESI-MS: m/z = 537 (M+H⁺).

### Reference Example 55

### 1-Benzyloxycarbonyl-4-(4-(4-methoxyphenyl)-5-(4-tolyl)oxazol-2-yl)piperidine

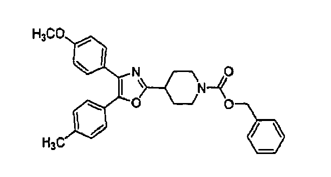

A title compound (519 mg, 1.07 mmol, 89%) was obtained as a colorless amorphous product from (2-(4-methoxyphenyl)-1-(4-tolyl)-2-oxoethyl) 1-benzyloxycarbonyl-4-piperidinecarboxylate (Reference Example 35) (604 mg, 1.20 mmol) in the same manner as in

### Reference Example 49.

¹H-NMR (400 MHz, CDCl₃) δ: 1.91 (2H, m), 2.12 (2H, m), 2.36 (3H, s), 2.98-3.12 (3H, m), 3.83 (3H, s), 4.21 (2H, m), 5.15 (2H, s), 6.89 (2H, d, J = 8.8 Hz), 7.16 (2H, d, J = 8.3 Hz), 7.31-7.38 (5H, m), 7.44 (2H, d, J = 8.3 Hz), 7.55 (2H, d, J = 8.8 Hz).

### Reference Example 56

### 1-Benzyloxycarbonyl-4-(5-(4-chlorophenyl)-4-(4-methoxyphenyl)oxazol-2-yl)piperidine

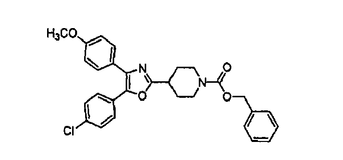

A title compound (616 mg, 1.22 mmol, 93%) was obtained as a colorless amorphous product from (1-(4-chlorophenyl)-2-(4-methoxyphenyl)-2-oxoethyl) 1-benzyloxycarbonyl-4-piperidinecarboxylate (Reference Example 36) (685 mg, 1.31 mmol) in the same manner as in Reference Example 49.

¹H-NMR (400 MHz, CDCl₃) δ: 1.90 (2H, m), 2.11 (2H, m), 2.97-3.12 (3H, m), 3.84 (3H, s), 4.21 (2H, m), 5.15 (2H, s), 6.91 (2H, d, J = 8.8 Hz), 7.30-7.38 (7H, m), 7.49 (2H, d, J = 8.3 Hz), 7.52 (2H, d, J = 8.8 Hz).

### Reference Example 57

### 1-tert-Butoxycarbonyl-4-(5-(4-fluorophenyl)-4-(4-methoxyphenyl)oxazol-2-yl)piperidine

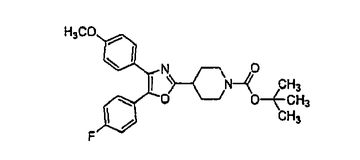

A title compound (304 mg, 0.654 mmol, 83%) was obtained as a white amorphous product from (1-(4-fluorophenyl)-2-(4-methoxyphenyl)-2-oxoethyl) 1-tert-butoxycarbonyl-4-piperidinecarboxylate (Reference Example 37) (370 mg, 0.784 mmol) in the same manner as in Reference Example 49.

¹H-NMR (400 MHz, CDCl₃) δ: 1.47 (9H, s), 1.87 (2H, m), 2.09 (2H, m), 2.95 (2H, m), 3.02 (1H, m), 3.84 (3H, s), 4.15 (2H, m), 6.90 (2H, d, J = 8.8 Hz), 7.04 (2H, t, J = 8.5 Hz), 7.50-7.55 (4H, m).

### Reference Example 58

### 1-Benzyloxycarbonyl-4-(5-phenyl-4-(4-trifluoromethylphenyl)oxazol-2-yl)piperidine

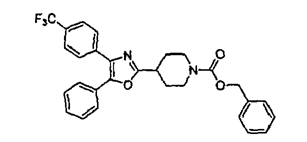

A title compound (161 mg, 0.318 mmol, 69%) was obtained as a colorless amorphous product from (1-phenyl-2-(4-trifluoromethylphenyl)-2-oxoethyl) 1-benzyloxycarbonyl-4-piperidinecarboxylate (Reference Example 38) (241 mg, 0.458 mmol) in the same manner as in Reference Example 49.

¹H-NMR (400 MHz, CDCl₃) δ: 1.92 (2H, m), 2.14 (2H, m), 3.02-3.13 (3H, m), 4.22 (2H, m), 5.16 (2H, s), 7.30-7.42 (8H, m), 7.55 (2H, d, J = 8.1 Hz), 7.60 (2H, d, J = 8.3 Hz), 7.76 (2H, d, J = 8.1Hz).

### Reference Example 59

### Methyl 3-(1,2-bis(4-methoxyphenyl)-2-oxoethyloxy)-3-oxopropionate

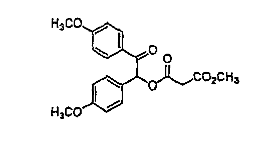

To a solution of p-anisoin (1.00 g, 3.67 mmol) in dichloromethane (20 ml) was added methyl malonyl chloride (0.5 ml, 4.65 mmol) and triethylamine (0.95 ml, 7.34 mmol). The reaction solution was stirred at room temperature for 3 hours, and 1M hydrochloric acid was then added, followed by extraction with dichloromethane. The organic layer was dried over sodium sulfate and concentrated under a reduced pressure. The residue was purified by flash chromatography (silica gel, n-hexane/ethyl acetate) to give a title compound (1.39 g, 3.73 mmol, quant.) as a colorless oil product.

¹H-NMR (400 MHz, CDCl₃) δ: 3.54 (2H, s), 3.74 (3H, s), 3.78 (3H, s), 3.82 (3H, s), 6.85 (1H, s), 6.86 (2H, d, J = 9.0 Hz), 6.88 (2H, d, J = 8.8Hz), 7.37 (2H, d, J = 8.8 Hz), 7.90 (2H, d, J = 9.0 Hz).

### Reference Example 60

### Methyl 4,5-bis(4-methoxyphenyl)oxazole-2-acetate

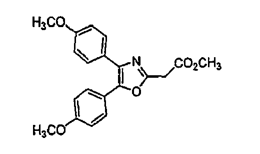

A title compound (540 mg, 1.52 mmol, 41%) was obtained as a brown oil product from methyl 1,2-bis(4-methoxyphenyl)-2-oxoethyloxy-3-oxopropionate (Reference Example 59) (1.39 g, 3.73 mmol) in the same manner as in Reference Example 49.

¹H-NMR (400 MHz, CDCl₃) δ: 3.78 (3H, s), 3.83 (6H, s), 3.93 (2H, s), 6.89 (2H, d, J = 8.8 Hz), 6.89 (2H, d, J = 8.8 Hz), 7.51 (2H, d, J = 8.8 Hz), 7.56 (2H, d, J = 8.8 Hz).

### Reference Example 61

### N-benzyloxycarbonyl-N,N-diethanolamine

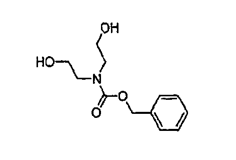

To a solution of N,N-diethanolamine (2.73 ml, 28.5 mmol) in 5% aqueous sodium bicarbonate (100 ml) was added benzyl chloroformate (4.0 ml, 28.1 mmol), and the resultant was stirred at room temperature for 24 hours. Ethyl acetate was added to the reaction solution, followed by extraction. The organic layer was dried over magnesium sulfate and then concentrated under a reduced pressure. The residue was purified via column chromatography (n-hexane/ethyl acetate = 1/4) to give a title compound (3.88 g, 16.2 mmol, 57%) as a colorless liquid.

¹H-NMR (400 MHz, CDCl₃) δ: 3.21 (2H, brs), 3.51 (4H, m), 3.78 (2H, m), 3.86 (2H, m), 5.15 (2H, s), 7.30-7.39 (5H, m).

ESI-MS: m/z = 240 (M+H⁺).

### Reference Example 62

### N-Benzyloxycarbonyl-N,N-bis(2-bromoethyl)amine

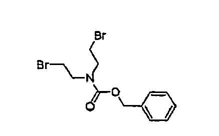

To a solution of N-benzyloxycarbonyl-N,N-diethanolamine (Reference Example 61) (3.85 g, 16.1 mmol) in dichloromethane (100 ml) was added triphenylphosphine (8.73 g, 33.3 mmol) and carbon tetrabromide (11.0 mmol, 33.1 mmol), and the resultant was stirred at room temperature for 5 hours. The reaction solution was concentrated under a reduced pressure, n-hexane was added to the residue, and insoluble matters were filtered. The filtrate was concentrated under a reduced pressure, and the residue was purified by column chromatography (silica gel, n-hexane/ethyl acetate = 4/1) to give a title compound (2.15 g, 5.89 mmol, 36%) as a colorless liquid.

¹H-NMR (400 MHz, CDCl₃) δ: 3.43 (2H, t, J = 7.1 Hz), 3.54 (2H, t, J = 7.1 Hz), 3.71-3.75 (4H, m), 5.16 (2H, s), 7.31-7.40 (5H, m).

ESI-MS: m/z = 366 (M+H⁺).

### Reference Example 63

### Methyl 1-benzyloxycarbonyl-4-(4,5-bis(4-methoxyphenyl)oxazol-2-yl)-4-piperidinecarboxylate

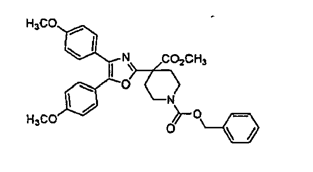

To a solution of methyl 4,5-bis(4-methoxyphenyl)oxazole-2-acetate (Reference Example 60) (340 mg, 0.960 mmol) and N-benzyloxycarbonyl-N,N-bis(2-bromoethyl)amine (Reference Example 62) (467 mg, 1.28 mmol) in N,N-dimethylformamide (2.0 ml) was added 55% sodium hydride (104 mg, 2.38 mmol) at 50°C. The reaction solution was stirred at the same temperature for 9 hours and then cooled to room temperature. 1M hydrochloric acid was added to the reaction solution, followed by extraction with ethyl acetate. The organic layer was washed with water, dried over magnesium sulfate, and then concentrated under a reduced pressure. The residue was purified by flash chromatography (silica gel, n-hexane/ethyl acetate) to give a title compound (104 mg, 0.186 mmol, 19%) as a yellow oil product.

¹H-NMR (400 MHz, CDCl₃) δ: 2.30 (2H, m), 2.44 (2H, m), 3.46 (2H, m), 3.75 (3H, m), 3.78 (2H, m), 3.83 (3H, s), 3.83 (3H, s), 5.13 (2H, s), 6.89 (2H, d, J = 8.8 Hz), 6.89 (2H, d, J = 8.8 Hz), 7.30-7.38 (5H, m), 7.48 (2H, d, J = 8.8 Hz), 7.56 (2H, d, J = 8.8 Hz).

ESI-MS: m/z = 557 (M+H⁺).

### Reference Example 64

### 1-Acetyl-4-(4-(4-chlorophenyl)-5-(4-methoxyphenyl)oxazol-2-yl)piperidine

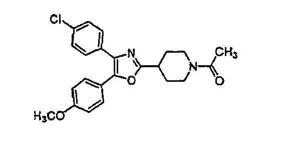

A title compound (710 mg, 1.73 mmol, 77%) was obtained as a white amorphous product from (2-(4-chlorophenyl)-1-(4-methoxyphenyl)-2-oxoethyl) 1-acetyl-4-piperidinecarboxylate (Reference Example 39) (970 mg, 2.25 mmol) in the same manner as in

### Reference Example 49.

¹H-NMR (400 MHz, CDCl₃) δ: 1.81-1.99 (2H, m), 2.13 (3H, s), 2.15 (2H, m), 2.92 (1H, m), 3.12 (1H, m), 3.26 (1H, m), 3.84 (3H, s), 3.90 (1H, m), 4.54 (1H, m), 6.90 (2H, d, J = 8.8 Hz), 7.32 (2H, d, J = 8.5 Hz), 7.46 (2H, d, J = 8.5 Hz), 7.57 (2H, d, J = 8.8 Hz).

### Reference Example 65

### 1-Acetyl-4-(4-(4-chlorophenyl)-5-(4-tolyl)oxazol-2-yl)piperidine

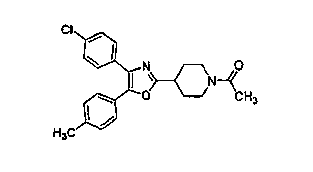

A title compound (174 mg, 0.441 mmol, 46%) was obtained as a light yellow solid from (2-(4-chlorophenyl)-1-(4-tolyl)-2-oxoethyl) 1-acetyl-4-piperidinecarboxylate (Reference Example 40) (394 mg, 0.951 mmol) in the same manner as in Reference Example 49.

¹H-NMR (400 MHz, CDCl₃) δ: 1.92 (2H, m), 2.10-2.21 (5H, m), 2.38 (3H, s), 2.92 (1H, m), 3.13 (1H, m), 3.26 (1H, m), 3.91 (1H, m), 4.54 (1H, m), 7.18 (2H, d, J = 8.3 Hz), 7.32 (2H, d, J = 8.5 Hz), 7.43 (2H, d, J = 8.3 Hz), 7.57 (2H, d, J= 8.5 Hz).

### Reference Example 66

### 1-tert-Butoxycarbonyl-4-(4-(4-fluorophenyl)-5-phenyloxazol-2-yl)piperidine

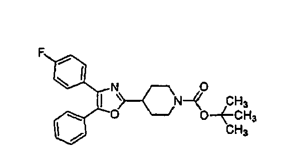

A title compound (179 mg, 0.423 mmol, 77%) was obtained as a white solid from (2-(4-fluorophenyl)-1-phenyl-2-oxoethyl) 1-tert-butoxycarbonyl-4-piperidinecarboxylate (Reference Example 41) (244 mg, 0.552 mol) in the same manner as in Reference Example 49.

¹H-NMR (400 MHz, CDCl₃) δ: 1.48 (9H, s), 1.88 (2H, m), 2.10 (2H, m), 2.96 (2H, m), 3.04 (1H, m), 4.13 (2H, m), 7.05 (2H, m), 7.32-7.39 (3H, m), 7.54 (2H, m), 7.60 (2H, m).

### Reference Example 67

### 1-tert-Butoxycarbonylpiperidinecarbaldehyde

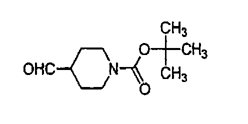

To a solution of triethylamine (2.3 ml, 16 mmol) and 1-tert-butoxycarbonyl-4-hydroxymethylpiperidine (500 mg, 2.3 mmol) in dimethyl sulfoxide (15 ml) was added a sulfur trioxide pyridine complex (1.11 g, 7.0 mmol), and the resultant was stirred at room temperature for 4 hours. An aqueous solution of saturated ammonium chloride was added to the reaction solution, followed by extraction with ethyl acetate. The organic layer was dried over magnesium sulfate and then concentrated under a reduced pressure. The residue was purified by flash chromatography (silica gel, n-hexane/ethyl acetate = 10/1 to 2/1) to give a title compound (513 mg, 2.3 mmol, quant.) as a colorless oil product.

¹H-NMR (400 MHz, CDCl₃) δ: 1.46 (9H, s), 1.51-1.62 (2H, m), 1.88-1.91 (2H, m), 2.38-2.45 (1H, m), 2.88-2.98 (2H, m), 3.94-4.02 (2H, m), 9.67 (1H, s).

EI-MS: m/z = 214 (M+H⁺).

### Reference Example 68

### 4,4'-Dimethoxybenzil

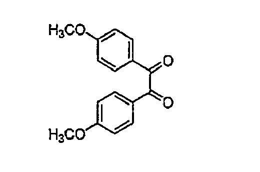

To a solution of copper sulfate (2.34 g, 14.7 mmol) in pyridine (10 ml) was added distilled water (2 ml). The reaction solution was heated under reflux for 10 minutes and then cooled to room temperature. p-Anisoin (2.0 g, 7.3 mmol) was added to the reaction solution, and the mixture was heated under reflux for 3 hours and then cooled to room temperature. Distilled water was added to the reaction solution, and the precipitated crystal was filtered. The obtained crystal was washed with distilled water and methanol and then dried under a reduced pressure to give a title compound (1.8 g, 6.5 mmol, 89%) as a yellow crystal.

¹H-NMR (400 MHz, CDCl₃) δ: 3.89 (6H, s), 6.98 (4H, d, J = 8.8 Hz), 7.95 (4H, d, J = 8.8 Hz).

IR (KBr, cm⁻¹): 1656, 1600, 1574, 1510, 1425, 1312, 1263, 1227, 1163, 1018, 879, 833.

EI-MS: m/z = 271 (M+H⁺).

### Reference Example 69

### 4-(4,5-Bis(4-methoxyphenyl)-1H-imidazol-2-yl)piperidine

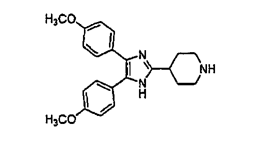

To a solution of 4,4'-dimethoxybenzil (Reference Example 68) (975 mg, 3.6 mmol) and 1-tert-butoxycarbonylpiperidinecarbaldehyde (Reference Example 67) (769 mg, 3.6 mmol) in acetic acid (40 ml), ammonium acetate (5.6 g 72 mmol) was added. The reaction solution was heated at 120°C for 4 hours with stirring and then cooled to room temperature. The reaction solution was concentrated under a reduced pressure, and saturated aqueous sodium bicarbonate was added to the residue, followed by extraction with chloroform. The organic layer was washed with distilled water, dried over magnesium sulfate, and concentrated under a reduced pressure. The residue was purified by flash chromatography (amine silica gel, chloroform/methanol = 50/1 to 10/1) to give a title compound (910 mg, 2.5 mmol, 69%) as a light yellow solid.

¹H-NMR (400 MHz, CDCl₃) δ: 1.67-1.78 (2H, m), 1.91 (1H, brs), 2.02-2.04 (2H, m), 2.69-2.74 (2H, m), 2.87-2.95 (1H, m), 3.14-3.17 (2H, m), 3.81 (6H, s), 6.85-6.87 (4H, m), 7.31-7.59 (4H, m), 9.29 (1H, brs).

IR (KBr, cm⁻¹): 2938, 2834, 1615, 1539, 1516, 1493, 1444, 1364, 1295, 1248, 1174, 1107, 1034, 970, 834.

EI-MS: m/z = 364 (M+H⁺).

### Reference Example 70

### 1-tert-Butoxycarbonyl-4-(4,5-bis(4-methoxyphenyl)-1H-imidazol-2-yl)piperidine

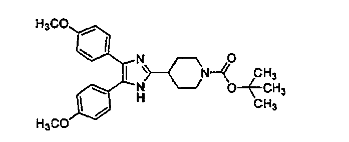

To a solution of 4-(4,5-bis(4-methoxyphenyl)-1H-imidazol-2-yl)piperidine (Reference Example 69) (858 mg, 2.4 mmol) in chloroform (15 ml) was added an aqueous solution of 1M sodium hydroxide (2.5 ml) and di-tert-butyl dicarbonate (541 mg, 2.5 mmol). After the reaction solution was stirred at room temperature for 30 minutes, saturated sodium bicarbonate water was added, followed by extraction with chloroform. The organic layer was dried over sodium sulfate and then concentrated under a reduced pressure. The residue was purified by flash chromatography (silica gel, n-hexane/ethyl acetate = 1/1 to 1/2) to give a title compound (1.09 g, 2.4 mmol, quant) as a white crystal.

¹H-NMR (400 MHz, CDCl₃) δ: 1.45 (9H, s), 1.60-1.73 (2H, m), 1.86-1.89 (2H, m), 2.63-2.82 (3H, m), 3.86 (6H, s), 4.19-4.20 (2H, m), 6.82 (2H, d, J = 8.4 Hz), 6.85 (2H, d, J = 8.4 Hz), 7.33 (2H, d, J = 8.4 Hz), 7.49 (2H, d, J = 8.4 Hz), 10.27 (1H, s).

IR (KBr, cm⁻¹): 2971, 1691, 1516, 1426, 1365, 1248, 1174, 1035, 834.

ESI-MS: m/z = 464 (M+H⁺).

### Reference Example 71

### 1-tert-Butoxycarbonyl-4-(4,5-bis(4-methoxyphenyl)-1-methoxycarbonylmethylimidazol-2-yl)piperidine

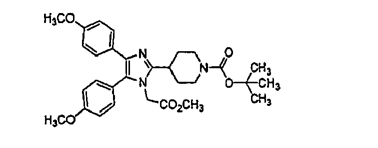

To a solution of 1-tert-butoxycarbonyl-4-(4,5-bis(4-methoxyphenyl)-1H-imidazol-2-yl)piperidine (Reference Example 70) (742 mg, 1.6 mmol) and methyl bromoacetate (490 mg, 3.2 mmol) in N,N-dimethylformamide (30 ml) was added potassium carbonate (442 mg, 3.2 mmol). The reaction solution was stirred at room temperature for 22 hours, and distilled water was added, followed by extraction with ethyl acetate. The organic layer was dried over sodium sulfate and then concentrated under a reduced pressure. The residue was purified by column chromatography (silica gel, n-hexane/ethyl acetate = 5/1 to 1/1) to give a title compound (722 mg, 1.3 mmol, 84%) as a colorless amorphous product.

¹H-NMR (400 MHz, CDCl₃) δ: 1.48 (9H, s), 1.84-1.88 (2H, m), 2.00-2.08 (2H, m), 2.63-2.69 (1H, m), 2.85 (2H, brs), 3.72 (3H, s), 3.75 (3H, s), 3.86 (3H, s), 4.24 (2H, brs), 4.45 (2H, s), 6.74 (2H, d, J = 8.8 Hz), 6.95 (2H, d, J = 8.8 Hz), 7.19 (2H, d, J = 8.8 Hz), 7.39 (2H, d, J = 8.8 Hz).

IR (KBr, cm⁻¹): 3448, 2953, 1749, 1686, 1616, 1570, 1521, 1492, 1438, 1364, 1337, 1291, 1247, 1177, 1123, 1069, 1031, 979, 840, 769.
ESI-MS: m/z = 537 (M+H⁺).

### Reference Example 72

### 1-Benzyloxycarbonyl-4-(4,5-bis(4-methoxyphenyl)-1H-pyrrol-2-yl)piperidine

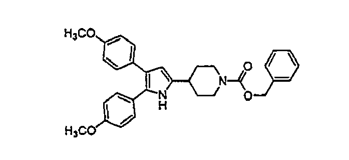

To a solution of 1-benzyloxycarbonyl-4-(3,4-bis(4-methoxyphenyl)-1,4-dioxobutyl)piperidine (Reference Example 47) (790 mg, 1.53 mmol) in acetic acid (10 ml) was added ammonium acetate (1.18 g, 15.3 mmol). The reaction solution was stirred at 90°C for 4 hours and then concentrated under a reduced pressure, and the residue was dissolved in ethyl acetate. The resulting solution was washed with saturated aqueous sodium bicarbonate, dried over sodium sulfate, and concentrated under a reduced pressure. The residue was purified by column chromatography (silica gel, n-hexane/ethyl acetate = 5/1 to 3/1) to give a title compound (456 mg, 0.92 mmol, 60%) as a brown amorphous product.

¹H-NMR (400 MHz, CDCl₃) δ: 1.62-1.67 (2H, m), 2.00-2.02 (2H, m), 2.78-2.92 (3H, m), 3.79 (3H, s), 3.80 (3H, s), 4.42-4.30 (2H, m), 5.14 (2H, s), 6.06 (1H, d, J = 2.4 Hz), 6.81 (2H, d, J = 8.8 Hz), 6.83 (2H, d, J = 8.4 Hz), 7.23-7.25 (4H, m), 7.29-7.37 (5H, m), 7.94 (1H, brs).

IR (KBr, cm⁻¹): 3337, 1696, 1519, 1439, 1225, 1221, 1176, 1030, 834.

ESI-MS: m/z = 497 (M+H⁺).

### Reference Example 73

### 1-Benzyloxycarbonyl-4-(4,5-bis(4-methoxyphenyl)-1-tert-butoxycarbonylmethylpyrrol-2-yl)piperidine

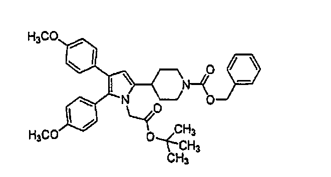

To a solution of 1-benzyloxycarbonyl-4-(4,5-bis(4-methoxyphenyl)-1H-pyrrol-2-yl)piperidine (Reference Example 72) (150 mg, 0.30 mmol) in N,N-dimethylformamide (2 ml) was added 55% sodium hydride (14.4 mg, 0.33 mmol) at 0°C, and then added tert-butyl bromoacetate (64 mg, 0.33 mmol). The reaction solution was stirred at room temperature for 4 hours, and an aqueous solution of saturated ammonium chloride was then added, followed by extraction with ethyl acetate. The organic layer was dried over sodium sulfate and then concentrated under a reduced pressure. The residue was purified by column chromatography (silica gel, n-hexane/ethyl acetate = 5/1) to give a title compound (130 mg, 0.21 mmol, 71%) as a brown oil product.

¹H-NMR (400 MHz, CDCl₃) δ: 1.41 (9H, s), 1.65-1.73 (2H, m), 1.93-1.96 (2H, m), 2.50-2.56 (1H, m), 2.82-2.91 (2H, m), 3.74 (3H, s), 3.83 (3H, s), 4.28-4.36 (2H, m), 4.34 (2H, s), 5.16 (2H, s), 6.16 (1H, s), 6.70 (2H, d, J = 8.8 Hz), 6.88 (2H, d, J = 8.8 Hz), 7.07 (2H, d, J = 8.8 Hz), 7.18 (2H, d, J = 8.8 Hz), 7.31-7.38 (5H, m).

IR (KBr, cm⁻¹): 2938, 1744, 1699, 1518, 1246, 1228, 1153, 1033, 836.

ESI-MS: m/z = 611 (M+H⁺).

### Reference Example 74

### 1-Benzyloxycarbonyl-4-(4,5-bis(4-methoxyphenyl)-1-hexylpyrrol-2-yl)piperidine

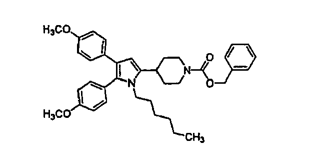

A title compound (124 mg, 0.21 mmol, 49%) was obtained as a light yellow oil product from 1-benzyloxycarbonyl-4-(4,5-bis(4-methoxyphenyl)-1H-pyrrol-2-yl)piperidine (Reference Example 72) (215 mg, 0.43 mmol) and 1-iodohexane (137 mg, 0.65 mmol) in the same manner as in Reference Example 73.

¹H-NMR (400 MHz, CDCl₃) δ: 0.80 (3H, t, J = 6.8 Hz), 1.10-1.19 (6H, m), 1.42-1.46 (2H, m), 1.68-1.74 (2H, m), 1.95-1.98 (2H, m), 2.67-2.73 (1H, m), 2.88-2.94 (2H, m), 3.73 (3H, s), 3.84 (3H, s), 4.32-4.36 (2H, m), 5.17 (2H, s), 6.11 (1H, s), 6.69 (2H, d, J = 8.8 Hz), 6.91 (2H, d, J = 8.8 Hz), 7.04 (2H, d, J = 8.8 Hz), 7.20 (2H, d, J = 8.8 Hz), 7.32-7.39 (5H, m).

IR (KBr, cm⁻¹): 2931, 2670, 1517, 1466, 1441, 1246, 1225, 1178, 1034, 835.

ESI-MS: m/z = 581 (M+H⁺).

### Reference Example 75

### 1-Benzyloxycarbonyl-4-(4,5-bis(4-methoxyphenyl)thiophen-2-yl)piperidine

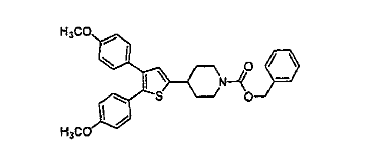

To a solution of 1-benzyloxycarbonyl-4-(3,4-bis(4-methoxyphenyl)-1,4-dioxobutyl)piperidine (Reference Example 47) (150 mg, 0.29 mmol) in toluene (5 ml) was added a Lawesson's reagent (59 mg, 0.145 mmol). The reaction solution was heated under reflux for 24 hours and then concentrated under a reduced pressure. The residue was purified by column chromatography (silica gel, n-hexane/ethyl acetate = 5/1) to give a title compound (105 mg, 0.20 mmol, 70%) as a light yellow oil product.

¹H-NMR (400 MHz, CDCl₃) δ: 1.67-1.70 (2H, m), 2.05-2.08 (2H, m), 2.84-2.90 (1H, m), 2.96-2.98 (2H, m), 3.80 (3H, s), 3.83 (3H, s), 4.20-4.28 (2H, m), 5.15 (2H, s), 6.11 (1H, s), 6.82 (2H, d, J = 8.8 Hz), 6.88 (2H, d, J = 8.8 Hz), 7.30 (2H, d, J = 8.8 Hz), 7.32-7.38 (5H, m), 7.42 (2H, d, J = 8.8 Hz).

ESI-MS: m/z = 514 (M+H⁺).

### Reference Example 76

### 1-Benzyloxycarbonyl-4-(1,5-bis(4-methoxyphenyl)pyrazol-3-yl)piperidine

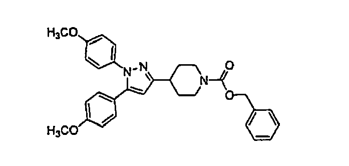

To a solution of 1-benzyloxycarbonyl-4-(4-methoxyphenyl)-1,3-dioxopropylpiperidine (Reference Example 48) (527 mg, 1.33 mmol) in ethanol (10 ml) was added 4-methoxyphenylhydrazine (232 mg, 1.33 mmol) and triethylamine (161 mg, 1.59 mmol). The reaction solution was heated under reflux for 20 hours and then cooled to room temperature. Water was added to the reaction solution, followed by extraction with ethyl acetate. The organic layer was dried over sodium sulfate and then concentrated under a reduced pressure. The residue was purified by column chromatography (silica gel, n-hexane/ethyl acetate = 2/1) to give a title compound (394 mg, 0.79 mmol, 60%) as a brown amorphous product.

¹H-NMR (400 MHz, CDCl₃) δ: 1.68-1.77 (2H, m), 2.02-2.05 (2H, m), 2.92 (1H, tt, J = 11.6, 3.6 Hz), 2.94-2.98 (2H, m), 3.79 (3H, s), 3.80 (3H, s), 4.28 (2H, m), 5.15 (2H, s), 6.23 (1H, s), 6.81 (2H, d, J = 8.4 Hz), 6.84 (2H, d, J = 8.4 Hz), 7.13 (2H, d, J = 8.4 Hz), 7.19 (2H, d, J = 8.4 Hz), 7.30-7.38 (5H, m).

IR (KBr, cm⁻¹): 3421, 2934, 1695, 1517, 1437, 1251, 1220, 1031, 835.

ESI-MS: m/z = 498 (M+H⁺).

### Reference Example 77

### 1-Benzyloxycarbonyl-4-(5-(4-methoxyphenyl)-1-phenylpyrazol-3-yl)piperidine

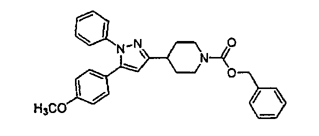

A title compound (203 mg, 0.43 mmol, 88%) was obtained as a light yellow oil product from 1-benzyloxycarbonyl-4-(4-methoxyphenyl)-1,3-dioxopropylpiperidine (Reference Example 48) (195 mg, 0.493 mmol) in the same manner as in Reference Example 76.

¹H-NMR (400 MHz, CDCl₃) δ: 1.72-1.75 (2H, m), 2.03-2.07 (2H, m), 2.93 (1H, tt, J = 11.6, 3.6 Hz), 2.93-2.97 (2H, m), 3.79 (3H, s), 4.28 (2H, m), 5.15 (2H, s), 6.25 (1H, s), 6.82 (2H, d, J = 9.2 Hz), 7.13 (2H, d, J = 9.2 Hz), 7.27-7.38 (10H, m).

ESI-MS: m/z = 468 (M+H⁺).

### Reference Example 78

### 4-(4,5-Bis(4-methoxyphenyl)oxazol-2-yl)piperidine

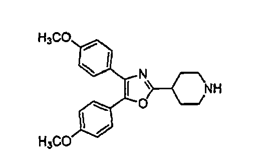

To a solution of 1-benzyloxycarbonyl-4-(4,5-bis(4-methoxyphenyl)oxazol-2-yl)piperidine (Reference Example 49) (21.0 g, 41.9 mmol) in ethanol (200 ml) was added 10% Pd-C (50% wet, 2.01 g), and the mixture was stirred under a hydrogen atmosphere at room temperature for 3 hours. A catalyst was removed from the reaction solution by filtration, and the filtrate was concentrated to give a title compound (15.2 g, 41.7 mmol, 99%) as a light yellow oil product.

¹H-NMR (400 MHz, CDCl₃) δ: 1.86 (2H, dq, J = 3.9, 13.2 Hz), 2.10 (2H, dd, J = 2.7, 13.2 Hz), 2.76 (2H, dt, J = 2.7, 12.4 Hz), 2.99 (1H, m), 3.20 (2H, dt, J = 3.9, 12.4 Hz), 3.83 (6H, s), 6.88 (2H, d, J = 9.0 Hz), 6.89 (2H, d, J = 9.0 Hz), 7.49 (2H, d, J = 9.0 Hz), 7.55 (2H, d, J = 9.0 Hz).

### Reference Example 79

### 3-(4,5-Bis(4-methoxyphenyl)oxazol-2-yl)piperidine

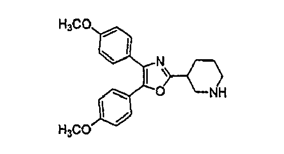

A title compound (685 mg, 1.88 mmol, quant.) was obtained as a colorless oil product from 1-benzyloxycarbonyl-3-(4,5-bis(4-methoxyphenyl)oxazol-2-yl)piperidine (Reference Example 50) (940 mg, 1.88 mmol) in the same manner as in Reference Example 78.

¹H-NMR (400 MHz, CDCl₃) δ: 1.53-1.65 (1H, m), 1.76-1.81 (1H, m), 1.85-1.95 (1H, m), 2.21-2.24 (1H, m), 2.74 (1H, ddd, J = 13.6, 10.8, 3.2 Hz), 2.97-3.05 (3H, m), 3.38-3.43 (1H, m), 3.83 (6H, s), 6.88 (2H, d, J = 8.8 Hz), 6.89 (2H, d, J = 8.8 Hz), 7.49 (2H, d, J = 8.8 Hz), 7.55 (2H, d, J = 8.8 Hz).

IR (KBr, cm⁻¹): 3263, 2934, 2839, 1605, 1571, 1508, 1461, 1297, 1252, 1178, 1029, 834.

### Reference Example 80

### 4-(4-(4-tert-Butylphenyl)-5-(4-methoxyphenyl)oxazol-2-yl)piperidine

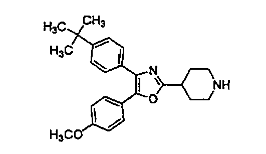

A title compound (348 mg, 0.89 mmol, quant.) was obtained as a white amorphous product from 1-benzyloxycarbonyl-4-(4-(4-tert-butylphenyl)-5-(4-methoxyphenyl)oxazol-2-yl)piperidine (Reference Example 51) (468 mg, 0.89 mmol) in the same manner as in Reference Example 78.

¹H-NMR (400 MHz, CDCl₃) δ: 1.33 (9H, s), 1.81-1.91 (2H, m), 2.09-2.12 (2H, m), 2.76 (2H, dt, J = 12.0, 2.4 Hz), 3.00 (1H, tt, J = 11.2, 3.6 Hz), 3.20 (2H, dt, J = 12.4, 3.6 Hz), 3.84 (3H, s), 6.90 (2H, d, J = 8.8 Hz), 7.36 (2H, d, J = 8.8 Hz), 7.53 (2H, d, J = 8.8 Hz), 7.56 (2H, d, J = 8.8 Hz).

IR (KBr, cm⁻¹): 3398, 2960, 1576, 1520, 1499, 1463, 1296, 1252, 1177, 1032, 834.

ESI-MS: m/z = 391 (M+H⁺).

### Reference Example 81

### 4-(5-(4-Methoxyphenyl)-4-(4-tolyl)oxazol-2-yl)piperidine

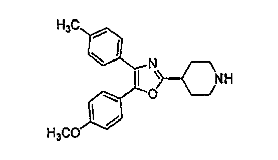

A title compound (737 mg, 2.11 mmol, 99%) was obtained as a white amorphous product from 1-benzyloxycarbonyl-4-(5-(4-methoxyphenyl)-4-(4-tolyl)oxazol-2-yl)piperidine (Reference Example 52) (1.03 g, 2.13 mmol) in the same manner as in Reference Example 78.

¹H-NMR (400 MHz, CDCl₃) δ: 1.87 (2H, m), 2.11 (2H, m), 2.36 (3H, s), 2.76 (2H, m), 3.00 (1H, m), 3.19 (2H, m), 3.83 (3H, m), 6.88 (2H, d, J = 8.3 Hz), 7.16 (2H, d, J = 8.3 Hz), 7.49-7.53 (4H, m).

### Reference Example 82

### 4-(4-(4-Isopropyloxyphenyl)-5-(4-methoxyphenyl)oxazol-2-yl)piperidine

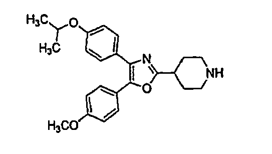

A title compound (322 mg, 0.82 mmol, quant.) was obtained as a colorless oil product from 1-benzyloxycarbonyl-4-(4-(4-isopropyloxyphenyl)-5-(4-methoxyphenyl)oxazol-2-yl)piperidine (Reference Example 53) (431 mg, 0.82 mmol) in the same manner as in

### Reference Example 78.

¹H-NMR (400 MHz, CDCl₃) δ: 1.35 (6H, d, J = 6.0 Hz), 1.82-1.91 (3H, m), 2.09-2.13 (2H, m), 2.77 (2H, dt, J = 12.4, 2.8 Hz), 3.00 (1H, tt, J = 11.2, 3.6 Hz), 3.21 (2H, dt, J = 12.4, 3.6 Hz), 3.83 (3H, s), 4.57 (1H, sept, J = 6.0 Hz), 6.87 (2H, d, J = 8.8 Hz), 6.88 (2H, d, J = 8.8 Hz), 7.50 (2H, d, J = 8.8 Hz), 7.53 (2H, d, J = 8.8 Hz).

IR (KBr, cm⁻¹): 3421, 2976, 1518, 1499, 1294, 1250, 1179, 1119, 1031, 953, 834.

ESI-MS: m/z = 393 (M+H⁺).

### Reference Example 83

### 4-(5-(4-Methoxyphenyl)-4-(4-trifluoromethylphenyl)oxazol-2-yl)piperidine

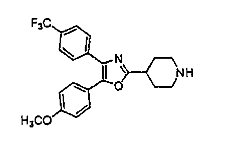

A title compound (225 mg, 0.56 mmol, quant.) was obtained as a white solid from 1-benzyloxycarbonyl-4-(5-(4-methoxyphenyl)-4-(4-trifluoromethylphenyl)oxazol-2-yl)piperidine (Reference Example 54) (302 mg, 0.56 mmol) in the same manner as in

### Reference Example 78.

¹H-NMR (400 MHz, CDCl₃) δ: 1.91-2.01 (2H, m), 2.10-2.19 (2H, brd), 2.81-2.87 (2H, brt), 3.06 (1H, tt, J = 10.8, 4.0 Hz), 3.25-3.28 (2H, brd), 3.52 (1H, brs), 3.85 (3H, s), 6.92 (2H, d, J = 8.8 Hz), 7.48 (2H, d, J = 8.4 Hz), 7.59 (2H, d, J = 8.8 Hz), 7.76 (2H, d, J = 8.4 Hz).

IR (KBr, cm⁻¹): 3405, 2945, 1620, 1568, 1523, 1502, 1327, 1252, 1164, 1120, 1069, 834.

ESI-MS: m/z = 403 (M+H⁺).

### Reference Example 84

### 4-(4-(4-Methoxyphenyl)-5-(4-tolyl)oxazol-2-yl)piperidine

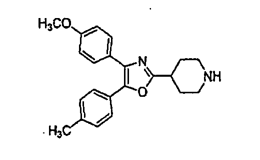

A title compound (378 mg, 1.08 mmol, 74%) was obtained as a colorless amorphous product from 1-benzyloxycarbonyl-4-(4-(4-methoxyphenyl)-5-(4-tolyl)oxazol-2-yl)piperidine (Reference Example 55) (510 mg, 1.46 mmol) in the same manner as in Reference Example 78.

¹H-NMR (400 MHz, CDCl₃) δ: 1.87 (2H, m), 2.11 (2H, m), 2.36 (3H, s), 2.76 (2H, m), 3.00 (1H, s), 3.20 (2H, m), 3.83 (3H, s), 6.89 (2H, d, J = 8.8 Hz), 7.15 (2H, d, J = 8.3 Hz), 7.45 (2H, d, J = 8.3 Hz), 7.56 (2H, d, J = 8.8 Hz).

### Reference Example 85

### 4-(4-(4-Methoxyphenyl)-5-phenyloxazol-2-yl)piperidine

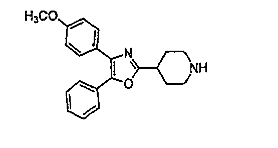

A title compound (420 mg, 1.22 mmol, quant.) was obtained as a white solid from 1-benzyloxycarbonyl-4-(5-(4-chlorophenyl)-4-(4-methoxyphenyl)oxazol-2-yl)piperidine (Reference Example 56) (616 mg, 1.22 mmol) in the same manner as in Reference Example 78.

¹H-NMR (400 MHz, CDCl₃) δ: 2.33-2.54 (4H, m), 3.17-3.31 (3H, m), 3.54 (2H, m), 3.84 (3H, s), 6.91 (2H, d, J = 8.8 Hz), 7.31-7.38 (3H, m), 7.55 (4H, d, J = 8.8 Hz), 9.73 (1H, brs).

### Reference Example 86

### 4-(5-(4-Fluorophenyl)-4-(4-methoxyphenyl)oxazol-2-yl)piperidine

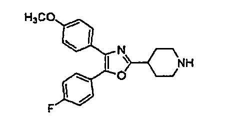

To a solution of 1-tert-butoxycarbonyl-4-(5-(4-fluorophenyl)-4-(4-methoxyphenyl)oxazol-2-yl)piperidine (Reference Example 57) (303 mg, 0.652 mmol) in dichloromethane (3.0 ml) was added trifluoroacetic acid (0.25 ml, 3.26 mmol), and the resultant was stirred at room temperature for 16 hours. The reaction solution was concentrated under a reduced pressure, and the residue was neutralized with the addition of an aqueous solution of 1M sodium hydroxide, followed by extraction with ethyl acetate. The organic layer was dried over magnesium sulfate and then concentrated under a reduced pressure. The residue was purified by column chromatography (amine silica gel, dichloromethane/methanol = 50/1) to give a title compound (209 mg, 0.593 mmol, 91 %) as a yellow amorphous product.

¹H-NMR (400 MHz, CDCl₃) δ: 1.90 (2H, m), 2.13 (2H, m), 2.78 (2H, m), 3.01 (1H, m), 3.22 (2H, m), 3.84 (3H, s), 6.91 (2H, d, J = 8.8 Hz), 7.04 (2H, t, J = 8.5 Hz), 7.52-7.56 (4H, m).

### Reference Example 87

### 4-(5-Phenyl-4-(4-trifluoromethylphenyl)oxazol-2-yl)piperidine

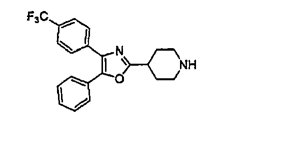

A title compound (113 mg, 0.303 mmol, 97%) was obtained as a yellow amorphous product from 1-benzyloxycarbonyl-4-(5-phenyl-4-(4-trifluoromethylphenyl)oxazol-2-yl)piperidine (Reference Example 66) (158 mg, 0.311 mmol) in the same manner as in Reference Example 78.

¹H-NMR (400 MHz, CDCl₃) δ: 1.90 (2H, m), 2.14 (2H, m), 2.78 (2H, m), 3.03 (1H, m), 3.22 (2H, m), 7.34-7.42 (3H, m), 7.56 (2H, m), 7.61 (2H, d, J = 8.3 Hz), 7.78 (2H, d, J = 8.3 Hz).

### Reference Example 88

### 4-(4-(4-Chlorophenyl)-5-(4-tolyl)oxazol-2-yl)piperidine

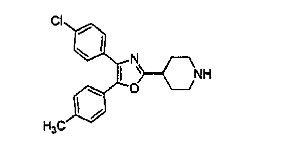

To a solution of 1-acetyl-4-(4-(4-chlorophenyl)-5-(4-tolyl)oxazol-2-yl)piperidine (Reference Example 65) (252 mg, 0.638 mmol) in ethanol (4 ml) was added 85% potassium hydroxide (214 mg, 3.24 mmol). The reaction solution was stirred at 85°C for 22 hours and then cooled to room temperature. Distilled water was added to the reaction solution, followed by extraction with ethyl acetate. The organic layer was dried over magnesium sulfate and then concentrated under a reduced pressure to give a title compound (209 mg, 0.592 mmol, 93%) as a yellow oil product.

¹H-NMR (400 MHz, CDCl₃) δ: 1.88 (2H, m), 2.11 (2H, m), 2.38 (3H, s), 2.76 (2H, m), 3.00 (1 H, m), 3.20 (2H, m), 7.18 (2H, d, J = 8.1 Hz), 7.32 (2H, d, J = 8.5 Hz), 7.43 (2H, d, J = 8.1 Hz), 7.58 (2H, d, J = 8.5 Hz).

### Reference Example 89

### 4-(4-(4-Chlorophenyl)-5-(4-methoxyphenyl)oxazol-2-yl)piperidine

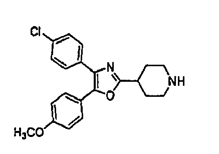

A title compound (601 mg, 1.63 mmol, 95%) was obtained as a white amorphous product from 1-acetyl-4-(4-(4-chlorophenyl)-5-(4-methoxyphenyl)oxazol-2-yl)piperidine (Reference Example 64) (706 mg, 1.72 mmol) in the same manner as in Reference Example 88.

¹H-NMR (400 MHz, CDCl₃) δ: 1.87 (2H, m), 2.12 (2H, m), 2.77 (2H, m), 3.00 (1H, m), 3.21 (2H, m), 3.84 (3H, s), 6.90 (2H, d, J = 8.8 Hz), 7.32 (2H, d, J = 8.5 Hz), 7.47 (2H, d, J = 8.5 Hz), 7.58 (2H, d, J = 8.8 Hz).

### Reference Example 90

### 4-(4-(4-Fluorophenyl)-5-phenyloxazol-2-yl)piperidine

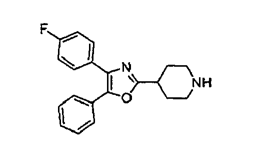

To a solution of 1-tert-butoxycarbonyl-4-(4-(4-fluorophenyl)-5-phenyloxazol-2-yl)piperidine (Reference Example 66) (176 mg, 0.416 mmol) in dichloromethane (3.0 ml) was added trifluoroacetic acid (1.0 ml). The reaction solution was stirred at room temperature for 14 hours and concentrated under a reduced pressure. An aqueous solution of 1M sodium hydroxide was added to the residue, followed by extraction with ethyl acetate. The organic layer was dried over magnesium sulfate and then concentrated under a reduced pressure. The residue was purified by column chromatography (amine silica gel, dichloromethane/methanol = 50/1) to give a title compound (126 mg, 0.391 mmol, 94%) as a colorless oil product.

¹H-NMR (400 MHz, CDCl₃) δ: 1.93 (2H, m), 2.14 (2H, m), 2.81 (2H, m), 3.03 (1H, m), 3.23 (2H, m), 7.06 (2H, m), 7.31-7.38 (3H, m), 7.53-7.63 (4H, m).

### Reference Example 91

### Methyl 4-(4,5-bis(4-methoxyphenyl)oxazol-2-yl)-4-piperidinecarboxylate

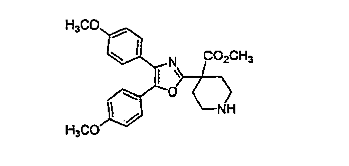

A title compound (56 mg, 0.13 mmol, 69%) was obtained as a white amorphous product from methyl 1-benzyloxycarbonyl-4-(4,5-bis(4-methoxyphenyl)oxazol-2-yl)-4-piperidinecarboxylate (Reference Example 63) (104 mg, 0.186 mmol) in accordance with the method of Reference Example 78.

¹H-NMR (400 MHz, CDCl₃) δ: 2.31 (2H, m), 2.41 (2H. m), 2.90-3.00 (4H, m), 3.75 (3H, s), 3.83 (6H, s), 6.89 (2H, d, J = 8.8 Hz), 6.90 (2H, d, J = 8.8 Hz), 7.49 (2H, d, J = 8.8 Hz), 7.58 (2H, d, J = 8.8 Hz).

ESI-MS: m/z = 423 (M+H⁺).

### Reference Example 92

### 4-(4,5-Bis(4-methoxyphenyl)oxazol-2-yl)piperazine

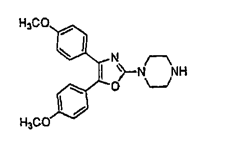

To a solution of 2-chloro-4,5-bis(4-methoxyphenyl)oxazole (Reference Example 26) (406 mg, 1.28 mmol) and piperazine (1.12 g, 13.0 mmol) in N,N-dimethylformamide (3.0 ml) was added cesium carbonate (503 mg, 1.54 mmol). The reaction solution was stirred at 80°C for 3 hours and then cooled to room temperature. Distilled water was added to the reaction solution, followed by extraction with ethyl acetate. The organic layer was washed with water, dried over magnesium sulfate, and then concentrated under a reduced pressure. The residue was purified by column chromatography (silica gel, dichloromethane/methanol = 10/1) to give a title compound (382 mg, 1.04 mmol, 81%) as a white amorphous product.

¹H-NMR (400 MHz, CDCl₃) δ: 3.99 (4H, m), 3.57 (4H, m), 3.81 (3H, s), 3.82 (3H, s), 6.84 (2H, d, J = 8.8 Hz), 6.87 (2H, d, J = 8.8 Hz), 7.41 (2H, d, J = 8.8 Hz), 7.54 (2H, d, J = 8.8 Hz).
ESI-MS: m/z = 366 (M+H⁺).

### Reference Example 93

### 4-(4,5-Bis(4-methoxyphenyl)thiazol-2-yl)piperidine

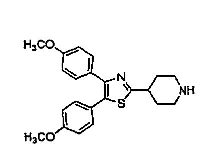

A solution of 1-tert-butoxycarbonyl-4-piperidine thioamide (350 mg, 1.4 mmol) and 2-bromo-1,2-bis(4-methoxyphenyl)ethanone (Reference Example 14) (480 mg, 1.4 mmol) in methanol (15 ml) was heated under reflux for 4 hours and then cooled to room temperature. The reaction solution was concentrated under a reduced pressure, and the residue was dissolved with the addition of 10% hydrochloric acid-methanol (15 ml), and the resultant was heated under reflux for 14 hours. The reaction solution was cooled to room temperature and then concentrated under a reduced pressure. Saturated aqueous sodium bicarbonate was added to the residue, followed by extraction with ethyl acetate. The organic layer was dried over magnesium sulfate and then concentrated under a reduced pressure. The residue was purified by flash chromatography (amine silica gel, chloroform) to give a title compound (263mg, 0.69 mmol, 48%) as a colorless oil product.

¹H-NMR (400 MHz, CDCl₃) δ: 1.67 (1H, s), 1.71-1.81 (2H, m), 2.15-2.18 (2H, m), 2.74-2.81 (2H, m), 3.11-3.23 (3H, m), 3.80 (3H, s), 3.82 (3H, s), 6.82 (2H, d, J = 8.8 Hz), 6.85 (2H, d, J = 8.8 Hz), 7.26 (2H, d, J = 8.8 Hz), 7.45 (2H, d, J = 8.8 Hz).

IR (neat, cm⁻¹): 3341, 2937, 2836, 2736, 1736, 1608, 1575, 1538, 1514, 1496, 1464, 1372, 1319, 1293, 1250, 1176, 1141, 1106, 1034, 969, 880, 834, 795.
ESI-MS: m/z = 381 (M+H⁺).

### Reference Example 94

### 4-(4-(4-Chlorophenyl)-5-(4-tolyl)-2-thiazolyl)piperidine hydrochloride

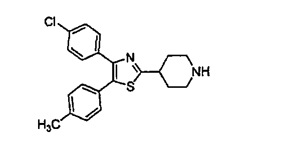

A solution of 1-tert-butoxycarbonyl-4-piperidine thioamide (500 mg, 2.05 mmol) and 2-bromo-1-(4-chlorophenyl)-2-(4-tolyl)ethanone (662 mg, 2.05 mmol) in methanol (15 ml) was heated under reflux for 4 hours and then cooled to room temperature. The reaction solution was concentrated under a reduced pressure, and the residue was dissolved with the addition of 10% hydrochloric acid-methanol (10 ml), and the resultant was heated under reflux for 4 hours. The reaction solution was cooled to room temperature and then concentrated under a reduced pressure. Chloroform was added to the residue to filter the slurry, and a title compound (831 mg, 2.05 mmol, quant.) was obtained as a yellow powder.

¹H-NMR (400 MHz, CDCl₃) δ: 1.71-1.82 (3H, m), 2.14-2.18 (2H, m), 2.36 (3H, s), 2.74-2.82 (2H, m), 3.11-3.23 (3H, m), 7.13 (2H, d, J = 8.8 Hz), 7.18-7.26 (4H, m), 7.45 (2H, d, J = 8.8 Hz).

IR (KBr, cm⁻¹): 3426, 2921, 1736, 1637, 1491, 1445, 1318, 1244, 1089, 1014, 970, 835,812.

ESI-MS: m/z = 369 (M+H⁺).

### Reference Example 95

### 4-(4-(4-Chlorophenyl)-5-phenylthiazol-2-yl)piperidine

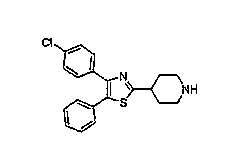

A title compound (270 mg, 0.76 mmol, 94%) was obtained as a colorless oil product from 2-bromo-1-(4-chlorophenyl)-2-phenylethanone (250 mg, 0.81 mmol) in the same manner as in Reference Example 93.

¹H-NMR (400 MHz, CDCl₃) δ: 1.65 (1H, s), 1.73-1.83 (2H, m), 2.15-2.20 (2H, m), 2.76-2.82 (2H, m), 3.14-3.25 (3H, m), 7.25 (2H, d, J = 8.8 Hz), 7.32-7.33 (5H, m), 7.45 (2H, d, J = 8.8 Hz).

IR (neat, cm⁻¹): 3307, 3058, 2940, 2849, 2811, 2736, 1598, 1572, 1495, 1443, 1400, 1360, 1319, 1245, 1178, 1140, 1090, 1071, 1014, 969, 877, 835, 759, 697.

ESI-MS: m/z = 355 (M+H⁺).

### Reference Example 96

### 4-(4,5-Diphenylthiazol-2-yl)piperidine hydrochloride

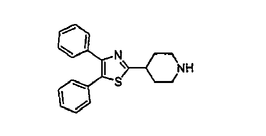

A title compound (506 mg, 1.41 mmol, 87%) was obtained as a white solid from 2-bromo-1,2-diphenylethanone (450 mg, 1.6 mmol) in the same manner as in Reference Example 94.

¹H-NMR (400 MHz, DMSO-d₆) δ: 1.98-2.08 (2H, m), 2.24-2.30 (2H, m), 3.05-3.12 (2H, m), 3.34-3.45 (3H, m), 7.31-7.45 (10H, m), 9.07 (1H, brs).

IR (KBr, cm⁻¹): 3434, 2926, 2810, 2714, 2479, 1580, 1498, 1445, 1312, 1227, 1071, 1005, 968, 880, 763, 698.

ESI-MS: m/z = 321 (M+H⁺).

### Reference Example 97

### 4-(4,5-Bis(4-methoxyphenyl)-1-methoxycarbonylmethylimidazol-2-yl)piperidine

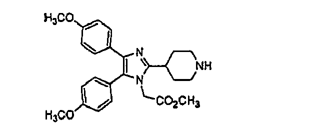

A mixture of 1-tert-butoxycarbonyl-4-(4,5-bis(4-methoxyphenyl)-1-methoxycarbonylmethylimidazol-2-yl)piperidine (Reference Example 71) (708 mg, 1.3 mmol) and 10% hydrochloric acid-methanol (30 ml) was stirred at 70°C for 4 hours and then cooled to room temperature. The reaction solution was concentrated under a reduced pressure, and saturated aqeous sodium bicarbonate was added to the residue, followed by extraction with chloroform. The organic layer was dried over sodium sulfate and then concentrated under a reduced pressure. The residue was purified by column chromatography (amine silica gel, chloroform/methanol = 10/1) to give a title compound (546 mg, 1.3 mmol, 95%) as a colorless amorphous product.

¹H-NMR (400 MHz, CDCl₃) δ: 1.67 (1H, brs), 1.85-1.88 (2H, m), 1.94-2.04 (2H, m), 2.63-2.75 (3H, m), 3.22-3.25 (2H, m), 3.72 (3H, s), 3.75 (3H, s), 3.86 (3H, s), 4.45 (2H, s), 6.74 (2H, d, J = 8.8 Hz), 6.94 (2H, d, J = 8.8 Hz), 7.19 (2H, d, J = 8.8 Hz), 7.41 (2H, d, J = 8.8 Hz).

IR (KBr, cm⁻¹): 3421, 2952, 1749, 1616, 1585, 1521, 1444, 1335, 1290, 1250, 1177, 1107, 1033, 837.

EI-MS: m/z = 436 (M+H⁺).

### Reference Example 98

### 4-(4,5-Bis(4-methoxyphenyl)-1H-pyrrol-2-yl)piperidine

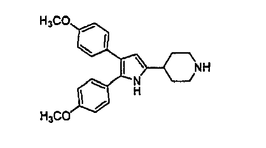

A title compound (31 mg, 0.085 mmol, 89%) was obtained as a yellow oil product from 1-benzyloxycarbonyl-4-(4,5-bis(4-methoxyphenyl)-1H-pyrrol-2-yl)piperidine (Reference Example 72) (47 mg, 0.095 mmol) in the same manner as in Reference Example 78.

¹H-NMR (400 MHz, CDCl₃) δ: 1.72 (2H, dq, J = 12.3, 4.0 Hz), 2.00-2.03 (2H, m), 2.34 (1H, brs), 2.72-2.79 (3H, m), 3.20-3.23 (2H, m), 3.79 (3H, s), 3.80 (3H, s), 6.07 (1H, d, J = 3.2 Hz), 6.80 (2H, d, J = 8.8 Hz), 6.83 (2H, d, J = 8.8 Hz), 7.24-7.26 (4H, m), 8.06 (1H, brs).

ESI-MS: m/z = 581 (M+H⁺).

### Reference Example 99

### 4-(4,5-Bis(4-methoxyphenyl)-1-tert-butoxycarbonylmethylpyrrol-2-yl)piperidine

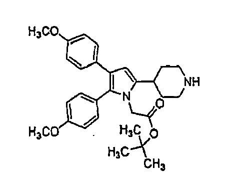

A title compound (91 mg, 0.19 mmol, 91%) was obtained as a yellow oil product from 1-benzyloxycarbonyl-4-(4,5-bis(4-methoxyphenyl)-1-tert-butoxycarbonylmethylpyrrol-2-yl)piperidine (Reference Example 73) (130 mg, 0.21 mmol) in the same manner as in

### Reference Example 78.

¹H-NMR (400 MHz, CDCl₃) δ: 1.41 (9H, s), 1.80-1.86 (2H, m), 1.99-2.02 (2H, m), 2.50-2.56 (1H, m), 2.75-2.81 (2H, m), 3.12-3.15 (1H, m), 3.29-3.32 (2H, m), 3.74 (3H, s), 3.83 (3H, s), 4.33 (2H, s), 6.22 (1H, s), 6.70 (2H, d, J = 8.8 Hz), 6.88 (2H, d, J = 8.8 Hz), 7.07 (2H, d, J = 8.8 Hz), 7.18 (2H, d, J = 8.8 Hz).

ESI-MS: m/z = 477 (M+H⁺).

### Reference Example 100

### 4-(4,5-Bis(4-methoxyphenyl)-1-hexylpyrrol-2-yl)piperidine

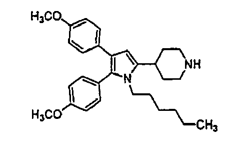

A title compound (87 mg, 0.195 mmol, 91%) was obtained as a brown oil product from 1-benzyloxycarbonyl-4-(4,5-bis(4-methoxyphenyl)-1-hexylpyrrol-2-yl)piperidine (Reference Example 74) (124 mg, 0.214 mmol) in the same manner as in Reference Example 78.

¹H-NMR (400 MHz, CDCl₃) δ: 0.81 (3H, t, J = 6.8 Hz), 1.10-1.21 (6H, m), 1.42-1.48 (2H, m), 1.94-2.05 (4H, m), 2.54-2.60 (3H, m), 3.17-3.20 (2H, m), 3.67-3.71 (2H, m), 3.73 (3H, s), 3.84 (3H, s), 6.17 (1H, s), 6.69 (2H, d, J = 8.8 Hz), 6.90 (2H, d, J = 8.8 Hz), 7.04 (2H, d, J = 8.8 Hz), 7.20 (2H, d, J = 8.8 Hz).

ESI-MS: m/z = 447 (M+H⁺).

### Reference Example 101

### 4-(4,5-Bis(4-methoxyphenyl)furan-2-yl)piperidine

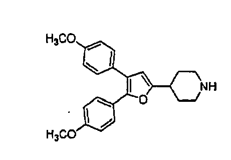

1-Benzyloxycarbonyl-4-(3,4-bis(4-methoxyphenyl)-1,4-dioxobutyl)piperidine (Reference Example 47) (121 mg, 0.28 mmol) was dissolved in concentrated sulfuric acid (0.5 ml) at 0°C and stirred at room temperature for 12 hours. The reaction solution was poured into saturated aqueous sodium bicarbonate, followed by extraction with ethyl acetate. The organic layer was dried over sodium sulfate and then concentrated under a reduced pressure to give a title compound (80 mg, 0.22 mmol, 96%) as a yellow amorphous product.

¹H-NMR (400 MHz, CDCl₃) δ: 1.62-1.68 (2H, m), 2.05-2.07 (2H, m), 2.62-2.81 (3H, m), 3.16-3.19 (2H, m), 3.80 (3H, s), 3.83 (3H, s), 3.87-3.92 (2H, m), 6.10 (1H, s), 6.81 (2H, d, J = 8.8 Hz), 6.88 (2H, d, J = 8.8 Hz), 7.31 (2H, d, J = 8.8 Hz), 7.43 (2H, d, J = 8.8 Hz).

ESI-MS: m/z = 364 (M+H⁺).

### Reference Example 102

### 4-(4,5-Bis(4-methoxyphenyl)thiophen-2-yl)piperidine

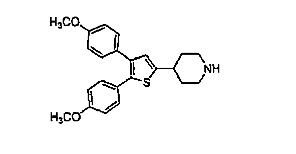

A title compound (35 mg, 0.092 mmol, 53%) was obtained as a brown oil product from 1-benzyloxycarbonyl-4-(4,5-bis(4-methoxyphenyl)thiophen-2-yl)piperidine (Reference Example 75) (90 mg, 0.175 mmol) in the same manner as in Reference Example 78.

¹H-NMR (400 MHz, CDCl₃) δ: 1.68-1.73 (2H, m), 2.06-2.09 (2H, brd), 2.74-2.85 (3H, m), 3.19-3.22 (2H, m), 3.80 (3H, s), 3.83 (3H, s), 6.11 (1H, s), 6.82 (2H, d, J = 8.8 Hz), 6.88 (2H, d, J = 8.8 Hz), 7.31 (2H, d, J = 8.8 Hz), 7.43 (2H, d, J = 8.8 Hz).

ESI-MS: m/z = 380 (M+H⁺).

### Reference Example 103

### 4-(1,5-Bis(4-methoxyphenyl)-1H-pyrazol-3-yl)piperidine

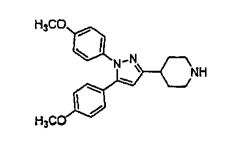

A title compound (262 mg, 0.723 mmol, quant.) was obtained as a brown oil product from 1-benzyloxycarbonyl-4-(1,5-bis(4-methoxyphenyl)-1H-pyrazol-3-yl)piperidine (Reference Example 76) (360 mg, 0.723 mmol) in the same manner as in Reference Example 78.

¹H-NMR (400 MHz, CDCl₃) δ: 1.69-1.76 (2H, m), 2.02-2.05 (2H, m), 2.77 (2H, dt, J = 12.0, 2.4 Hz), 2.87 (2H, tt, J = 11.6, 4.0 Hz), 3.19 (2H, dt, J = 12.0, 3.0 Hz), 3.79 (3H, s), 3.80 (3H, s), 6.26 (1H, s), 6.81 (2H, d, J = 8.8 Hz), 6.84 (2H, d, J = 8.8 Hz), 7.14 (2H, d, J = 8.8 Hz), 7.20 (2H, d, J = 8.8 Hz).

### Reference Example 104

### 4-(5-(4-Methoxyphenyl)-1-phenyl-1H-pyrazol-3-yl)piperidine

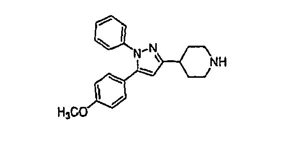

A title compound (143 mg, 0.428 mmol, quant.) was obtained as a colorless oil product from 1-benzyloxycarbonyl-4-(5-(4-methoxyphenyl)-1-phenyl-1H-pyrazol-3-yl)piperidine (Reference Example 77) (200 mg, 0.428 mmol) in the same manner as in Reference Example 78.

¹H-NMR (400 MHz, CDCl₃) δ: 1.82-1.92 (2H, m), 2.09-2.13 (2H, m), 2.30 (1H, brs), 2.86 (2H, dt, J = 12.0, 2.4 Hz), 2.94 (2H, tt, J = 11.6, 4.0 Hz), 3.30 (2H, dt, J = 12.0, 3.0 Hz), 3.80 (3H, s), 6.29 (1H, s), 6.82 (2H, d, J = 8.8 Hz), 7.14 (2H, d, J = 8.8 Hz), 7.24-7.34 (5H, m).

### Reference Example 105

### Benzyl 1-tert-butoxycarbonyl-4-piperidinecarboxylate

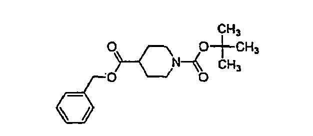

To a solution of 1-tert-butoxycarbonyl-4-piperidinecarboxylic acid (647 mg, 2.82 mg) in acetone (30 ml) was added benzyl bromide (0.36 ml, 3.0 mmol) and potassium carbonate (480 mg, 3.47 mmol). The resultant was heated under reflux for 2 hours and then cooled to room temperature. The reaction solution was concentrated under a reduced pressure, and distilled water was added to the residue, followed by extraction with ethyl acetate. The organic layer was dried and then concentrated under a reduced pressure. The residue was purified by flash chromatography (silica gel, n-hexane/ethyl acetate) to give a title compound (750 mg, 2.35 mmol, 83%) as a colorless oil product.

¹H-NMR (400 MHz, CDCl₃) δ: 1.45 (9H, s), 1.60-1.70 (2H, m), 1.88-1.96 (2H, m), 2.46-2.54 (1H, m), 2.78-2.87 (2H, m), 3.93-4.12 (2H, m), 5.13 (2H, s), 7.30-7.39 (5H, m).

### Reference Example 106

### Benzyl 1-tert-butoxycarbonyl-4-methyl-4-piperidinecarboxylate

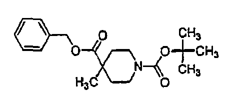

To a solution of benzyl 1-tert-butoxycarbonyl-4-piperidinecarboxylate (Reference Example 105) (710 mg, 2.22 mmol) in tetrahydrofuran (10 ml) was added a solution or 0.5M potassium bis(trimethylsilyl)amide/toluene (5.3 ml) at -78°C, and the resultant was stirred at the same temperature for 1 hour. Methyl iodide (165 µl, 2.65 mmol) was added to the reaction solution, and the mixture was gradually heated to room temperature. Distilled water was added to the reaction solution, followed by extraction with ethyl acetate. The organic layer was dried and then concentrated under a reduced pressure. The residue was purified by flash chromatography (silica gel, n-hexane/ethyl acetate) to give a title compound (596 mg, 1.78 mmol, 80%) as a colorless liquid.

¹H-NMR (400 MHz, CDCl₃) δ: 1.22 (3H, s), 1.33-1.43 (2H, m), 1.44 (9H, s), 2.04-2.12 (2H, m), 2.91-3.04 (2H, m), 3.65-3.82 (2H, m), 5.14 (2H, s), 7.30-7.40 (5H, m).

### Reference Example 107

### 1-tert-Butoxycarbonyl-4-methyl-4-piperidinecarboxylic acid

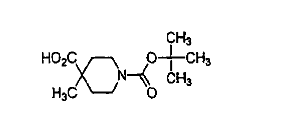

To a solution of benzyl 1-tert-butoxycarbonyl-4-methyl-4-piperidinecarboxylate (Reference Example 106) (593 mg, 1.77 mmol) in methanol (10 ml) was added 10% Pd-C (63 mg), and the resultant was stirred under a hydrogen atmosphere for 2 hours. The reaction solution was filtered, and the filtrate was concentrated under a reduced pressure to give a title compound (408 mg, 1.67 mmol, 94%) as a white solid.

¹H-NMR (400 MHz, CDCl₃) δ: 1.27 (3H, s), 1.34-1.48 (2H, m), 1.45 (9H, s), 2.00-2.11 (2H, m), 2.97-3.15 (2H, m), 3.67-3.87 (2H, m).

ESI-MS: m/z = 244 (M+H⁺).

### Reference Example 108

### (1,2-Bis(4-methoxyphenyl)-2-oxoethyl) 1-tert-butoxycarbonyl-4-methyl-4-piperidinecarboxylate

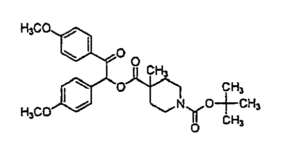

A title compound (384 mg, 0.771 mmol, 92%) was obtained as a white amorphous product from 1-tert-butoxycarbonyl-4-methyl-4-piperidinecarboxylic acid (Reference Example 107) (204 mg, 0.838 mmol) in the same manner as in Reference Example 31.

¹H-NMR (400 MHz, CDCl₃) δ: 1.28 (3H, s), 1.34-1.46 (2H, m), 1.45 (9H, s), 2.08-2.21 (2H, m), 2.92-3.06 (1H, m), 3.20-3.30 (1H, m), 3.73-3.90 (8H, m), 6.78 (1H, s), 6.87 (2H, d, J = 8.8 Hz), 6.87 (2H, d, J = 8.8 Hz), 7.35 (2H, d, J = 8.8 Hz), 7.91 (2H, d, J = 8.8 Hz).

### Reference Example 109

### 4-(4,5-Bis(4-methoxyphenyl)oxazol-2-yl)-4-methylpiperidine

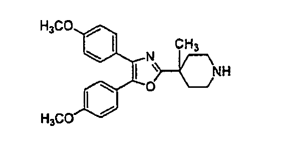

To a solution of (1,2-bis(4-methoxyphenyl)-2-oxoethyl) 1-tert-butoxycarbonyl-4-methyl-4-piperidinecarboxylate (Reference Example 108) (380 mg, 0.736 mmol) in acetic acid (3.0 ml) was added ammonium acetate (652 mg, 8.45 mmol), and the resultant was stirred at 90°C for 10 hours and then cooled to 0°C. The reaction solution was neutralized with the addition of an aqueous solution of 5M sodium hydroxide and saturated aqueous sodium bicarbonate at 0°C, followed by extraction with ethyl acetate. The organic layer was dried and then concentrated under a reduced pressure. The residue was dissolved with the addition of dichloromethane (5.0 ml). Trifluoroacetic acid (1.0 ml) was added to the reaction solution, the mixture was stirred at room temperature for 5 hours, and the resultant was then concentrated under a reduced pressure. An aqueous solution of 1M sodium hydroxide was added to the residue, followed by extraction with ethyl acetate. The organic layer was dried and then concentrated under a reduced pressure. The residue was purified by column chromatography (silica gel, n-hexane/silica gel) to give a title compound (274 mg, 0.724 mmol, 95%) as a light yellow oil product.

¹H-NMR (400 MHz, CDCl₃) δ: 1.42 (3H, s), 1.60-1.67 (2H, m), 2.30-2.38 (2H, m), 2.83-2.90 (2H, m), 2.94-3.00 (2H, m), 3.83 (6H, s), 6.89 (2H, d, J = 8.8 Hz), 6.90 (2H, d, J = 8.8 Hz), 7.50 (2H, d, J = 8.8 Hz), 7.57 (2H, d, J = 8.8 Hz).

ESI-MS: m/z = 379 (M+H⁺).

### Reference Example 110

### 4-Benzyloxycarbonyl-1-tert-butoxycarbonyl-4-piperidinecarboxylic acid

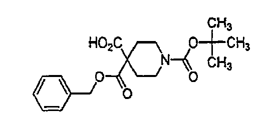

To a solution of benzyl 1-tert-butoxycarbonyl-4-piperidinecarboxylate (Reference Example 105) (800 mg, 2.50 mmol) in tetrahydrofuran (10 ml) was added 0.7M potassium bis(trimethylsilyl)amide (5.0 ml, 3.5 mmol) at -78°C, the mixture was stirred at the same temperature for 2 hours, and dry ice was added to the reaction solution. The reaction solution was gradually heated to room temperature, and distilled water was added thereto, followed by extraction with ethyl acetate. The aqueous layer was acidified with 1M hydrochloric acid, followed by extraction with ethyl acetate. The organic layer was dried over magnesium sulfate and then concentrated under a reduced pressure to give a title compound (621 mg, 1.70 mmol, 68%) as a light yellow solid.

¹H-NMR (400 MHz, CDCl₃) δ: 1.44 (9H, s), 2.05-2.13 (4H, m), 3.37-3.50 (4H, m), 5.20 (2H, s), 7.29-7.38 (5H, m).

### Reference Example 111

### (1,2-Bis(4-methoxyphenyl)-2-oxoethyl) 4-benzyloxycarbonyl-1-tert-butoxycarbonyl-4-piperidinecarboxylate

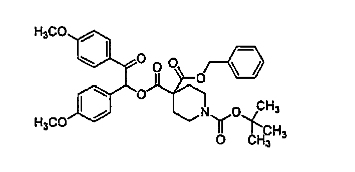

To a solution of benzyl 1-tert-butoxycarbonyl-4-piperidinecarboxylate (Reference Example 105) (800 mg, 2.50 mmol) in tetrahydrofuran (10 ml) was added 0.7M potassium bis(trimethylsilyl)amide (5.0 ml, 3.5 mmol) at -78°C, the mixture was stirred at the same temperature for 1 hour, and carbon dioxide was then introduced into the reaction solution for the period of 20 minutes. The reaction solution was gradually heated to room temperature, and the reaction solution was acidified with 1M hydrochloric acid, followed by extraction with ethyl acetate. The organic layer was dried over magnesium sulfate and then concentrated under a reduced pressure. The residue was dissolved with the addition of N,N-dimethylformamide (3.0 ml), 2-bromo-1,2-bis(4-methoxyphenyl)ethanone (Reference Example 14) (602 mg, 1.79 mmol) and cesium carbonate (817 mg, 2.50 mmol) were added thereto, and the resultant was stirred at room temperature for 3 hours. Distilled water was added to the reaction solution, followed by extraction with ethyl acetate. The organic layer was washed with water, dried over magnesium sulfate, and then concentrated under a reduced pressure. The residue was purified by flash chromatography (silica gel, n-hexane/ethyl acetate) to give a title compound (957 mg, 1.55 mmol, 62%) as a white amorphous product.

¹H-NMR (400 MHz, CDCl₃) δ: 1.44 (9H, s), 1.98-2.05 (1H, m), 2.12-2.29 (3H, m), 3.34-3.42 (1H, m), 3.49-3.58 (2H, m), 3.59-3.66 (1H, m), 3.77 (3H, s), 3.82 (3H, s), 5.10 (1H, d, J = 12.4 Hz), 5.16 (1H, d, J = 12.4 Hz), 6.77 (1H, s), 6.84 (2H, d, J = 8.8 Hz), 6.86 (2H, d, J = 8.8 Hz), 7.22-7.31 (7H, m), 7.88 (2H, d, J = 8.8 Hz).

### Reference Example 112

### Benzyl 4-(4,5-bis(4-methoxyphenyl)oxazol-2-yl)piperidine-4-carboxylate

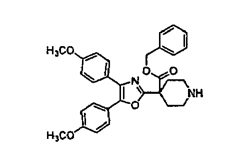

A title compound (638 mg, 1.28 mmol, 83%) was obtained as a light yellow oil product from (1,2-bis(4-methoxyphenyl)-2-oxoethyl) 4-benzyloxycarbonyl-1-tert-butoxycarbonyl-4-piperidinecarboxylate (Reference Example 111) (954 mg, 1.54 mmol) in the same manner as in Reference Example 31.

¹H-NMR (400 MHz, CDCl₃) δ: 2.27-2.46 (4H, m), 2.83-3.02 (4H, m), 3.83 (6H, s), 5.20 (2H, s), 6.87 (2H, d, J = 8.8 Hz), 6.90 (2H, d, J = 8.8 Hz), 7.24-7.30 (2H, d, J = 8.8 Hz), 7.43 (2H, d, J = 8.8 Hz), 7.56 (2H, d, J = 8.8 Hz).

ESI-MS: m/z = 499 (M+H⁺).

### Reference Example 113

### Methyl 1-(benzyloxycarbonyl)piperidine-4-carboxylate

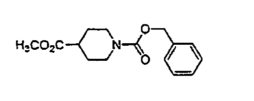

To a solution of 1-(benzyloxycarbonyl)piperidine-4-carboxylic acid (Reference Example 27) (540 mg, 2.05 mmol) in methanol was added concentrated sulfuric acid (two drops with a Pasteur pipette), and the resultant was heated under reflux for 5 hours. The reaction solution was cooled to room temperature and then concentrated under a reduced pressure. Saturated aqueous sodium bicarbonate was added to the residue, followed by extraction with ethyl acetate. The organic layer was dried over magnesium sulfate and then concentrated under a reduced pressure to give a title compound (532 mg, 1.92 mmol, 93%) as a colorless oil product.

¹H-NMR (400 MHz, CDCl₃) δ: 1.57-1.73 (2H, m), 1.82-1.96 (2H, m), 2.43-2.55 (1H, m), 2.85-2.97 (2H, m), 3.69 (3H, s), 4.02-4.17 (2H, m), 5.12 (2H, s), 7.29-7.40 (5H, m).

IR (neat, cm⁻¹): 1732, 1696, 1450, 1433, 1223, 1192, 1175, 1040.

### Reference Example 114

### 1-Benzyloxycarbonyl-4-methoxycarbonyl-4-piperidinecarboxylic acid

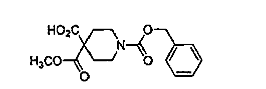

A title compound (1.19 g, 3.70 mmol, 87%) was obtained as a light yellow oil product from methyl 1-(benzyloxycarbonyl)piperidine-4-carboxylate (Reference Example 113) (1.18 g, 4.25 mmol) in the same manner as in Reference Example 110.

¹H-NMR (400 MHz, CDCl₃) δ: 2.05-2.16 (4H, m), 3.49-3.58 (4H, m), 3.77 (3H, s), 5.13 (2H, s), 7.29-7.39 (5H, m).

### Reference Example 115

### (1,2-Bis(4-methoxyphenyl)-2-oxoethyl) 1-benzyloxycarbonyl-4-methoxycarbonyl-4-piperidinecarboxylate

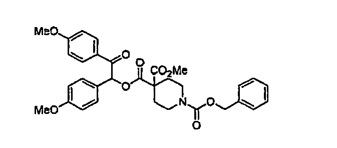

To a solution of 1-benzyloxycarbonyl-4-methoxycarbonyl-4-piperidinecarboxylic acid (Reference Example 114) (1.19 g, 3.70 mmol) and 1,2-bis(4-methoxyphenyl)-2-bromoethanone (Reference Example 14) (1.24 g, 3.70 mmol) in N,N-dimethylformamide (4.0 ml) was added cesium carbonate (1.33 g, 4.08 mmol), and the mixture was stirred at room temperature for 2 hours. Distilled water was added to the reaction solution, followed by extraction with ethyl acetate. The organic layer was washed with water, dried, and then concentrated under a reduced pressure. The residue was purified by column chromatography (silica gel, n-hexane/ethyl acetate) to give a title compound (1.88 g, 3.26 mmol, 88%) as a white amorphous product.

¹H-NMR (400 MHz, CDCl₃) δ: 2.09-2.30 (4H, m), 3.40-3.50 (1H, m), 3.58-3.71 (6H, s), 3.77 (3H, s), 3.83 (3H,s), 5.13 (2H, s), 6.81 (1H, s), 6.86 (2H, d, J = 8.8 Hz), 6.88 (2H, d, J = 8.8 Hz), 7.28-7.39 (7H, m), 7.89 (2H, d, J = 8.8 Hz).

### Reference Example 116

### Methyl 1-benzyloxycarbonyl-4-(4,5-bis(4-methoxyphenyl)oxazol-2-yl)-4-piperidinecarboxylate

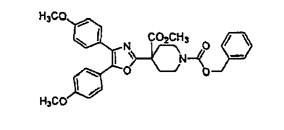

A title compound (1.40 g, 2.51 mmol, 77%) was obtained as a light yellow amorphous product from (1,2-bis(4-methoxyphenyl)-2-oxoethyl) 1-tert-butoxycarbonyl-4-methoxycarbonyl-4-piperidinecarboxylate (Reference Example 115) (1.88g, 3.26 mmol) in the same manner as in Reference Example 49.

¹H-NMR (400 MHz, CDCl₃) δ: 2.25-2.50 (4H, m), 3.40-3.50 (2H, m), 3.71-3.82 (5H, s), 3.83 (6H, s), 5.13 (2H, s), 6.89 (2H, d, J = 8.8 Hz), 6.90 (2H, d, J = 8.8 Hz), 7.28-7.39 (5H, m), 7.48 (2H, d, J = 8.8 Hz), 7.56 (2H, d, J = 8.8 Hz).

### Reference Example 117

### 1-Benzyloxycarbonyl-4-(4,5-bis(4-methoxyphenyl)oxazol-2-yl)-4-hydroxymethylpiperidine

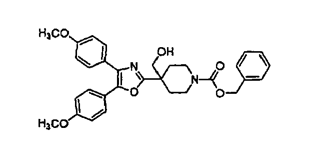

To a solution of methyl 1-benzyloxycarbonyl-4-(4,5-bis(4-methoxyphenyl)oxazol-2-yl)-4-piperidinecarboxylate (Reference Example 116) (200 mg, 0.359 mmol) in tetrahydrofuran (4.0 ml) was added lithium borohydride (39 mg, 1.79 mmol), and the mixture was stirred at room temperature for 17 hours. Distilled water was added to the reaction solution, followed by extraction with ethyl acetate. The organic layer was dried over magnesium sulfate and then concentrated under a reduced pressure. The residue was purified by flash chromatography (silica gel, n-hexane/ethyl acetate) to give a title compound (184 mg, 0.348 mmol, 97%) as a white amorphous product.

¹H-NMR (400 MHz, CDCl₃) δ:1.68-1.78 (2H, m), 2.24-2.32 (2H, m), 3.08-3.14 (1H, m), 3.39-3.48 (2H, m), 3.78-3.91 (10H, m), 5.15 (2H, s), 6.90 (4H, d, J = 8.8 Hz), 7.29-7.38 (5H, m), 7.48 (2H, d, J = 8.8 Hz), 7.56 (2H, d, J = 8.8 Hz).

### Reference Example 118

### 4-Acetoxymethyl-1-benzyloxycarbonyl-4-(4,5-bis(4-methoxyphenyl)oxazol-2-yl)piperidine

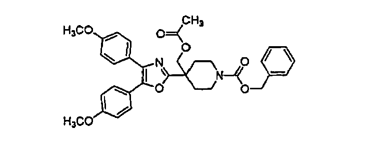

To a solution of 1-benzyloxycarbonyl-4-(4,5-bis(4-methoxyphenyl)oxazol-2-yl)-4-hydroxymethylpiperidine (Reference Example 117) (180 mg, 0.34 mmol) in dichloromethane (4.0 ml) were added acetic anhydride (40 µl, 0.41 mmol), pyridine (35 µl, 0.43 mmol) and 4,4-dimethylaminopyridine (16 mg, 0.13 mmol), and the mixture was stirred at room temperature for 2 hours. The reaction solution was concentrated under a reduced pressure, and 1 M hydrochloric acid was added to the residue, followed by extraction with ethyl acetate. The organic layer was dried over magnesium sulfate and then concentrated under a reduced pressure. The residue was purified by flash chromatography (silica gel, n-hexane/ethyl acetate) to give a title compound (194 mg, 0.336 mmol, quant.) as a colorless oil product.

¹H-NMR (400 MHz, CDCl₃) δ: 1.68-1.80 (2H, m), 2.05 (2H, m), 2.35-2.43 (2H, m), 3.05-3.25 (2H, m), 3.84 (6H, s), 4.02-4.15 (2H, m), 4.26 (2H, s), 5.14 (2H, s), 6.89 (2H, d, J = 8.8 Hz), 6.90 (2H, d, J = 8.8 Hz), 7.28-7.37 (5H, m), 7.48 (2H, d, J = 8.8 Hz), 7.56 (2H, d, J = 8.8 Hz).

### Reference Example 119

### 4-Acetoxymethyl-4-(4,5-bis(4-methoxyphenyl)oxazol-2-yl)piperidine

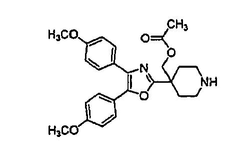

A title compound (147 mg, 0.336 mmol, quant.) was obtained as a colorless oil product from 4-acetoxymethyl-1-benzyloxycarbonyl-4-(4,5-bis(4-methoxyphenyl)oxazol-2-yl)piperidine (Reference Example 118) (190 mg, 0.333 mmol) in the same manner as in Reference Example 78.

¹H-NMR (400 MHz, CDCl₃) δ: 1.72-1.80 (2H, m), 2.02 (2H, s), 2.35-2.42 (2H, m), 2.82-2.91 (2H, m), 2.99-3.07 (2H, m), 3.84 (6H, s), 4.26 (2H, s), 6.89 (2H, d, J = 8.8 Hz), 6.90 (2H, d, J = 8.8 Hz), 7.49 (2H, d, J = 8.8 Hz), 7.57 (2H, d, J = 8.8 Hz).

### Reference Example 120

### Benzyl 1-tert-butoxycarbonyl-4-carbamoyl-4-piperidinecarboxylate

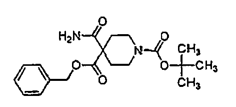

To a solution of 4-benzyloxycarbonyl-1-tert-butoxycarbonyl-4-piperidinecarboxylic acid (Reference Example 110) (206 mg, 0.567 mmol) in dichloromethane (4.0 ml) was added triethylamine (93 µl, 0.68 mmol) and ethyl chloroformate (60 µl, 0.63 mmol) at 0°C, and the mixture was stirred at the same temperature for 1 hour. 28% aqueous ammonia (2.0 ml) was added to the reaction solution, and the mixture was stirred at room temperature for 3 hours. The organic layer was separated from the reaction solution, dried over sodium sulfate, and then concentrated under a reduced pressure to give a title compound (205 mg, 0.567 mmol, quant.) as a white solid.

¹H-NMR (400 MHz, CDCl₃) δ: 1.44 (9H, s), 1.95-2.02 (2H, m), 2.12-2.22 (2H, m), 3.03-3.12 (2H, m), 3.69-3.81 (2H, m), 5.22 (2H, s), 5.38 (1H, brs), 5.90 (1H, brs), 7.31-7.40 (5H, m).

### Reference Example 121

### 1-tert-Butoxycarbonyl-4-carbamoyl-4-piperidinecarboxylic acid

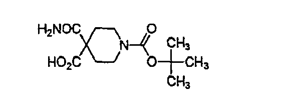

A title compound (154 mg, 0.567 mmol, quant.) was obtained as a white amorphous product from benzyl 1-tert-butoxycarbonyl-4-carbamoyl-4-piperidinecarboxylate (Reference Example 120) (205 mg, 0.567 mmol) in the same manner as in Reference Example 107.

¹H-NMR (400 MHz, CDCl₃) δ: 1.45 (9H, s), 1.88-1.99 (2H, m), 2.09-2.19 (2H, m), 3.05-3.23 (3H, m), 3.68-3.79 (2H, m), 3.87-4.29 (2H, m), 6.57 (1H, brs).

### Reference Example 122

### (1,2-Bis(4-methoxyphenyl)-2-oxoethyl) 1-tert-butoxycarbonyl-4-carbamoyl-4-piperidinecarboxylate

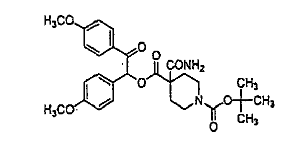

A title compound (252 mg, 0.478 mmol, 84%) was obtained as a white amorphous product from 1-tert-butoxycarbonyl-4-carbamoyl-4-piperidinecarboxylic acid (Reference Example 121) (154 mg, 0.567 mmol) and 1,2-(bis(4-methoxyphenyl)-2-bromoethanone (Reference Example 14) (193 mg, 0.575 mmol) in the same manner as in Reference Example 115.

¹H-NMR (400 MHz, CDCl₃) δ: 1.44 (9H, s), 1.95-2.27 (4H, m), 2.70-2.91 (1H, m), 2.99-3.14 (1H, m), 3.79 (3H, s), 3.84 (3H, s), 3.85-4.03 (2H, m), 5.48 (1H, brs), 6.88 (2H, d, J = 8.8 Hz), 6.89 (2H, d, J = 8.8 Hz), 7.33 (2H, d, J = 8.8 Hz), 7.51 (1H, brs), 7.91 (2H, d, J = 8.8 Hz).

### Reference Example 123

### 4-(4,5-(4-Methoxyphenyl)oxazol-2-yl)-1-tert-butoxycarbonylpiperidine-4-carboxamide

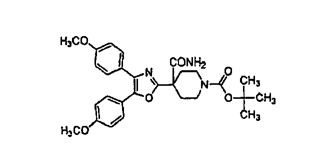

A title compound (370 mg, 0.729 mmol, 76%) was obtained as a white amorphous product from (1,2-bis(4-methoxyphenyl)-2-oxoethyl) 1-tert-butoxycarbonyl-4-carbamoyl-4-piperidinecarboxylate (Reference Example 122) (503 mg, 0.955 mmol) in the same manner as in Reference Example 49.

¹H-NMR (400 MHz, CDCl₃) δ: 1.45 (9H, s), 2.21-2.30 (2H, m), 2.41-2.50 (2H, m), 3.02-3.13 (2H, m), 3.84 (3H, s), 3.84 (3H, s), 3.90-4.05 (2H, m), 5.37 (1H, brs), 6.25 (1H, brs), 6.90 (2H, d, J = 8.8 Hz), 6.92 (2H, d, J = 8.8 Hz), 7.48 (2H, d, J = 8.8 Hz), 7.57 (2H, d, J = 8.8 Hz).

### Reference Example 124

### 4-(4,5-Bis(4-methoxyphenyl)oxazol-2-yl)piperidine-4-carboxamide

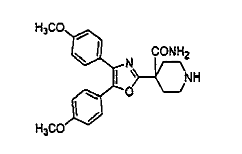

A title compound (114 mg, 0.280 mmol, 59%) was obtained as a white amorphous product from (1,2-bis(4-methoxyphenyl)-2-oxoethyl) 1-tert-butoxycarbonyl-4-carbamoyl-4-piperidinecarboxylate (Reference Example 123) in the same manner as in Reference Example 86.

¹H-NMR (400 MHz, CDCl₃) δ: 2.19-2.28 (2H, m), 2.43-2.50 (2H, m), 2.77-2.82 (2H, m), 3.04-3.12 (2H, m), 3.84 (3H, s), 3.84 (3H, s), 5.34 (1H, brs), 6.23 (1H, brs), 6.89 (2H, d, J = 8.8 Hz), 6.92 (2H, d, J = 8.8 Hz), 7.49 (2H, d, J = 8.8 Hz), 7.57 (2H, d, J = 8.8 Hz).

### Reference Example 125

### 4-(4,5-Bis(4-methoxyphenyl)oxazol-2-yl)-1-tert-butoxycarbonyl-4-cyanopiperidine

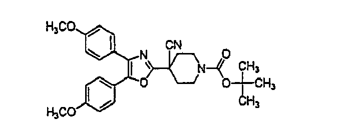

To a solution of 4-(4,5-(4-methoxyphenyl)oxazol-2-yl)-1-tert-butoxycarbonylpiperidine-4-carboxamide (Reference Example 123) (267 mg, 0.526 mmol) in dichloromethane (5.0 ml) was added (methoxycarbonylsulfamoyl)triethylammonium hydroxide inner salt (a Burgess reagent) (383 mg, 1.60 mmol), and the mixture was stirred at room temperature for 22 hours. The reaction solution was concentrated under a reduced pressure, and the residue was purified by flash chromatography (silica gel, n-hexane/ethyl acetate) to give a title compound (185 mg, 0.378 mmol, 72%) as a white amorphous product.

¹H-NMR (400 MHz, CDCl₃) δ: 1.47 (9H, s), 2.19-2.28 (2H, m), 2.34-2.41 (2H, m), 3.27-3.38 (2H, m), 3.83 (3H, s), 3.84 (3H, s), 4.05-4.16 (2H, m), 6.91 (4H, d, J = 8.8 Hz), 7.51 (2H, d, J = 8.8 Hz), 7.55 (2H, d, J = 8.8 Hz).

IR (KBr, cm⁻¹): 2237, 1697, 1613, 1521, 1500, 1422, 1251.

### Reference Example 126

### 4-(4,5-Bis(4-methoxyphenyl)oxazol-2-yl)-4-cyanopiperidine

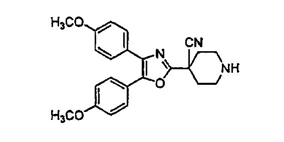

A title compound (144 mg, 0.372 mmol, quant.) was obtained as a white amorphous product from 4-(4,5-bis(4-methoxyphenyl)oxazol-2-yl)-1-tert-butoxycarbonyl-4-cyanopiperidine (Reference Example 125) (182 mg, 0.372 mmol) in the same manner as in Reference Example 86.

¹H-NMR (400 MHz, CDCl₃) δ: 2.38-2.54 (4H, m), 3.18-3.27 (2H, m), 3.29-3.37 (2H, m), 3.83 (3H, s), 3.84 (3H, s), 6.91 (4H, d, J = 8.8 Hz), 7.51 (2H, d, J = 8.8 Hz), 7.55 (2H, d, J = 8.8 Hz).

ESI-MS: m/z = 390 (M+H⁺).

### Reference Example 127

### 1-Benzyloxycarbonyl-4-phenyl-4-piperidinecarboxylic acid

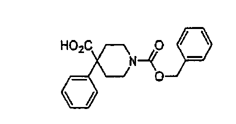

To a solution of 4-phenyl-4-piperidinecarboxylic acid p-toluene sulfonate (113 mg, 3.00 mmol) in water (15 ml) was added sodium carbonate (954 mg, 9.00 mmol) and benzyl chloroformate (511 mg, 3.00 mmol), and the mixture was stirred at room temperature for 15 hours. Distilled water was added to the reaction solution, followed by extraction with ether. The aqueous layer was acidified with concentrated hydrochloric acid, and the precipitate was filtered and dried to give a title compound (860 mg, 2.53 mmol, 84%) as a white powder.

¹H-NMR (400 MHz, CDCl₃) δ:1.82-1.98 (2H, m), 2.48-2.58 (2H, m), 3.11-3.26 (2H, m), 3.95-4.11 (2H, m), 5.12 (2H, s), 7.27-7.41 (10H, m).

### Reference Example 128

### (1,2-Bis(4-methoxyphenyl)-2-oxoethyl) 1-benzyloxycarbonyl-4-phenyl-4-piperidinecarboxylate

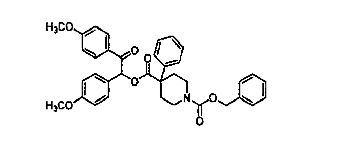

A title compound (571 mg, 0.962 mmol, 96%) was obtained as a white solid from 1-benzyloxycarbonyl-4-phenyl-4-piperidinecarboxylate (Reference Example 127) (335 mg, 1.00 mmol) in the same manner as in Reference Example 115.

¹H-NMR (400 MHz, CDCl₃) δ:1.83-1.93 (2H, m), 2.64 (2H, m), 3.14 (1H, m), 3.58 (1H, m), 3.76 (3H, s), 3.81 (3H, s), 4.13 (2H, m), 5.13 (2H, s), 6.70 (1H, s), 6.82 (2H, d, J = 8.8 Hz), 6.84 (2H, d, J = 8.8 Hz), 7.24-7.38 (12H, m), 7.86 (2H, d, J = 8.8 Hz).

### Reference Example 129

### 1-Benzyloxycarbonyl-4-(4,5-bis(4-methoxyphenyl)oxazol-2-yl)-4-phenylpiperidine

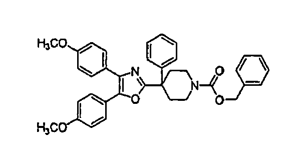

A title compound (481 mg, 0.837 mmol, 96%) was obtained as a light yellow amorphous product from (1,2-bis(4-methoxyphenyl)-2-oxoethyl) 1-benzyloxycarbonyl-4-phenyl-4-piperidinecarboxylate (Reference Example 128) (520 mg, 0.876 mmol) in the same manner as in Reference Example 49.

¹H-NMR (400 MHz, CDCl₃) δ: 2.14 (2H, m), 2.78 (2H, brd, J = 13.2 Hz), 3.17 (2H, m), 3.81 (3H, s), 3.82 (3H, s), 4.15 (2H, m), 5.14 (2H, s), 6.85 (2H, d, J = 8.8 Hz), 6.90 (2H, d, J = 8.8 Hz), 7.26-7.36 (10H, m), 7.42 (2H, d, J = 8.8 Hz), 7.57 (2H, d, J = 8.8 Hz).

### Reference Example 130

### 4-(4,5-Bis(4-methoxyphenyl)oxazol-2-yl)-4-phenylpiperidine

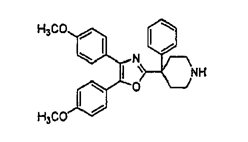

A title compound (342 mg, 0.778 mmol, quant.) was obtained as a light brown oil product from 1-benzyloxycarbonyl-4-(4,5-bis(4-methoxyphenyl)oxazol-2-yl)-4-phenylpiperidine (Reference Example 129) (447 mg, 0.778 mmol) in the same manner as in Reference Example 78.

¹H-NMR (400 MHz, CDCl₃) δ: 2.36-2.50 (4H, m), 2.84-2.90 (4H, m), 3.11 (1H, brt, J = 12.0 Hz), 3.36 (1H, brd, J = 12.8 Hz), 3.82 (3H, s), 3.84 (3H, s), 6.86 (2H, d, J = 8.8 Hz), 6.91 (2H, d, J = 8.8 Hz), 7.30-7.38 (5H, m), 7.43 (2H, d, J = 8.8 Hz), 7.58 (2H, d, J = 8.8 Hz).

### Reference Example 131

### 4-(5-(4-Fluorophenyl)-4-(4-methoxyphenyl)thiazol-2-yl)piperidine

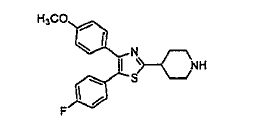

A solution of 2-bromo-2-(4-fluorophenyl)-1-(4-methoxyphenyl)ethanone (Reference Example 22) (199 mg, 0.616 mmol) and 1-tert-butoxycarbonylpiperidine-4-thiocarboxamide (153 mg, 0.613 mmol) in methanol (5.0 ml) was stirred at room temperature for 4 hours. The reaction solution was concentrated under a reduced pressure, and the residue was dissolved with the addition of dichloromethane. Trifluoroacetic acid (1.0 ml) was added, the mixture was stirred at room temperature for 3 hours, and the resultant was then concentrated under a reduced pressure. The residue was neutralized with the addition of an aqueous solution of 1M sodium hydroxide, followed by extraction with ethyl acetate. The organic layer was dried and then concentrated under a reduced pressure. The residue was purified by flash chromatography (silica gel, n-hexane/ethyl acetate) to give a title compound (177 mg, 0.480 mmol, 78 %) as a white amorphous product.

¹H-NMR (400 MHz, CDCl₃) δ: 1.71-1.83 (2H, m), 2.12-2.20 (2H, m), 2.75-2.82 (2H, m), 3.07-3.26 (3H, m), 3.80 (3H, s), 6.81 (2H, d, J = 8.8 Hz), 7.00 (2H, t, J = 8.8 Hz), 7.29 (2H, dd, J = 5.4, 3.4 Hz), 7.41 (2H, d, J = 8.8 Hz).

### Reference Example 132

### 4-(4-(4-Methoxyphenyl)-5-(4-tolyl)thiazol-2-yl)piperidine

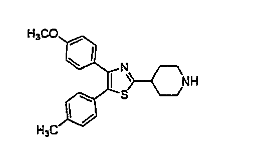

A title compound (247 mg, 0.677 mmol, 84%) was obtained as a yellow oil product from 2-bromo-1-(4-methoxyphenyl)-2-(4-tolyl)ethanone (Reference Example 20) (258 mg, 0.808 mmol) and 1-tert-butoxycarbonyl-4-thiocarbamoylpiperidine (198 mg, 0.811) in the same manner as in Reference Example 131.

¹H-NMR (400 MHz, CDCl₃) δ: 1.70-1.82 (2H, m), 2.12-2.20 (2H, m), 2.35 (3H, s), 2.74-2.81 (2H, m), 3.10-3.25 (3H, m), 3.80 (6H, s), 6.81 (2H, d, J = 8.8 Hz), 7.11 (2H, d, J = 8.0 Hz), 7.22 (2H, d, J = 8.0 Hz), 7.45 (2H, d, J = 8.8 Hz).

### Reference Example 133

### Benzyl 1-tert-butoxycarbonyl-4-thiocarbamoyl-4-piperidinecarboxylate

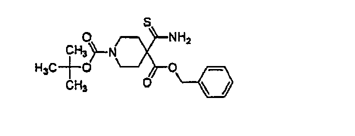

To a solution of benzyl 1-tert-butoxycarbonyl-4-carbamoyl-4-piperidinecarboxylate (Reference Example 120) (1.34 g, 3.69 mmol) in toluene (15 ml) was added a Lawesson's reagent (772 mg, 1.91 mmol), the mixture was stirred at 100°C for 4 hours, and the resultant was then cooled to room temperature. Distilled water was added to the reaction solution, followed by extraction with ethyl acetate. The organic layer was dried over magnesium sulfate and then concentrated under a reduced pressure. The residue was purified by flash chromatography (silica gel, n-hexane/ethyl acetate) to give a title compound (277 mg, 0.732 mmol, 20%) as a yellow amorphous product.

¹H-NMR (400 MHz, CDCl₃) δ: 1.44 (9H, s), 1.99-2.09 (2H, m), 2.32-2.39 (2H, m), 2.73-2.96 (2H, m), 3.87-4.08 (2H, m), 5.22 (2H, s), 7.23 (1H, brs), 7.31-7.41 (5H, m), 7.49 (1H, brs).

### Reference Example 134

### Benzyl 4-(4,5-bis(4-methoxyphenyl)thiazol-2-yl)-4-piperidinecarboxylate

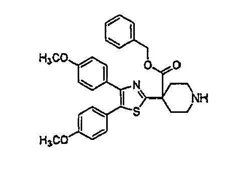

A title compound (311 mg, 0.604 mmol, 84%) was obtained as a white amorphous product from 1,2-bis(4-methoxyphenyl)-2-bromoethanone (Reference Example 14) (283 mg, 0.844 mmol) and benzyl 1-tert-butoxycarbonyl-4-thiocarbamoyl-4-piperidinecarboxylate (Reference Example 133) (273 mg, 0.721 mmol) in the same manner as in Reference Example 131.

¹H-NMR (400 MHz, CDCl₃) δ: 2.17-2.29 (2H, m), 2.48-2.57 (2H, m), 2.81-2.91 (2H, m), 2.98-3.08 (2H, m), 3.80 (3H, s), 3.83 (3H, s), 5.22 (2H, s), 6.80 (2H, d, J = 8.8 Hz), 6.85 (2H, d, J = 8.8 Hz), 7.23-7.32 (7H, m), 7.44 (2H, d, J = 8.8 Hz).

### Reference Example 135

### 1-tert-Butoxycarbonyl-4-methylpiperidine-4-carboxamide

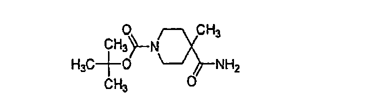

A title compound (277 mg, 1.14 mmol, 92%) was obtained as a white solid from 1-tert-butoxycarbonyl-4-methyl-4-piperidinecarboxylic acid (Reference Example 107) (301 mg, 1.24 mmol) in the same manner as in Reference Example 120.

¹H-NMR (400 MHz, CDCl₃) δ: 1.24 (3H, s), 1.45 (9H, s), 1.67-1.88 (2H, brs), 1.89-1.99 (2H, m), 3.22-3.32 (2H, m), 3.52-3.69 (2H, m), 5.44-5.67 (2H, brs).

### Reference Example 136

### 1-tert-Butoxycarbonyl-4-methylpiperidine-4-thiocarboxamide

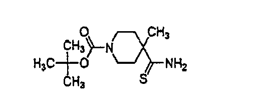

To a solution of 1-tert-butoxycarbonyl-4-methylpiperidine-4-carboxamide (Reference Example 135) (277 mg, 1.14 mmol) in 1,4-dioxane (10 ml) was added a Lawesson's reagent (257 mg, 0.635 mmol), and the mixture was stirred at room temperature for 15 hours. The reaction solution was concentrated under a reduced pressure, and the residue was purified by flash chromatography (silica gel, n-hexane/ethyl acetate) to give a title compound (107 mg, 0.414 mmol, 36%) as a white solid.

¹H-NMR (400 MHz, CDCl₃) δ: 1.35 (3H, s), 1.45 (9H, s), 1.59-1.69 (2H, m), 2.06-2.21 (2H, m), 3.45-3.48 (4H, m), 6.99 (1H, brs), 7.63 (1H, brs).

### Reference Example 137

### 4-(4,5-Bis(4-methoxyphenyl)thiazol-2-yl)-4-methylpiperidine

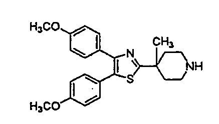

A title compound (158 mg, 0.402 mmol, quant.) was obtained as a light yellow amorphous product from 1-tert-butoxycarbonyl-4-methylpiperidine-4-thiocarboxamide (Reference Example 136) (104 mg, 0.402 mmol) and 1,2-bis(4-methoxyphenyl)-2-bromoethanone (Reference Example 14) (137mg, 0.408 mmol) in the same manner as in Reference Example 131.

¹H-NMR (400 MHz, CDCl₃) δ:1.68-1.87 (2H, m), 2.22-2.34 (2H, m), 2.97-3.00 (4H, m), 3.80 (3H, s), 3.82 (3H, s), 6.81 (2H, d, J = 8.8 Hz), 6.85 (2H, d, J = 8.8 Hz), 7.27 (2H, d, J = 8.8 Hz), 7.47 (2H, d, J = 8.8 Hz).

ESI-MS: m/z = 395 (M+H⁺).

### Reference Example 138

### Benzyl 1-tert-butoxycarbonyl-4-cyano-4-piperidinecarboxylate

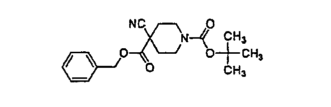

A title compound (599 mg, 1.74 mmol, 86%) was obtained as a colorless oil product from benzyl 1-tert-butoxycarbonyl-4-carbamoyl-4-piperidinecarboxylate (Reference Example 120) (735 mg, 2.03 mmol) in the same manner as in Reference Example 125.

¹H-NMR (400 MHz, CDCl₃) δ: 1.45 (9H, s), 1.90-2.00 (2H, m), 2.02-2.11 (2H, m), 3.01-3.20 (2H, m), 3.99-4.23 (2H, m), 5.26 (2H, s), 7.32-7.43 (5H, m).

### Reference Example 139

### 1-tert-Butoxycarbonyl-4-cyano-4-piperidinecarboxylic acid

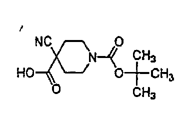

A title compound (429 mg, 1.68 mmol, 97%) was obtained as a white amorphous product from benzyl 1-tert-butoxy-4-cyano-4-piperidinecarboxylate (Reference Example 138) (595 mg, 1.73 mmol) in the same manner as in Reference Example 107.

¹H-NMR (400 MHz, CDCl₃) δ:1.47 (9H, s), 1.91-2.02 (2H, m), 2.03-2.12 (2H, m), 3.02-3.17 (2H, m), 4.02-4.21 (2H, m).

ESI-MS: m/z = 253 (M-1).

### Reference Example 140

### 1-tert-Butoxycarbonyl-4-cyano-4-piperidinecarboxamide

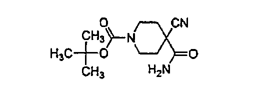

To a solution of 1-tert-butoxycarbonyl-4-cyano-4-piperidinecarboxylic acid (Reference Example 139) (424 mg, 1.67 mmol) in dichloromethane (8.0 ml) was added triethylamine (275 µl, 2.01 mmol) and isobutyl chloroformate (0.25 ml, 1.9 mmol) at 0°C, and the resultant was stirred at the same temperature for 1 hour. 28% aqueous ammonia (1.0 ml) was added to the reaction solution, and the resultant was stirred at room temperature for 3 hours. Water was added to the reaction solution then the solution was separated. The organic layer was dried and then concentrated under a reduced pressure. The residue was purified by column chromatography (silica gel, n-hexane/ethyl acetate) to give a title compound (266 mg, 1.05 mmol, 63%) as a white amorphous product.

¹H-NMR (400 MHz, CDCl₃) δ: 1.46 (3H, s), 1.91-1.99 (2H, m), 2.02-2.12 (2H, m), 2.92-3.15 (2H, m), 4.08-4.37 (2H, m), 5.67 (1H, brs), 6.30 (1H, brs).

ESI-MS: m/z = 252 (M-1).

### Reference Example 141

### 1-tert-Butoxycarbonyl-4-cyano-4-piperidinethiocarboxamide

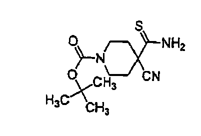

A title compound (191 mg, 0.709 mmol, 71%) was obtained as a white solid from 1-tert-butoxycarbonyl-4-cyano-4-piperidinecarboxamide (Reference Example 140) (266 mg, 1.05 mmol) in the same manner as in Reference Example 136.

¹H-NMR (400 MHz, CDCl₃) δ: 1.47 (9H, s), 1.87-1.99 (2H, m), 2.27-2.41 (2H, m), 2.92-3.16 (2H, m), 4.13-4.42 (2H, m), 7.61 (2H, brs).

### Reference Example 142

### 4-(4,5-Bis(4-methoxyphenyl)thiazol-2-yl)-4-cyanopiperidine

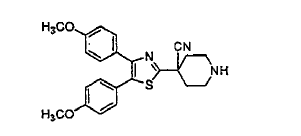

A title compound (269 mg, 0.663 mmol, 95%) was obtained as a white amorphous product from 1-tert-butoxycarbonyl-4-cyano-4-piperidinethiocarboxamide (Reference Example 141) (188 mg, 0.698 mmol) and 1,2-bis(4-methoxyphenyl)-2-bromoethanone (Reference Example 14) (249 mg, 0.742 mmol) in the same manner as in Reference Example 131.

¹H-NMR (400 MHz, CDCl₃) δ: 2.19-2.28 (2H, m), 2.36-2.42 (2H, m), 3.09-3.17 (2H, m), 3.19-3.26 (2H, m), 3.80 (3H, s), 3.83 (3H, s), 6.82 (2H, d, J = 8.8 Hz), 6.87 (2H, d, J = 8.8 Hz), 7.28 (2H, d, J = 8.8 Hz), 7.47 (2H, d, J = 8.8 Hz).

### Reference Example 143

### 4-(4,5-Bis(4-methoxyphenyl)oxazol-2-yl)-1-tert-butoxycarbonylpiperidine

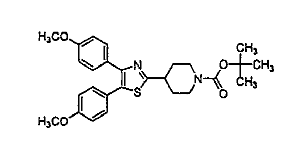

To a solution of 1,2-bis(4-methoxyphenyl)-2-bromoethanone (Reference Example 14) (1.05 g, 3.29 mmol) in methanol (10 ml) was added 1-tert-butoxycarbonylpiperidine-4-thiocarboxamide (804 mg, 3.29 mmol), and the mixture was stirred at room temperature for 2 hours. The reaction solution was concentrated under a reduced pressure, and an aqueous solution of 1M sodium hydroxide was added to the residue, followed by extraction with dichloromethane. The organic layer was dried over sodium sulfate and then concentrated under a reduced pressure. The residue was purified by flash chromatography on silica gel (n-hexane/ethyl acetate) to give a title compound (745 mg, 1.55 mmol, 47%) as a light yellow oil product.

¹H-NMR (400 MHz, CDCl₃) δ: 1.48 (9H, s), 1.72-1.83 (2H, m), 2.10-2.18 (2H, m), 2.85-2.95 (2H, m), 3.12-3.21 (1H, s), 3.80 (3H, s), 3.82 (3H, s), 4.16-4.27 (2H, m), 6.81 (2H, d, J = 8.8 Hz), 6.85 (2H, d, J = 8.8 Hz), 7.25 (2H, d, J = 8.8 Hz), 7.44 (2H, d, J = 8.8 Hz).

### Reference Example 144

### N-Benzylidene-4-toluenesulfonamide

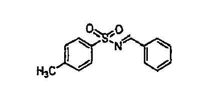

To a solution of p-toluenesulfonamide (8.05 g, 47.0 mmol) in toluene (100 ml), molecular sieves (4A, 8.0 g) was added Amberlyst (150 mg), and benzaldehyde (5.00 g, 47.1 mmol), and the mixture was heated under reflux for 21 hours with azeotropic dehydration. The reaction solution was cooled to room temperature, and the precipitate was filtered. The filtrate was concentrated under a reduced pressure, n-hexane was added to the residue, and the resulting slurry was filtered and washed to give a title compound (8.74 g, 33.7 mmol, 72%) as a white powder.

¹H-NMR (400 MHz, CDCl₃) δ: 2.44 (3H, s), 7.35 (2H, d, J = 8.0 Hz), 7.49 (2H, d, J = 8.0 Hz), 7.62 (1H, t, J = 7.6 Hz), 7.88-7.94 (4H, m), 9.04 (1H, s).

ESI-MS: m/z = 260 (M+H⁺).

### Reference Example 145

### 3-Phenyl-2-(4-toluenesulfonyl)-1,2-oxaziridine

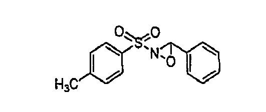

To a mixed solution of saturated aqueous sodium bicarbonate (12 ml) and chloroform (10 ml), benzyltriethylammonium chloride (290 mg, 1.27 mmol) and N-benzylidene-4-toluenesulfonamide (Reference Example 144) (3.00 g, 11.6 mmol) were added, and a solution of m-chloroperbenzoic acid (2.58 g, 12.7 mmol) in chloroform (24 ml) was added dropwise thereto at 0°C with stirring. Thereafter, the resultant was stirred at the same temperature for 2 hours, and the mixture was gradually heated to room temperature. The reaction solution was extracted with chloroform, the organic layer was washed with saturated aqueous sodium bicarbonate, an aqueous solution of 5% sodium bisulfite, and brine, dried (over potassium carbonate), and then concentrated under a reduced pressure. Hexane was added to the residue, the resulting slurry was filtered and washed, and a title compound (3.03 g,11.0 mmol, 95%) was obtained as a white powder.

¹H-NMR (400 MHz, CDCl₃) δ: 2.50 (3H, s), 5.45 (1H, s), 7.40-7.47 (7H, m), 7.93 (2H, d, J = 8.4 Hz).

ESI-MS: m/z = 276 (M+H⁺).

### Reference Example 146

### 4-(4,5-Bis(4-methoxyphenyl)oxazol-2-yl)-1-tert-butoxycarbonyl-4-hydroxypiperidine

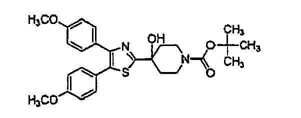

To a solution of 4-(4,5-bis(4-methoxyphenyl)oxazol-2-yl)-1-tert-butoxycarbonylpiperidine (Reference Example 143) (1.51 g, 3.14mmol) in tetrahydrofuran (15 ml) was added a solution of 2.6M n-butyllithium/n-hexane (1.33 ml) at -78°C, and the mixture was stirred at the same temperature for 1 hour. 3-Phenyl-2-(4-toluenesulfonyl)-1,2-oxaziridine (Reference Example 145) (1.26 g, 3.46 mmol) was added to the reaction solution while stirring the solution, and the mixture was gradually heated to room temperature. 0.1M hydrochloric acid was added to the reaction solution, followed by extraction with ether. The organic layer was washed with brine, dried over magnesium sulfate, and then concentrated under a reduced pressure. The residue was purified by flash chromatography (silica gel, n-hexane/ethyl acetate) to give a title compound (1.25 g, 2.51 mmol, 80%) as a white amorphous product.

¹H-NMR (400 MHz, CDCl₃) δ: 1.67 (9H, s), 1.87-96 (2H, m), 2.08-2.19 (2H, m), 3.26-3.37 (3H, m), 3.80 (3H, s), 3.82 (3H, s), 3.94-4.14 (2H, m), 6.81 (2H, d, J = 8.8 Hz), 6.85 (2H, d, J = 8.8 Hz), 7.25 (2H, d, J = 8.8 Hz), 7.45 (2H, d, J = 8.8 Hz).

ESI-MS: m/z = 497 (M+H⁺).

### Reference Example 147

### 4-(4,5-Bis(4-methoxyphenyl)thiazol-2-yl)-4-hydroxypiperidine

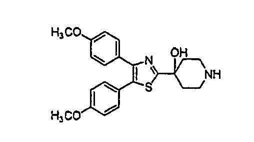

A title compound (148 mg, 0.37 mmol, 91%) was obtained as a light yellow amorphous product from 1-tert-butoxycarbonyl-4-(4,5-bis(4-methoxyphenyl)thiazol-2-yl)-4-hydroxypiperidine (Reference Example 146) (204 mg, 0.41 mmol) in the same manner as in Reference Example 86.

¹H-NMR (400 MHz, CDCl₃) δ: 1.90-1.95 (2H, m), 2.10-2.19 (2H, m), 3.00-3.07 (2H, m), 3.09-3.18 (2H, m), 3.80 (3H, s), 3.83 (3H, s), 6.82 (2H, d, J = 8.8 Hz), 6.86 82H, d, J = 8.8 Hz), 7.27 (2H, d, J = 8.8 Hz), 7.46 (2H, d, J = 8.8 Hz).

### Reference Example 148

### 1-tert-Butoxycarbonyl-4-(4,5-bis(4-methoxyphenyl)thiazol-2-yl)-4-methoxypiperidine

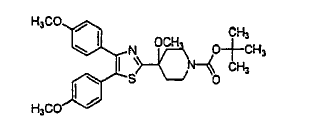

To a solution of 1-tert-butoxycarbonyl-4-(4,5-bis(4-methoxyphenyl)thiazol-2-yl)-4-hydroxypiperidine (Reference Example 146) (250 mg, 0.50 mmol) in N,N-dimethylformamide (2.5 ml) was added 60% sodium hydride (60 mg, 1.50 mmol), the mixture was stirred at room temperature for 10 minutes, methyl iodide (429 mg, 3.0 mmol) was added, and the mixture was stirred at room temperature for 13 hours. 1M hydrochloric acid was added to the reaction solution, followed by extraction with ethyl acetate. The organic layer was washed with brine, dried over sodium sulfate, and then concentrated under a reduced pressure. The residue was purified by flash chromatography (silica gel, n-hexane/ethyl acetate) to give a title compound (108 mg, 0.21 mmol, 42%) as a light yellow amorphous product.

¹H-NMR (400 MHz, CDCl₃) δ: 1.46(9H, s), 2.08-2.11(2H, m), 2.17-2.24(2H, m), 3.27(3H, s), 3.29-3.36(2H, m), 3.80(3H, s), 3.82(3H, s), 6.82(2H, d, J = 8.8 Hz), 6.87(2H, d, J = 8.8 Hz), 7.26(2H, d, J = 8.8 Hz), 7.45(2H, d, J = 8.8 Hz).

### Reference Example 149

### 4-(4,5-Bis(4-methoxyphenyl)thiazol-2-yl)-4-methoxypiperidine

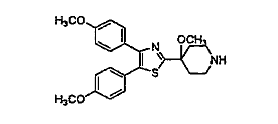

A title compound (43 mg, 0.11 mmol, 60%) was obtained as a light yellow amorphous product from 1-tert-butoxycarbonyl-4-(4,5-bis(4-methoxyphenyl)thiazol-2-yl)-4-methoxypiperidine (Reference Example 148) (90 mg, 0.18 mmol) in the same manner as in Reference Example 86.

¹H-NMR (400 MHz, CDCl₃) δ: 2.06-2.09(2H, m), 2.18-2.25(2H, m), 2.94-3.12(4H, m), 3.27(3H, s), 3.80(3H, s), 3.82(3H, s), 6.82(2H, d, J = 8.8 Hz), 6.86(2H, d, J = 8.8 Hz), 7.27(2H, d, J = 8.8 Hz), 7.46(2H, d, J = 8.8 Hz).

### Reference Example 150

### 1-tert-Butoxycarbonyl-4-thiocarbamoylpiperazine

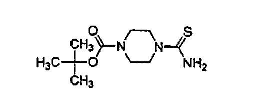

To a solution of 1-tert-butoxycarbonylpiperazine (294 mg, 1.57 mmol) in dichloromethane (5.0 ml) was added pyridine and thiophosgene (120 µl, 1.57 mmol) at 0°C, and the mixture was stirred at the same temperature for 1 hour. 28% aqueous ammonia was added to the reaction solution at 0°C, the mixture was stirred at room temperature for 2 hours, and the reaction solution was concentrated under a reduced pressure. Distilled water was added to the residue, followed by extraction with ethyl acetate. The organic layer was dried over magnesium sulfate and then concentrated under a reduced pressure. The residue was purified by flash chromatography (silica gel, n-hexane/ethyl acetate) to give a title compound (88 mg, 0.35 mmol, 22%) as a light brown solid.

¹H-NMR (400 MHz, CDCl₃) δ: 1.47 (9H, s), 3.50-3.59 (4H, m), 3.74-3.95 (4H, m), 5.95 (2H, brs).

### Reference Example 151

### 1-tert-Butoxycarbonyl-4-(4,5-bis(4-methoxyphenyl)thiazol-2-yl)piperazine

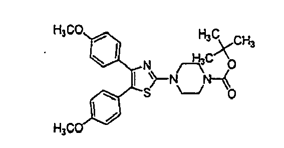

To a solution of 1-tert-butoxycarbonyl-4-thiocarbamoylpiperazine (Reference Example 150) (88mg, 0.35 mmol) in acetonitrile (20 ml) was added 1,2-bis(4-methoxyphenyl)-2-bromo-1-ethanone (Reference Example 14) (117 mg, mmol), and the mixture was stirred at room temperature for 2 hours. The reaction solution was concentrated under a reduced pressure, and the residue was purified by chromatography (silica gel, n-hexane/ethyl acetate) to give a title compound (81 mg, 0.17 mmol, 48%) as a white solid.

¹H-NMR (400 MHz, CDCl₃) δ: 1.49 (9H, s), 3.47-3.61 (8H, m), 3.79 (3H, s), 3.81 (3H, s), 6.78 (2H, d, J = 8.8 Hz), 6.82 (2H, d, J = 8.8 Hz), 7.21 (2H, d, J = 8.8 Hz), 7.44 (2H, d, J = 8.8 Hz).

### Reference Example 152

### 4-(4,5-Bis(4-methoxyphenyl)thiazol-2-yl)piperazine

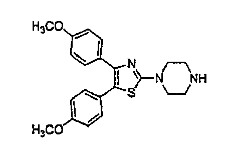

A title compound (64 mg, 0.17 mmol, quant.) was obtained as a light yellow amorphous product from 1-tert-butoxycarbonyl-4-(4,5-bis(4-methoxyphenyl)thiazol-2-yl)piperazine (Reference Example 151) (81 mg, 0.17 mmol) in the same manner as in Reference Example 86.

¹H-NMR (400 MHz, CDCl₃) δ: 3.04-3.11 (4H, m), 3.54-3.61 (4H, m), 3.79 (3H, s), 3.81 (3H, s), 6.78 (2H, d, J = 8.8 Hz), 6.82 (2H, d, J = 8.8 Hz), 7.21 (2H, d, J = 8.8 Hz), 7.21 (2H, d, J = 8.8 Hz), 7.43 (2H, d, J = 8.8 Hz).

ESI-MS: m/z = 383 (M+H⁺).

### Reference Example 153

### 2-Methyl-4-(2-nitrovinyl)anisole

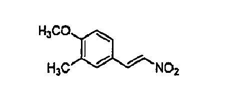

To a solution of 4-methoxy-3-methylbenzaldehyde (1.50 g, 10.0 mmol) in methanol (3.5 ml), nitromethane (0.6 ml, 11.2 mmol) was added ethylenediamine diacetate (182 mg, 1.01 mmol), and the mixture was stirred at room temperature for 24 hours. Ethanol (5.0 ml) was added to the reaction solution, and the mixture was stirred at 0°C for 30 minutes, followed by filtration. The resulting powder was washed with cooled ethanol to give a title compound (1.04 g, 5.38 mmol, 54%) as a yellow powder.

¹H-NMR (400 MHz, CDCl₃) δ: 2.24 (3H, s), 3.89 (3H, s), 6.87 (1H, d, J = 8.5 Hz), 7.35 (1H, d, J = 2.2 Hz), 7.39 (1H, dd, J = 2.2, 8.5 Hz), 7.52 (1H, d, J = 13.4 Hz), 7.96 (1H, d, J = 13.4 Hz).

### Reference Example 154

### 2-Fluoro-4-(2-nitrovinyl)anisole

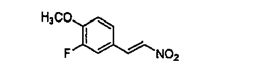

A title compound (1.16 g, 5.88 mmol, 59%) was obtained as a yellow powder from 3-fluoro-4-methoxybenzaldehyde (1.54 g, 10.0 mmol) in the same manner as in Reference Example 153.

¹H-NMR (400 MHz, CDCl₃) δ: 3.96 (3H, s), 7.02 (1H, t, J = 8.5 Hz), 7.28 (1H, d, J = 2.0 Hz), 7.32(1H, dd, J = 2.0, 8.5 Hz), 7.49 (1H, d, J = 13.6 Hz), 7.93 (1H, d, J = 13.6 Hz).

### Reference Example 155

### 1,2-Dimethoxy-4-(2-nitrovinyl)benzene

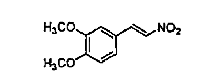

A title compound (1.94 g, 9.27 mmol, 93%) was obtained as a yellow powder from 3,4-dimethoxybenzaldehyde (1.66 g, 10.0 mmol) in the same manner as in Reference Example 153.

¹H-NMR (400 MHz, CDCl₃) δ: 3.93 (3H, s), 3.95 (3H, s), 6.92 (1H, d, J = 8.3 Hz), 7.01 (1H, d, J = 2.0 Hz), 7.18 (1H, dd, J = 2.0, 8.3 Hz), 7.54 (1H, d, J = 13.6 Hz), 7.97 (1H, d, J = 13.6 Hz).

### Reference Example 156

### 1,3-Dimethoxy-4-(2-nitrovinyl)benzene

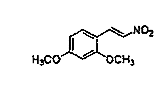

A title compound (1.92 g, 9.18 mmol, 92%) was obtained as a yellow powder from 2,4-dimethoxybenzaldehyde (1.66 g, 10.0 mmol) in the same manner as in Reference Example 153.

¹H-NMR (400 MHz, CDCl₃) δ: 3.87 (3H, s), 3.93 (3H, s), 6.49 (1H, d, J = 2.4 Hz), 6.56 (1H, dd, J = 2.4, 8.8 Hz), 7.39 (1H, d, J = 8.8 Hz), 7.84 (1H, d, J = 13.4 Hz), 8.09 (1H, d, J = 13.4 Hz).

### Reference Example 157

### 2-Methoxy-5-(2-nitrovinyl)pyridine

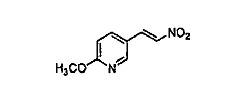

A title compound (717mg, 4.0mmol, 79%) was obtained as a yellow powder from 2-methoxy-5-pyridinecarbaldehyde (690mg, 5.0mmol) in the same manner as in Reference Example 153.

¹H-NMR (400 MHz, CDCl₃) δ: 4.0 (3H, s), 6.83 (1H, d, J = 8.8 Hz), 7.53 (1H, d, J = 13.6 Hz), 7.75 (1H, dd, J = 2.2, 8.8 Hz), 7.98 (1H, d, J = 13.6 Hz), 8.35 (1H, d, J = 2.2 Hz).

### Reference Example 158

### 2-Methoxy-3-(2-nitrovinyl)pyridine

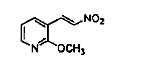

A title compound (478 mg, 2.65 mmol, 56%) was obtained as a yellow powder from 2-methoxy-3-pyridinecarbaldehyde (478 mg, 4.75 mmol) in the same manner as in Reference Example 153.

¹H-NMR (400 MHz, CDCl₃) δ: 4.10 (3H, s), 7.01 (1H, dd, J = 5.1, 7.5 Hz), 7.75 (1H, dd, J = 1.9, 7.5 Hz), 7.94 (1H, d, J = 13.6 Hz), 8.00 (1H, d, J = 13.6 Hz), 8.27 (1H, dd, J = 1.9, 5.1 Hz).

### Reference Example 159

### 1-tert-Butoxycarbonyl-4-(5-(3-fluoro-4-methoxyphenyl)-1-(4-methoxyphenyl)-1H-pyrazol-3-yl)piperidine

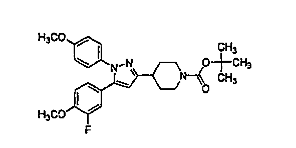

To a solution of 1-tert-butoxycarbonylpiperidinecarbaldehyde (Reference Example 67) (320 mg, 1.50 mmol) in methanol (6.0 ml) was added 4-methoxyphenylhydrazine (208 mg, 1.50 mmol), the mixture was stirred at room temperature for 1 hour, 2-fluoro-4-(2-nitrovinyl)anisole (Reference Example 154) (267 mg, 1.35 mmol) was added thereto, and the resultant was stirred under an air atmosphere for 24 hours. The reaction solution was concentrated under a reduced pressure, and the residue was purified by flash chromatography (silica gel, n-hexane/ethyl acetate) to give a title compound (367 mg, 0.762 mmol, 56%) as a yellow amorphous product.

¹H-NMR (400 MHz, CDCl₃) δ: 1.47 (9H, s), 1.62-1.75 (2H, m), 1.96-2.04 (2H, m), 2.80-2.93 (3H, m), 3.81 (3H, s), 3.88 (3H, s), 4.08-4.29 (2H, m), 6.25 (1H, s), 6.83-6.96 (5H, m), 7.19 (2H, d, J = 8.8 Hz).

### Reference Example 160

### 1-tert-Butoxycarbonyl-4-(1-(4-methoxyphenyl)-5-(4-methoxy-3-tolyl)-1H-pyrazol-3-yl)piperidine

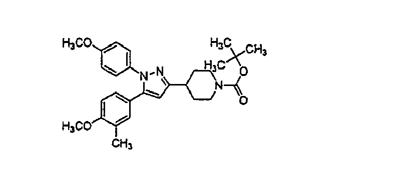

A title compound (449 mg, 0.940 mmol, 69%) was obtained as a yellow amorphous product from 4-methoxyphenylhydrazine (208 mg, 1.50 mmol), 1-tert-butoxycarbonylpiperidinecarbaldehyde (Reference Example 67) (320 mg, 1.50 mmol), and 2-methyl-4-(2-nitrovinyl)anisole (Reference Example 153) (261 mg, 1.35 mmol) in the same manner as in Reference Example 159.

¹H-NMR (400 MHz, CDCl₃) δ: 1.47 (9H, s), 1.62-1.75 (2H, m), 1.97-2.04 (2H, m), 2.15 (3H, s), 2.80-2.94 (3H, m), 3.80 (3H, s), 3.81 (3H, s), 4.09-4.27 (2H, m), 6.22 (1H, s), 6.70 (1H, d, J = 8.3 Hz), 6.84 (2H, d, J = 8.8 Hz), 6.93 (1H, dd, J = 2.2, 8.3 Hz), 7.04 (1H, d, J = 2.2 Hz), 7.20 (2H, d, J = 8.8 Hz).

### Reference Example 161

### 1-tert-Butoxycarbonyl-4-(5-(3,4-dimethoxyphenyl)-1-(4-methoxyphenyl)-1H-pyrazol-3-yl)piperidine

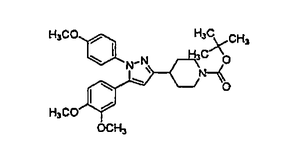

A title compound (320 mg, 0.648 mmol, 48%) was obtained as a yellow amorphous product from 4-methoxyphenylhydrazine (208 mg, 1.50 mmol), 1-tert-butoxycarbonylpiperidinecarbaldehyde (Reference Example 67) (320 mg, 1.50 mmol), and 1,2-dimethoxy-4-(2-nitrovinyl)benzene (Reference Example 155) (282 mg, 1.35 mmol) in the same manner as in Reference Example 159.

¹H-NMR (400 MHz, CDCl₃) δ: 1.47 (9H, s), 1.64 (2H, m), 1.98 (2H, m), 2.81-2.95 (3H, m), 3.66 (3H, s), 3.80 (3H, s), 3.87 (3H, s), 4.10-4.28 (2H, m), 6.26 (1H, s), 6.66(1H, d, J = 1.5 Hz), 6.79-6.81 (2H, m), 6.85 (2H, d, J = 8.8 Hz), 7.21 (2H, d, J = 8.8 Hz).

### Reference Example 162

### 1-tert-Butoxycarbonyl-4-(5-(2,4-dimethoxyphenyl)-1-(4-methoxyphenyl)-1H-pyrazol-3-yl)piperidine

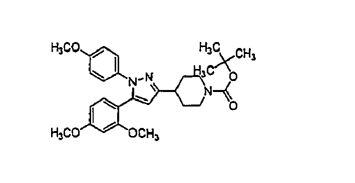

A title compound (304 mg, 0.616 mmol, 45%) was obtained as a yellow amorphous product from 4-methoxyphenylhydrazine (208 mg, 1.50 mmol), 1-tert-butoxycarbonylpiperidinecarbaldehyde (Reference Example 67) (320 mg, 1.50 mmol), and 1,3-dimethoxy-4-(2-nitrovinyl)benzene (Reference Example 156) (282 mg, 1.35 mmol) in the same manner as in Reference Example 159.

¹H-NMR (400 MHz, CDCl₃) δ:1.47 (9H, s), 1.64-1.76 (2H, m), 1.99-2.07 (2H, m), 2.80-2.94 (3H, m), 3.42 (3H, s), 3.77 (3H, s), 3.81 (3H, s), 4.08-4.25 (2H, m), 6.21 (1H, s), 6.36 (1H, d, J = 2.4 Hz), 6.47 (1H, dd, J = 2.4, 8.5 Hz), 6.78 (2H, d, J = 8.8 Hz), 7.13 (1H, d, J = 8.5 Hz), 7.16 (2H, d, J = 8.8 Hz).

### Reference Example 163

### 4-(1-(4-Methoxyphenyl)-5-(2-methoxypyridin-5-yl)-1H-pyrazol-3-yl)piperidine

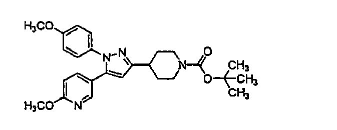

A title compound (380 mg, 0.82 mmol, 60%) was obtained as a yellow amorphous product from 4-methoxyphenylhydrazine (208 mg, 1.5 mmol), 1-tert-butoxycarbonylpiperidinecarbaldehyde (Reference Example 67) (319 mg, 1.5 mmol), and 2-methoxy-5-(2-nitrovinyl)pyridine (Reference Example 157) (245 mg, 1.4 mmol) in the same manner as in Reference Example 159.

¹H-NMR (400 MHz, CDCl₃) δ:1.48 (9H, s), 1.63-1.76 (2H, m), 1.97-2.06 (2H, m), 2.80-2.94 (3H, m), 3.81 (3H, s), 3.93 (3H, s), 4.14-4.27 (2H, m), 6.27 (1H, s), 6.64 (1H, d, J = 8.8 Hz), 6.86 (2H, d, J = 8.8 Hz), 7.19 (2H, d, J = 8.8 Hz), 7.32 (1H, dd, J = 2.2, 8.8 Hz), 8.08 (1H, d, J = 2.2 Hz).

### Reference Example 164

### 1-tert-Butoxycarbonyl-4-(1-(4-methoxyphenyl)-5-(2-methoxypyridin-3-yl)-1H-pyrazol-3-yl)piperidine

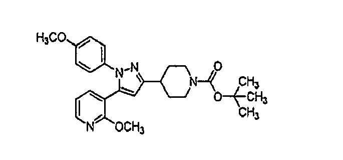

A title compound (207 mg, 0.44 mmol, 44%) was obtained as an orange oil product from 4-methoxyphenylhydrazine (174 mg, 1.00 mmol), 1-tert-butoxycarbonylpiperidinecarbaldehyde (Reference Example 67) (213 mg, 1.00 mmol), and 2-methoxy-3-(2-nitrovinyl)pyridine (Reference Example 158) (162 mg, 0.90 mmol).

¹H-NMR (400 MHz, CDCl₃) δ: 1.47 (9H, s), 1.65-1.77 (2H, m), 2.00-2.07 (2H, m), 2.79-2.98 (3H, m), 3.68 (3H, s), 3.78 (3H, s), 4.09-4.28 (2H, m), 6.33 (1H, s), 6.80 (2H, d, J = 8.8 Hz), 6.86 (1H, dd, J = 4.9, 7.3 Hz), 7.14 (2H, d, J = 8.8 Hz), 7.44 (1H, dd, J = 1.9, 7.3 Hz), 8.13 (1H, dd, J = 1.9, 4.9 Hz).

### Reference Example 165

### 4-(1-(4-Methoxyphenyl)-5-(4-methoxy-3-tolyl)-1H-pyrazol-3-yl)piperidine

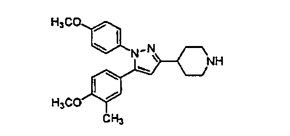

A title compound (286 mg, 0.762 mmol, quant.) was obtained as a light brown solid from 1-tert-butoxycarbonyl-4-(1-(4-methoxyphenyl)-5-(4-methoxy-3-tolyl)-1H-pyrazol-3-yl)piperidine (Reference Example 160) (363 mg, 0.760 mmol) in the same manner as in Reference Example 86.

¹H-NMR (400 MHz, CDCl₃) δ:1.81-1.93 (2H, m), 2.08-2.16 (2H, m), 2.15 (3H, s), 2.83-2.99 (5H, m), 3.27-3.35 (2H, m), 3.81 (3H, s), 3.81 (3H, s), 6.25 (2H, s), 6.70 (1H, d, J = 8.3 Hz), 6.84 (2H, d, J = 8.8 Hz), 6.94 (1H, dd, J = 2.2, 8.3 Hz), 7.04 (1H, d, J = 2.2 Hz), 7.20 (2H, d, J = 8.8 Hz).

### Reference Example 166

### 4-(5-(3-Fluoro-4-methoxyphenyl)-1-(4-methoxyphenyl)-1H-pyrazol-3-yl)piperidine

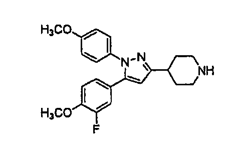

A title compound (286 mg, 0.752 mmol, quant.) was obtained as a light brown amorphous product from 1-tert-butoxycarbonyl-4-(5-(3-fluoro-4-methoxyphenyl)-1-(4-methoxyphenyl)-1H-pyrazol-3-yl)piperidine (Reference Example 159) (362 mg, 0.752 mmol) in the same manner as in Reference Example 86.

¹H-NMR (400 MHz, CDCl₃) δ: 1.69-1.81 (2H, m), 2.01-2.09 (2H, m), 2.76-2.84 (2H, m), 2.85-2.93 (1H, m), 3.19-3.27 (2H, m), 3.81 (3H, s), 3.88 (3H, s), 6.17 (1H, s), 6.83-6.96 (5H, m), 7.19 (2H, d, J = 8.8 Hz).

### Reference Example 167

### 4-(5-(2,4-Dimethoxyphenyl)-1-(4-methoxyphenyl)-1H-pyrazol-3-yl)piperidine

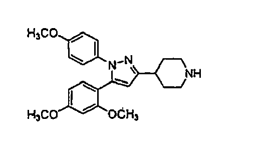

A title compound (159 mg, 0.404 mmol, 65%) was obtained as a light yellow solid from 1-tert-butoxycarbonyl-4-(5-(2,4-dimethoxyphenyl)-1-(4-methoxyphenyl)-1H-pyrazol-3-yl)piperidine (Reference Example 162) (304 mg, 0.616 mmol) in the same manner as in Reference Example 86.

¹H-NMR (400 MHz, CDCl₃) δ:1.63-1.77 (2H, m), 2.00-2.08 (2H, m), 2.71-2.80 (2H, m), 2.83-2.92 (1H, m), 3.13-3.21 (2H, m), 3.41 (3H, s), 3.77 (3H, s), 3.81 (3H, s), 6.23 (1H, s), 6.36 (1H, d, J = 2.2 Hz), 6.47 (1H, dd, J = 2.2, 8.3 Hz), 6.77 (2H, d, J = 8.8 Hz), 7.14 (1H, d, J = 8.3 Hz), 7.16 (2H, d, J = 8.8 Hz).

### Reference Example 168

### 4-(1-(4-Methoxyphenyl)-5-(2-methoxypyridin-5-yl)-1H-pyrazol-3-yl)piperidine

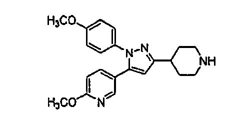

A title compound (117 mg, 0.32 mmol, 78%) was obtained as a light yellow amorphous product from 1-tert-butoxycarbonyl-4-(1-(4-methoxyphenyl)-5-(2-methoxypyridin-5-yl)-1H-pyrazol-3-yl)piperidine (Reference Example 163) (192 mg, 0.41 mmol) in accordance with the method of Reference Example 86.

¹H-NMR (400 MHz, CDCl₃) δ: 2.07-2.16(2H, m), 2.24-2.27(2H, m), 3.09-3.11(3H, m), 3.55-3.58(2H, m), 3.81(3H, s), 3.95(3H, s), 6.39(1H, s), 6.73(1H, d, J = 8.8 Hz), 6.90(2H, d, J = 8.8 Hz), 7.17(2H, d, J = 8.8 Hz), 7.39(1H, dd, J = 2.0, 8.8 Hz), 8.12(1H, m).

### Reference Example 169

### 4-(1-(4-Methoxyphenyl)-5-(2-methoxypyridin-3-yl)-1H-pyrazol-3-yl)piperidine

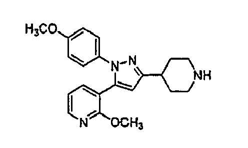

A title compound (157 mg, 0.43 mmol, 98%) was obtained as a brown amorphous product from 1-tert-butoxycarbonyl-4-(1-(4-methoxyphenyl)-5-(2-methoxypyridin-3-yl)-1H-pyrazol-3-yl)piperidine (Reference Example 164) (203 mg, 0.437 mmol) in the same manner as in Reference Example 86.

¹H-NMR (400 MHz, CDCl₃) δ:1.69-1.81 (2H, m), 2.03-2.11 (2H, m), 2.75-2.83 (2H, m), 2.86-2.96 (1H, m), 3.18-3.25 (2H, m), 3.67 (3H, s), 3.78 (3H, s), 6.36 (1H, s), 6.80 (2H, d, J = 8.8 Hz), 6.86 (1H, dd, J = 4.9, 7.3 Hz), 7.15 (2H, d, J = 8.8 Hz), 7.45 (1H, dd, J = 1.9, 7.3 Hz), 8.13 (1H, dd, J = 1.9, 4.9 Hz).

### Reference Example 170

### 8-Benzyl-8-azabicyclo[3.2.1]octan-3-one

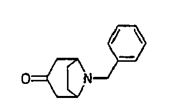

A solution of 2,5-dimethoxytetrahydrofuran (19.8 g, 149.8 mmol) in 0.1M hydrochloric acid (180 ml, 18.0 mmol) was stirred at 80°C for 2 hours. The reaction solution was cooled to 0°C, acetonedicarboxylic acid (19.8 g, 149.8 mmol), concentrated hydrochloric acid (13.8 ml, 164.8 mmol), sodium acetate (14.8 g, 179.8 mmol), and benzylamine (17.7 g, 164.8 mmol) were added to the reaction solution, and the mixture was stirred at the same temperature for 12 hours and at 55°C for 2 hours. The reaction solution was cooled to room temperature, and an aqueous solution of 4M sodium hydroxide (70 ml) was added, followed by extraction with chloroform. The organic layer was dried over sodium sulfate and then concentrated under a reduced pressure. The residue was purified by flash chromatography (silica gel, n-hexane/ethyl acetate) to give a title compound (23.3 g, 108 mmol, 72%) as a light yellow oil product.

¹H-NMR (400 MHz, CDCl₃) δ:1.61-1.66 (2H, m), 2.10-2.13 (2H, m), 2.19-2.23 (2H, m), 2.67-2.72 (2H, m), 3.50 (2H, m), 3.75 (2H, s), 7.26-7.43 (5H, m).

ESI-MS: m/z = 216 (M+H⁺).

### Reference Example 171

### 8-Benzyloxycarbonyl-8-azabicyclo[3.2.1]octan-3-one

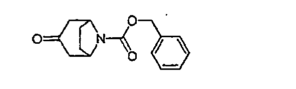

To a solution of 8-benzyl-8-azabicyclo[3.2.1]octan-3-one (Reference Example 170) (10.0 g, 46.4 mmol) in acetic acid (100 ml) was added ASCA-2 catalyst (2.0 g, hydrous product, NE Chemcat Corporation), and the mixture was stirred under a hydrogen atmosphere (1atm) at room temperature for 5 hours. The reaction solution was filtered and then concentrated under a reduced pressure. Tetrahydrofuran (100 ml) was added to dissolve the residue, an aqueous solution of sodium bicarbonate (100 ml) and benzyl chloroformate (6.94 ml, 48.8 mmol) were added thereto, and the resultant was stirred at room temperature for 1 hour. Distilled water was added to the reaction solution, followed by extraction with ethyl acetate. The organic layer was washed with brine, dried over magnesium sulfate, and then concentrated under a reduced pressure. Dichloromethane (100 ml) was added to dissolve the residue, acetic anhydride (4.38 ml, 46.4 mmol) and pyridine (3.37 ml, 46.4 mmol) were added thereto, and the resultant was stirred at room temperature for 1 hour. An aqueous solution of 1M hydrochloric acid was added to the reaction solution, followed by extraction with dichloromethane. The organic layer was washed with an aqueous solution of 1M hydrochloric acid and then with brine, dried over sodium sulfate, and then concentrated under a reduced pressure. The residue was purified by flash chromatography (silica gel, n-hexane) to give a title compound (10.6 g, 40.7 mmol, 88%) as a colorless oil product.

¹H-NMR (400 MHz, CDCl₃) δ: 1.66-1.72 (2H, m), 2.09-2.12 (2H, m), 2.34-2.38 (2H, m), 2.58-2.75 (2H, m), 4.59 (2H, m), 5.19 (2H, s), 7.33-7.38 (5H, m).

### Reference Example 172

### 8-Benzyloxycarbonyl-8-azabicyclo[3.2.1]octan-3-carbaldehyde

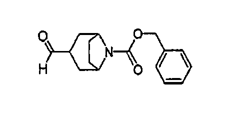

To a solution of (methoxymethyl)triphenylphosphonium chloride (7.93 g, 23.1 mmol) in tetrahydrofuran (38.5 ml) was added potassium tert-butoxide (2.60 g, 23.1 mmol) at -15°C, and the resultant was stirred at the same temperature for 30 minutes. A solution of 8-benzyloxycarbonyl-8-azabicyclo[3.2.1]octan-3-one (Reference Example 171) (5.00 g, 10.9 mmol) in tetrahydrofuran (38.5 ml) was added dropwise to the reaction solution at -15°C, and the mixture was then stirred at room temperature for 15 hours. An aqueous solution of 6M hydrochloric acid was added to the reaction solution, and the mixture was then stirred for 5 hours. Distilled water was added to the reaction solution, followed by extraction with ethyl acetate. The organic layer was washed with brine, dried over sodium sulfate, and then concentrated under a reduced pressure. The residue was purified by flash chromatography (silica gel, n-hexane/ethyl acetate) to give a title compound (2.23 g, 8.16 mmol, 42%) as a colorless oil product.

¹H-NMR (400 MHz, CDCl₃) δ:1.63-1.84 (6H, m), 2.01-2.08 (2H, m), 2.72-2.77 (1H, m), 4.41-4.44 (2H, m), 5.14 (2H, s), 7.29-7.37 (5H, m), 9.54 (1H, m).

ESI-MS: m/z = 274 (M+H⁺).

### Reference Example 173

### 8-Benzyloxycarbonyl-3-(1,5-bis(4-methoxyphenyl)-1H-pyrazol-3-yl)-8-azabicyclo[3.2.1]octane

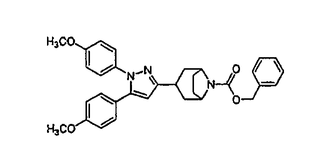

To a solution of 8-benzyloxycarbonyl-8-azabicyclo[3.2.1]octan-3-carbaldehyde (Reference Example 172) (2.20 g, 8.04 mmol) in methanol (38 ml) was added 4-methoxyphenylhydrazine (1.11 g, 8.04 mmol), and the resultant was stirred at room temperature for 1.5 hours. 4-(2-Nitrovinyl)anisole (1.30 g, 7.24 mmol) was added to the reaction solution, and the resultant was stirred under an air atmosphere for 20 hours. Distilled water was added to the reaction solution, followed by extraction with ethyl acetate. The organic layer was washed with distilled water and then with brine, dried over magnesium sulfate, and then concentrated under a reduced pressure. The residue was purified by flash chromatography (amine silica gel, n-hexane/ethyl acetate) to give a title compound (390 mg, 0.74 mmol, 9%) as a light yellow amorphous product.

¹H-NMR (400 MHz, CDCl₃) δ:1.83-2.05 (8H, m), 3.30-3.39 (1H, m), 3.80-3.81 (6H, m), 4.41-4.44 (2H, m), 5.17-5.18 (2H, m), 6.17 (1H, s), 6.82 (4H, dd, J = 10.0, 9.2 Hz), 7.11 (2H, d, J = 8.8 Hz), 7.18 (2H, d, J = 8.8 Hz).
ESI-MS: m/z = 524 (M+H⁺).

### Reference Example 174

### 3-(1,5-Bis(4-methoxyphenyl)-1H-pyrazol-3-yl)-8-azabicyclo[3.2.1]octane

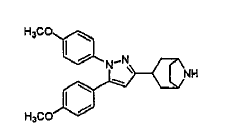

To a mixed solution of 8-benzyloxycarbonyl-3-(1,5-bis(4-methoxyphenyl)-1H-pyrazol-3-yl)-8-azabicyclo[3.2.1]octane (Reference Example 173) (380 mg, 0.73 mmol) in methanol (3.0 ml) and ethyl acetate (1.0 ml) was added ASCA-2 catalyst (152 mg, hydrous product, NE Chemcat Corporation), and the resultant was stirred under a hydrogen atmosphere for 2 hours. The reaction solution was filtered and then concentrated under a reduced pressure. The residue was purified by flash chromatography (amine silica gel, chloroform/methanol) to give a title compound (212 mg, 0.54 mmol, 75%) as a light yellow amorphous product.

¹H-NMR (400 MHz, CDCl₃) δ: 1.78-1.92 (8H, m), 3.16-3.25 (1H, m), 3.62 (2H, m), 3.79 (3H, s), 3.80 (3H, s), 6.30 (1H, s), 6.82 (4H, dd, J = 8.8, 11.2 Hz), 7.12 (2H, d, J = 8.8 Hz), 7.19 (2H, d, J = 8.8 Hz).

ESI-MS: m/z = 390 (M+H⁺).

### Reference Example 175

### 1-Benzyloxycarbonyl-4-(3-(4-tolyl)-1,3-dioxopropyl)piperidine

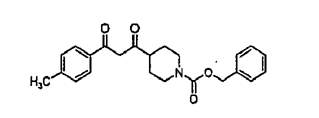

To a solution of 1-(benzyloxycarbonyl)piperidine-4-carboxylic acid (Reference Example 27) (2.08 g, 7.9 mmol) in dichloromethane (10 ml) was added oxalyl chloride (0.83 ml, 9.5 mmol) and N,N-dimethylformamide (two drops with a Pasteur pipette) at 0°C, and the resultant was stirred at room temperature for 2 hours. The reaction solution was concentrated under a reduced pressure, and tetrahydrofuran (20 ml) was added to dissolve the residue to give an acid chloride solution.

Tetrahydrofuran (80 ml) was introduced into the other reaction vessel, and 0.5M potassium (bistrimethylsilyl)amide (23.7 ml, 12 mmol) and 4-methylacetophenone (1.59 g, 12 mmol) were added at -78°C. After the mixture was stirred at the same temperature for 1 hour, the acid chloride solution that had been prepared in advance was added thereto, and the resultant was stirred at the same temperature for 1 hour. The saturated aqueous ammonium chloride solution was added to the reaction solution, followed by extraction with ethyl acetate. The organic layer was dried over sodium sulfate and then concentrated under a reduced pressure. The residue was purified via flash chromatography (silica gel, n-hexane/ethyl acetate) to give a title compound (2.54 g, 6.7 mmol, 85%) as a light yellow oil product.

¹H-NMR (400 MHz, CDCl₃) δ: 1.62-1.98 (4H, m), 2.41 (3H, s), 2.67-2.75 (1H, m), 2.78-2.96 (2H, m), 4.18-38 (2H, m), 5.14 (2H, s), 6.15 (1H, s), 7.25-7.39 (7H, m), 7.78 (2H, d, J = 8.5 Hz).

### Reference Example 176

### 1-Benzyloxycarbonyl-4-(1-(4-methoxyphenyl)-5-(4-tolyl)-1H-pyrazol-3-yl)piperidine

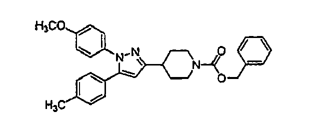

To a solution of 1-benzyloxycarbonyl-4-(3-(4-tolyl)-1,3-dioxopropyl)piperidine (Reference Example 175) (1.5 g, 4.0 mmol) in ethanol (8.2 ml) was added 4-methoxyphenylhydrazine hydrochloride (1.35 g, 4.3 mmol) and triethylamine (1.25 ml, 2.2 mmol), and the mixture was stirred at 80°C for 16 hours and then cooled to room temperature. Distilled water was added to the reaction solution, followed by extraction with ethyl acetate. The organic layer was dried over magnesium sulfate and concentrated under a reduced pressure. The residue was purified by flash chromatography (silica gel, toluene/ethyl acetate) to give a title compound (800 mg, 1.7 mmol, 42%) as a yellow oil product.

¹H-NMR (400 MHz, CDCl₃) δ: 1.72-1.74 (2H, m), 2.03-2.04 (2H, m), 2.33 (3H, s), 2.89-2.95 (3H, m), 3.79 (3H, s), 4.20-4.40 (2H, m), 5.15 (2H, s), 6.26 (1H, s), 6.83 (2H, d, J = 8.8 Hz), 7.10 (4H, s), 7.19 (2H, d, J = 8.8 Hz), 7.32-7.37 (5H, m).

### Reference Example 177

### 4-(1-(4-Methoxyphenyl)-5-(4-tolyl)-1H-pyrazol-3-yl)piperidine

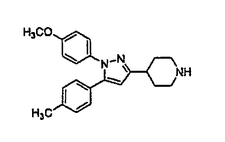

To a solution of 1-benzyloxycarbonyl-4-(1-(4-methoxyphenyl)-5-(4-tolyl)-1H-pyrazol-3-yl)piperidine (Reference Example 176) (800 mg, 1.7 mmol) in methanol (10 ml) was added ASCA-2 catalyst (100 mg, hydrous product, NE Catchem Corporation), and the resultant was stirred under a hydrogen atmosphere at room temperature for 2 hours. The reaction solution was filtered and concentrated under a reduced pressure to give a title compound (577 mg, 1.7 mmol, quant.) as a light yellow amorphous product.

¹H-NMR (400 MHz, CDCl₃) δ: 2.15-2.37 (7H, m), 3.04-3.13 (3H, m), 3.48-3.57 (2H, m), 3.81 (3H, s), 6.31 (1H, s), 6.84 (2H, d, J = 8.8 Hz), 7.08 (4H, s), 7.18 (2H, d, J = 8.8 Hz).

### Reference Example 178

### 1-(4-(Benzyloxy)phenyl)ethanone

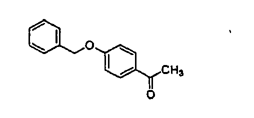

To a solution of 1-(4-hydroxyphenyl)ethanone (2.00 g, 14.7 mmol) in N,N-dimethylformamide (13.8 ml) was added potassium carbonate (2.53 g, 18.4 mmol) and benzyl bromide (2.00 ml, 9.81 mmol), and the resultant was stirred at room temperature for 3 hours. Distilled water was added to the reaction solution, followed by extraction with diethyl ether. The organic layer was washed with distilled water and then with brine, dried over magnesium sulfate, and then concentrated under a reduced pressure. The residue was recrystallized (ethyl acetate/n-hexane) to give a title compound (2.11 g, 9.33 mmol, 64%) as a white powder.

¹H-NMR (400 MHz, CDCl₃) δ: 2.55 (3H, s), 5.14 (2H, s), 7.01 (2H, d, J = 8.8 Hz), 7.35-7.44 (5H, m), 7.94 (2H, d, J = 8.8 Hz).

### Reference Example 179

### 1-Benzyloxycarbonyl-4-(3-(4-(benzyloxy)phenyl)-1,3-dioxopropyl)piperidine

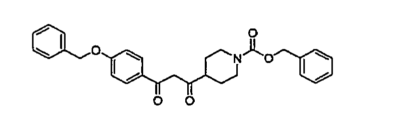

To a solution of 1-(4-(benzyloxy)phenyl)ethanone (Reference Example 178) (2.11 g, 9.33 mmol) in tetrahydrofuran (20.0 ml) was added a solution of lithium(bistrimethylsilyl)amide in tetrahydrofuran (1.0M, 10.3 ml, 10.3 mmol) dropwise at 0°C over the period of 15 minutes, and the resultant was stirred at the same temperature for 3 hours to give a solution of lithium enolate in tetrahydrofuran.

In the other flask, N,N'-carbonyldiimidazole (1.66 g, 10.2 mmol) was added at 0°C to a solution of 1-(benzyloxycarbonyl)piperidine-4-carboxylic acid (Reference Example 27) (2.45 g, 9.34 mmol) in tetrahydrofuran (10 ml), and the mixture was stirred at the same temperature for 2 hours. The solution of lithium enolate in tetrahydrofuran that had been prepared in advance was added to the above mixture at 0°C, and the resultant was stirred at the same temperature for 2 hours and then at room temperature for 1 hour. 1M hydrochloric acid was added to the reaction solution, followed by extraction with ethyl acetate. The organic layer was washed with distilled water and then with brine, dried over magnesium sulfate, and then concentrated under a reduced pressure. The residue was purified by flash chromatography (silica gel, n-hexane/ethyl acetate) to give a title compound (700 mg, 1.48 mmol, 16%) as a yellow powder.

¹H-NMR (400 MHz, CDCl₃) δ:1.66-1.69 (2H, m), 1.89 (2H, m), 2.43-2.47 (1H, m), 2.88 (2H, m), 4.28 (2H, m), 5.13 (2H, s), 5.14 (2H, s), 6.11 (2H, s), 7.02 (2H, d, J = 8.8 Hz), 7.36-7.43 (10H, m), 7.86 (2H, d, J = 8.8 Hz), 13.7 (1H, s).

### Reference Example 180

### 1-Benzyloxycarbonyl-4-(5-(4-(benzyloxy)phenyl)-1-(4-methoxyphenyl)-1H-pyrazol-3-yl)piperidine

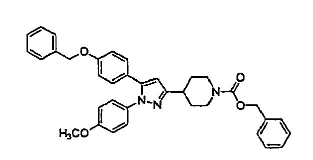

To a solution of 4-methoxyphenylhydrazine hydrochloride (133 mg, 0.42 mmol) in ethanol (1.1 ml) was added triethylamine (130 µl, 0.93 mmol), the mixture was stirred for 30 minutes, and 1-benzyloxycarbonyl-4-(3-(4-(benzyloxy)phenyl)-1,3-dioxopropyl)piperidine (Reference Example 179) (200 mg, 0.42 mmol) was then added to the reaction solution. The reaction solution was stirred at 80°C for 15 hours, the reaction solution was cooled to room temperature, and distilled water was then added thereto, followed by extraction with ethyl acetate. The organic layer was washed with distilled water and then with brine, dried over magnesium sulfate, and then concentrated under a reduced pressure. The residue was purified by flash chromatography (silica gel, n-hexane/ethyl acetate) to give a title compound (123 mg, 0.21 mmol, 50%) as a light yellow amorphous product.

¹H-NMR (400 MHz, CDCl₃) δ:1.71-1.74 (2H, m), 2.02-2.05 (2H, m), 2.89-2.95 (3H, m), 3.81 (3H, s), 4.28 (2H, m), 5.04 (2H, s), 5.15 (2H, s), 6.23 (1H, s), 6.84 (2H, d, J = 8.8 Hz), 6.89 (2H, d, J = 8.8 Hz), 7.13 (2H, d, J = 8.8 Hz), 7.19 (2H, d, J = 8.8 Hz), 7.33-7.41 (10H,m).

ESI-MS m/z = 574 (M+H⁺).

### Reference Example 181

### 1-(Benzyloxy)-4-iodobenzene

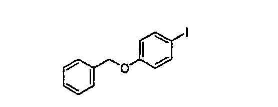

To a solution of 4-iodophenol (2.00 g, 9.09 mmol) in N,N-dimethylformamide (10.0 ml) was added potassium carbonate (1.57 g, 11.4 mmol) and benzyl bromide (1.24 ml, 10.4 mmol), and the mixture was stirred at room temperature for 2 hours. Distilled water was added to the reaction solution, followed by extraction with diethyl ether. The organic layer was washed with distilled water and then with brine, dried over magnesium sulfate, and then concentrated under a reduced pressure. The residue was purified by flash chromatography (silica gel, n-hexane/ethyl acetate) to give a title compound (2.81 g, 9.03 mmol, 99%) as a white powder.

¹H-NMR (400 MHz, CDCl₃) δ: 5.03 (2H, s), 6.75 (2H, d, J = 9.2 Hz), 7.30-7.42 (5H, m), 7.56 (2H, d, J = 8.8 Hz).

### Reference Example 182

### N-Amino-N-tert-butoxycarbonyl(4-benzyloxy)aniline

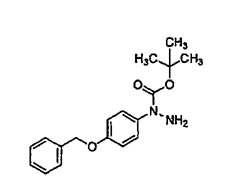

To a solution of 1-(benzyloxy)-4-iodobenzene (Reference Example 181) (2.80 g, 9.03 mmol), tert-butyl hydrazinecarboxylate (1.43 g, 10.1 mmol), cesium carbonate (4.21 g, 12.6 mmol), and 1,10-phenanthroline (325 mg, 1.81 mmol) in N,N-dimethylformamide (9.03 ml) was added copper(I) iodide (86 mg, 0.45 mmol), and the mixture was stirred at 80°C for 15 hours. The reaction solution was cooled to room temperature, diethyl ether was added thereto, and the mixture was then stirred. After the reaction solution was filtered, the filtrate was removed by distillation under a reduced pressure. The residue was purified by flash chromatography (silica gel, n-hexane/ethyl acetate) to give a title compound (1.40 g, 4.45 mmol, 49%) as a white powder.

¹H-NMR (400 MHz, CDCl₃) δ: 1.48 (9H, s), 4.43 (2H, brs), 5.05 (2H, s), 6.91 (2H, d, J = 9.2 Hz), 7.30-7.44 (7H, m).

### Reference Example 183

### 1-Benzyloxycarbonyl-4-(1-(4-(benzyloxy)phenyl)-5-(4-methoxyphenyl)-1H-pyrazol-3-yl)piperidine

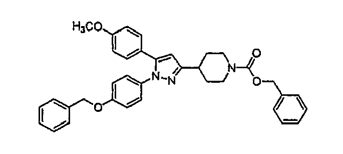

To a solution of N-amino-N-tert-butoxycarbonyl(4-benzyloxy)aniline (Reference Example 182) (1.24 g, 3.94 mmol) in methanol (3.94 ml) was added a solution of 10% hydrochloric acid-methanol (12 ml) at room temperature, and the mixture was stirred for 3 hours. The resultant was concentrated under a reduced pressure to give (4-(benzyloxy)phenyl)hydrazine hydrochloride. Ethanol (2.6 ml) was added to dissolve the residue, triethylamine (156 µl, 1.11 mmol) was added thereto, and the mixture was stirred for 30 minutes. 1-Benzyloxycarbonyl-4-(3-(4-methoxyphenyl)-1,3 -dioxopropyl)piperidine (Reference Example 48) (200 mg, 0.50 mmol) was added to the reaction solution, and the mixture was stirred at 80°C for 15 hours. The reaction solution was cooled to room temperature, and distilled water was added, followed by extraction with ethyl acetate. The organic layer was washed with distilled water and then with brine, dried over magnesium sulfate, and then concentrated under a reduced pressure. The residue was purified by flash chromatography (silica gel, n-hexane/ethyl acetate) to give a title compound (95 mg, 0.16 mmol, 33%) as a light yellow amorphous product.

¹H-NMR (400 MHz, CDCl₃) δ:1.71-1.74 (2H, m), 2.02-2.05 (2H, m), 2.87-2.95 (3H, m), 3.80 (3H, s), 4.27 (2H, m), 5.06 (2H, s), 5.15 (2H, s), 6.23 (1H, s), 6.81 (2H, d, J = 8.8 Hz), 6.91 (2H, d, J = 9.2 Hz), 7.13 (2H, d, J = 8.8 Hz), 7.19 (2H, d, J = 9.2 Hz), 7.33-7.41 (10H, m).

ESI-MS m/z = 574 (M+H⁺).

### Reference Example 184

### 1-Benzyloxycarbonyl-4-(1,5-bis(4-(benzyloxy)phenyl)-1H-pyrazol-3-yl)piperidine

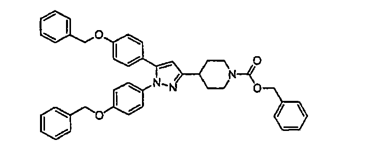

To a solution of (4-(benzyloxy)phenyl)hydrazine hydrochloride (the intermediate of Reference Example 183) (116 mg, 0.47 mmol) in ethanol (4.7 ml) was added triethylamine (130 µl, 0.93 mmol), and the mixture was stirred for 30 minutes. 1-Benzyloxycarbonyl-4-(3-(4-(benzyloxy)phenyl)-1,3-dioxopropyl)piperidine (Reference Example 179) (200 mg, 0.42 mmol) was added to the reaction solution, and the mixture was stirred at 80°C for 15 hours. The reaction solution was cooled to room temperature, and distilled water was added, followed by extraction with ethyl acetate. The organic layer was washed with distilled water and then with brine, dried over magnesium sulfate, and then concentrated under a reduced pressure. The residue was purified by flash chromatography (silica gel, n-hexane/ethyl acetate) to give a title compound (150 mg, 0.23 mmol, 54%) as a light yellow powder.

¹H-NMR (400 MHz, CDCl₃) δ:1.71-1.74 (2H, m), 2.02-2.05 (2H, m), 2.87-2.95 (3H, m), 4.28 (2H, m), 5.04 (2H, s), 5.06 (2H, s), 5.15 (2H, s), 6.23 (1H, s), 6.86-6.93 (4H, m), 6.91 (2H, d, J = 9.2 Hz), 7.13 (2H, d, J = 8.8 Hz), 7.31-7.43 (15H, m).

ESI-MS m/z = 650 (M+H⁺).

### Reference Example 185

### 1-Benzyloxycarbonyl-4-(1,5-bis(4-(hydroxyphenyl)-1H-pyrazol-3-yl)piperidine

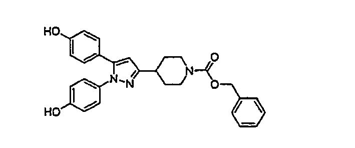

To a solution of 1-benzyloxycarbonyl-4-(1,5-bis(4-(benzyloxy)phenyl)-1H-pyrazol-3-yl)piperidine (Reference Example 184) (150 mg, 0.23 mmol) in ethanol (4.6 ml) was added ASCA-2 catalyst (50 mg, hydrous product, NE Chemcat Corporation), and the mixture was stirred under a hydrogen atmosphere for 5 hours. The reaction solution was filtered, and the filtrate was concentrated under a reduced pressure. Tetrahydrofuran (2.3 ml) and methanol (2.3 ml) were added to dissolve the residue, an aqueous solution of 1M sodium hydroxide (1.15 ml) and benzyl chloroformate (119 µl, 0.76 mmol) were added thereto, and the mixture was stirred for 6 hours. The reaction solution was concentrated under a reduced pressure, and the residue was purified by flash chromatography (silica gel, n-hexane/ethyl acetate) to give a title compound (80 mg, 0.17 mmol, 74%) as a white powder.

¹H-NMR (400 MHz, CDCl₃) δ: 2.01-2.05 (2H, m), 2.87-2.93 (3H, m), 4.20 (2H, m), 5.13-5.16 (2H, m), 6.22 (1H, s), 6.55 (2H, d, J = 9.2 Hz), 6.68 (2H, d, J = 8.8 Hz), 6.91-6.94 (4H, m), 7.30-7.37 (5H, m), 7.50 (1H, brs), 9.01 (1H, brs).

ESI-MS m/z = 470 (M+H⁺).

### Reference Example 186

### 1-Benzyloxycarbonyl-4-(1,5-bis(4-(acetoxy)phenyl)-1H-pyrazol-3-yl)piperidine-1-carboxylate

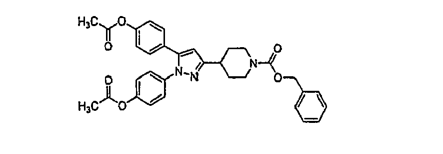

To a solution of 1-benzyloxycarbonyl-4-(1,5-bis(4-(hydroxyphenyl)-1H-pyrazol-3-yl)piperidine (Reference Example 185) (80 mg, 0.17 mmol) in tetrahydrofuran (2.0 ml) was added acetic anhydride (500 µl) and pyridine (500 µl), and the mixture was stirred for 3 hours. 1M hydrochloric acid was added to the reaction solution, followed by extraction with ethyl acetate. The organic layer was washed with 1M hydrochloric acid and then with brine, dried over magnesium sulfate, and then concentrated under a reduced pressure. The residue was purified by flash chromatography (silica gel, n-hexane/ethyl acetate) to give a title compound (90 mg, 0.16 mmol, 96%) as a colorless oil product.

¹H-NMR (400 MHz, CDCl₃) δ: 1.72-1.74 (2H, m), 2.02-2.06 (2H, m), 2.29 (3H, s), 2.30 (3H, s), 2.88-2.96 (3H, m), 4.28 (2H, m), 5.15 (2H, s), 6.29 (1H, s), 7.03-7.08 (4H, m), 7.22-7.38 (9H, m).

ESI-MS m/z = 554 (M+H⁺).

### Reference Example 187

### Allyl 1-tert-butoxycarbonyl-4-piperidinecarboxylate

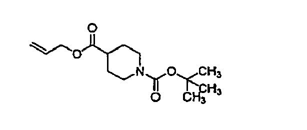

To a solution of 1-tert-butoxycarbonyl-4-piperidinecarboxylic acid (1.50 g, 6.54 mmol) in N,N-dimethylformamide (14 ml) was added potassium carbonate (1.36 g, 7.85 mmol) and allyl bromide (679 µl, 9.81 mmol), and the mixture was stirred at room temperature for 3 hours. Distilled water was added to the reaction solution, followed by extraction with ether. The organic layer was washed with distilled water and then with brine, dried over magnesium sulfate, and then concentrated under a reduced pressure. The residue was purified by flash chromatography (silica gel, n-hexane/ethyl acetate) to give a title compound (1.50 g, 5.57 mmol, 85%) as a colorless oil product.

¹H-NMR (400 MHz, CDCl₃) δ: 1.46 (9H, s), 1.59-1.69 (2H, m), 1.88-1.91 (2H, m), 2.44-2.52 (1H, m), 2.81-2.87 (2H, m), 4.02 (2H, m), 4.59 (2H, dd, J = 1.2, 5.6 Hz), 5.24 (1H, dd, J = 1.2, 10.4 Hz), 5.34 (1H, dd, J = 1.2, 17.2 Hz), 5.91 (1H, m).

### Reference Example 188

### 1-tert-Butoxycarbonyl-4-allyl-4-piperidinecarboxylic acid

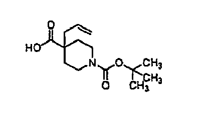

To a solution of allyl 1-tert-butoxycarbonyl-4-piperidinecarboxylate (Reference Example 187) (1.40 g, 5.20 mmol) in tetrahydrofuran (10 ml) was added a solution of 1M lithium bis(trimethylsilyl)amide/tetrahydrofuran (6.24 ml, 6.24 mmol) dropwise at -78°C, and the mixture was stirred at the same temperature for 1 hour. Chlorotrimethylsilane (792 µl, 6.24 mmol) was added dropwise to the reaction solution at -78°C, and the mixture was stirred at room temperature for 1 hour. Ether was added to the reaction solution, and the precipitate was filtered. The filtrate was concentrated under a reduced pressure, toluene (26 ml) was added to the residue, and the mixture was stirred at 110°C for 15 hours. The reaction solution was cooled to room temperature, 1M hydrochloric acid was added thereto, and the mixture was stirred at room temperature for 1 hour. Distilled water was added to the reaction solution, followed by extraction with ethyl acetate. The organic layer was washed with brine, dried over magnesium sulfate, and then concentrated under a reduced pressure. The residue was purified by flash chromatography (silica gel, n-hexane/ethyl acetate) to give a title compound (682 mg, 2.53 mmol, 49%) as a colorless oil product.

¹H-NMR (400 MHz, CDCl₃) δ:1.39-1.45 (13H, m), 2.31-2.33 (2H, m), 3.00 (2H, m), 3.87 (2H, m), 5.06-5.11 (2H, m), 5.67-5.76 (1H, m).

ESI-MS m/z = 270 (M+H⁺).

### Reference Example 189

### (1,2-Bis(4-methoxyphenyl)-2-oxoethyl) 1-tert-butoxycarbonyl-4-allyl-4-piperidinecarboxylate

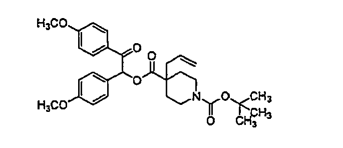

To a mixed solution of 1-tert-butoxycarbonyl-4-allyl-4-piperidinecarboxylic acid (Reference Example 188) (520 mg, 1.93 mmol) in tetrahydrofuran (2.0 ml) and toluene (2.0 ml) was added triphenylphosphine (608 mg, 2.32 mmol), p-anisoin (526 mg, 1.93 mmol), and diisopropyl azodicarboxylate (478 µl, 2.41 mmol), and the mixture was stirred at room temperature for 3 hours. The reaction solution was concentrated under a reduced pressure, and the residue was purified by flash chromatography (silica gel, n-hexane/ethyl acetate) to give a title compound (660 mg, 1.24 mmol, 65%) as a colorless oil product.

¹H-NMR (400 MHz, CDCl₃) δ: 1.39-1.45 (11H, m), 2.09-2.20 (2H, m), 2.33 (2H, d, J = 8.0 Hz), 2.94-3.20 (2H, m), 3.75-3.89 (8H, m), 4.99-5.06 (2H, m), 5.68-5.79 (1H, m), 6.78 (1H, s), 6.86-6.89 (4H, m), 7.34 (2H, d, J = 8.8 Hz), 7.91 (2H, d, J = 8.8 Hz).

ESI-MS m/z = 524 (M+H⁺).

### Reference Example 190

### 1-tert-Butoxycarbonyl-4-allyl-4-(1,5-bis(4-methoxyphenyl)oxazol-2-yl)piperidine

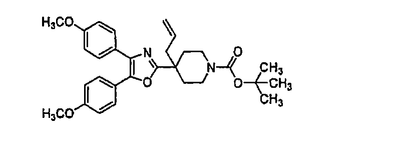

To a solution of (1,2-bis(4-methoxyphenyl)-2-oxoethyl) 1-tert-butoxycarbonyl-4-allyl-4-piperidinecarboxylate (Reference Example 189) (650 mg, 1.24 mmol) in acetic acid (2.5 ml) was added ammonium acetate (1.91 g, 24.8 mmol), and the mixture was stirred at 60°C for 15 hours. The reaction solution was cooled to room temperature, and the reaction solution was added dropwise to an aqueous solution of saturated sodium carbonate. Water was added to the reaction solution, followed by extraction with ether. The organic layer was washed with distilled water and then with brine, dried over magnesium sulfate, and then concentrated under a reduced pressure. The residue was purified by flash chromatography (silica gel, n-hexane/ethyl acetate) to give a title compound (165 mg, 0.33 mmol, 26%) as a white amorphous product.

¹H-NMR (400 MHz, CDCl₃) δ: 1.45 (9H, s), 1.57-1.63 (2H, m), 2.33-2.37 (2H, m), 2.47 (2H, d, J = 8.0 Hz), 3.06 (2H, m), 3.83-3.92 (8H, m), 5.01-5.05 (2H, m), 5.63-5.72 (1H, m), 6.88-6.91 (4H, m), 7.48 (2H, d, J = 8.8 Hz), 7.56 (2H, d, J = 8.8 Hz).

### Reference Example 191

### 4-(4,5-Bis(4-tolyl)oxazol-2-yl)piperidine

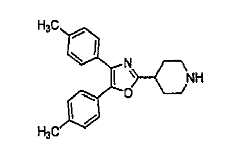

A light yellow oil product (1.43 g, 2.94 mmol, 74%) was obtained from 1-benzyloxycarbonylisonipecotic acid chloride (1.05g, 3.99 mmol) and dimethylbenzoin (1.00 g, 4.16 mmol) in the same manner as in Reference Example 28. From this light yellow oil product (1.40 g, 2.88 mmol), another light yellow oil product (1.12 mg, 2.40 mmol, 83%) was obtained in the same manner as in Reference Example 49. To a solution of the resulting yellow oil product (667 mg, 1.43 mmol) in ethanol (5.0 ml) was added 10% Pd-C (50% hydrous, 60 mg), and the mixture was stirred under a hydrogen atmosphere for 18 hours. The reaction solution was filtered, and the filtrate was concentrated under a reduced pressure to give a title compound (480 mg, 1.43 mmol, quant.) as a brown oil product.

¹H-NMR (400 MHz, CDCl₃) δ: 1.81-1.91 (2H, m), 2.06-2.14 (2H, m), 2.36 (3H, s), 2.37 (3H, s), 2.72-2.80 (2H, m), 2.90-3.12 (1H, m), 3.16-3.23 (2H, m), 7.16 (2H, d, J = 8.8 Hz), 7.16 (2H, d, J = 8.8 Hz), 7.46 (2H, d, J = 8.8 Hz), 7.52 (2H, d, J = 8.8 Hz).

### Reference Example 192

### Methyl 4-(2-(benzyloxy)ethoxy)phenylacetate

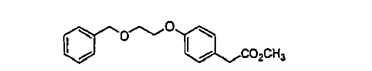

To a solution of methyl 4-hydroxyphenylacetate (500 mg, 3.01 mmol) in N,N-dimethylformamide (2.0 ml), cesium carbonate (1.00 g, 3.07 mmol) and benzyl 2-bromoethyl ether (0.5 ml, 3.2 mmol) were added, and the mixture was stirred at 50°C for 2 hours and then cooled to room temperature. Distilled water was added to the reaction solution, followed by extraction with ethyl acetate. The organic layer was washed with water, dried, and then concentrated under a reduced pressure. The residue was purified via flash chromatography (silica gel, n-hexane/ethyl acetate) to give a title compound (679 mg, 2.26 mmol, 75 %) as a colorless liquid.

¹H-NMR (400 MHz, CDCl₃) δ: 3.56 (2H, s), 3.68 (3H, s), 3.82 (2H, t, J = 4.88 Hz), 4.14 (2H, t, J = 4.88 Hz), 4.63 (2H, s), 6.88 (2H, d, J = 8.5 Hz), 7.18 (2H, d, J = 8.5 Hz), 7.27-7.39 (5H, m).

### Reference Example 193

### Methyl 2-(4-(2-(benzyloxy)ethoxy)phenyl)-3-(4-methoxyphenyl)-3-oxopropanoate

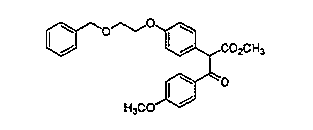

A title compound (725 mg, 1.66 mmol, 74%) was obtained as a yellow liquid from anisic acid (341 mg, 2.24 mmol) and methyl 4-(2-(benzyloxy)ethoxy)phenylacetate (Reference Example 192) (676 mg, 2.25 mmol) in the same manner as in Reference Example 1.

¹H-NMR (400 MHz, CDCl₃) δ: 3.75 (3H, s), 3.80 (2H, t, J = 4.88 Hz), 3.83 (3H, s), 4.11 (2H, t, J = 4.88 Hz), 4.61 (2H, s), 5.53 (2H, s), 6.88 (2H, d, J = 8.8 Hz), 6.90 (2H, d, J = 8.5 Hz), 7.25-7.35 (7H, m), 7.92 (2H, d, J = 8.8 Hz).

### Reference Example 194

### 2-(4-(2-(Benzyloxy)ethoxy)phenyl)-1-(4-methoxyphenyl)ethanone

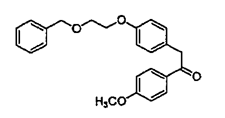

A title compound (359 mg, 0.953 mmol, 57%) was obtained as a light yellow solid from methyl 2-(4-(2-(benzyloxy)ethoxy)phenyl)-3-(4-methoxyphenyl)-3-oxopropanoate (Reference Example 193) (725 mg, 1.66 mmol) in the same manner as in Reference Example 6.

¹H-NMR (400 MHz, CDCl₃) δ: 3.81 (2H, t, J = 4.9 Hz), 3.86 (3H, s), 4.12 (2H, t, J = 4.9 Hz), 4.16 (2H, s), 4.62 (2H, s), 6.88 (2H, d, J = 8.5 Hz), 6.92 (2H, d, J = 8.8 Hz), 7.17 (2H, d, J = 8.5 Hz), 7.27-7.37 (5H, m), 7.98 (2H, d, J = 8.8 Hz).

### Reference Example 195

### 2-(4-(2-(Benzyloxy)ethoxy)phenyl)-2-bromo-1-(4-methoxyphenyl)ethanone

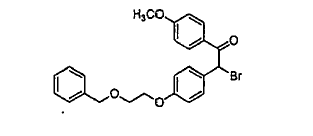

A title compound (430 mg, 0.946 mmol, quant.) was obtained as a brown oil product from 2-(4-(2-(benzyloxy)ethoxy)phenyl)-1-(4-methoxyphenyl)ethanone (Reference Example 194) (356 mg, 0.946 mmol) in the same manner as in Reference Example 14.

¹H-NMR (400 MHz, CDCl₃) δ: 3.81 (2H, t, J = 4.9 Hz), 3.85 (3H, s), 4.13 (2H, t, J = 4.9 Hz), 4.62 (2H, s), 6.36 (1H, s), 6.90 (2H, d, J = 8.8 Hz), 6.91 (2H, d, J = 8.8 Hz), 7.28-7.46 (7H, m), 7.96 (2H, d, J = 8.8 Hz).

### Reference Example 196

### (2-(4-(2-(Benzyloxy)ethyloxy)phenyl)-1-(4-methoxyphenyl)-2-oxoethyl) 1-tert-butoxycarbonyl-4-piperidinecarboxylate

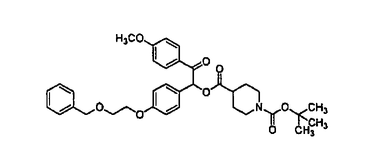

A title compound (376 mg, 0.623 mmol, 66%) was obtained as a light yellow amorphous product from 2-(4-(2-(benzyloxy)ethoxy)phenyl)-2-bromo-1-(4-methoxyphenyl)ethanone (Reference Example 195) (473 mg, 0.623 mmol) and 1-tert-butoxycarbonyl-4-piperidinecarboxylic acid (229 mg, 1.00 mmol) in the same manner as in Reference Example 28.

¹H-NMR (400 MHz, CDCl₃) δ: 1.45 (9H, s), 1.63-1.79 (2H, m), 1.85-1.92 (1H, m), 1.97-2.04 (1H, m), 2.58-2.66 (1H, m), 2.82-2.94 (2H, m), 3.80 (2H, t, J = 4.9 Hz), 3.82 (5H, s), 3.90-4.09 (2H, m), 4.11 (2H, t, J = 4.9 Hz), 4.61 (2H, s), 6.78 (1H, s), 6.86 (2H, d, J = 8.8 Hz), 6.90 (2H, d, J = 8.8 Hz), 7.27-7.37 (7H, m), 7.90 (2H, d, J = 8.8 Hz).

### Reference Example 197

### 5-((2-(Benzyloxy)ethyloxy)phenyl)-4-(4-methoxyphenyl)piperidine

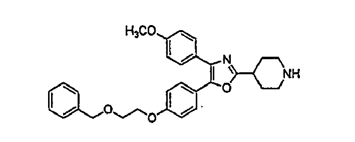

A title compound (258 mg, 0.532 mmol, 86%) was obtained as a light yellow amorphous product from (2-(4-(2-(benzyloxy)ethyloxy)phenyl)-1-(4-methoxyphenyl)-2-oxoethyl) 1-tert-butoxycarbonyl-4-piperidinecarboxylate (Reference Example 196) (372 mg, 0.616 mmol) in the same manner as in Reference Example 49 and in Reference Example 86.

¹H-NMR (400 MHz, CDCl₃) δ: 1.80-1.90 (2H, m), 2.06-2.13 (2H, m), 2.72-2.79 (2H, m), 2.95-3.03 (1H, m), 3.15-3.25 (2H, m), 3.83 (3H, s), 3.84 (2H, t, J = 4.9 Hz), 4.17 (2H, t, J = 4.9 Hz), 4.64 (2H, s), 6.89 (2H, d, J = 8.8 Hz), 6.90 (2H, d, J = 8.8 Hz), 7.28-7.37 (5H, m), 7.48 (2H, d, J = 8.8 Hz), 7.55 (2H, d, J = 8.8 Hz).

### Reference Example 198

### 5-((2-(Benzyloxy)ethyloxy)phenyl)-4-(4-methoxyphenyl)-1-(N-hydroxy-N-methylcarbamoyl)piperidine

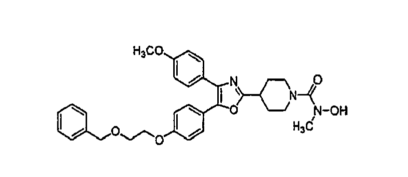

To a solution of 5-((2-(benzyloxy)ethyloxy)phenyl)-4-(4-methoxyphenyl)piperidine (Reference Example 197) (254 mg, 0.524 mmol) in tetrahydrofuran (3.0 ml) was added a solution of triphosgene (65 mg, 0.22 mmol) and triethylamine (65 mg, 0.64 mmol) in tetrahydrofuran (1.0 ml) at 0°C, and the mixture was stirred at room temperature for 2 hours. A solution of N-methylhydroxylamine hydrochloride (55 mg, 0.66 mmol) and triethylamine (140 mg, 1.4 mmol) in tetrahydrofuran (1.0 ml) was added to the reaction solution, and the mixture was stirred at 50°C for 6 hours. The reaction solution was cooled to room temperature and then concentrated under a reduced pressure. Distilled water was added to the residue, followed by extraction with ethyl acetate. The organic layer was dried over magnesium sulfate and then concentrated under a reduced pressure. The residue was purified by flash chromatography (amine silica gel, n-hexane/ethyl acetate) to give a title compound (222 mg, 0.398 mmol, 76%) as a white amorphous product.

¹H-NMR (400 MHz, CDCl₃) δ: 1.92-2.02 (2H, m), 2.13-2.19 (2H, m), 2.99 (3H, s), 3.08-3.17 (3H, m), 3.83 (3H, s), 3.84 (2H, t, J = 4.9 Hz), 4.04-4.10 (2H, m), 4.64 (2H, s), 6.80 (2H, d, J = 9.0 Hz), 6.91 (2H, d, J = 9.0 Hz), 7.28-7.38 (5H, m), 7.47 (2H, d, J = 8.8 Hz), 7.54 (2H, d, J = 8.8 Hz).

### Reference Example 199

### 4-(4,5-Bis(4-methoxyphenyl)thiazol-2-yl)-1-(N-methylcarbamoyl)piperidine

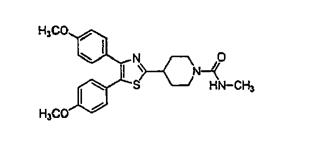

To a solution of methylamine hydrochloride (246 mg, 3.64 mmol) in acetonitrile (10 ml), was added sodium bicarbonate (671 mg, 7.99 mmol) and 4-nitrophenyl chloroformate (588 mg, 2.92 mmol), and the mixture was stirred at room temperature for 6 hours. 4-(4,5-Bis(4-methoxyphenyl)oxazol-2-yl)piperidine (Reference Example 93) (861 mg, 2.26 mmol) and triethylamine (463 µl, 3.4 mmol) were added to the reaction solution, and the mixture was stirred at room temperature for 24 hours. Distilled water was added to the reaction solution, followed by extraction with ethyl acetate. The organic layer was dried over magnesium sulfate and then concentrated. The residue was purified by flash chromatography (amine silica gel, n-hexane/ethyl acetate) to give a title compound (990 mg, 2.26 mmol, quant.) as a white amorphous product.

¹H-NMR (400 MHz, CDCl₃) δ: 1.75-1.87 (2H, m), 2.13-2.21 (2H, m), 2.83 (3H, d, J = 4.4 Hz), 2.92 -3.01 (2H, m), 3.15-3.23 (1H, m), 3.80 (3H, s), 3.82 (3H, s), 4.01-4.09 (2H, m), 4.47 (1H, bd, J = 4.4 Hz), 6.81 (2H, d, J = 8.8 Hz), 6.84 (2H, d, J = 8.8 Hz), 7.24 (2H, d, J = 8.8 Hz), 7.43 (2H, d, J = 8.8 Hz).

ESI-MS: m/z = 438 (M+H⁺).

As the compounds of the present invention, compounds of Examples 1 to 77 below were synthesized. Compounds used for synthesizing compounds of Examples, which are not described in terms of methods of synthesis, are commercially available compounds.

### Example 1

### 4-(4,5-Bis(4-methoxyphenyl)oxazol-2-yl)-1-(N-hydroxy-N-methylcarbamoyl)piperidine

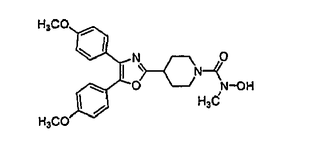

To a solution of triphosgene (2.17 g, 7.31 mmol) in dichloromethane (10 ml) was added a solution of 4-(4,5-bis(4-methoxyphenyl)oxazol-2-yl)piperidine (Reference Example 78) (8.11 g, 22.2 mmol) in dichloromethane (22 ml) and a solution of pyridine (1.8 ml) in dichloromethane (10 ml) dropwise at -78°C. The reaction solution was stirred at room temperature for 3 hours and then concentrated under a reduced pressure. The residue was dissolved in ethyl acetate and washed with 1M hydrochloric acid. The organic layer was dried over magnesium sulfate and concentrated under a reduced pressure to give 4-(4,5-bis(4-methoxyphenyl)oxazol-2-yl)piperidine-1-carbonyl chloride (7.12 g, 16.6 mmol, 74%) as an orange amorphous product.

¹H-NMR (400 MHz, CDCl₃) δ: 2.05 (2H, m), 2.20 (2H, m), 3.15-3.26 (2H, m), 3.41 (1H, m), 3.83 (3H, s), 3.83 (3H, s), 4.29 (2H, m), 6.89 (2H, d, J = 9.0 Hz), 6.90 (2H, d, J = 9.0 Hz), 7.48 (2H, d, J = 9.0 Hz), 7.55 (2H, d, J = 9.0 Hz).

To a solution of 4-(4,5-bis(4-methoxyphenyl)oxazol-2-yl)piperidine-1-carbonyl chloride (7.12 g, 16.6 mmol) in dichloromethane (100 ml) was added N-methylhydroxylamine hydrochloride (1.52 g 18.2 mmol) and triethylamine (5.70 ml, 41.7 mmol), and the mixture was stirred at room temperature for 2 hours. 1M hydrochloric acid was added to the reaction solution then the solution was separated. The organic layer was washed with brine, dried over sodium sulfate, and then concentrated under a reduced pressure. The residue was purified by column chromatography (silica gel, n-hexane/ethyl acetate = 2/5), and the resulting solid was recrystallized (ethyl acetate/n-hexane = 150 ml/75 ml) to give a title compound (5.80 g, 13.2 mmol, 59%) as a white powder.

¹H-NMR (400 MHz, CDCl₃) δ: 1.99 (2H, dq, J = 3.6, 13.6 Hz), 2.16 (2H, d, J = 3.6, 13.6), 2.99 (3H, s), 3.09-3.18 (3H, m), 3.83 (3H, s), 3.83 (3H, s), 4.07 (2H, dt, J = 3.6, 13.6 Hz), 6.80 (1H, brs), 6.88 (2H, d, J = 9.0 Hz), 6.89 (2H, d, J = 9.0 Hz), 7.48 (2H, d, J = 9.0 Hz), 7.54 (2H, d, J = 9.0 Hz).

IR (KBr, cm⁻¹): 3294, 1659, 1520, 1501, 1442, 1253.

Elementary analysis: measured value: C, 65.85, H, 6.29, N, 9.34; calculated value: (C₂₄H₂₇N₃O₅) C, 65.89, 6.22, N, 9.60.

### Example 2

### 3-(4,5-Bis(4-methoxyphenyl)oxazol-2-yl)-1-(N-hydroxy-N-methylcarbamoyl)piperidine

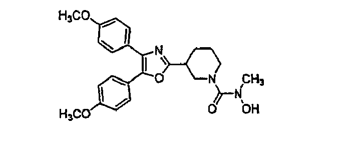

To a solution of triphosgene (67 mg, 0.22 mmol) in dichloromethane (5.0 ml) was added a solution of 3-(4,5-bis(4-methoxyphenyl)oxazol-2-yl)piperidine (Reference Example 79) (245 mg, 0.67 mmol) in dichloromethane (5.0 ml) and a solution of pyridine (53 mg, 0.67 mmol) in dichloromethane (2.0 ml) at -78°C, and the mixture was gradually heated to room temperature. The reaction solution was concentrated under a reduced pressure, and 1M hydrochloric acid was added to the residue, followed by extraction with ethyl acetate. The organic layer was washed with brine, dried over sodium sulfate, and concentrated under a reduced pressure. The residue was dissolved with the addition of dichloromethane (10 ml), N-methylhydroxylamine hydrochloride (67 mg, 0.80 mmol) and triethylamine (203 mg, 2.0 mmol) were added, and the mixture was stirred at room temperature for 17 hours. An aqueous solution of saturated ammonium was added to the reaction solution, followed by extraction with ethyl acetate. The organic layer was washed with brine, dried over sodium sulfate, and concentrated under a reduced pressure. The residue was purified by column chromatography (silica gel, n-hexane/ethyl acetate = 5/1 to 3/1) to give a title compound (170 mg, 0.39 mmol, 58%) as a light yellow amorphous product.

¹H-NMR (400 MHz, CDCl₃) δ:1.64-1.71 (1H, m), 1.82-1.87 (1H, m), 1.92-2.02 (1H, m), 2.26-2.30 (1H, m), 3.01 (3H, s), 3.07-3.19 (2H, m), 3.47 (1H, dd, J = 13.2, 9.6Hz), 3.84 (6H, s), 3.89-3.91 (1H, m), 4.24 (1H, dd, J = 13.2, 4.0 Hz), 6.89 (2H, d, J = 8.8 Hz), 6.90 (2H, d, J = 8.8 Hz), 7.49 (2H, d, J = 8.8 Hz), 7.53 (2H, d, J = 8.8 Hz).

IR (KBr, cm⁻¹): 3246, 2934, 2835, 1614, 1579, 1519, 1500, 1440, 1297, 1253, 1176, 1031, 834.

### Example 3

### 4-(4-(4-tert-Butylphenyl)-5-(4-methoxyphenyl)oxazol-2-yl)-1-(N-hydroxy-N-methylcarbamoyl)piperidine

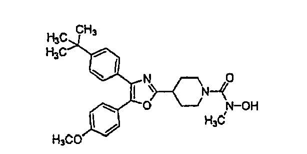

To a solution of 4-(4-(4-tert-butylphenyl)-5-(4-methoxyphenyl)oxazol-2-yl)piperidine (Reference Example 80) (350 mg 0.90 mmol) in dichloromethane (10 ml) was added triphosgene (89 mg, 0.30 mmol) and triethylamine (91 mg, 0.90 mmol) at -78°C, and the mixture was gradually heated to room temperature. N-methylhydroxylamine hydrochloride (90 mg, 1.1 mmol) and triethylamine (181 mg, 1.79 mmol) were added to the reaction solution, and the mixture was stirred at room temperature for 15 hours. An aqueous solution of saturated ammonium chloride was added to the reaction solution, followed by extraction with ethyl acetate. The organic layer was dried over sodium sulfate and then concentrated under a reduced pressure. The residue was purified by column chromatography (amine silica gel, n-hexane/ethyl acetate = 1/1) to give a title compound (172 mg, 0.37 mmol, 41%) as a light yellow amorphous product.

¹H-NMR (400 MHz, CDCl₃) δ:1.33 (9H, s), 1.92-2.02 (2H, m), 2.14-2.19 (2H, m), 2.99 (3H, s), 3.09-3.18 (3H, m), 3.84 (3H, s), 4.04-4.09 (2H, m), 6.82 (1H, brs), 6.90 (2H, d, J = 8.8 Hz), 7.37 (2H, d, J = 8.8 Hz), 7.52 (2H, d, J = 8.8 Hz), 7.55 (2H, d, J = 8.8 Hz).

IR (KBr, cm⁻¹): 3229, 2960, 1615, 1520, 1499, 1441, 1253, 1177, 1032, 835.

EI-MS: m/z = 463 (M⁺).

HR-MS (EI): measured value: 463.2435, calculated value: (C₂₇H₃₃N₃O₄), 463.2471.

### Example 4

### 4-(5-(4-Methoxyphenyl)-4-(4-tolyl)oxazol-2-yl)-1-(N-hydroxy-N-methylcarbamoyl)piperidine

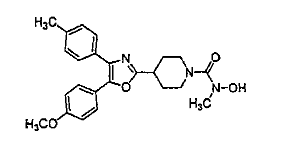

To a solution of triphosgene (109 mg, 0.367 mmol) in tetrahydrofuran (4.0 ml) was added a solution of 4-(5-(4-methoxyphenyl)-4-(4-tolyl)oxazol-2-yl)piperidine (Reference Example 81) (316 mg, 0.907 mmol) in tetrahydrofuran (3.0 ml) and a solution of triethylamine (120 mg, 1.18 mmol) in tetrahydrofuran (2.0 ml) at 0°C, and the mixture was stirred at room temperature for 3 hours. A solution of N-methylhydroxylamine hydrochloride (95 mg, 1.13 mmol) and triethylamine (243 mg, 2.40 mmol) in tetrahydrofuran (2.0 ml) was added to the reaction solution, and the mixture was stirred at 50°C for 17 hours and then cooled to room temperature. Insoluble matters were removed from the reaction solution by filtration, and the filtrate was concentrated under a reduced pressure. The residue was purified by flash chromatography (amine silica gel, n-hexane/ethyl acetate), and the resulting solid was recrystallized (methanol) to give a title compound (253 mg, 0.600 mmol, 66%) as a light yellow crystal.

¹H-NMR (400 MHz, CDCl₃) δ:1.97 (2H, m), 2.16 (2H, m), 2.36 (3H, s), 2.99 (3H, s), 3.10-3.18 (3H, m), 3.83 (3H, s), 4.07 (2H, m), 6.78 (1H, brs), 6.89 (2H, d, J = 8.8 Hz), 7.16 (2H, d, J = 8.3 Hz), 7.49 (2H, d, J = 8.8 Hz), 7.51 (2H, d, J = 8.3 Hz).

IR (KBr, cm⁻¹): 3278, 1659, 1521, 1501, 1467, 1441, 1255.

ESI-MS: m/z = 422 (M+H⁺).

Elementary analysis: measured value: C, 68.22, H, 6.54, N, 9.91; calculated value: (C₂₄H₂₇N₃O₄) C, 68.39, 6.46, N, 9.97.

### Example 5

### 4-(4-(4-Isopropyloxyphenyl)-5-(4-methoxyphenyl)oxazol-2-yl)-1-(N-hydroxy-N-methylcarbamoyl)piperidine

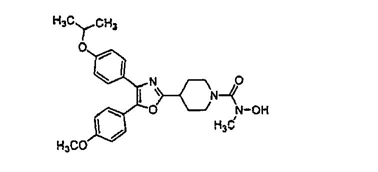

To a solution of 4-(4-(4-isopropyloxyphenyl)-5-(4-methoxyphenyl)oxazol-2-yl)piperidine (Reference Example 82) (343 mg, 0.87 mmol) in dichloromethane (10 ml) was added triphosgene (86 mg, 0.29 mmol) and triethylamine (88 mg, 0.87 mmol) at -78°C, and the mixture was gradually heated to room temperature. N-methylhydroxylamine hydrochloride (87 mg, 1.1 mmol) and triethylamine (177 mg, 1.75 mmol) were added to the reaction solution, and the mixture was stirred at room temperature for 17 hours. An aqueous solution of saturated ammonium chloride was added to the reaction solution, followed by extraction with ethyl acetate. The organic layer was dried over sodium sulfate and concentrated under a reduced pressure. The residue was purified by flash chromatography (amine silica gel, n-hexane/ethyl acetate) to give a title compound (183 mg, 0.39 mmol, 45%) as a white amorphous product.

¹H-NMR (400 MHz, CDCl₃) δ: 1.34 (6H, d, J = 6.0 Hz), 1.91-2.00 (2H, m), 2.13-2.18 (2H, m), 2.99 (3H, s), 3.07-3.16 (3H, m), 3.83 (3H, s), 4.06-4.13 (2H, m), 4.57 (1H, sept, J = 6.0 Hz), 6.87 (2H, d, J = 8.8 Hz), 6.88 (2H, d, J = 8.8 Hz), 7.49 (2H, d, J = 8.8 Hz), 7.52 (2H, d, J = 8.8 Hz).

IR (KBr, cm⁻¹): 3238, 2975, 2931, 1613, 1518, 1499, 1441, 1250, 1179, 1118, 1031, 952, 834.

EI-MS: m/z = 465 (M⁺).

HR-MS (EI): measured value: 465.2246, calculated value: (C₂₆H₃₁N₃O₅), 465.2264.

### Example 6

### 4-(5-(4-Methoxyphenyl)-4-(4-trifluoromethylphenyl)oxazol-2-yl)-1-(N-hydroxy-N-methylcarbamoyl)piperidine

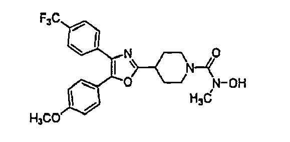

To a solution of 4-(5-(4-methoxyphenyl)-4-(4-trifluoromethylphenyl)oxazol-2-yl)piperidine (Reference Example 83) (207 mg, 0.51 mmol) in dichloromethane (10 ml) was added triphosgene (51 mg, 0.17 mmol) and triethylamine (52 mg, 0.51 mmol) at -78°C, and the mixture was gradually heated to room temperature. N-methylhydroxylamine hydrochloride (51 mg, 0.62 mmol) and triethylamine (104 mg, 1.0 mmol) were added to the reaction solution, and the mixture was stirred at room temperature for 4 hours. An aqueous solution of saturated ammonium chloride was added to the reaction solution, followed by extraction with ethyl acetate. The organic layer was dried over sodium sulfate and concentrated under a reduced pressure. The residue was purified by flash chromatography (amine silica gel, n-hexane/ethyl acetate) to give a title compound (120 mg, 0.25 mmol, 49%) as a white solid.

¹H-NMR (400 MHz, CDCl₃) δ: 1.92-2.02 (2H, m), 2.15-2.20 (2H, m), 3.00 (3H, s), 3.10-3.19 (3H, m), 3.85 (3H, s), 4.05-4.11 (2H, m), 6.76 (1H, brs), 6.93 (2H, d, J = 9.0 Hz), 7.48 (2H, d, J = 9.0 Hz), 7.60 (2H, d, J = 8.5 Hz), 7.76 (2H, d, J = 8.5 Hz).

IR (KBr, cm⁻¹): 3280, 2933, 2843, 1660, 1620, 1524, 1503, 1445, 1326, 1257, 1164, 1125, 1069, 1033, 854.

EI-MS: m/z = 475 (M⁺).

HR-MS (EI): measured value: 475.1695, calculated value: (C₂₄H₂₄F₃N₃O₄), 475.1719.

### Example 7

### 4-(4-(4-Methoxyphenyl)-5-(4-tolyl)oxazol-2-yl)-1-(N-hydroxy-N-methylcarbamoyl)piperidine

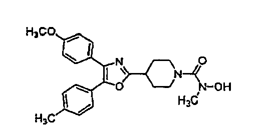

To a solution of triphosgene (109 mg, 0.36 mmol) in dichloromethane (2.0 ml) was added a solution of 4-(4-(4-methoxyphenyl)-5-(4-tolyl)oxazol-2-yl)piperidine (Reference Example 84) (378 mg, 1.08 mmol) in dichloromethane (3.0 ml) and triethylamine (147 µl, 1.07 mmol) at -78°C, and the mixture was gradually heated to room temperature. 1M hydrochloric acid was added to the reaction solution, followed by extraction with ethyl acetate. The organic layer was washed with brine, dried over magnesium sulfate, and then concentrated under a reduced pressure. The residue was dissolved in dichloromethane (10 ml), N-methylhydroxylamine hydrochloride (106 mg, 1.27 mmol) and triethylamine (180 mg, 1.3 mmol) were added thereto, and the mixture was stirred at room temperature for 14 hours. Distilled water was added to the reaction solution then the solution was separated. The organic layer was dried over sodium sulfate and concentrated under a reduced pressure. The residue was purified by flash chromatography (amine silica gel, n-hexane/ethyl acetate) to give a title compound (185 mg, 0.439 mmol, 40%) as a white solid.

¹H-NMR (400 MHz, CDCl₃) δ:1.97 (2H, m), 2.17 (2H, m), 2.37 (3H, s), 2.99 (3H, s), 3.08-3.18 (3H, m), 3.83 (3H, s), 4.08 (2H, m), 6.69 (1H, brs), 6.89 (2H, d, J = 9.0 Hz), 7.16 (2H, d, J = 8.3 Hz), 7.44 (2H, d, J = 8.3 Hz), 7.55 (2H, d, J = 9.0 Hz).

IR (KBr, cm⁻¹): 3296, 1663, 1520, 1499, 1438, 1252.

EI-MS: m/z = 421 (M⁺).

HR-MS (EI): measured value: 421.2003, calculated value: (C₂₄H₂₇N₃O₄), 421.2002.

### Example 8

### 4-(4-(4-Methoxyphenyl)-5-phenyloxazol-2-yl)-1-(N-hydroxy-N-methylcarbamoyl)piperidine

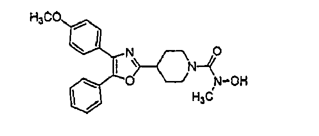

To a solution of triphosgene (99 mg, 0.33 mmol) in dichloromethane (2.0 ml) was added a solution of 4-(4-(4-methoxyphenyl)-5-phenyloxazol-2-yl)piperidine (Reference Example 85) (368 mg, 1.10 mmol) in dichloromethane (2.0 ml) and triethylamine (75 µl, 0.55 mmol) at -78°C, and the mixture was gradually heated to room temperature. 1M hydrochloric acid was added to the reaction solution, followed by extraction with ethyl acetate. The organic layer was dried over magnesium sulfate and then concentrated under a reduced pressure. The residue was dissolved with the addition of dichloromethane (4.0 ml), N-methylhydroxylamine hydrochloride (79 mg, 250 mmol) and triethylamine (250 µl, 1.82 mmol) were added thereto, and the mixture was stirred at room temperature for 1 hour. 1M hydrochloric acid was added to the reaction solution then the solution was separated. The organic layer was dried over sodium sulfate and concentrated under a reduced pressure. The residue was purified by flash chromatography (amine silica gel, n-hexane/ethyl acetate) to give a title compound (306 mg, 0.751 mmol, 76%) as a white amorphous product.

¹H-NMR (400 MHz, CDCl₃) δ: 1.98 (2H, m), 2.18 (2H, m), 3.00 (3H, s), 3.11-3.18 (3H, m), 3.84 (3H, s), 4.08 (2H, m), 6.79 (1H, brs), 6.90 (2H, d, J = 8.8 Hz), 7.30-7.37 (3H, m), 7.54-7.58 (4H, m).

IR (KBr, cm⁻¹): 3236, 1649, 1618, 1568, 1514, 1250.

EI-MS: m/z = 407 (M⁺).

HR-MS (EI): measured value: 407.1824, calculated value: (C₂₃H₂₅N₃O₄), 407.1845.

### Example 9

### 4-(5-(4-Fluorophenyl)-4-(4-methoxyphenyl)oxazol-2-yl)-1-(N-hydroxy-N-methylcarbamoyl)-piperidine

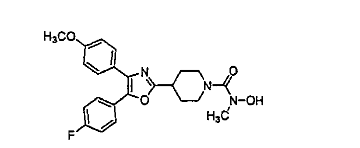

To a solution of triphosgene (59 mg, 0.199 mmol) in dichloromethane (1.0 ml) was added 4-(5-(4-fluorophenyl)-4-(4-methoxyphenyl)oxazol-2-yl)piperidine (Reference Example 86) (204 mg, 0.579 mmol) and triethylamine (95 µl, 0.69 mmol) at -78°C, and the mixture was gradually heated to room temperature. 1M hydrochloric acid was added to the reaction solution, followed by extraction with ethyl acetate. The organic layer was dried over magnesium sulfate and then concentrated under a reduced pressure. Dichloromethane (3.0 ml) was added to the residue, N-methylhydroxylamine hydrochloride (55 mg, 0.66 mmol) and triethylamine (143 µl, 1.03 mmol) were added thereto, and the mixture was stirred at room temperature for 4 hours. The reaction solution was concentrated under a reduced pressure, and the residue was purified by column chromatography (amine silica gel, n-hexane/ethyl acetate = 1/3 to 1/5) to give a title compound (106 mg, 0.249 mmol, 44%) as a white amorphous product.

¹H-NMR (400 MHz, CDCl₃) δ: 1.97 (2H, m), 2.17 (2H, m), 3.00 (3H, s), 3.10-3.18 (3H, m), 3.84 (3H, s), 4.08 (2H, m), 6.78 (1H, brs), 6.91 (2H, d, J = 8.8 Hz), 7.05 (2H, t, J = 8.5 Hz), 7.50-7.55 (4H, m).

EI-MS: m/z = 425 (M⁺).

HR-MS (EI): measured value: 425.1728, calculated value: (C₂₃H₂₄FN₃O₄), 425.1751.

### Example 10

### 4-(5-Phenyl-4-(4-trifluoromethylphenyl)oxazol-2-yl)-1-(N-hydroxy-N-methylcarbamoyl)piperidine

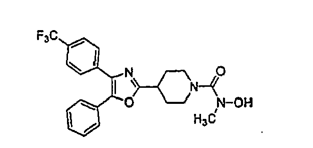

To a solution of triphosgene (29 mg, 0.098 mmol) in dichloromethane (1.0 ml) was added 4-(5-phenyl-4-(4-trifluoromethylphenyl)oxazol-2-yl)piperidine (Reference Example 87) (107 mg, 0.287 mmol) and triethylamine (47 µl, 0.34 mmol) at -78°C, and the mixture was gradually heated to room temperature. 1M hydrochloric acid was added to the reaction solution, followed by extraction with ethyl acetate. The organic layer was dried over magnesium sulfate and then concentrated under a reduced pressure. Dichloromethane (2.0 ml) was added to the residue, N-methylhydroxylamine hydrochloride (40 mg, 0.47 mmol) and triethylamine (71 µl, 0.51 mmol) were added thereto, and the mixture was stirred at room temperature for 16 hours. The reaction solution was concentrated under a reduced pressure, and the residue was purified by column chromatography (amine silica gel, n-hexane/ethyl acetate = 1/1 to 1/3) to give a title compound (51 mg, 0.11 mmol, 38%) as a white amorphous product.

¹H-NMR (400 MHz, CDCl₃) δ: 1.98 (2H, m), 2.19 (2H, m), 3.01 (3H, s), 3.12-3.19 (3H, m), 4.09 (2H, m), 6.78 (1H, brs), 7.38-7.43 (3H, m), 7.55 (2H, d, J = 9.5 Hz), 7.61 (2H, d, J = 8.1 Hz), 7.77 (2H, d, J = 8.1 Hz).

ESI-MS: m/z = 446 (M+H⁺).

### Example 11

### 4-(4-(4-Chlorophenyl)-5-(4-tolyl)oxazol-2-yl)-1-(N-hydroxy-N-methylcarbamoyl)piperidine

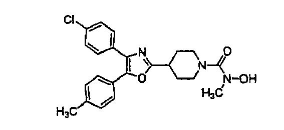

A title compound (86 mg, 0.20 mmol, 34%) was obtained as a light yellow amorphous product from 4-(4-(4-chlorophenyl)-5-(4-tolyl)oxazol-2-yl)piperidine (Reference Example 88) (209 mg, 0.59 mmol) in accordance with the method of Example 1.

¹H-NMR (400 MHz, CDCl₃) δ: 1.96 (2H, m), 2.17 (2H, m), 2.38 (3H, s), 3.00 (3H, s), 3.11-3.18 (3H, m), 4.08 (2H, m), 6.77 (1H, s), 7.18 (2H, d, J = 8.1 Hz), 7.32 (2H, d, J = 8.8 Hz), 7.42 (2H, d, J = 8.1 Hz), 7.57 (2H, d, J = 8.8 Hz).

ESI-MS: m/z = 426 (M+H⁺).

### Example 12

### 4-(4-(4-Chlorophenyl)-5-(4-methoxyphenyl)oxazol-2-yl)-1-(N-hydroxy-N-methylcarbamoyl)-piperidine

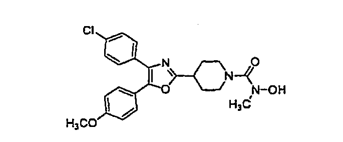

To a solution of triphosgene (98 mg, 0.33 mmol) in tetrahydrofuran (5.0 ml) was added a solution of 4-(4-(4-chlorophenyl)-5-(4-methoxyphenyl)oxazol-2-yl)piperidine (Reference Example 89) (301 mg, 0.816 mmol) in tetrahydrofuran (3.0 ml) and a solution of triethylamine (102 mg, 1.01 mmol) in tetrahydrofuran (2.0 ml) at 0°C, and the mixture was stirred at room temperature for 1 hour. N-methylhydroxylamine hydrochloride (76 mg, 0.91 mmol) and a solution of triethylamine (204 mg, 2.02 mmol) in tetrahydrofuran (2.0 ml) were added to the reaction solution, and the mixture was stirred at 50°C for 1.5 hours and then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (300 mg, 0.679 mmol, 83%) as a white solid.

¹H-NMR (400 MHz, CDCl₃) δ: 1.96 (2H, m), 2.16 (2H, m), 3.00 (3H, s), 3.08-3.18 (3H, m), 3.84 (3H, s), 4.07 (2H, m), 6.77 (1H, s), 6.91 (2H, d, J = 8.8 Hz), 7.32 (2H, d, J = 8.5 Hz), 7.46 (2H, d, J = 8.5 Hz), 7.56 (2H, d, J = 8.8 Hz).

IR (KBr, cm⁻¹): 3277, 1659, 1513, 1256.

EI-MS: m/z = 441 (M⁺).

HR-MS (EI): measured value: 441.1476, calculated value: (C₂₃H₂₄N₃O₄), 441.1455.

### Example 13

### 4-(4-(4-Fluorophenyl)-5-phenyloxazol-2-yl)-1-(N-hydroxy-N-methylcarbamoyl)piperidine

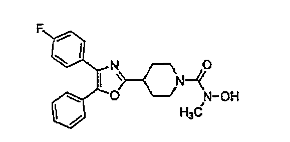

To a solution of 4-(4-(4-fluorophenyl)-5-phenyloxazol-2-yl)piperidine (Reference Example 90) (125 mg, 0.388 mmol) in tetrahydrofuran (2.0 ml) was added triphosgene (42 mg, 0.14 mmol) and triethylamine (58 µl, 0.42 mmol) at 0°C, and the mixture was stirred at room temperature for 1.5 hours. N-methylhydroxylamine hydrochloride (34 mg, 0.40 mmol) and triethylamine (116 µl, 0.848 mmol) were added to the reaction solution, and the mixture was stirred at room temperature for 24 hours. 1M hydrochloric acid was added to the reaction solution, followed by extraction with ethyl acetate. The organic layer was dried over magnesium sulfate and concentrated under a reduced pressure. The residue was purified by column chromatography (amine silica gel, n-hexane/ethyl acetate = 1/3) to give a title compound (92 mg, 0.23 mmol, 59%) as a white amorphous product.

¹H-NMR (400 MHz, CDCl₃) δ: 1.98 (2H, m), 2.18 (2H, m), 3.00 (3H, s), 3.12-3.18 (3H, m), 4.08 (2H, m), 6.78 (1H, brs), 7.06 (2H, t, J = 8.8 Hz), 7.31-7.39 (3H, m), 7.52-7.54 (2H, m), 7.60 (2H, dd, J = 5.4, 8.8 Hz).

IR (KBr, cm⁻¹): 3246, 1653, 1626, 1511, 1445, 1225.

ESI-MS: m/z = 396 (M+H⁺).

### Example 14

### Methyl 4-(4,5-bis(4-methoxyphenyl)oxazol-2-yl)-1-(N-hydroxy-N-methylcarbamoyl)-4-piperidinecarboxylate

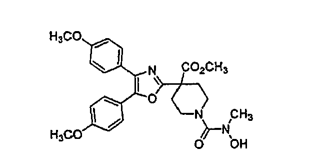

To a solution of triphosgene (13 mg, 0.044 mmol) in dichloromethane (1.0 ml) was added methyl 4-(4,5-bis(4-methoxyphenyl)oxazol-2-yl)-4-piperidinecarboxylate (Reference Example 91) (53 mg, 0.12 mmol) and triethylamine (17 µl, 0.12 mmol) at -78°C, and the mixture was gradually heated to room temperature. 1M hydrochloric acid was added to the reaction solution to separate the solution. The organic layer was dried over sodium sulfate and concentrated under a reduced pressure. The residue was dissolved with the addition of dichloromethane (2.0 ml), N-methylhydroxylamine hydrochloride (11 mg, 0.13 mmol) and triethylamine (20 µl, 0.14 mmol) were added thereto, and the mixture was stirred at room temperature for 14 hours. The reaction solution was concentrated under a reduced pressure, and the residue was purified by flash chromatography (amine silica gel, n-hexane/ethyl acetate) to give a title compound (30 mg, 0.060 mmol, 50%) as a white amorphous product.

¹H-NMR (400 MHz, CDCl₃) δ: 2.34 (2H, m), 2.48 (2H, m), 2.97 (3H, s), 3.45 (2H, m), 3.72 (2H, m), 3.76 (3H, s), 3.83 (3H, s), 3.84 (3H, s), 6.72 (1H, brs), 6.89 (2H, d, J = 8.8 Hz), 6.90 (2H, d, J = 9.0 Hz), 7.48 (2H, d, J = 8.8 Hz), 7.57 (2H, d, J = 9.0 Hz).

IR (KBr, cm⁻¹): 3248, 1737, 1649, 1614, 1520, 1500, 1252.

EI-MS: m/z = 495 (M⁺).

HR-MS (EI): measured value: 495.2009, calculated value: (C₂₆H₂₉N₃O₇), 495.2006.

### Example 15

### 4-(4,5-Bis(4-methoxyphenyl)oxazol-2-yl)-1-(N-isopropyl-N-hydroxycarbamoyl)piperidine

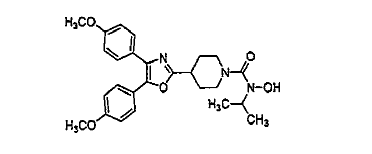

To a solution of triphosgene (49 mg, 0.17 mmol) in dichloromethane (5.0 ml) was added 4-(4,5-bis(4-methoxyphenyl)oxazol-2-yl)piperidine (Reference Example 78) (182 mg, 0.50 mmol) and triethylamine (101 mg, 1.00 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (5.0 ml), N-isopropylhydroxylamine hydrochloride (58 mg, 0.52 mmol) and triethylamine (105 mg, 1.03 mmol) were added thereto, and the mixture was stirred at room temperature for 24 hours. An aqueous solution of saturated ammonium chloride was added to the reaction solution, followed by extraction with ethyl acetate. The organic layer was dried over sodium sulfate and concentrated under a reduced pressure. The residue was purified by flash chromatography (amine silica gel, n-hexane/ethyl acetate) to give a title compound (141 mg, 0.30 mmol, 61%) as a colorless amorphous product.

¹H-NMR (400 MHz, CDCl₃) δ: 1.18 (6H, d, J = 6.6 Hz), 1.91-2.01 (2H, m), 2.14-2.18 (2H, m), 3.09-3.17 (3H, m), 3.49-3.56 (1H, m), 3.83 (3H, s), 3.83 (3H, s), 4.01-4.06 (2H, m), 6.36 (1H, s), 6.87-6.91 (4H, m), 7.48 (2H, d, J = 8.0 Hz), 7.55 (2H, d, J = 8.0 Hz).

IR (KBr, cm⁻¹): 3376, 2932, 1613, 1520, 1500, 1441, 1364, 1298, 1251, 1176, 1032, 834.

EI-MS: m/z = 465 (M⁺).

HR-MS (EI): measured value: 465.2264, calculated value: (C₂₆H₃₁N₃O₅), 465.2264.

### Example 16

### 4-(4,5-Bis(4-methoxyphenyl)oxazol-2-yl)-1-(N-cyclohexyl-N-hydroxycarbamoyl)piperidine

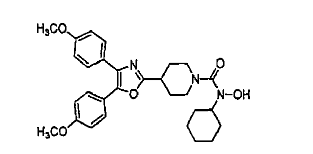

To a solution of triphosgene (49 mg, 0.17 mmol) in dichloromethane (5.0 ml) was added 4-(4,5-bis(4-methoxyphenyl)oxazol-2-yl)piperidine (Reference Example 78) (182 mg, 0.50 mmol) and triethylamine (101 mg, 1.00 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (5.0 ml), N-cyclohexylhydroxylamine hydrochloride (79 mg, 0.52 mmol) and triethylamine (101 mg, 1.00 mmol) were added thereto, and the mixture was stirred at room temperature for 8 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (150 mg, 0.30 mmol, 59%) as a colorless amorphous product.

¹H-NMR (400 MHz, CDCl₃) δ: 1.21-1.28 (2H, m), 1.61-1.73 (6H, m), 1.82-1.86 (2H, m), 1.92-2.01 (2H, m), 2.14-2.18 (2H, m), 3.07-3.18 (4H, m), 3.83 (3H, s), 3.83 (3H, s), 4.00-4.05 (2H, m), 6.44 (1H, s), 6.89 (2H, d, J = 8.0 Hz), 6.90 (2H, d, J = 8.0 Hz), 7.49 (2H, d, J = 8.0 Hz), 7.55 (2H, d, J = 8.0 Hz).

IR (KBr, cm⁻¹): 3423, 2931, 2854, 1613, 1520, 1500, 1442, 1298, 1251, 1176, 1032, 834.

ESI-MS: m/z = 506 (M+H⁺).

HR-MS (ESI): measured value: 506.2670, calculated value: (C₂₉H₃₆N₃O₅+H⁺), 506.2655.

### Example 17

### 4-(4,5-Bis(4-methoxyphenyl)oxazol-2-yl)-1-(N-benzyloxycarbamoyl)piperidine

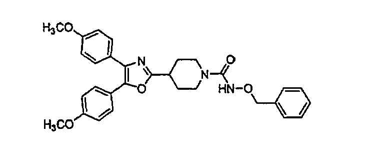

To a solution of O-benzylhydroxylamine (533 mg, 3.34 mmol) in acetonitrile (10 ml) was added sodium bicarbonate (1.34 g, 15.9 mmol) and p-nitrophenyl chloroformate (632 mg, 3.13 mmol). The reaction solution was stirred at room temperature for 4 hours, a solution of 4-(4,5-bis(4-methoxyphenyl)oxazol-2-yl)piperidine (Reference Example 78) (1.02 g, 2.80 mmol) in acetonitrile (5 ml) and triethylamine (0.7 ml, 5.12 mmol) were added thereto, and the mixture was stirred at room temperature for 1 hour. Distilled water was added to the reaction solution, extraction was carried out with ethyl acetate, the resultant was dried over magnesium sulfate, and the resultant was then concentrated under a reduced pressure. The residue was purified by column chromatography (silica gel, n-hexane/ethyl acetate = 1/2 to 1/4) to give a title compound (1.31 g, 2.55 mmol, 91 %) as a white amorphous product.

¹H-NMR (400 MHz, CDCl₃) δ: 1.92 (2H, m), 2.12 (2H, m), 3.00-3.11 (3H, m), 3.83 (3H, s), 3.83 (3H, s), 3.98 (2H, m), 4.88 (2H, s), 6.89 (2H, d, J = 8.8 Hz), 6.91 (2H, d, J = 8.8 Hz), 7.01 (1H, s), 7.34-7.43 (5H, m), 7.48 (2H, d, J = 8.8 Hz), 7.54 (2H, d, J = 8.8 Hz).

### Example 18

### 4-(4,5-Bis(4-methoxyphenyl)oxazol-2-yl)-1-(N-hydroxycarbamoyl)piperidine

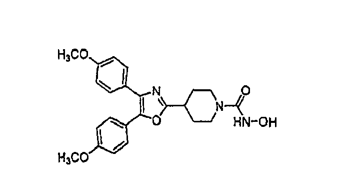

To a solution of hydroxylamine hydrochloride (249 mg, 3.58 mmol) in acetonitrile (10 ml) was added sodium bicarbonate (868 mg, 10.3 mmol) and 4-nitrophenyl chloroformate (706 mg, 3.50 mmol), and the mixture was stirred at room temperature for 4 hours. 4-(4,5-Bis(4-methoxyphenyl)oxazol-2-yl)piperidine (Reference Example 78) (1.30 g, 3.56 mmol) and triethylamine (1.4 ml, 10.2 mmol) were added to the reaction solution, and the mixture was stirred at room temperature for 5 hours. The reaction solution was concentrated under a reduced pressure, and distilled water was added to the residue, followed by extraction with ethyl acetate. The organic layer was washed with 1M hydrochloric acid, dried over magnesium sulfate, and then concentrated under a reduced pressure. The residue was purified by column chromatography (silica gel, n-hexane/ethyl acetate = 1/3 to 1/5) to give a title compound (682 mg, 1.61 mmol, 45%) as a white amorphous product.

¹H-NMR (400 MHz, CDCl₃) δ: 1.92-2.01 (2H, m), 2.14-2.17 (2H, m), 3.05-3.15 (3H, m), 3.83 (3H, s), 3.84 (3H, s), 3.98 (2H, dt, J = 13.6, 4.0 Hz), 6.44 (1H, brs), 6.71 (1H, brs), 6.89 (2H, d, J = 8.4 Hz), 6.90 (2H, d, J = 8.4 Hz), 7.48 (2H, d, J = 8.4 Hz), 7.54 (2H, d, J = 8.4 Hz).

IR (KBr, cm⁻¹): 3348, 3196, 2928, 1647, 1519, 1500, 1297, 1255, 1175, 1031, 835.

### Example 19

### 4-(4,5-Bis(4-methoxyphenyl)oxazol-2-yl)-1-(N-hydroxy-N-ethylcarbamoyl)piperidine

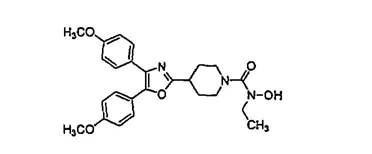

To a solution of 4-(4,5-bis(4-methoxyphenyl)oxazol-2-yl)-1-(N-hydroxycarbamoyl)piperidine (Example 18) (99 mg, 0.234 mmol) in N,N-dimethylformamide (1.0 ml) was added cesium carbonate (76 mg, 0.233 mmol) and bromoethane (17.5 µl). The reaction solution was stirred at room temperature for 18 hours, and distilled water was added, followed by extraction with ethyl acetate. The organic layer was washed with water, dried over magnesium sulfate, and then concentrated under a reduced pressure. The residue was purified by preparative thin-layer chromatography (silica gel, n-hexane/ethyl acetate = 1/3) to give a title compound (11 mg, 0.024 mmol, 10%) as a colorless amorphous product.

¹H-NMR (400 MHz, CDCl₃) δ: 1.24 (3H, t, J = 7.1 Hz), 1.96 (2H, m), 2.16 (2H, m), 3.10-3.22 (5H, m), 3.83 (3H, s), 3.84 (3H, s), 4.05 (2H, m), 6.66 (1H, brs), 6.89 (2H, d, J = 8.8 Hz), 6.90 (2H, d, J = 8.8 Hz), 7.48 (2H, d, J = 8.8 Hz), 7.54 (2H, d, J = 8.8 Hz).

ESI-MS: m/z = 452 (M+H⁺).

### Example 20

### 4-(5-(4-Chlorophenyl)-4-(4-methoxyphenyl)oxazol-2-yl)-1-(N-hydroxy-N-methylcarbamoyl)-piperidine

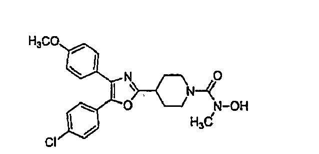

To a solution of (1-(4-chlorophenyl)-2-(4-methoxyphenyl)-2-oxoethyl) 1-(N-hydroxy-N-methylcarbamoyl)-4-piperidinecarboxylate (Reference Example 44) (97 mg, 0.21 mmol) in acetic acid (1.0 ml) was added ammonium acetate (165 mg, 2.14 mmol), and the mixture was stirred at 90°C for 4 hours. The reaction solution was cooled to room temperature and neutralized with the addition of saturated aqeous sodium bicarbonate, followed by extraction with ethyl acetate. The organic layer was dried over magnesium sulfate and concentrated under a reduced pressure. The residue was purified by flash chromatography (amine silica gel, n-hexane/ethyl acetate) to give a title compound (54 mg, 0.12 mmol, 57 %) as a white amorphous product.

¹H-NMR (400 MHz, CDCl₃) δ: 1.97 (2H, m), 2.17 (2H, m), 3.00 (3H, s), 3.07-3.19 (3H, m), 3.84 (3H, s), 4.08 (2H, m), 6.77 (1H, brs), 6.91 (2H, d, J = 8.8 Hz), 7.32 (2H, d, J = 8.3 Hz), 7.49 (2H, d, J = 8.3 Hz), 7.52 (2H, d, J = 8.8 Hz).

IR (KBr, cm⁻¹): 3247, 1649, 1624, 1513, 1488, 1251.

EI-MS: m/z = 441 (M⁺).

HR-MS (EI): measured value: 441.1429, calculated value: (C₂₃H₂₄ClN₃O₄), 441.1455.

### Example 21

### 4-(4,5-Bis(4-methoxyphenyl)oxazol-2-yl)-1-(N-hydroxy-N-methylcarbamoyl)piperazine

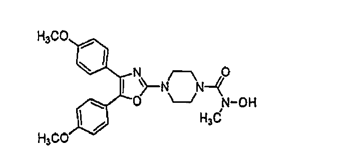

A title compound (73 mg, 0.17 mmol, 41%) was obtained as a white amorphous product from 4-(4,5-bis(4-methoxyphenyl)oxazol-2-yl)piperazine (Reference Example 92) (150 mg, 0.41 mmol) in accordance with the method of Example 3.

¹H-NMR (400 MHz, CDCl₃) δ: 3.02 (3H, s), 3.62 (8H, m), 3.82 (3H, s), 3.82 (3H, s), 6.65 (1H, s), 6.85 (2H, d, J = 8.8 Hz), 6.88 (2H, d, J = 8.8 Hz), 7.41 (2H, d, J = 8.8 Hz), 7.54 (2H, d, J = 8.8 Hz).

ESI-MS: m/z = 439 (M+H⁺).

### Example 22

### 4-(4,5-Bis(4-methoxyphenyl)oxazol-2-yl)-1-(N-methoxy-N-methylcarbarnoyl)piperazine

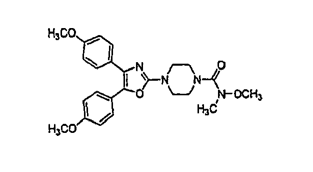

A title compound (38 mg, 0.084 mmol, 25%) was obtained as a white solid from 4-(4,5-bis(4-methoxyphenyl)oxazol-2-yl)piperazine (Reference Example 92) (120 mg, 0.33 mmol) in accordance with the method of Example 1.

¹H-NMR (400 MHz, CDCl₃) δ: 3.01 (3H, s), 3.61 (11H, m), 3.82 (3H, s), 3.83 (3H, s), 6.85 (2H, d, J = 8.8 Hz), 6.88 (2H, d, J = 8.8 Hz), 7.41 (2H, d, J = 8.8 Hz), 7.54 (2H, d, J = 8.8 Hz).

ESI-MS: m/z = 453 (M+H⁺).

### Example 23

### 4-(4,5-Bis(4-methoxyphenyl)oxazol-2-yl)-1-(N-methoxycarbonyloxy-N-methylcarbamoyl)piperidine

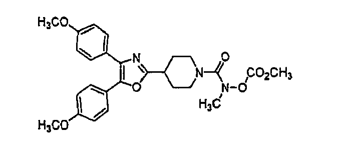

To a solution of 4-(4,5-bis(4-methoxyphenyl)oxazol-2-yl)-1-(N-hydroxy-N-methylcarbamoyl)piperidine (Example 1) (145 mg, 0.331 mmol) in dichloromethane (5.0 ml) was added triethylamine (54 µl, 0.39 mmol) and methyl chloroformate (36 µl, 0.34 mmol). The reaction solution was stirred at room temperature for 1 hour and then concentrated under a reduced pressure. Distilled water was added to the residue, followed by extraction with ethyl acetate. The organic layer was dried over magnesium sulfate and concentrated under a reduced pressure. The residue was purified by column chromatography (silica gel, n-hexane/ethyl acetate = 1/2) to give a title compound (134 mg, 0.270 mmol, 81%) as a white amorphous product.

¹H-NMR (400 MHz, CDCl₃) δ: 1.96 (2H, m), 2.15 (2H, m), 3.06-3.16 (6H, m), 3.83 (3H, s), 3.83 (3H, s), 3.88 (3H, s), 4.04 (2H, m), 6.89 (2H, d, J = 8.8 Hz), 6.90 (2H, d, J = 8.8 Hz), 7.48 (2H, d, J = 8.8 Hz), 7.54 (2H, d, J = 8.8 Hz).

### Example 24

### 4-(4,5-Bis(4-methoxyphenyl)oxazol-2-yl)-1-(N-methoxy-N-methylcarbamoyl)piperidine

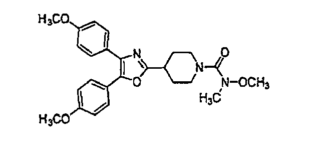

A title compound (198 mg, 0.439 mmol, 43%) was obtained as a white amorphous product from 4-(4,5-bis(4-methoxyphenyl)oxazol-2-yl)piperidine (Reference Example 78) (369 mg, 1.01 mmol) in accordance with the method of Example 1.

¹H-NMR (400 MHz, CDCl₃) δ: 1.90-2.00 (2H, m), 2.15 (2H, dd, J = 13.2, 3.2 Hz), 2.98 (3H, s), 3.03-3.12 (3H, m), 3.61 (3H, s), 3.83 (6H, s), 4.11 (2H, dt, J = 13.2, 3.2 Hz), 6.89 (2H, d, J = 8.8 Hz), 6.89 (2H, d, J = 8.4 Hz), 7.49 (2H, d, J = 8.8 Hz), 7.55 (2H, d, J = 8.4 Hz).

IR (neat, cm⁻¹): 2932, 1660, 1612, 1579, 1519, 1500, 1432, 1297, 1250, 1175, 1031, 834.

ESI-MS: m/z = 452 (M+H⁺).

### Example 25

### 4-(4,5-Bis(4-methoxyphenyl)oxazol-2-yl)-1-(N-tert-butoxycarbonylinethyloxy-N-methylcarbamoyl)piperidine

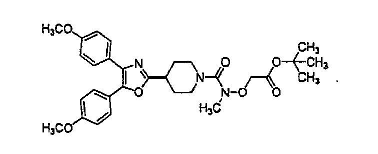

To a solution of 4-(4,5-bis(4-methoxyphenyl)oxazol-2-yl)-1-(N-hydroxy-N-methylcarbamoyl)piperidine (Example 1) (149 mg, 0.34 mmol) in dimethylformamide (5 ml) was added 55% sodium hydride (16.3 mg, 0.37 mmol) at 0°C. The reaction solution was stirred at room temperature for 10 minutes, tert-butyl bromoacetate (73 mg, 0.37 mmol) was added at 0°C, and the resultant was stirred at room temperature for 3 hours. An aqueous solution of saturated ammonium chloride was added to the reaction solution, followed by extraction with ethyl acetate. The organic layer was dried over sodium sulfate and then concentrated under a reduced pressure. The residue was purified by column chromatography (silica gel, n-hexane/ethyl acetate = 5/1 to 3/1) to give a title compound (170 mg, 0.308 mmol, 91%) as a light yellow amorphous product.

¹H-NMR (400 MHz, CDCl₃) δ: 1.48 (9H, s), 1.94 (2H, dq, J = 11.0, 4.0 Hz), 2.12-2.16 (2H, m), 3.03-3.10 (3H, m), 3.05 (3H, s), 3.83 (6H, s), 4.08 (2H, dt, J = 13.7, 3.4 Hz), 4.22 (2H, s), 6.88 (2H, d, J = 9.0 Hz), 6.89 (2H, d, J = 9.0 Hz), 7.49 (2H, d, J = 9.0 Hz), 7.55 (2H, d, J = 9.0 Hz).

IR (KBr, cm⁻¹): 2932, 1750, 1680, 1520, 1501, 1298, 1251, 1175, 1031, 835.

### Example 26

### 4-(4,5-Bis(4-methoxyphenyl)oxazol-2-yl)-1-(N-carboxymethyloxy-N-methylcarbamoyl)piperidine

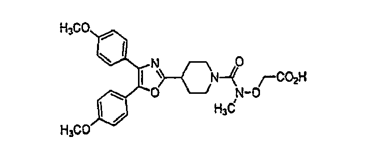

To a solution of 4-(4,5-bis(4-methoxyphenyl)oxazol-2-yl)-1-(N-tert-butoxycarbonylmethyloxy-N-methylcarbamoyl)piperidine (Example 25) (160 mg, 0.29 mmol) in dichloromethane (2.0 ml) was added 2,6-lutidine (337 µl, 2.9 mmol) and trimethylsilyl triflate (262 µl, 1.45 mmol) at 0°C. The reaction solution was stirred at room temperature for 21 hours, and 1M hydrochloric acid was added, followed by extraction with dichloromethane. The organic layer was dried over sodium sulfate and then concentrated under a reduced pressure. The residue was purified by column chromatography (diol-silica gel, ethyl acetate) to give a title compound (13.3 mg, 0.027 mmol, 9%) as a white amorphous product.

¹H-NMR (400 MHz, CDCl₃) δ: 1.98 (2H, dq, J = 14.0, 3.6 Hz), 2.16-2.20 (2H, m), 3.10-3.18 (3H, m), 3.13 (3H, s), 3.83 (6H, s), 3.95-3.98 (2H, m), 4.48 (2H, s), 6.89 (2H, d, J = 8.8 Hz), 6.90 (2H, d, J = 8.8 Hz), 7.48 (2H, d, J = 8.8 Hz), 7.54 (2H, d, J = 8.8 Hz).

### Example 27

### 4-(4,5-Bis(4-methoxyphenyl)thiazol-2-yl)-1-(N-hydroxy-N-methylcarbamoyl)piperidine

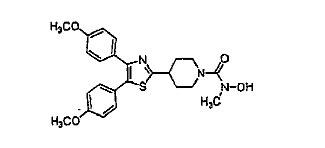

To a solution of triphosgene (66 mg, 0.22 mmol) in dichloromethane (5.0 ml) was added 4-(4,5-bis(4-methoxyphenyl)thiazol-2-yl)piperidine (Reference Example 93) (249 mg, 0.65 mmol) and triethylamine (80 µl, 0.58 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (5.0 ml), N-methylhydroxylamine hydrochloride (57 mg, 0.69 mmol) and triethylamine (142 µl, 1.38 mmol) were added thereto, and the resultant was stirred at room temperature for 24 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (118 mg, 0.26 mmol, 40%) as a colorless amorphous product.

¹H-NMR (400 MHz, CDCl₃) δ: 1.81-1.91 (2H, m), 2.18-2.22 (2H, m), 3.00 (3H, s), 3.03-3.10 (2H, m), 3.20-3.28 (1H, m), 3.79 (3H, s), 3.81 (3H, s), 4.14-4.17 (2H, m), 6.81 (2H, d, J = 8.0 Hz), 6.84 (2H, d, J = 8.0 Hz), 6.91 (1H, s), 7.24 (2H, d, J = 8.0 Hz), 7.43 (2H, d, J = 8.0 Hz).

IR (KBr, cm⁻¹): 3245, 2930, 1735, 1609, 1515, 1495, 1440, 1294, 1249, 1176, 1109, 1033, 890, 829; 753.

EI-MS: m/z = 453 (M⁺).

HR-MS (EI): measured value: 453.1700, calculated value: (C₂₄H₂₇N₃O₄S), 453.1722.

### Example 28

### 4-(4-(4-Chlorophenyl)-5-(4-tolyl)thiazol-2-yl)-1-(N-hydroxy-N-methylcarbamoyl)piperidine

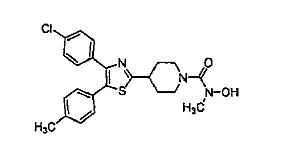

To a solution of triphosgene (90 mg, 0.30 mmol) in dichloromethane (10 ml) was added 4-(4-(4-chlorophenyl)-5-(4-tolyl)thiazol-2-yl)piperidine hydrochloride (Reference Example 94) (180 mg, 0.44 mmol) and triethylamine (184 µl, 1.78 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (10 ml), N-methylhydroxylamine hydrochloride (78 mg, 0.94 mmol) and triethylamine (193 µl, 1.87 mmol) were added thereto, and the resultant was stirred at room temperature for 20 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (67 mg, 0.15 mmol, 34%) as a colorless amorphous product.

¹H-NMR (400 MHz, CDCl₃) δ: 1.81-1.92 (2H, m), 2.19-2.22 (2H, m), 2.36 (3H, s), 3.00 (3H, s), 3.04-3.11 (2H, m), 3.22-3.28 (1H, m), 4.14-4.18 (2H, m), 6.83 (1H, s), 7.12-7.26 (6H, m), 7.44 (2H, d, J = 8.4 Hz).

IR (KBr, cm⁻¹): 3253, 2920, 1628, 1490, 1440, 1399, 1309, 1183, 1090, 1013, 878, 836,814,754.

EI-MS: m/z = 442 (M+H⁺).

### Example 29

### 4-(4-(4-Chlorophenyl)-5-phenylthiazol-2-yl)-1-(N-hydroxy-N-methylcarbamoyl)piperidine

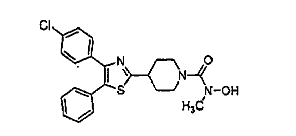

To a solution of triphosgene (74 mg, 0.25 mmol) in dichloromethane (5.0 ml) was added 4-(4-(4-chlorophenyl)-5-phenylthiazol-2-yl)piperidine (Reference Example 95) (261 mg, 0.74 mmol) and triethylamine (160 µl, 1.17 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (10 ml), N-methylhydroxylamine hydrochloride (65 mg, 0.77 mmol) and triethylamine (160 µl, 1.17 mmol) were added thereto, and the resultant was stirred at room temperature for 24 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (95 mg, 0.22 mmol, 30%) as a colorless amorphous product.

¹H-NMR (400 MHz, CDCl₃) δ: 1.83-1.93 (2H, m), 2.20-2.23 (2H, m), 3.01 (3H, s), 3.04-3.11 (2H, m), 3.22-3.30 (1H, m), 4.15-4.19 (2H, m), 6.85 (1H, s), 7.23-7.34 (7H, m), 7.41 (2H, d, J = 8.0 Hz).

IR (KBr, cm⁻¹): 3427, 2921, 1627, 1494, 1442, 1399, 1308, 1090, 1013, 876, 836, 760, 696.
EI-MS: m/z = 428 (M+H⁺).

### Example 30

### 4-(4,5-Diphenylthiazol-2-yl)-1-(N-hydroxy-N-methylcarbamoyl)piperidine

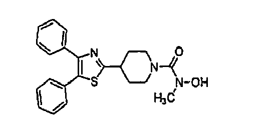

To a solution of triphosgene (85 mg, 0.29 mmol) in dichloromethane (10 ml) was added 4-(4,5-diphenylthiazol-2-yl)piperidine hydrochloride (Reference Example 96) (300 mg, 0.841 mmol) and triethylamine (174 µl, 1.27 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (10 ml), N-methylhydroxylamine hydrochloride (74 mg, 0.88 mmol) and triethylamine (183 µl, 1.34 mmol) were added thereto, and the resultant was stirred at room temperature for 21 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (210 mg, 0.53 mmol, 63%) as a colorless amorphous product.

¹H-NMR (400 MHz, CDCl₃) δ: 1.83-1.93 (2H, m), 2.20-2.24 (2H, m), 3.00 (3H, s), 3.03-3.11 (2H, m), 3.24-3.31 (1H, m), 4.15-4.19 (2H, m), 6.94 (1H, s), 7.25-7.33 (8H, m), 7.48-7.50 (2H, m).

IR (KBr, cm⁻¹): 3256, 2921, 1627, 1484, 1441, 1398, 1308, 1253, 1179, 1127, 1071, 1005,890,758,697.

EI-MS: m/z = 394 (M+H⁺).

### Example 31

### 4-(4,5-Bis(4-methoxyphenyl)-1H-imidazol-2-yl)-1-(N-hydroxy-N-methylcarbamoyl)piperidine

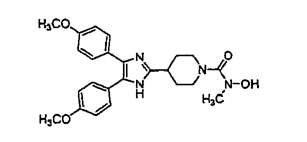

To a solution of triphosgene (137 mg, 0.46 mmol) in dichloromethane (5.0 ml) was added a solution of 4-(4,5-bis(4-methoxyphenyl)-1H-imidazol-2-yl)piperidine (Reference Example 69) (510 mg, 1.4 mmol) in tetrahydrofuran (10 ml) and pyridine (113 µl, 1.40 mmol) at -78°C, and the mixture was gradually heated to room temperature. Distilled water was added to the reaction solution, followed by extraction with ethyl acetate. The organic layer was dried over magnesium sulfate and then concentrated under a reduced pressure. The residue was dissolved with the addition of dichloromethane (10 ml), N-methylhydroxylamine hydrochloride (101 mg, 1.21 mmol) and triethylamine (350 µl, 2.56 mmol) were added thereto, and the resultant was stirred at room temperature for 21 hours. An aqueous solution of saturated ammonium chloride was added to the reaction solution, followed by extraction with ethyl acetate. The organic layer was dried over magnesium sulfate and concentrated under a reduced pressure. The residue was purified by flash chromatography (amine silica gel, n-hexane/ethyl acetate) to give a title compound (230 mg, 0.53 mmol, 38%) as a white solid.

¹H-NMR (400 MHz, CD₃OD) δ: 1.82-1.92 (2H, m), 1.97-2.01 (2H, m), 2.94-3.03 (6H, m), 3.30 (1H, s), 3.78 (6H, s), 4.21-4.25 (2H, m), 6.85-6.87 (4H, m), 7.28-7.31 (4H, m).

IR (KBr, cm⁻¹): 3212, 2937, 1657, 1517, 1442, 1402, 1294, 1248, 1176, 1107, 1033, 972, 836.

EI-MS: m/z = 436 (M⁺).

HR-MS (EI): measured value: 436.2093, calculated value: (C₂₄H₂₈N₄O₄), 436.2111.

### Example 32

### 4-(4,5-Bis(4-methoxyphenyl)-1-methoxycarbonylmethyl-1H-imidazol-2-yl)-1-(N-hydroxy-N-methylcarbamoyl)piperidine

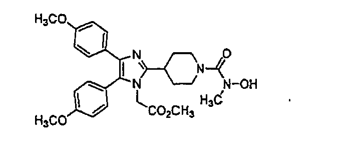

To a solution of 4-(4,5-bis(4-methoxyphenyl)-l-methoxycarbonylmethyl-1H-imidazol-2-yl)piperidine (Reference Example 97) (306 mg, 0.70 mmol) in dichloromethane (10 ml) was added triphosgene (69 mg, 0.23 mmol) and triethylamine (106 mg, 1.0 mmol) at - 78°C, and the mixture was gradually heated to room temperature. N-methylhydroxylamine hydrochloride (76 mg, 0.91 mmol) and triethylamine (212 mg, 2.1 mmol) were added to the reaction solution, and the resultant was stirred at room temperature for 3 hours. Distilled water was added to the reaction solution, followed by extraction with ethyl acetate. The organic layer was dried over sodium sulfate and then concentrated under a reduced pressure. The residue was purified by flash chromatography (silica gel, n-hexane/ethyl acetate) to give a title compound (300 mg, 0.59 mmol, 84%) as a white amorphous product.

¹H-NMR (400 MHz, CDCl₃) δ: 1.62-1.70 (1H, m), 1.93-1.96 (2H, m), 2.06-2.15 (2H, m), 2.72-2.78 (1H, m), 3.01 (3H, s), 3.03-3.08 (2H, m), 3.73 (3H, s), 3.75 (3H, s), 3.86 (3H, s), 4.18-4.21 (2H, m), 4.45 (2H, s), 6.74 (2H, d, J = 8.0 Hz), 6.95 (2H, d, J = 8.0 Hz), 7.19 (2H, d, J = 8.0 Hz), 7.39 (2H, d, J = 8.0Hz).

IR (KBr, cm⁻¹): 3436, 2953, 1750, 1618, 1521, 1442, 1334, 1291, 1250, 1178, 1110, 1032, 981, 838, 748.

ESI-MS: m/z = 509 (M+H⁺).

### Example 33

### 4-(4,5-Bis(4-methoxyphenyl)-1-carboxymethyl-1H-imidazol-2-yl)-1-(N-hydroxy-N-methylcarbamoyl)piperidine

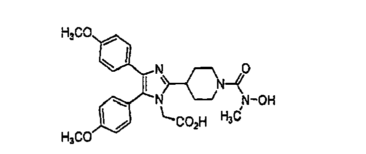

To a solution of 4-(4,5-bis(4-methoxyphenyl)-1-methoxycarbonylmethyl-1H-imidazol-2-yl)-1-(N-hydroxy-N-methylcarbamoyl)piperidine (Example 32) (46 mg, 0.090 mmol) in methanol (2.0 ml) was added lithium hydroxide monohydrate (5.7 mg, 0.136 mmol) and four drops of distilled water, and the resultant was stirred at room temperature for 24 hours. Acetic acid (0.2 ml) was added to the reaction solution and then concentrated under a reduced pressure. The residue was purified by column chromatography (silica gel, dichloromethane/methanol = 20/1 to 10/1) to give a title compound (35 mg, 0.071 mmol, 79%) as a white amorphous product.

¹H-NMR (400 MHz, DMSO-d₆) δ: 1.70-1.83 (4H, m), 2.83 (3H, s), 2.83-2.89 (3H, m), 3.68 (3H, s), 3.79 (3H, s), 4.07-4.11 (2H, m), 4.27 (2H, s), 6.74 (2H, d, J = 8.8 Hz), 6.99 (2H, d, J = 8.8 Hz), 7.19 (2H, d, J = 8.8 Hz), 7.27 (2H, d, J = 8.8 Hz), 9.25 (1H, brs).

IR (KBr, cm⁻¹): 3437, 2931, 1614, 1521, 1502, 1442, 1384, 1293, 1252, 1180, 1031, 837.

ESI-MS: m/z = 495 (M+H⁺).

### Example 34

### 4-(4,5-Bis(4-methoxyphenyl)-1H-pyrrol-2-yl)-1-(N-hydroxy-N-methylcarbamoyl)piperidine

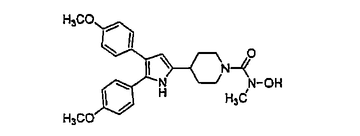

To a solution of (4-(4,5-bis(4-methoxyphenyl)-1H-pyrrol-2-yl)piperidine (Reference Example 98) (31 mg, 0.084 mmol) in dichloromethane (5.0 ml) were added triphosgene (9 mg, 0.029 mmol) and triethylamine (9 mg, 0.09 mmol) at -78°C, and the mixture was gradually heated to room temperature. N-methylhydroxylamine hydrochloride (8 mg, 0.09 mmol) and triethylamine (26 mg, 0.26 mmol) were added to the reaction solution, and the mixture was stirred at room temperature for 17 hours. Distilled water was added to the reaction solution, followed by extraction with ethyl acetate. The organic layer was dried over sodium sulfate and concentrated under a reduced pressure. The residue was purified by preparative thin-layer chromatography (silica gel, n-hexane/ethyl acetate = 1/1) to give a title compound (16.5 mg, 0.0379 mmol, 45%) as a light yellow amorphous product.

¹H-NMR (400 MHz, CDCl₃) δ: 1.72 (2H, dq, J = 12.4, 4.0 Hz), 2.04-2.07 (2H, m), 2.85 (1H, tt, J = 11.6, 4.0 Hz), 2.98-3.04 (2H, m), 2.99 (3H, s), 3.79 (3H, s), 3.81 (3H, s), 4.13-4.17 (2H, m), 6.07 (1H, d, J = 2.8 Hz), 6.80 (1H, s), 6.81 (2H, d, J = 8.8 Hz), 6.84 (2H, d, J = 8.8 Hz), 7.23 (2H, d, J = 8.8 Hz), 7.24 (2H, d, J = 8.8 Hz), 7.92 (1H, brs).

IR (KBr, cm⁻¹): 3407, 2935, 1636, 1520, 1441, 1246, 1179, 1033, 834.

EI-MS: m/z = 435 (M⁺).

HR-MS (EI): measured value: 435.2148, calculated value: (C₂₅H₂₉N₃O₄), 435.2158.

### Example 35

### 4-(4,5-Bis(4-methoxyphenyl)-1-tert-butoxycarbonylmethylpyrrol-2-yl)-1-(N-hydroxy-N-methylcarbamoyl)piperidine

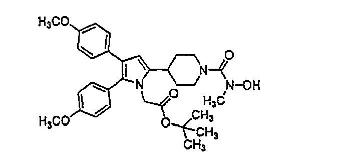

To a solution of 4-(4,5-bis(4-methoxyphenyl)-1-tert-butoxycarbonylmethylpyrrol-2-yl)piperidine (Reference Example 99) (90 mg, 0.19 mmol) in dichloromethane (3.0 ml) was added triphosgene (19 mg, 0.063 mmol) and triethylamine (19 mg, 0.19 mmol) at -78°C, and the mixture was gradually heated to room temperature. N-methylhydroxylamine hydrochloride (18 mg, 0.21 mmol) and triethylamine (57 mg, 0.56 mmol) were added to the reaction solution, and the mixture was stirred at room temperature for 5 hours. An aqueous solution of saturated ammonium chloride was added to the reaction solution, followed by extraction with ethyl acetate. The organic layer was dried over sodium sulfate and concentrated under a reduced pressure. The residue was purified by column chromatography (amine silica gel, n-hexane/ethyl acetate = 1/1) to give a title compound (51 mg, 0.093 mmol, 49%) as a colorless oil product.

¹H-NMR (400 MHz, CDCl₃) δ: 1.41 (9H, s), 1.73 (2H, dq, J = 13.2, 4.0 Hz), 1.98-2.01 (2H, m), 2.59 (1H, tt, J = 11.6, 4.0 Hz), 2.91-2.97 (2H, m), 3.00 (3H, s), 3.73 (3H, s), 3.83 (3H, s), 4.17-4.20 (2H, m), 4.34 (2H, s), 6.17 (1H, s), 6.70 (2H, d, J = 8.8 Hz), 6.88 (2H, d, J = 8.8 Hz), 7.07 (2H, d, J = 8.8 Hz), 7.18 (2H, d, J = 8.8 Hz).

IR (KBr, cm⁻¹): 2936, 1743, 1614, 1519, 1442, 1288, 1246, 1177, 1153, 1034, 836.

ESI-MS: m/z = 550 (M+H⁺).

### Example 36

### 4-(4,5-Bis(4-methoxyphenyl)-1-carboxymethylpyrrol-2-yl)-1-(N-hydroxy-N-methylcarbamoyl)piperidine

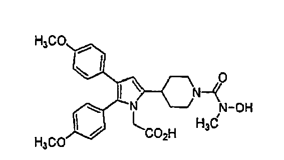

To a solution of 4-(4,5-bis(4-methoxyphenyl)-1-tert-butoxycarbonylmethylpyrrol-2-yl)-1-(N-hydroxy-N-methylcarbamoyl)piperidine (Example 35) (51 mg, 0.093 mmol) in dichloromethane (2.0 ml) was added 2,6-lutidine (108 µl, 0.93 mmol) and trimethylsilyl triflate (84 µl, 0.46 mmol) at 0°C. The reaction solution was stirred at room temperature for 18 hours, and 1M hydrochloric acid was added thereto, followed by extraction with dichloromethane. The organic layer was dried over sodium sulfate and concentrated under a reduced pressure. The residue was purified by preparative thin-layer chromatography (silica gel, dichloromethane/methanol/acetic acid = 9/1/1) to give a title compound (30 mg, 0.061 mmol, 66%) as a light yellow amorphous product.

¹H-NMR (400 MHz, CDCl₃) δ: 1.43-1.52 (2H, m), 1.85-1.90 (2H, m), 2.63-2.69 (1H, m), 2.76-2.81 (2H, m), 2.81 (3H, s), 3.66 (3H, s), 3.76 (3H, s), 4.08-4.11 (2H, m), 4.14 (2H, s), 6.00 (1H, s), 6.69 (2H, d, J = 8.8 Hz), 6.89 (2H, d, J = 8.4 Hz), 6.96 (2H, d, J = 8.8 Hz), 7.14 (2H, d, J = 8.4 Hz), 9.27 (1H, brs).

IR (KBr, cm⁻¹): 3412, 2938, 1612, 1518, 1442, 1288, 1246, 1179, 1033, 836.

ESI-MS: m/z = 494 (M+H⁺).

### Example 37

### 4-(4,5-Bis(4-methoxyphenyl)-1-hexylpyrrol-2-yl)-1-(N-hydroxy-N-methylcarbamoyl)piperidine

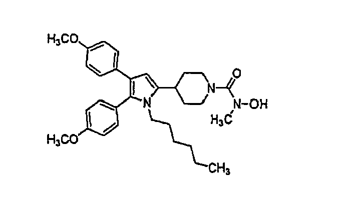

To a solution of 4-(4,5-bis(4-methoxyphenyl)-1-hexylpyrrol-2-yl)piperidine (Reference Example 100) (87 mg, 0.195 mmol) in dichloromethane (2.0 ml) was added triphosgene (19 mg, 0.065 mmol) and triethylamine (20 mg, 0.065 mmol) at -78°C, and the mixture was gradually heated to room temperature. N-methylhydroxylamine hydrochloride (20 mg, 0.23 mmol) and triethylamine (40 mg, 0.39 mmol) were added to the reaction solution, and the mixture was stirred at room temperature for 19 hours. An aqueous solution of saturated ammonium chloride was added to the reaction solution then the solution was separated. The organic layer was dried over sodium sulfate and concentrated under a reduced pressure. The residue was purified by column chromatography (amine silica gel, n-hexane/ethyl acetate = 1/1) to give a title compound (27 mg, 0.053 mmol, 27%) as a light yellow amorphous product.

¹H-NMR (400 MHz, CDCl₃) δ: 0.81 (3H, t, J = 6.8 Hz), 1.11-1.20 (6H, m), 1.43-1.47 (2H, m), 1.74 (2H, dq, J = 12.8, 3.6 Hz), 2.01-2.03 (2H, m), 2.76 (1H, tt, J = 11.6, 3.6 Hz), 2.96-3.03 (2H, m), 3.01 (3H, s), 3.69-3.73 (2H, m), 3.73 (3H, s), 3.85 (3H, s), 4.19-4.23 (2H, m), 6.12 (1H, s), 6.69 (2H, d, J = 8.8 Hz), 6.91 (2H, d, J = 8.4 Hz), 7.04 (2H, d, J = 8.8 Hz), 7.20 (2H, d, J = 8.4 Hz).

IR (KBr, cm⁻¹): 3253, 2930, 1613, 1517, 1465, 1441, 1286, 1246, 1178, 1034, 835.

ESI-MS: m/z = 519 (M+H⁺).

### Example 38

### 4-(4,5-bis(4-methoxyphenyl)furan-2-yl)-1-(N-hydroxy-N-methylcarbamoyl)piperidine

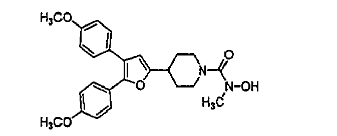

To a solution of 4-(4,5-bis(4-methoxyphenyl)furan-2-yl)piperidine (Reference Example 101) (80 mg, 0.22 mmol) in dichloromethane (2.0 ml) was added triphosgene (22 mg, 0.073 mmol) and triethylamine (22 mg, 0.22 mmol) at -78°C, and the mixture was gradually heated to room temperature. N-methylhydroxylamine hydrochloride (20 mg, 0.24 mmol) and triethylamine (66 mg, 0.66 mmol) were added to the reaction solution, and the mixture was stirred at room temperature for 5 hours. An aqueous solution of saturated ammonium chloride was added to the reaction solution, followed by extraction with ethyl acetate. The organic layer was dried over sodium sulfate and concentrated under a reduced pressure. The residue was purified by preparative thin-layer chromatography (silica gel, n-hexane/ethyl acetate = 1/1) to give a title compound (36 mg, 0.082 mmol, 37%) as a light yellow amorphous product.

¹H-NMR (400 MHz, CDCl₃) δ: 1.74 (2H, dq, J = 11.6, 4.0 Hz), 2.09-2.13 (2H, m), 2.94 (1H, tt, J = 11.6, 3.6 Hz), 2.99 (3H, s), 3.02-3.10 (2H, m), 3.80 (3H, s), 3.83 (3H, s), 4.10-4.13 (2H, m), 6.12 (1H, d, J = 0.7 Hz), 6.82 (2H, d, J = 8.8 Hz), 6.88 (2H, d, J = 8.8 Hz), 7.30 (2H, d, J = 8.8 Hz), 7.42 (2H, d, J = 8.8 Hz).

IR (KBr, cm⁻¹): 3261, 2933, 1612, 1518, 1497, 1441, 1295, 1250, 1176, 1029, 834.

ESI-MS: m/z = 437 (M+H⁺).

### Example 39

### 4-(4,5-Bis(4-methoxyphenyl)thiophen-2-yl)-1-(N-hydroxy-N-methylcarbamoyl)piperidine

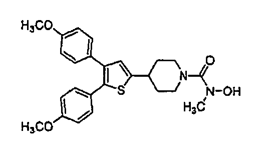

To a solution of 4-(4,5-bis(4-methoxyphenyl)thiophen-2-yl)piperidine (Reference Example 102) (35 mg, 0.092 mmol) in dichloromethane (1.0 ml) was added triphosgene (9 mg, 0.030 mmol) and triethylamine (9 mg, 0.09 mmol) at -78°C, and the mixture was gradually heated to room temperature. N-methylhydroxylamine hydrochloride (9 mg, 0.11 mmol) and triethylamine (19 mg, 0.18 mmol) were added to the reaction solution, and the mixture was stirred at room temperature for 2 hours. Saturated aqueous ammonium chloride was added to the reaction solution, followed by extraction with ethyl acetate. The organic layer was dried over sodium sulfate and concentrated under a reduced pressure. The residue was purified by preparative thin-layer chromatography (silica gel, n-hexane/ethyl acetate = 2/3) to give a title compound (16 mg, 0.035 mmol, 38%) as a light yellow amorphous product.

¹H-NMR (400 MHz, CDCl₃) δ: 1.69-1.79 (2H, m), 2.09-2.13 (2H, m), 2.91-2.97 (1H, m), 2.99 (3H, s), 3.02-3.10 (2H, m), 3.80 (3H, s), 3.83 (3H, s), 4.10-4.13 (2H, m), 6.12 (1H, d, J = 1.2 Hz), 6.81 (1H, s), 6.82 (2H, d, J = 8.8 Hz), 6.88 (2H, d, J = 8.8 Hz), 7.30 (2H, d, J = 8.8 Hz), 7.43 (2H, d, J = 8.8 Hz).

IR (KBr, cm⁻¹): 2928, 2853, 1613, 1519, 1498, 1441, 1248, 1176, 1029, 834.

EI-MS: m/z = 452 (M⁺).
HR-MS (EI): measured value: 452.1749, calculated value: (C₂₅H₂₈N₂O₄S), 452.1770.

### Example 40

### 4-(1,5-Bis(4-methoxyphenyl)-1H-pyrazol-3-yl)-1-(N-hydroxy-N-methylcarbamoyl)piperidine

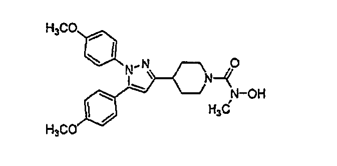

To a solution of 4-(1,5-bis(4-methoxyphenyl)-1H-pyrazol-3-yl)piperidine (Reference Example 103) (266 mg, 0.732 mmol) in dichloromethane (5.0 ml) was added triphosgene (72 mg, 0.24 mmol) and triethylamine (74 mg, 1.0 mmol) at 0°C, and the mixture was stirred at room temperature for hours. N-methylhydroxylamine hydrochloride (67 mg, 0.81 mmol) and triethylamine (222 mg, 2.2 mmol) were added to the reaction solution, and the mixture was stirred at room temperature for 1 hour. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (161 mg, 0.368 mmol, 50%) as a light yellow amorphous product.

¹H-NMR (400 MHz, CDCl₃) δ: 1.78 (2H, dq, J = 4.0, 16.0 Hz), 2.08-2.11 (2H, m), 2.95-3.01 (1H, m), 2.99 (3H, s), 3.04 (2H, dt, J = 2.8, 13.6 Hz), 3.80 (3H, s), 3.81 (3H, s), 4.13-4.17 (2H, m), 6.24 (1H, s), 6.81 (2H, d, J = 8.8 Hz), 6.84 (2H, d, J = 8.8 Hz), 6.87 (1H, s), 7.12 (2H, d, J = 8.8 Hz), 7.19 (2H, d, J = 8.8 Hz).

IR (KBr, cm⁻¹): 3423, 2933, 1646, 1614, 1517, 1441, 1298, 1251, 1179, 1032, 836.

ESI-MS: m/z = 437 (M+H⁺).

Elementary analysis: measured value: C, 65.93, H, 6.53, N, 12.72; calculated value: (C₂₄H₂₈N₄O₄) C, 66.04, 6.47, N, 12.84.

### Example 41

### 4-(5-(4-Methoxyphenyl)-1-phenyl-1H-pyrazol-3-yl)-1-(N-hydroxy-N-methylcarbamoyl)piperidine

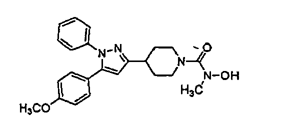

To a solution of 4-(5-(4-methoxyphenyl)-1-phenyl-1H-pyrazol-3-yl)piperidine (Reference Example 104) (146 mg, 0.438 mmol) in dichloromethane (10 ml) was added triphosgene (43 mg, 0.15 mmol) and pyridine (35 mg, 0.44 mmol) at 0°C, and the mixture was stirred at room temperature for 13 hours. Distilled water was added to the reaction solution to separate the solution, and the aqueous layer was extracted with ethyl acetate. The organic layer was mixed, dried over sodium sulfate, and concentrated under a reduced pressure. The residue was dissolved with the addition of dichloromethane, N-methylhydroxylamine hydrochloride (40 mg, 0.48 mmol) and triethylamine (89 mg, 0.88 mmol) were added thereto, and the mixture was stirred at room temperature for 3 hours. Distilled water was added to the reaction solution, followed by extraction with ethyl acetate. The organic layer was dried over magnesium sulfate and concentrated under a reduced pressure. The residue was purified by preparative thin-layer chromatography (silica gel, n-hexane/ethyl acetate = 1/1) to give a title compound (15 mg, 0.038 mmol, 9%) as a light yellow amorphous product.

¹H-NMR (400 MHz, CDCl₃) δ: 1.79 (2H, dq, J = 4.0, 11.6 Hz), 2.08-2.11 (2H, m), 2.95-3.03 (1H, m), 2.99 (3H, s), 3.05 (2H, dt, J = 2.8, 12.0 Hz), 3.80 (3H, s), 4.13-4.17 (2H, m), 6.26 (1H, s), 6.82 (2H, d, J = 8.8 Hz), 6.88 (1H, s), 7.13 (2H, d, J = 8.8 Hz), 7.25-7.35 (5H, m).

IR (KBr, cm⁻¹): 3226, 2922, 2853, 1614, 1508, 1438, 1372, 1250, 1178, 1032, 836.

ESI-MS: m/z = 407 (M+H⁺).

### Example 42

### 4-(4,5-Bis(4-methoxyphenyl)oxazol-2-yl)-1-(N-hydroxy-N-methylcarbamoyl)-4-methylpiperidine

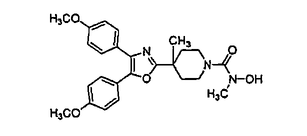

To a solution of 4-(4,5-bis(4-methoxyphenyl)oxazol-2-yl)-4-methylpiperidine (Reference Example 109) (273 mg, 0.721 mmol) in tetrahydrofuran (7.5 ml) was added triphosgene (86 mg, 0.29 mmol) and triethylamine (91 mg, 0.90 mmol) at 0°C, and the mixture was stirred at room temperature for 1 hour. N-methylhydroxylamine hydrochloride (93 mg, 1.11 mmol) and triethylamine (202 mg, 1.99 mmol) were added to the reaction solution, the mixture was stirred at 70°C for 0.5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (272 mg, 0.602 mmol, 83%) as a white amorphous product.

¹H-NMR (400 MHz, CDCl₃) δ: 1.43 (3H, s), 1.63-1.72 (2H, m), 2.35-2.43 (2H, m), 2.96 (3H, s), 3.22-3.31 (2H, m), 3.83 (3H, s), 3.84 (3H, s), 6.81 (1H, s), 6.89 (2H, d, J = 8.8 Hz), 6.90 (2H, d, J = 8.8 Hz), 7.49 (2H, d, J = 8.8 Hz), 7.56 (2H, d, J = 8.8 Hz).

IR (KBr, cm⁻¹): 3390, 1628, 1615, 1514, 1495, 1441, 1250.

ESI-MS: m/z = 452 (M+H⁺).

### Example 43

### Benzyl 4-(4,5-bis(4-methoxyphenyl)oxazol-2-yl)-1-(N-hydroxy-N-methylcarbamoyl)piperidine-4-carboxylate

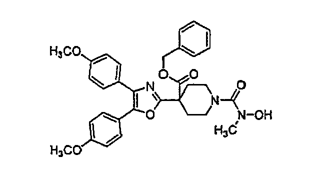

To a solution of benzyl 4-(4,5-bis(4-methoxyphenyl)oxazol-2-yl)piperidine-4-carboxylate (Reference Example 112) (632 mg, 1.27 mmol) in tetrahydrofuran (12.5 ml) was added triphosgene (153 mg, 0.515 mmol) and triethylamine (155 mg, 1.53 mmol) at 0°C, and the mixture was stirred at room temperature for 1 hour. N-methylhydroxylamine hydrochloride (139 mg, 1.66 mmol) and triethylamine (325 mg, 3.21 mmol) were added to the reaction solution, the mixture was stirred at 50°C for 5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (535 mg, 0.936 mmol, 74%) as a white amorphous product.

¹H-NMR (400 MHz, CDCl₃) δ: 2.31-2.40 (2H, m), 2.46-2.54 (2H, m), 2.96 (3H, s), 3.42-3.51 (2H, m), 3.67-3.75 (2H, m), 3.84 (3H, s), 3.84 (3H, s), 5.20 (2H, s), 6.72 (1H, brs), 6.88 (2H, d, J = 8.8 Hz), 6.90 (2H, d, J = 8.8 Hz), 7.41 (2H, d, J = 8.8 Hz), 7.55 (2H, d, J = 8.8 Hz).

ESI-MS: m/z = 572 (M+H⁺).

### Example 44

### 4-Acetoxymethyl-4-(4,5-bis(4-methoxyphenyl)oxazol-2-yl)-1-(N-hydroxy-N-methylcarbamoyl)piperidine

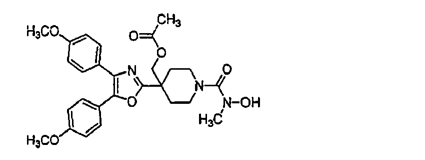

To a solution of 4-acetoxymethyl-4-(4,5-bis(4-methoxyphenyl)oxazol-2-yl)piperidine (Reference Example 119) (142 mg, 0.325 mmol) in tetrahydrofuran (5.0 ml) was added triphosgene (41 mg, 0.14 mmol) and triethylamine (55 µl 0.40 mmol) at 0°C, and the mixture was stirred at the same temperature for 1 hour. N-methylhydroxylamine hydrochloride (38 mg, 0.45 mmol) and triethylamine (110 µl, 0.80 mmol) were added to the reaction solution, the mixture was stirred at 50°C for 4 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (139 mg, 0.273 mmol, 84%) as a white amorphous product.

¹H-NMR (400 MHz, CDCl₃) δ: 1.75 (2H, m), 2.04 (3H, s), 2.38-2.46 (2H, m), 2.96 (3H, s), 3.16-3.24 (2H, m), 3.84 (3H, s), 3.84 (3H, s), 3.96-4.04 (2H, m), 4.25 (2H, s), 6.76 (1H, s), 6.90 (2H, d, J = 8.8 Hz), 6.91 (2H, d, J = 8.8 Hz), 7.48 (2H, d, J = 8.8 Hz), 7.56 (2H, d, J = 8.8 Hz).

### Example 45

### 4-(4,5-Bis(4-methoxyphenyl)oxazol-2-yl)-4-hydroxymethyl-1-(N-hydroxy-N-methylcarbamoyl)piperidine

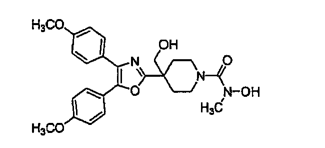

To a solution of 4-acetoxymethyl-4-(4,5-bis(4-methoxyphenyl)oxazol-2-yl)-1-(N-hydroxy-N-methylcarbamoyl)piperidine (Example 44) (135 mg, 0.265 mmol) in methanol (3.0 ml) was added potassium carbonate (45 mg, 0.32 mmol), and the mixture was stirred at room temperature for 2 hours. The reaction solution was concentrated under a reduced pressure, and distilled water was added to the residue, followed by extraction with ethyl acetate. The organic layer was dried and concentrated under a reduced pressure. The residue was purified by flash chromatography (amine silica gel, chloroform/methanol) to give a title compound (106 mg, 0.226 mmol, 85%) as a white amorphous product.

¹H-NMR (400 MHz, CDCl₃) δ: 1.74-1.83 (2H, m), 2.29-2.37 (2H, m), 2.93-2.98 (4H, m), 3.37-3.46 (2H, m), 3.81-3.84 (10H, m), 6.77 (1H, s), 6.90 (2H, d, J = 8.8 Hz), 6.90 (2H, d, J = 8.8 Hz), 7.49 (2H, d, J = 8.8 Hz), 7.56 (2H, d, J = 8.8 Hz).

ESI-MS: m/z = 468 (M+H⁺).

### Example 46

### 4-(4,5-Bis(4-methoxyphenyl)oxazol-2-yl)-1-(N-hydroxy-N-methylcarbamoyl)piperidine-4-carboxamide

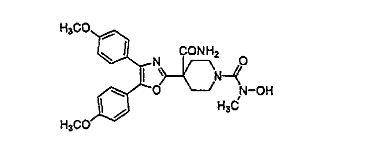

To a solution of 4-(4,5-bis(4-methoxyphenyl)oxazol-2-yl)piperidine-4-carboxamide (Reference Example 124) (110 mg, 0.270 mmol) in tetrahydrofuran (5.0 ml) was added triphosgene (32 mg, 0.11 mmol) and triethylamine (40 mg, 0.39 mmol) at 0°C, and the mixture was stirred at the same temperature for 1 hour. N-methylhydroxylamine hydrochloride (32 mg, 0.38 mmol) and triethylamine (80 mg, 0.79 mmol) were added to the reaction solution, the mixture was stirred at 50°C for 5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, chloroform/methanol) to give a title compound (100 mg, 0.208 mmol, 77%) as a white solid.

¹H-NMR (400 MHz, CDCl₃) δ: 2.29-2.39 (2H, m), 2.47-2.55 (2H, m), 2.97 (3H, s), 3.21-3.30 (2H, m), 3.85 (6H, s), 3.91-3.99 (2H, m), 5.41 (1H, brs), 6.26 (1H, brs), 6.75 (1H, brs), 6.91 (2H, d, J = 8.8 Hz), 6.92 (2H, d, J = 8.8 Hz), 7.49 (2H, d, J = 8.8 Hz), 7.57 (2H, d, J = 8.8 Hz).

IR (KBr, cm⁻¹): 3376, 3223, 1687, 1642, 1615, 1520, 1501, 1446, 1294, 1252, 1178, 1033,836.

ESI-MS: m/z = 481 (M+H⁺).

### Example 47

### 4-(4,5-Bis(4-methoxyphenyl)oxazol-2-yl)-4-cyano-1-(N-hydroxy-N-methylcarbamoyl)piperidine

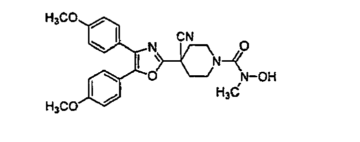

To a solution of 4-(4,5-bis(4-methoxyphenyl)oxazol-2-yl)-4-cyanopiperidine (Reference Example 126) (144 mg, 0.372 mmol) in tetrahydrofuran (5.0 ml) was added triphosgene (49 mg, 0.16 mmol) and triethylamine (60 µl, 0.44 mmol) at 0°C, and the mixture was stirred at the same temperature for 1 hour. N-methylhydroxylamine hydrochloride (42 mg, 0.50 mmol) and triethylamine (120 µl, 0.88 mmol) were added to the reaction solution, the mixture was stirred at 50°C for 5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (145 mg, 0.313 mmol, 84%) as a white amorphous product.

¹H-NMR (400 MHz, CDCl₃) δ: 2.28-2.37 (2H, m), 2.40-2.47 (2H, m), 3.02 (3H, s), 3.41-3.52 (2H, m), 3.84 (3H, s), 3.85 (3H, s), 4.04-4.11 (2H, m), 6.58 (1H, brs), 6.91 (4H, d, J = 8.8 Hz), 7.51 (2H, d, J = 8.8 Hz), 7.56 (2H, d, J = 8.8 Hz).

### Example 48

### 4-(4,5-Bis(4-methoxyphenyl)oxazol-2-yl)-(N-hydroxy-N-methylcarbamoyl)-4-phenylpiperidine

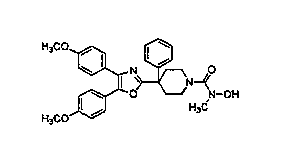

To a solution of 4-(4,5-bis(4-methoxyphenyl)oxazol-2-yl)-4-phenylpiperidine (Reference Example 130) (365 mg, 0.778 mmol) in tetrahydrofuran (10 ml) was added triphosgene (92 mg, 0.31 mmol) and triethylamine (79 mg, 0.78 mmol) at 0°C, and the mixture was stirred at room temperature for 14 hours. N-methylhydroxylamine hydrochloride (97 mg, 1.2 mmol) and triethylamine (158 mg, 1.6 mmol) were added to the reaction solution, the mixture was stirred at 50°C for 9 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (57 mg, 0.11 mmol, 14%) as a light yellow amorphous product.

¹H-NMR (400 MHz, CDCl₃) δ: 2.19 (2H, dt, J = 4.4, 8.8 Hz), 2.82 (2H, brd, J = 14.0 Hz), 2.97 (3H, s), 3.27 (2H, m), 3.82 (3H, s), 3.84 (3H, s), 4.07 (2H, brd, J = 14.0 Hz), 6.78 (1H, s), 6.86 (2H, d, J = 8.8 Hz), 6.91 (2H, d, J = 8.8 Hz), 7.26 (1H, m), 7.34-7.35 (4H, m), 7.43 (2H, d, J = 8.8 Hz), 7.59 (2H, d, J = 8.8 Hz).

### Example 49

### 4-(4,5-Bis(4-methoxyphenyl)oxazol-2-yl)-(N-hydroxy-N-ethoxycarbonylethylcarbamoyl)piperidine

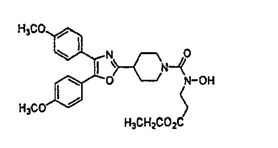

To a solution of 4-(4,5-bis(4-methoxyphenyl)oxazol-2-yl)piperidine-1-carbonyl chloride (311 mg, 0.729 mmol) in tetrahydrofuran (10 ml) was added ethyl N-hydroxy-3-aminopropionate (304 mg, 1.45 mmol) and triethylamine (74 mg, 0.73 mmol), and the mixture was stirred at room temperature for 4 hours. Distilled water was added to the reaction solution, followed by extraction with ethyl acetate. The organic layer was washed with 1M hydrochloric acid and with distilled water, dried over sodium sulfate, and concentrated under a reduced pressure. The residue was purified by flash chromatography (amine silica gel, n-hexane/ethyl acetate) to give a title compound (140 mg, 0.267 mmol, 37%) as a light yellow amorphous product.

¹H-NMR (400 MHz, CDCl₃) δ: 1.28 (3H, t, J = 7.2 Hz), 1.94-2.03 (2H, m), 2.16-2.19 (2H, m), 2.74 (2H, t, J = 6.4 Hz), 3.09-3.18 (3H, m), 3.42 (2H, t, J = 6.4 Hz), 3.83 (3H, s), 3.84 (3H, s), 4.11-4.18 (2H, m), 4.16 (2H, q, J = 7.2 Hz), 6.82 (1H, s), 6.82 (2H, d, J = 8.8 Hz), 6.89 (2H, d, J = 8.8 Hz), 7.48 (2H, d, J = 8.8 Hz), 7.54 (2H, d, J = 8.8 Hz).

### Example 50

### 4-(4,5-Bis(4-methoxyphenyl)oxazol-2-yl)-(N-hydroxy-N-carboxyethylcarbamoyl)piperidine

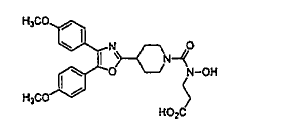

To a solution of 4-(4,5-bis(4-methoxyphenyl)oxazol-2-yl)-(N-hydroxy-N-ethoxycarbonylethylcarbamoyl)piperidine (Example 49) (135 mg, 0.258 mmol) in methanol (2.0 ml) was added lithium hydroxide monohydrate (16 mg, 0.39 mmol) and distilled water (5 drops with a Pasteur pipette), and the mixture was stirred at room temperature for 24 hours. The reaction solution was acidified with the addition of 1M hydrochloric acid, followed by extraction with ethyl acetate. The organic layer was dried over sodium sulfate and concentrated under a reduced pressure. The residue was purified by flash chromatography (diol-silica gel, chloroform/methanol) to give a title compound (118 mg, 0.238 mmol, 92%) as a white amorphous product.

¹H-NMR (400 MHz, CDCl₃) δ: 1.92-2.01 (2H, m), 2.14-2.17 (2H, m), 2.77 (2H, t, J = 6.4 Hz), 3.09-3.16 (3H, m), 3.45 (2H, t, J = 6.4 Hz), 3.83 (6H, s), 4.13-4.17 (2H, m), 6.88 (2H, d, J = 8.8 Hz), 6.89 (2H, d, J = 8.8 Hz), 7.47 (2H, d, J = 8.8 Hz), 7.52 (2H, d, J = 8.8 Hz).

### Example 51

### 4-(5-(4-Fluorophenyl)-4-(4-methoxyphenyl)thiazol-2-yl)-1-(N-hydroxy-N-methylcarbamoyl)-piperidine

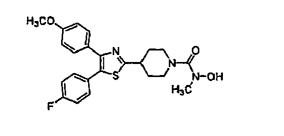

To a solution of 4-(5-(4-fluorophenyl)-4-(4-methoxyphenyl)thiazol-2-yl)piperidine (Reference Example 131) (275 mg, 0.746 mmol) in tetrahydrofuran (10 ml) was added triphosgene (89 mg, 0.30 mmol) and triethylamine (104 mg, 1.0 mmol) at 0°C, and the mixture was stirred at the same temperature for 1 hour. N-methylhydroxylamine hydrochloride (110 mg, 1.32 mmol) and triethylamine (209 mg, 2.1 mmol) were added to the reaction solution, the mixture was stirred at 50°C for 4.5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (288 mg, 0.652 mmol, 87%) as a white amorphous product.

¹H-NMR (400 MHz, CDCl₃) δ: 1.81-1.93 (2H, m), 2.17-2.25 (2H, m), 3.00 (3H, s), 3.05-3.12 (2H, m), 3.22-3.30 (1H, m), 3.80 (3H, s), 4.13-4.20 (2H, m), 6.78 (1H, s), 6.82 (2H, d, J = 8.8 Hz), 7.01 (2H, t, J = 8.8 Hz), 7.29 (2H, dd, J = 5.4, 8.8 Hz), 7.40 (2H, d, J = 8.8 Hz).

IR (KBr, cm⁻¹): 3405, 1626, 1610, 1514, 1494, 1250.

ESI-MS: m/z = 442 (M+H⁺).

### Example 52

### 4-(4-(4-Methoxyphenyl)-5-(4-tolyl)thiazol-2-yl)-1-(N-hydroxy-N-methylcarbamoyl)piperidine

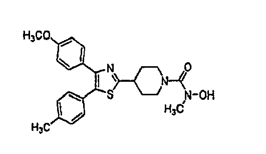

To a solution of 4-(4-(4-methoxyphenyl)-5-(4-tolyl)thiazol-2-yl)piperidine (Reference Example 132) (244 mg, 0.669 mmol) in tetrahydrofuran (10 ml) was added triphosgene (81 mg, 0.27 mmol) and triethylamine (110 µl, 0.80 mmol) at 0°C, and the mixture was stirred at the same temperature for 1 hour. N-methylhydroxylamine hydrochloride (76 mg, 0.91 mmol) and triethylamine (220 µl, 1.6 mmol) were added to the reaction solution, the mixture was stirred at 50°C for 9 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (228 mg, 0.521 mmol, 78%) as a white amorphous product.

¹H-NMR (400 MHz, CDCl₃) δ: 1.81-1.93 (2H, s), 2.17-2.25 (2H, m), 2.36 (3H, s), 3.00 (3H, s), 3.04-3.12 (2H, m), 3.21-3.30 (1H, m), 3.80 (3H, s), 4.13-4.17 (2H, m), 6.79 (1H, s), 6.81 (2H, d, J = 8.8 Hz), 7.11 (2H, d, J = 8.0 Hz), 7.21 (2H, d, J = 8.0 Hz), 7.44 (2H, d, J = 8.8 Hz).

IR (KBr, cm⁻¹): 3440, 3238, 1631, 1610, 1516, 1494, 1440, 1250.

ESI-MS: m/z = 481 (M+H⁺).

### Example 53

### Benzyl 4-(4,5-bis(4-methoxyphenyl)thiazol-2-yl)-1-(N-hydroxy-N-methylcarbamoyl)piperidine-4-carboxylate

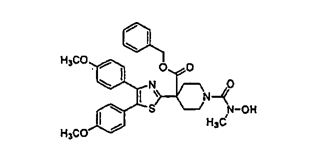

To a solution of benzyl 4-(4,5-bis(4-methoxyphenyl)thiazol-2-yl)piperidine-4-carboxylate (Reference Example 134) (311 mg, 0.604 mmol) in tetrahydrofuran (7.0 ml) was added triphosgene (74 mg, 0.25 mmol) and triethylamine (100 µl, 0.73 mmol) at 0°C, and the mixture was stirred at the same temperature for 1 hour. N-methylhydroxylamine hydrochloride (90 mg, 1.1 mmol) and triethylamine (300 µl, 2.2 mmol) were added to the reaction solution, the mixture was stirred at 50°C for 20 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (306 mg, 0.520 mmol, 86%) as a white amorphous product.

¹H-NMR (400 MHz, CDCl₃) δ: 2.32-2.40 (2H, m), 2.48-2.56 (2H, m), 2.96 (3H, s), 3.42-3.50 (2H, m), 3.67-3.75 (2H, m), 3.80 (3H, s), 3.83 (3H, s), 5.22 (2H, s), 6.71 (1H, s), 6.81 (2H, d, J = 8.8 Hz), 6.86 (2H, d, J = 8.8 Hz), 7.24 (2H, d, J = 8.8 Hz), 7.27-7.34 (5H, m), 7.43 (2H, d, J = 8.8 Hz).

### Example 54

### 4-(4,5-Bis(4-methoxyphenyl)thiazol-2-yl)-4-hydroxymethyl-1-(N-hydroxy-N-methylcarbamoyl)piperidine

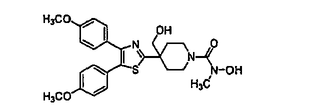

To a solution of benzyl 4-(4,5-bis(4-methoxyphenyl)thiazol-2-yl)-1-(N-hydroxy-N-methylcarbamoyl)piperidinecarboxylate (Example 53) (272 mg, 0.463 mmol) in tetrahydrofuran (4.0 ml) was added lithium borohydride, and the mixture was stirred at room temperature. Distilled water was added to the reaction solution, followed by extraction with ethyl acetate. The organic layer was dried and concentrated under a reduced pressure. The residue was purified by flash chromatography (silica gel, n-hexane/ethyl acetate) to give a title compound (162 mg, 0.335 mmol, 72%).

¹H-NMR (400 MHz, CDCl₃) δ: 1.92-2.00 (2H, m), 2.18-2.26 (2H, m), 2.98 (3H, s), 3.24-3.29 (1H, m), 3.50-3.58 (2H, m), 3.66-3.75 (2H, m), 3.81-3.83 (8H, m), 6.76 (1H, s), 6.82 (2H, d, J = 8.8 Hz), 6.87 (2H, d, J = 8.8 Hz), 7.27 (2H, d, J = 8.8 Hz), 7.44 (2H, d, J = 8.8 Hz).

IR (KBr, cm⁻¹): 3389, 3250, 1646, 1610, 1515, 1490, 1440, 1251.

### Example 55

### 4-(4,5-Bis(4-methoxyphenyl)thiazol-2-yl)-1-(N-hydroxy-N-methylcarbamoyl)-4-methylpiperidine

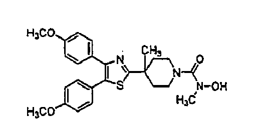

To a solution of 4-(4,5-bis(4-methoxyphenyl)thiazol-2-yl)-4-methylpiperidine (Reference Example 137) (158 mg, 0.402 mmol) in tetrahydrofuran (8.0 ml) was added triphosgene (48 mg, 0.16 mmol) and triethylamine (85 µl, 0.62 mmol) at 0°C, and the mixture was stirred at the same temperature for 1 hour. N-methylhydroxylamine hydrochloride (57 mg, 0.68 mmol) and triethylamine (175 µl, 1.3 mmol) were added to the reaction solution, the mixture was stirred at 50°C for 4 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (144 mg, 0.308 mmol, 77%) as a white amorphous product.

¹H-NMR (400 MHz, CDCl₃) δ: 1.45 (3H, s), 1.74-1.83 (2H, m), 2.30-2.37 (2H, m), 2.96 (3H, s), 3.38-3.47 (2H, m), 3.78-3.86 (8H, m), 6.82 (2H, d, J = 8.8 Hz), 6.86 (2H, d, J = 8.8 Hz), 7.27 (2H, d, J = 8.8 Hz), 7.46 (2H, d, J = 8.8 Hz).

IR (KBr, cm⁻¹): 3435, 3245, 1642, 1609, 1515, 1493, 1440, 1292, 1250.

ESI-MS: m/z = 468 (M+H⁺).

### Example 56

### 4-(4,5-Bis(4-methoxyphenyl)thiazol-2-yl)-4-cyano-1-(N-hydroxy-N-methylcarbamoyl)piperidine

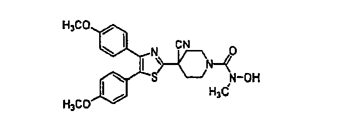

To a solution of 4-(4,5-bis(4-methoxyphenyl)thiazol-2-yl)-4-cyanopiperidine (Reference Example 142) (266 mg, 0.656 mmol) in tetrahydrofuran (8.0 ml) was added triphosgene (80 mg, 0.27 mmol) and triethylamine (135 µl, 0.99 mmol) at 0°C, and the mixture was stirred at the same temperature for 1 hour. N-methylhydroxylamine hydrochloride (76 mg, 0.91 mmol) and triethylamine (250 µl, 1.8 mmol) were added to the reaction solution, the mixture was stirred at 50°C for 1.5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (217 mg, 0.453 mmol, 69%) as a white amorphous product.

¹H-NMR (400 MHz, CDCl₃) δ: 2.27-2.36 (2H, m), 2.38-2.45 (2H, m), 3.03 (3H, s), 3.37-3.46 (2H, m), 3.81 (3H, s), 3.83 (3H, s), 4.15-4.23 (2H, m), 6.61 (1H, brs), 6.82 (2H, d, J = 8.8 Hz), 6.88 (2H, d, J = 8.8 Hz), 7.27 (2H, d, J = 8.8 Hz), 7.45 (2H, d, J = 8.8 Hz).

IR (KBr, cm⁻¹): 3391, 3248, 1635, 1609, 1515, 1493, 1440, 1294, 1251.

ESI-MS: m/z = 479 (M+H⁺).

### Example 57

### 4-(4,5-Bis(4-methoxyphenyl)thiazol-2-yl)-1-(N-hydroxy-N-methylcarbamoyl)piperazine

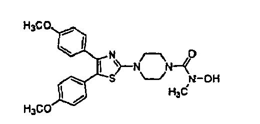

To a solution of 4-(4,5-bis(4-methoxyphenyl)thiazol-2-yl)piperazine (Reference Example 152) (64 mg, 0.17 mmol) in tetrahydrofuran (10 ml) was added triphosgene (21 mg, 0.070 mmol) and triethylamine (35 µl, 0.25 mmol) at 0°C, and the mixture was stirred at the same temperature for 1 hour. N-methylhydroxylamine hydrochloride (21 mg, 0.25 mmol) and triethylamine (70 µl, 0.50 mmol) were added to the reaction solution, the mixture was stirred at 80°C for 1 hour, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (66 mg, 0.14 mmol, 82%) as a white amorphous product.

¹H-NMR (400 MHz, CDCl₃) δ: 3.03 (3H, s), 3.52-3.59 (4H, m), 3.61-3.67 (4H, m), 3.79 (3H, s), 3.81 (3H, s), 6.67 (1H, brs), 6.79 (2H, d, J = 8.8 Hz), 6.82 (2H, d, J = 8.8 Hz), 7.21 (2H, d, J = 8.8 Hz), 7.43 (2H, d, J = 8.8 Hz).

IR (KBr, cm⁻¹), 3396, 3246, 1630, 1609, 1543, 1511, 1493, 1439, 1287, 1247.

ESI-MS: m/z = 455 (M+H⁺).

### Example 58

### 4-(4,5-Bis(4-methoxyphenyl)thiazol-2-yl)-4-hydroxy-1-(N-hydroxy-N-methylcarbamoyl)piperidine

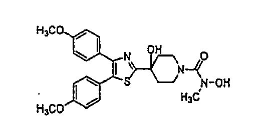

To a solution of 4-(4,5-bis(4-methoxyphenyl)thiazol-2-yl)-4-hydroxypiperidine (Reference Example 147) (143 mg, 0.36 mmol) in tetrahydrofuran (15 ml) was added triphosgene (45 mg, 0.15 mmol) and triethylamine (75 µl, 0.55 mmol) at 0°C, and the mixture was stirred at the same temperature for 1 hour. N-methylhydroxylamine hydrochloride (43 mg, 0.51 mmol) and triethylamine (150 µl, 1.1 mmol) were added to the reaction solution, the mixture was stirred at 50°C for 8 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (169 mg, 0.36 mmol, quant.) as a white amorphous product.

¹H-NMR (400 MHz, CDCl₃) δ: 1.94-2.01 (2H, m), 2.13-2.22 (2H, m), 3.01 (3H, s), 3.39 (1H, s), 3.43-3.52 (2H, s), 3.80 (3H, s), 3.83 (3H, s), 3.99-4.06 (2H, m), 6.80 (1H, s), 6.82 (2H, d, J = 8.8 Hz), 6.86 (2H, d, J = 8.8 Hz), 7.26 (2H, d, J = 8.8 Hz), 7.44 (2H, d, J = 8.8 Hz).

### Example 59

### 4-(4,5-Bis(4-methoxyphenyl)thiazol-2-yl)-1-(N-hydroxy-N-methylcarbamoyl)-4-methoxypiperidine

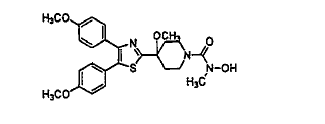

To a solution of 4-(4,5-bis(4-methoxyphenyl)thiazol-2-yl)-4-methoxypiperidine (Reference Example 149) (43 mg, 0.11 mmol) in tetrahydrofuran (4.5 ml) was added triphosgene (13 mg, 0.044 mmol) and triethylamine (22 µl, 0.16 mmol) at 0°C, and the mixture was stirred at the same temperature for 0.5 hours. N-methylhydroxylamine hydrochloride (12 mg, 0.15 mmol) and triethylamine (44 µl, 0.32 mmol) were added to the reaction solution, the mixture was stirred at 50°C for 4 hours, and the resultant was then cooled to room temperature. The reaction solution was concentrated under a reduced pressure, and distilled water was added to the residue, followed by extraction with ethyl acetate. The organic layer was dried over sodium sulfate and concentrated under a reduced pressure. The residue was purified by flash chromatography (amine silica gel, n-hexane/ethyl acetate) to give a title compound (51 mg, 0.11 mmol, quant.) as a white amorphous product.

¹H-NMR (400 MHz, CDCl₃) δ: 2.15-2.18 (2H, m), 2.24-2.30 (2H, m), 2.99 (3H, s), 3.28 (3H, s), 3.42-3.48 (2H, m), 3.80 (3H, s), 3.82 (3H, s), 3.84-3.89 (2H, m), 6.82 (2H, d, J = 8.8 Hz), 6.87 (2H, d, J = 8.8 Hz), 7.26 (2H, d, J = 8.8 Hz), 7.44 (2H, d, J = 8.8 Hz).

### Example 60

### 4-(4,5-Bis(4-methoxyphenyl)thiazol-2-yl)-1-(N-hydroxy-N-isopropylcarbamoyl)piperidine

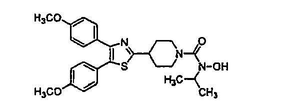

To a solution of 4-(4,5-bis(4-methoxyphenyl)thiazol-2-yl)piperidine (Reference Example 93) (201 mg, 0.528 mmol) in tetrahydrofuran (15 ml) was added triphosgene (65 mg, 0.22 mmol) and triethylamine (100 µl, 0.73 mmol) at 0°C, and the mixture was stirred at the same temperature for hours. N-isopropylhydroxylamine hydrochloride (82 mg, 0.74 mmol) and triethylamine (200 µl, 1.5 mmol) were added to the reaction solution, the mixture was stirred at 50°C for 5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (145 mg, 0.301 mmol, 57%) as a white amorphous product.

¹H-NMR (400 MHz, CDCl₃) δ: 1.19 (6H, d, J = 6.6 Hz), 1.81-1.92 (2H, m), 2.17-2.25 (2H, m), 3.03-3.13 (2H, m), 3.48-3.56 (1H, m), 3.80 (3H, s), 3.82 (3H, s), 4.09-4.17 (2H, m), 6.33 (1H, s), 6.82 (2H, d, J = 8.8 Hz), 6.85 (2H, d, J = 8.8 Hz), 7.25 (2H, d, J = 8.8 Hz), 7.44 (2H, d, J = 8.8 Hz).

IR (KBr, cm⁻¹): 3266, 1651, 1608, 1515, 1496, 1441, 1294, 1249, 1176.

### Example 61

### 4-(4,5-Bis(4-methoxyphenyl)thiazol-2-yl)-1-(N-methoxy-N-methylcarbamoyl)piperidine

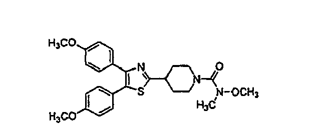

To a solution of 4-(4,5-bis(4-methoxyphenyl)thiazol-2-yl)piperidine (Reference Example 93) (200 mg, 0.526 mmol) in tetrahydrofuran (20 ml) was added triphosgene (62 mg, 0.21 mmol) and triethylamine (110 µl, 0.80 mmol) at 0°C, and the mixture was stirred at the same temperature for 1 hour. N,O-dimethylhydroxylamine hydrochloride (72 mg, 0.74 mmol) and triethylamine (220 µl, 1.6 mmol) were added to the reaction solution, the mixture was stirred at 75°C for 4 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (173 mg, (0.370 mmol, 70%) as a light yellow oil product.

¹H-NMR (400 MHz, CDCl₃) δ: 1.79-1.91 (2H, m), 2.16-2.23 (2H, m), 2.98 (3H, s), 2.98-3.97 (2H, m), 3.80 (3H, s), 3.82 (3H, s), 4.14-4.22 (2H, m), 6.81 (2H, d, J = 8.8 Hz), 6.84 (2H, d, J = 8.8 Hz), 7.25 (2H, d, J = 8.8 Hz), 7.44 (2H, d, J = 8.8 Hz).

### Example 62

### 4-(1-(4-Methoxyphenyl)-5-(4-methoxy-3-tolyl)-1H-pyrazol-3-yl)-1-(N-hydroxy-N-methylcarbamoyl)piperidine

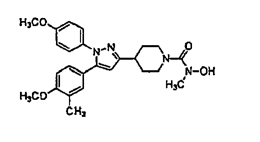

To a solution of 4-(1-(4-methoxyphenyl)-5-(4-methoxy-3-tolyl)pyrazol-3-yl)piperidine (Reference Example 165) (285 mg, 0.755 mmol) in tetrahydrofuran (15 ml) was added triphosgene (92 mg, 0.31 mmol) and triethylamine (150 µl, 1.1 mmol) at 0°C, and the mixture was stirred at the same temperature for hours. N-methylhydroxylamine hydrochloride (91 mg, 1.1 mmol) and triethylamine (300 µl, 2.2 mmol) were added to the reaction solution, the mixture was stirred at 50°C for 6 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (167 mg, 0.370 mmol, 49%) as a light orange amorphous product.

¹H-NMR (400 MHz, CDCl₃) δ: 1.59-1.72 (2H, m), 2.05-2.12 (2H, m), 2.15 (3H, s), 2.92-3.08 (6H, m), 3.81 (3H, s), 3.81 (3H, s), 4.12-4.18 (2H, m), 6.22 (1H, s), 6.70 (1H, d, J = 8.5 Hz), 6.84 (2H, d, J = 9.0 Hz), 6.87 (1H, s), 6.93 (1H, dd, J = 2.2, 8.5 Hz), 7.03 (1H, d, J = 2.2 Hz), 7.20 (2H, d, J = 9.0 Hz).

IR(KBr, cm⁻¹): 3407, 1648, 1614, 1517, 1500, 1440, 1250.

ESI-MS: m/z = 451 (M+H⁺).

### Example 63

### 4-(5-(3-Fluoro-4-methoxyphenyl)-1-(4-methoxyphenyl)-1H-pyrazol-3-yl)-1-(N-hydroxy-N-methylcarbamoyl)piperidine

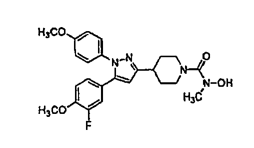

To a solution of 4-(5-(3-fluoro-4-methoxyphenyl)-1-(4-methoxyphenyl)-1H-pyrazol-3-yl)piperidine (Reference Example 166) (286 mg, 0.752 mmol) in tetrahydrofuran (15 ml) was added triphosgene (91 mg, 0.30 mmol) and triethylamine (150 µl, 1.1 mmol) at 0°C, and the mixture was stirred at the same temperature for hours. N-methylhydroxylamine hydrochloride (91 mg, 1.1 mmol) and triethylamine (300 µl, 2.2 mmol) were added to the reaction solution, the mixture was stirred at 50°C for 6 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (236 mg, 0.519 mmol, 69%) as a light orange amorphous product.

¹H-NMR (400 MHz, CDCl₃) δ: 1.71-1.84 (2H, m), 2.03-2.13 (2H, m), 2.92-3.08 (6H, m), 3.82 (3H, s), 3.88 (3H, s), 4.11-4.19 (2H, m), 6.25 (1H, s), 6.83-6.95 (6H, m), 7.18 (2H, d, J = 9.0 Hz).

ESI-MS: m/z = 455 (M+H⁺).

### Example 64

### 4-(5-(3,4-Dimethoxyphenyl)-1-(4-methoxyphenyl)-1H-pyrazol-3-yl)-1-(N-hydroxy-N-methylcarbamoyl)piperidine

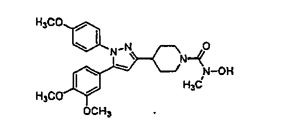

To a solution of 1-tert-butoxycarbonyl-4-(5-(3,4-dimethoxyphenyl)-1-(4-methoxyphenyl)-1H-pyrazol-3-yl)piperidine (Reference Example 161) (315 mg, 0.638 mmol) in dichloromethane (5.0 ml) was added trifluoroacetic acid (1.0 ml, 13 mmol), and the mixture was stirred at room temperature for 2 hours. The reaction solution was concentrated under a reduced pressure, and an aqueous solution of 1M sodium hydroxide was added to the residue, followed by extraction with ethyl acetate. The organic layer was dried over magnesium sulfate and then concentrated under a reduced pressure. The residue was dissolved with the addition of tetrahydrofuran (20 ml), triphosgene (76 mg, 0.25 mmol) and triethylamine (130 µl, 0.95 mmol) were added at 0°C, and the mixture was stirred at the same temperature for hours. N-methylhydroxylamine hydrochloride (76 mg, 1 mmol) and triethylamine (260 µl, 1.9 mmol) were added to the reaction solution, the mixture was stirred at 50°C for 23 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (181 mg, 0.388 mmol, 61%) as a white amorphous product.

¹H-NMR (400 MHz, CDCl₃) δ: 1.73-1.85 (2H, m), 2.06-2.13 (2H, m), 2.93-3.09 (6H, m), 3.66 (3H, s), 3.80 (3H, s), 3.87 (3H, s), 4.12-4.19 (2H, m), 6.26 (1H, s), 6.65 (1H, dd, J = 1.2 Hz), 6.79-6.86 (5H, m), 7.20 (2H, d, J = 8.8 Hz).

IR (KBr, cm⁻¹): 3541, 3390, 1635, 1512, 1442, 1250.

ESI-MS: m/z = 467 (M+H⁺).

### Example 65

### 4-(5-(2,4-Dimethoxyphenyl)-1-(4-methoxyphenyl)-1H-pyrazol-3-yl)-1-(N-hydroxy-N-methylcarbamoyl)piperidine

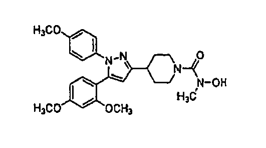

To a solution of 4-(5-(2,4-dimethoxyphenyl)-1-(4-methoxyphenyl)-1H-pyrazol-3-yl)piperidine (Reference Example 167) (156 mg, 0.396 mmol) in tetrahydrofuran (5.0 ml) was added triphosgene (52 mg, 0.18 mmol) and triethylamine (83 µl, 0.61 mmol) at 0°C, and the mixture was stirred at the same temperature for 1 hour. N-methylhydroxylamine hydrochloride (46 mg, 0.55 mmol) and triethylamine (160 µl, 1.2 mmol) were added to the reaction solution, the mixture was stirred at 90°C for 20 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (104 mg, 0.223 mmol, 56%) as a white amorphous product.

¹H-NMR (400 MHz, CDCl₃) δ: 1.73-1.84 (2H, m), 2.07-2.15 (2H, m), 2.94-3.08 (6H, m), 3.42 (3H, s), 3.77 (3H, s), 3.81 (3H, s), 4.10-4.18 (2H, m), 6.20 (1H, s), 6.36 (1H, d, J = 2.4 Hz), 6.47 (1H, dd, J = 2.4, 8.5 Hz), 6.78 (2H, d, J = 8.8 Hz), 6.87 (1H, s), 7.12 (1H, d, J = 8.5 Hz), 7.15 (2H, d, J = 8.8 Hz).

IR (KBr, cm⁻¹): 3405, 3232, 1643, 1620, 1512, 1446, 1300, 1250.

ESI-MS: m/z = 467 (M+H⁺).

### Example 66

### 4-(1-(4-Methoxyphenyl)-5-(2-methoxypyridin-5-yl)-1H-pyrazol-3-yl)-1-(N-hydroxy-N-methylcarbamoyl)piperidine

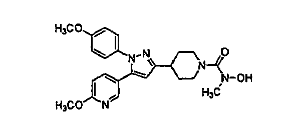

To a solution of 4-(1-(4-methoxyphenyl)-5-(2-methoxypyridin-5-yl)-1H-pyrazol-3-yl)piperidine (Reference Example 168) (117 mg, 0.32 mmol) in tetrahydrofuran (14 ml) was added triphosgene (40 mg, 0.14 mmol) and triethylamine (68 µl, 0.50 mmol) at 0°C, and the mixture was stirred at the same temperature for 0.5 hours. N-methylhydroxylamine hydrochloride (38 mg, 0.46 mmol) and triethylamine (136 µl, 1.0 mmol) were added to the reaction solution, the mixture was stirred at 50°C for 2 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (61 mg, 0.14 mmol, 43%) as a white amorphous product.

¹H-NMR (400 MHz, CDCl₃) δ: 1.73-1.83 (2H, m), 2.04-2.09 (2H, m), 2.99 (3H, s), 3.00-3.06 (3H, m), 3.80 (3H, s), 3.92 (3H, s), 4.14-4.17 (2H, m), 6.28 (1H, s), 6.65(1H, d, J = 8.8 Hz), 6.87 (2H, d, J = 8.8 Hz), 7.17 (2H, d, J = 8.8 Hz), 7.30 (1H, dd, J = 2.0, 8.8 Hz), 8.07 (1H, m).

### Example 67

### 4-(1-(4-Methoxyphenyl)-5-(2-methoxypyridin-3-yl)-1H-pyrazol-3-yl)-1-(N-hydroxy-N-methylcarbamoyl)piperidine

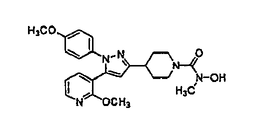

To a solution of 4-(1-(4-methoxyphenyl)-5-(2-methoxypyridin-3-yl)-1H-pyrazol-3-yl)piperidine (Reference Example 169) (157 mg, 0.43 mmol) in tetrahydrofuran (20 ml) was added triphosgene (55 mg, 0.18 mmol) and triethylamine (90 µl, 0.65 mmol) at 0°C, and the mixture was stirred at the same temperature for 1 hour. N-methylhydroxylamine hydrochloride (47 mg, 0.56 mmol) and triethylamine (180 µl, 1.30 mmol) were added to the reaction solution, the mixture was stirred at 75°C for 2 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (151 mg, 0.345 mmol, 80%) as an orange amorphous product.

¹H-NMR (400 MHz, CDCl₃) δ: 1.73-1.86 (2H, m), 2.07-2.15 (2H, m), 2.97-3.05 (6H, m), 3.68 (3H, s), 3.78 (3H, s), 4.12-4.19 (2H, m), 6.33 (1H, s), 6.81 (2H, d, J = 8.8 Hz), 6.86 (1H, dd, J = 4.9, 7.3 Hz), 7.14 (2H, d, J = 8.8 Hz), 7.43 (1H, dd, J = 1.9, 7.3 Hz), 8.14 (1H, dd, J = 1.9, 4.9 Hz).

### Example 68

### 3-(1,5-Bis(4-methoxyphenyl)-1H-pyrazol-3-yl)-8-(N-hydroxy-N-methylcarbamoyl)-8-azabicyclo[3.2.1]octane

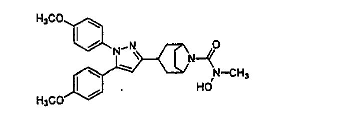

To a solution of 3-(1,5-bis(4-methoxyphenyl)-1H-pyrazol-3-yl)-8-azabicyclo[3.2.1]octane (Reference Example 174) (92 mg, 0.24 mmol) in tetrahydrofuran (4.7 ml) was added triphosgene (28 mg, 0.10 mmol) and triethylamine (43 µl, 0.31 mmol) at 0°C, and the mixture was stirred at room temperature for 12 hours. N-methylhydroxylamine hydrochloride (25 mg, 0.30 mmol) and triethylamine (82 µl, 0.59 mmol) were added to the reaction solution, the mixture was stirred at 60°C for 6 hours, and the resultant was then cooled to room temperature. Distilled water was added to the reaction solution, followed by extraction with ethyl acetate. The organic layer was washed with distilled water and then with brine, dried over sodium sulfate, and concentrated under a reduced pressure. The residue was purified by flash chromatography (amine silica, n-hexane/ethyl acetate) to give a title compound (91 mg, 0.20mmol), 84%) as a light yellow amorphous product.

¹H-NMR (400 MHz, CDCl₃) δ: 1.86-1.89 (2H, m), 1.98-2.05 (6H, m), 3.11 (3H, s), 3.30-3.35 (1H, m), 3.79 (3H, s), 3.80 (3H, s), 4.42 (2H, m), 6.24 (1H, s), 6.82 (4H, dd, J = 14.0, 8.8 Hz), 6.92 (1H, s), 7.11 (2H, d, J = 8.8 Hz), 7.18 (2H, d, J = 8.8 Hz).

ESI-MS: m/z = 463 (M+H⁺).

### Example 69

### 4-(1-(4-Methoxyphenyl)-5-(4-tolyl)-1H-pyrazol-3-yl)-1-(N-hydroxy-N-methylcarbamoyl)piperidine

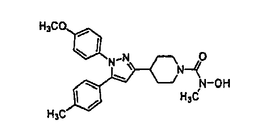

To a solution of 4-(1-(4-methoxyphenyl)-5-(4-tolyl)-1H-pyrazol-3-yl)piperidine (Reference Example 177) (521 mg, 1.50 mmol) in tetrahydrofuran (20 ml) was added triphosgene (178 mg, 0.60 mmol) and triethylamine (265 µl, 1.94 mmol) at 0°C, and the mixture was stirred at the same temperature for 1 hour. N-methylhydroxylamine hydrochloride (150 mg, 1.80 mmol) and triethylamine (530 µl, 3.87 mmol) were added to the reaction solution, the mixture was stirred at 60°C for 5 hours, and the resultant was then cooled to room temperature. The reaction solution was concentrated under a reduced pressure, and distilled water was added to the residue, followed by extraction with ethyl acetate. The organic layer was dried over magnesium sulfate and concentrated under a reduced pressure. The residue was purified by flash chromatography (amine silica gel, n-hexane/ethyl acetate) to give a title compound (466 mg, 1.11 mmol, 74%) as a white amorphous product.

¹H-NMR (400 MHz, CDCl₃) δ: 1.78 (2H, ddd, J = 4.0, 12.0, 24.8 Hz), 2.06-2.10 (2H, m), 2.33 (3H, s), 2.99 (3H, s), 2.95-3.07 (3H, m), 3.80 (3H, s), 4.13-4.17 (2H, m), 6.26 (1H, s), 6.84 (2H, d, J = 8.8 Hz), 7.10 (4H, s), 7.19 (2H, d, J = 8.8 Hz).

### Example 70

### 4-(5-(4-Hydroxyphenyl)-1-(4-methoxyphenyl)-1H-pyrazol-3-yl)-1-(N-hydroxy-N-methylcarbamoyl)piperidine

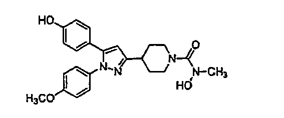

To a solution of 1-benzyloxycarbonyl-4-(5-(4-(benzyloxy)phenyl)-1-(4-methoxyphenyl)-1H-pyrazol-3-yl)piperidine (Reference Example 180) (123 mg, 0.21 mmol) in ethanol (2.14 ml) was added ASCA-2 catalyst (62 mg, hydrous product, NE Chemcat Corporation), and the mixture was stirred under a hydrogen atmosphere for 5 hours. The reaction solution was filtered, and the filtrate was removed by distillation under a reduced pressure. The residue was dissolved with the addition of tetrahydrofuran (9.0 ml), triphosgene (48 mg, 0.16 mmol) and triethylamine (75 µl, 0.54 mmol) were added at 0°C, and the mixture was stirred at room temperature for 15 hours. N-methylhydroxylamine hydrochloride (43 mg, 0.52 mmol) and triethylamine (143 µl, 1.03 mmol) were added to the reaction solution, and the mixture was stirred at 55°C for 3 hours. The reaction solution was cooled to room temperature, and distilled water was added to the reaction solution, followed by extraction with ethyl acetate. The organic layer was dried over sodium sulfate and concentrated under a reduced pressure. The residue was dissolved with the addition of methanol (2.14 ml), potassium carbonate (118 mg, 0.86 mmol) was added thereto, and the mixture was stirred at room temperature for 15 hours and then at 60°C for 4 hours. The reaction solution was cooled to room temperature, and distilled water was added, followed by extraction with ethyl acetate. The organic layer was washed with distilled water and then with brine, dried over sodium sulfate, and concentrated under a reduced pressure. The residue was purified by flash chromatography (silica gel, chloroform/methanol) to give a title compound (20 mg, 0.047 mmol, 22%) as a white powder.

¹H-NMR (400 MHz, CD₃OD) δ: 1.69-1.79 (2H, m), 1.98-2.00 (2H, m), 2.87-3.02 (6H, m), 3.80 (3H, s), 4.17-4.21 (2H, m), 6.33 (1H, s), 6.67 (2H, d, J = 8.8 Hz), 6.92 (2H, d, J = 9.2 Hz), 7.01 (2H, d, J = 8.8 Hz), 7.15 (2H, d, J = 9.2 Hz).

ESI-MS: m/z = 423 (M+H⁺).

### Example 71

### 4-(1-(4-Hydroxyphenyl)-5-(4-methoxyphenyl)-1H-pyrazol-3-yl)-1-(N-hydroxy-N-methylcarbamoyl)piperidine

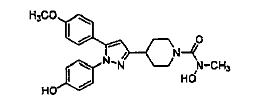

To a solution of 1-benzyloxycarbonyl-4-(1-(4-(benzyloxy)phenyl)-5-(4-methoxyphenyl)-1H-pyrazol-3-yl)piperidine (Reference Example 183) (90 mg, 0.16 mmol) in methanol (1.56 ml) was added ASCA-2 catalyst (45 mg, hydrous product, NE Chemcat Corporation), and the mixture was stirred under a hydrogen atmosphere at room temperature for 5 hours. The reaction solution was filtered, and the filtrate was concentrated under a reduced pressure. The residue was dissolved with the addition of tetrahydrofuran (9.0 ml), triphosgene (35 mg, 0.12 mmol) and triethylamine (55 µl, 0.39 mmol) were added at 0°C, and the mixture was stirred at room temperature for 15 hours. N-methylhydroxylamine hydrochloride (31 mg, 0.38 mmol) and triethylamine (105 µl, 0.75 mmol) were added to the reaction solution, and the mixture was stirred at 55°C for 3 hours. The reaction solution was cooled to room temperature, and distilled water was added, followed by extraction with ethyl acetate. The organic layer was washed with distilled water and then with brine, dried over sodium sulfate, and concentrated under a reduced pressure. The residue was dissolved with the addition of methanol (1.56 ml), potassium carbonate (86 mg, 0.62 mmol) was added thereto, and the mixture was stirred at 60°C for 5 hours. The reaction solution was cooled to room temperature, and distilled water was added to the reaction solution, followed by extraction with ethyl acetate. The organic layer was washed with distilled water and then with brine, dried over sodium sulfate, and concentrated under a reduced pressure. The residue was purified by flash chromatography (silica gel, chloroform/methanol) to give a title compound (4 mg, 0.010 mmol, 7%) as a white powder.

¹H-NMR (400 MHz, CDCl₃) δ: 1.72-1.82 (2H, m), 2.05-2.09 (2H, m), 2.94-3.06 (6H, m), 3.79 (3H, s), 4.13-4.16 (2H, m), 6.24 (1H, s), 6.65 (2H, d, J = 8.4 Hz), 6.80 (2H, d, J = 8.4 Hz), 6.93 (1 H, s), 7.04 (2H, d, J = 8.8 Hz), 7.11 (2H, d, J = 8.8 Hz).

ESI-MS: m/z = 423 (M+H⁺).

### Example 72

### 4-(1,5-Bis(4-hydroxyphenyl)-1H-pyrazol-3-yl)-1-(N-hydroxy-N-methylcarbamoyl)piperidine

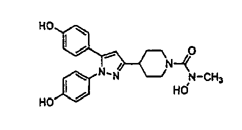

To a solution of 1-benzyloxycarbonyl-4-(1,5-bis(4-(acetoxy)phenyl)-1H-pyrazol-3-yl)piperidine (Reference Example 186) (90 mg, 0.16 mmol) in methanol (1.63 ml) was added ASCA-2 catalyst (90 mg, hydrous product, NE Chemcat Corporation), and the mixture was stirred under a hydrogen atmosphere for 15 minutes. The reaction solution was filtered, and the filtrate was removed by distillation under a reduced pressure. The residue was dissolved with the addition of tetrahydrofuran (9.0 ml), triphosgene (19 mg, 0.07 mmol) and triethylamine (28 µl, 0.20 mmol) were added at 0°C, and the mixture was stirred at room temperature for 4 hours. N-methylhydroxylamine hydrochloride (16 mg, 0.20 mmol) and triethylamine (56 µl, 0.41 mmol) were added to the reaction solution, and the mixture was stirred at 60°C for 2 hours. The reaction solution was cooled to room temperature, and distilled water was added to the reaction solution, followed by extraction with ethyl acetate. The organic layer was washed with distilled water and then with brine, dried over sodium sulfate, and concentrated under a reduced pressure. The residue was dissolved with the addition of methanol (1.63 ml), potassium carbonate (67 mg, 0.49 mmol) was added thereto, and the mixture was stirred at 80°C for 2 hours. The reaction solution was cooled to room temperature, and distilled water was added to the reaction solution, followed by extraction with ethyl acetate. The organic layer was washed with distilled water and then with brine, dried over sodium sulfate, and concentrated under a reduced pressure. The residue was purified by flash chromatography (amine silica gel, chloroform/methanol) to give a title compound (20 mg, 0.049 mmol, 30%) as a white amorphous product.

¹H-NMR (400 MHz, CD₃OD) δ: 1.59-1.75 (2H, m), 1.88-1.91 (2H, m), 2.78-2.93 (6H, m), 4.08-4.11 (2H, m), 6.22 (1H, s), 6.58 (2H, d, J = 8.8 Hz), 6.68 (2H, d, J = 8.8 Hz), 6.82 (1H, s), 6.92-6.97 (4H, m).

ESI-MS: m/z = 409 (M+H⁺).

### Example 73

### 4-Allyl-4-(1,5-bis(4-methoxyphenyl)oxazol-2-yl)-1-(N-hydroxy-N-methylcarbamoyl)piperidine

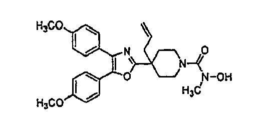

To a solution of 1-tert-butoxycarbonyl-4-allyl-4-(1,5-bis(4-methoxyphenyl)oxazol-2-yl)piperidine (Reference Example 190) (180 mg, 0.36 mmol) in dichloromethane (357 µl) was added trifluoroacetic acid (357 µl), and the mixture was stirred for 2 hours. The reaction solution was concentrated under a reduced pressure, and the residue was purified by flash chromatography (amine silica gel, n-hexane/ethyl acetate). To a solution of the resultant in tetrahydrofuran (3.8 ml) was added triphosgene (42 mg, 0.14 mmol) and triethylamine (66 µl, 0.47 mmol) at 0°C, and the mixture was stirred at room temperature for 6 hours. N-methylhydroxylamine hydrochloride (38 mg, 0.45 mmol) and triethylamine (132 µl, 0.95 mmol) were added to the reaction solution, and the mixture was stirred at 60°C for 15 hours. The reaction solution was cooled to room temperature, and distilled water was added to the reaction solution, followed by extraction with ethyl acetate. The organic layer was washed with distilled water and then with brine. The organic layer was dried over sodium sulfate and concentrated under a reduced pressure. The residue was purified by flash chromatography (amine silica gel, n-hexane/ethyl acetate) to give a title compound (110 mg, 0.23 mmol, 61 %) as a white amorphous product.

¹H-NMR (400 MHz, CDCl₃) δ: 1.65-1.73 (2H, m), 2.39-2.43 (2H, m), 2.47 (2H, d, J = 7.2 Hz), 2.95 (3H, s), 3.16-3.21 (2H, m), 3.84 (3H, s), 3.84 (3H, s), 3.94-3.96 (2H, m), 5.05-5.07 (2H, m), 5.63-5.70 (1H, m), 6.81 (1H, s), 6.89-6.92 (4H, m), 7.48 (2H, d, J = 8.8 Hz), 7.56 (2H, d, J = 8.8 Hz).

ESI-MS: m/z = 478 (M+H⁺).

### Example 74

### 4-(1,5-Bis(4-methoxyphenyl)oxazol-2-yl)-4-propyl-1-(N-hydroxy-N-methylcarbamoyl)piperidine

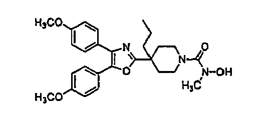

To a solution of 4-allyl-4-(1,5-bis(4-methoxyphenyl)oxazol-2-yl)-1-(N-hydroxy-N-methylcarbamoyl)piperidine (Example 73) (35 mg, 0.073 mmol) in ethanol (0.73 ml) was added 10%Pd-C (35 mg), and the mixture was stirred under a hydrogen atmosphere for 3 hours. The reaction solution was filtered, and the filtrate was concentrated under a reduced pressure. The residue was purified by flash chromatography (amine silica gel, n-hexane/ethyl acetate) to give a title compound (31 mg, 0.065 mmol, 89%) as a white amorphous product.

¹H-NMR (400 MHz, CDCl₃) δ: 1.12-1.28 (2H, m), 1.58-1.69 (6H, m), 2.43-2.46 (2H, m), 2.95 (3H, s), 3.10-3.17 (2H, m), 3.94 (3H, s), 3.98 (3H, s), 4.01-4.15 (2H, m), 6.82 (1H, s), 6.88-6.92 (4H, m), 7.48 (2H, d, J = 8.8 Hz), 7.57 (2H, d, J = 8.8 Hz).

ESI-MS: m/z = 480 (M+H⁺).

### Example 75

### 4-(1,5-Bis(4-tolyl)-1H-pyrazol-3-yl)-1-(N-hydroxy-N-methylcarbamoyl)piperidine

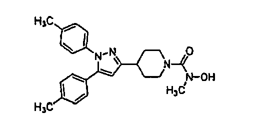

To a solution of 1-benzyloxycarbonyl-4-((4-tolyl)-1,3-dioxopropyl)piperidine (Reference Example 175) (1.27 g, 3.34 mmol) in ethanol (20 ml) was added 4-tolylhydrazine (530 mg, 3.34 mmol) and triethylamine (338 mg, 3.34 mmol), and the mixture was heated under reflux for 4 hours. The reaction solution was cooled to room temperature and concentrated under a reduced pressure. Distilled water was added to the residue, followed by extraction with ethyl acetate. The organic layer was dried over sodium sulfate and concentrated under a reduced pressure. The residue was purified by flash chromatography (silica gel, toluene/ethyl acetate) to give 1-benzyloxycarbonyl-4-(1,5-bis(4-tolyl)-1H-pyrazol-3-yl)piperidine (522 mg, 1.12 mmol, 34%).

To a solution of 1-benzyloxycarbonyl-4-(1,5-bis(4-tolyl)-1H-pyrazol-3-yl)piperidine (497 mg, 1.07 mmol) in ethanol (10 ml) was added 10% Pd-C (50% hydrous, 50 mg), and the mixture was stirred under a hydrogen atmosphere for 15 hours. The reaction solution was filtered, and the filtrate was concentrated under a reduced pressure. The residue was dissolved with the addition of tetrahydrofuran (20 ml), triphosgene (127 mg, 0.428 mmol) and triethylamine (108 mg, 1.07 mmol) were added at 0°C, and the mixture was stirred at the same temperature for 1 hour. N-methylhydroxylamine hydrochloride (134 mg, 1.61 mmol) and triethylamine (325 mg, 3.21 mmol) were added to the reaction solution, and the mixture was stirred 50°C for 4 hours to give a title compound (480 mg, 1.43 mmol, quant.) as a brown oil product. Distilled water was added to the reaction solution, followed by extraction with ethyl acetate. The organic layer was dried over sodium sulfate and concentrated under a reduced pressure. The residue was purified by flash chromatography (amine silica gel, n-hexane/ethyl acetate) to give a title compound (100 mg, 0.247 mmol, 23%) as a yellow amorphous product.

¹H-NMR (400 MHz, CDCl₃) δ: 1.73-1.84 (2H, m), 2.07-2.10 (2H, m), 2.33 (3H, s), 2.34 (3H, s), 2.99 (3H, s), 3.01-3.08 (2H, m), 4.13-4.17 (2H, m), 6.27 (2H, s), 6.87 (1H, brs), 7.09-7.17 (8H, m).

### Example 76

### 4-(4,5-Bis(4-tolyl)oxazol-2-yl)-1-(N-hydroxy-N-methylcarbamoyl)piperidine

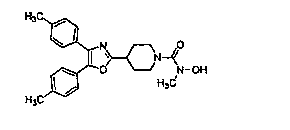

To a solution of triphosgene (169 mmol, 0.57 mmol) in tetrahydrofuran (15 ml) was added a solution of 4-(4,5-bis(4-tolyl)oxazol-2-yl)piperidine (Reference Example 191) (480 mg, 1.43 mmol) in tetrahydrofuran (5.0 ml) and triethylamine (145 mg, 1.43 mmol) at 0°C, and the mixture was stirred at the same temperature for 1 hour. N-methylhydroxylamine hydrochloride (239 mg, 2.86 mmol) and triethylamine (580 mg, 5.72 mmol) were added to the reaction solution, and the mixture was stirred at 50°C for 3 hours. Distilled water was added to the reaction solution, followed by extraction with ethyl acetate. The organic layer was dried over sodium sulfate and concentrated under a reduced pressure. The residue was purified by flash chromatography (amine silica gel, n-hexane/ethyl acetate) to give a title compound (411 mg, 1.01 mmol, 71 %) as a white amorphous product.

¹H-NMR (400 MHz, CDCl₃) δ: 1.94-2.02 (2H, m), 2.15-2.21 (2H, m), 2.37 (6H, s), 2.99 (3H, s), 3.11-3.17 (3H, m), 4.04-4.12 (2H, m), 6.82 (1H, brs), 7.16 (4H, d, J = 8.8 Hz), 7.45 (2H, d, J = 8.8 Hz), 7.51 (2H, d, J = 8.8 Hz).

### Example 77

### 5-((2-Hydroxy)ethyloxy)phenyl)-4-(4-methoxyphenyl)-1-(N-hydroxy-N-methylcarbamoyl)piperidine

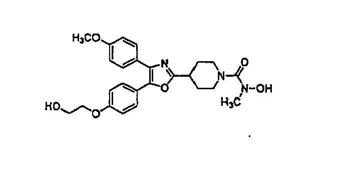

To a solution of 5-((2-(benzyloxy)ethyloxy)phenyl)-4-(4-methoxyphenyl)-1-(N-hydroxy-N-methyl)carbamoylpiperidine (Reference Example 198) (217 mg, 0.389 mmol) in methanol (5.0 ml) was added 10% Pd-C (20 mg), and the resultant was stirred under a hydrogen atmosphere for 2 hours. The reaction solution was filtered, and the filtrate was concentrated under a reduced pressure. The residue was purified by flash chromatography (amine silica gel, n-hexane/ethyl acetate) to give a title compound (122 mg, 0.261 mmol, 67%) as a white amorphous product.

¹H-NMR (400 MHz, CDCl₃) δ: 1.92-2.04 (2H, m), 2.13-2.20 (2H, m), 3.00 (3H, s), 3.08-3.18 (3H, m), 3.83 (3H, s), 3.96-4.15 (7H, m), 6.75 (1H, brs), 6.90 (2H, d, J = 8.8 Hz), 6.91 (2H, d, J = 8.8 Hz), 7.49 (2H, d, J = 8.8 Hz), 7.54 (2H, d, J = 8.8 Hz).

IR (KBr, cm⁻¹): 3395, 1640, 1614, 1519, 1500, 1251.

ESI-MS: m/z = 468 (M+H⁺).

Drug efficacy of the compound of the present invention was evaluated in Example 78 to 81 below.

### Example 78

### Evaluation of analgesic effects

ddY mice were grown for at least 16 hours under fasting conditions while free water intake was allowed, and a solution of the test compound or a solvent was orally administered thereto (0.1 ml/10 g). As a solvent for a solution of the test compound, dimethyl sulfoxide (DMSO):Tween 80:27% hydroxypropyl-β-cyclodextrin (hereafter abbreviated to "HP-β-CD") (1:1:8) or 27% HP-β-CD was used. A solution of 0.6% acetic acid (0.1 ml/10 g) was administered intraperitoneally 45 minutes after administration to induce a writhing reaction (i.e., a movement of stretching or arching the body). The number of the writhing reactions that had occurred for the period from 10 minutes after administration of the acetic acid solution to 10 minutes thereafter was counted, and the number of times was designated as an indicator of pain. The number of times of reactions in the group to which a solvent had been administered was designated as 100%, and the concentration of the test compound that had inhibited 50% of such reaction was designated as the ED₅₀ value.

The results are shown in Table 2.

**Table 2**

| Compound | ED₅₀ (mg/kg)[90% confidence limit] |
|---|---|
| Example 1 | 2.02 [1.20-2.91] |
| Example 4 | 4.02 [2.44-7.99] |
| Example 7 | 3.27 [1.35-8.75] |
| Example 9 | 2.63 [0.81-6.00] |
| Example 12 | 2.29 [1.25-3.55] |
| Example 13 | 2.62 [1.32-4.52] |
| Example 14 | 4.17 [2.35-10.05] |
| Example 20 | 6.45 [3.86-10.67] |
| Example 27 | 2.46 [1.30-3.98] |
| Example 40 | 1.64 [0.51-2.82] |
| Example 47 | 3.30 [2.10-5.33] |
| Example 56 | 4.61 [2.25-21.99] |
| Example 57 | 9.79 [6.61-14.55] |
| Example 67 | 2.51 [1.42-3.93] |
| Example 69 | 3.19 [1.79-5.78] |

The test results demonstrate that the compound of the present invention can provide excellent analgesic effects with oral administration.

### Example 79

### Effects on partial sciatic nerve ligation model in mice

Analgesic effects of the compound of the present invention were evaluated using the partial sciatic nerve ligation model (Seltzer models) in mice, with which neuropathic pain can be evaluated.

### Experimental method

Models of neuropathic pain were prepared in accordance with the method of Seltzer et al. ICR mice (5-week-old, male) were put under anesthesia with sodium pentobarbital (70 mg/kg, i.p.) to expose the sciatic nerve of the femoral region of the right posterior limb, and half of the sciatic nerve was strongly and triply ligated using 8-0 silk yarn (Natsume Seisakusho, Co., Ltd.) under a microscope (this group is designated as the "ligation group"). The group that had been subjected to exposure of the sciatic nerve without ligation was designated as a sciatic nerve control group (i.e., the "sham group"). Effects on neuropathic pain were evaluated (i.e., the von Frey test) by accommodating mice for at least 1 hour in an acrylic cage for measurement (Natsume Seisakusho, Co., Ltd.) provided on a mesh, applying tactile stimulus by applying a filament to soles of the both posterior legs for three seconds using a filament (North Coast Medical, Inc. CA, USA) that exerts pressure of 0.16 g, repeating such stimulus application three times at 3-second-intervals, scoring the levels of escape reactions upon application of mechanical tactile stimulus (i.e., 0: no reaction; 1: mild and insignificant escape reactions from stimuli; 2: quick escape reactions from stimuli that do not involve flinching (i.e., a movement of quickly and continuously shaking legs) or licking (i.e., a movement of licking legs); and 3: quick escape reactions that involve flinching or licking), and designating the total of the three scores as an indicator of analgesic effects. The von Frey tests were performed 1 hour, 2 hours, and 3 hours after the oral administration for determining the pre-values prior to the administration of the test substances, which took place 7 days after the ligation surgery of sciatic nerve, and such values were designated as the indicators of analgesic effects. Gabapentin (30 mg/kg, po) was used as a positive control. As a solvent for the test compound, 27%HP-β-CD was used.

### Results

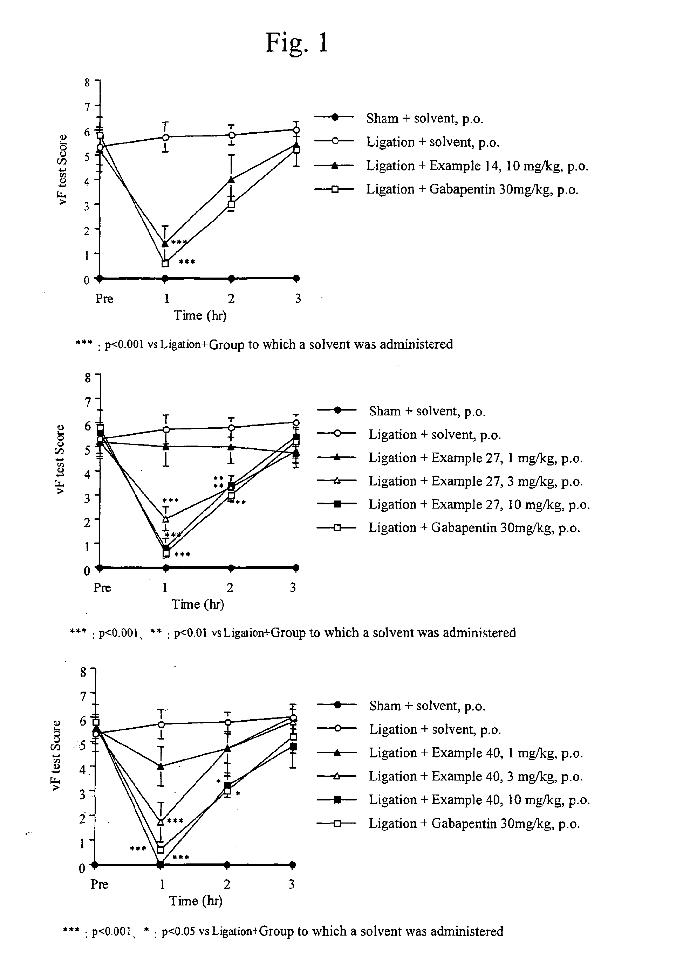

The results are shown in Fig. 1. The obtained data were processed by performing a paired t-test amongst several groups and correcting the data via Dunnett's method. The results of the von Frey test were significantly improved with oral administration of 10 mg/kg of the compound of Example 14 and with oral administration of 3 and 10 mg/kg of the compounds of Example 27 and Example 40, respectively. These test results demonstrate that the ureide derivative (I) of the present invention or a pharmaceutically acceptable salt thereof is effective against neuropathic pain.

### Example 80: Formalin test in mice

### Experimental method

ddY mice were grown for at least 16 hours under fasting conditions while free water intake was allowed, and a solution of the test compound or a solvent was orally administered thereto (0.1 ml/10 g). As a solvent for a solution of the test compound, 27% HP-β-CD was used. Thirty minutes after administration, 20 µl of a 2.5% formalin solution was subcutaneously administered to the ichnograms of right posterior legs of mice to induce a movement of licking. A movement of mice to lick the treated legs was designated as an indicator of pain, and the licking time was measured at 5-minute intervals for 30 minutes. The reaction that had occurred within 10 minutes immediately after administration of the formalin solution was designated as the phase I reaction, and the reaction that had occurred 10 to 30 minutes after administration was designated as the phase II reaction.

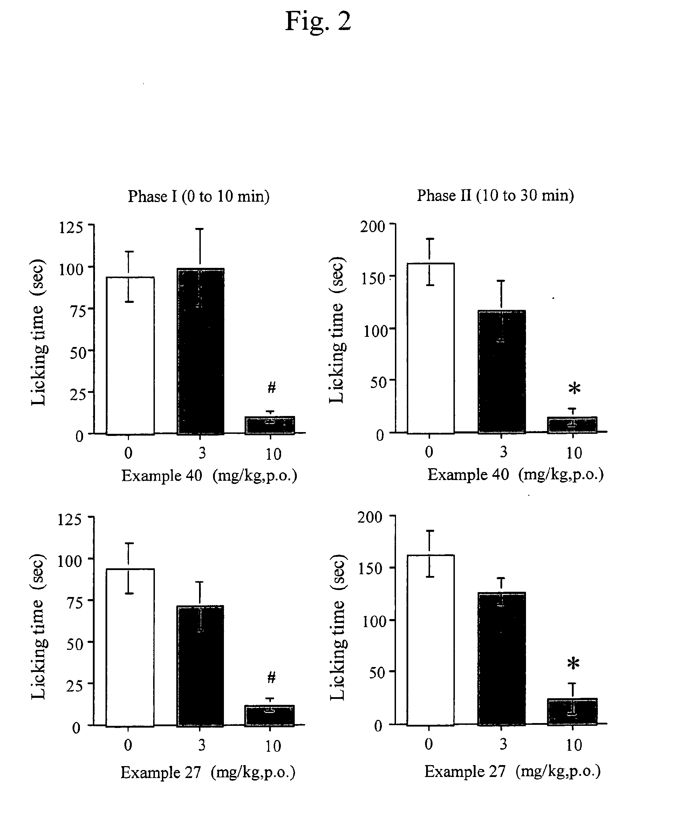

The results are shown in Fig. 2. Oral administration of the compounds of Example 27 and Example 40 in amounts of 10 mg/kg significantly improved the indicators of two-phase pains induced by formalin in both phase I and phase II.

The test results demonstrate that the compounds of the present invention exhibit analgesic effects with oral administration thereof to model animals of different types of pain.

### Example 81: Measurement of activity of inhibiting COX-1 and COX-2

### 1. Measurement of activity of inhibiting COX-1

Human blood that had been sampled with the use of heparin (19 units/ml) was used. Blood was fractionated to reaction tubes in amounts of 500 µl, the test compound or solvent was added thereto, and the resultants were incubated at 37°C for 60 minutes. Dimethyl sulfoxide (DMSO) was used as a solvent for the test compound. Thereafter, ionomycin (30 µM) was added, and the reaction was allowed to proceed for 30 minutes. After the completion of the reaction, the supernatant (blood plasma) was recovered via centrifugation at 4°C, and the amount of thromboxane B₂ in the plasma was quantified using a kit (519031, Cayman Chemical). The amount of thromboxane B₂ produced via the addition of ionomycin was designated as 100%, and the concentration of the test compound that inhibits 50% of such reaction was represented by the IC₅₀ value.

### 2. Measurement of activity of inhibiting COX-2

Human blood that had been sampled with the use of heparin (19 units/ml) was used. Blood was fractionated to reaction tubes in amounts of 500 µl, aspirin (12 µg/ml) was added thereto, and the resultants were incubated at 37°C for 6 hours. Thereafter, the test compound or solvent, and lipopolysaccharide (10 µg/ml) were added, and the reaction was allowed to proceed for 18 hours. After the completion of the reaction, the supernatant (blood plasma) was recovered via centrifugation at 4°C, and the amount of prostaglandin E₂ in the plasma was quantified using a kit (514010, Cayman Chemical). Dimethyl sulfoxide (DMSO) was used as a solvent for the test compound. The amount of prostaglandin E₂ produced via the addition of lipopolysaccharide was designated as 100%, and the concentration of the test compound that inhibits 50% of such reaction was represented by the IC₅₀ value.

The results are shown in Table 3.

**Table 3**

| Compound | IC₅₀ COX-1 (µM) | IC₅₀ COX-2 (µM) |
|---|---|---|
| Example 1 | <0.1 | >10 |
| Example 4 | <0.1 | >10 |
| Example 7 | <0.1 | >10 |
| Example 9 | <0.1 | >10 |
| Example 12 | <0.1 | >10 |
| Example 14 | <0.1 | >10 |
| Example 15 | <0.1 | >10 |
| Example 16 | <0.1 | >10 |
| Example 19 | <0.1 | >10 |
| Example 20 | <0.1 | >10 |
| Example 24 | <0.1 | >10 |
| Example 27 | <0.1 | >10 |
| Example 34 | <0.1 | >10 |
| Example 38 | <0.1 | >10 |
| Example 39 | <0.1 | >10 |
| Example 40 | <0.1 | >10 |
| Example 42 | <0.1 | >10 |
| Example 47 | <0.1 | >10 |
| Example 48 | <0.1 | >10 |
| Example 55 | <0.1 | >10 |
| Example 56 | <0.1 | >10 |
| Example 60 | <0.1 | >10 |
| Example 61 | <0.1 | >10 |
| Example 68 | <0.1 | >10 |
| Example 69 | <0.1 | >10 |
| Example 74 | <0.1 | >10 |
| Example 77 | <0.1 | >10 |

The test results demonstrate that the compound of the present invention has activity of selectively inhibiting COX-1.

### Comparative Example 1

### Mouse acetic acid writhing test

The compound of Reference Example 78 is disclosed in the claims of JP Patent Publication (kohyo) No. 2006-517535 A, the compounds of Reference Examples 93 and 199 are disclosed in the claims of JP Patent No. 3003148, and the compound of Reference Example 103 is disclosed in the claims of JP Patent Publication (kohyo) No. 2006-514095 A. Thus, these compounds were compared with the compounds of the present invention having similar structures in terms of analgesic effects with oral administration (Table 4) or subcutaneous administration (Table 5). Dimethyl sulfoxide (DMSO):Tween 80:27% HP-β-CD (1:1:8) or 27% HP-β-CD was used as a solvent for the test compound.

**Table 4**

| Oral administration | |
|---|---|
| Compound | ED₅₀ (mg/kg) [90% confidence limit] |
| Example 1 | 2.02 [1.20-2.91] |
| Reference Example 78 Sulfate | >100 |

The test results demonstrate that the compound of Example 1 of the present invention exhibits remarkably excellent analgesic effects compared with the compound of Reference Example 78 (i.e., sulfate) having a similar structure (i.e., at least 49 times higher in terms of activity ratio). Also, the ureide structure of the compound of the present invention was found to be important for the completion of the present invention.

**Table 5**

| Subcutaneous administration | |
|---|---|
| Compound | ED₅₀ (mg/kg) [90% confidence limit] |
| Example 27 | 1.82 [0.47-3.34] |
| Reference Example 93 Toluenesulfonate | >100 |
| Reference Example 199 | 34.4 [20.21-63.21] |
| Example 40 | 2.47 [1.64-3.49] |
| Reference Example 103 Sulfate | >100 |

The test results demonstrate that the compound of Example 27 of the present invention has analgesic effects apparently superior to those of the compound of Reference Example 93 (toluenesulfonate) and the compound of Reference Example 199 having similar structures (i.e., activity ratios of at least 54 times and about 19 times higher) and that the compound of Example 40 has analgesic effects apparently superior to those of the compound of Reference Example 103 (sulfate) having a similar structure (i.e., at least 40 times higher in terms of an activity ratio). Also, the ureide structure of the compound of the present invention, particularly, the oxyureide structure, was found to be particularly important for the completion of the invention.

### Comparative Example 2

### Mouse models of partial sciatic nerve ligation (Seltzer models)

The compound of Reference Example 78 is disclosed in the claims of JP Patent Publication (kohyo) No. 2006-517535 A, the compounds of Reference Example 93 and Reference Example 199 are disclosed in the claims of JP Patent No. 3003148, and the compound of Reference Example 103 is disclosed in the claims of JP Patent Publication (kohyo) No. 2006-514095 A. Accordingly, sulfate of Reference Example 78, toluenesulfonate of Reference Example 93, the free compound of Reference Example 199, and sulfate of Reference Example 103 were used as test compounds and evaluated using mouse models of partial sciatic nerve ligation, which were similar to those used in Example 79. 27% HP-β-CD was used as a solvent for the test compounds.

### Results

The results are shown in Fig. 3. The obtained data were processed by performing a paired t-test amongst several groups and correcting the data via Dunnett's method. The test results demonstrate that oral administration of the compound up to 100 mg/kg would not result in significant inhibitory effects. Accordingly, the ureide structure of the compound of the present invention was found to be important for the completion of the invention in respect of neuropathic pain.

All publications, patents, and patent applications cited herein are incorporated herein by reference in their entirety.

## Claims

1. An ureide derivative represented by general formula (I): 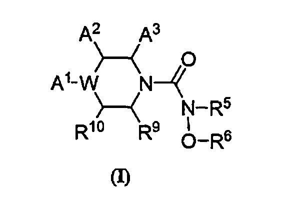 wherein either A¹ or A² represents a hydrogen atom, and the other represents formula (IIa), (IIb), or (IIc): 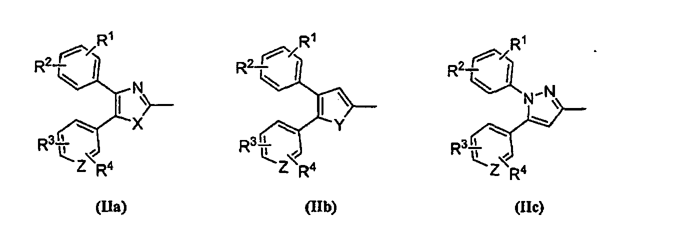 wherein A³ represents a hydrogen atom, a halogen atom, an alkyl group having 1 to 6 carbon atoms, a cyano group, or a hydroxyl group; X and Y each independently represent O, S, or NR⁷; Z represents CH or N; W represents CR⁸ or N; R¹, R², R³, and R⁴ each independently represent a hydrogen atom, a halogen atom, an alkyl group having 1 to 6 carbon atoms, a haloalkyl group having 1 to 3 carbon atoms, an alkoxy group having 1 to 4 carbon atoms, a hydroxyl group, or a 2-hydroxyethoxy group; R⁵ represents a hydrogen atom, an alkyl group having 1 to 6 carbon atoms, or -(CH₂)ₘCO₂R¹¹, wherein m is an integer between 0 and 3; R⁶ represents a hydrogen atom, an alkyl group having 1 to 6 carbon atoms, an aralkyl group having 7 to 12 carbon atoms, an acyl group having 2 to 8 carbon atoms, or -(CH₂)ₙCO₂R¹², wherein n is an integer between 0 and 3; R⁷ represents a hydrogen atom, an alkyl group having 1 to 6 carbon atoms, an acyl group having 2 to 8 carbon atoms, or -(CH₂)₁CO₂R¹³, wherein 1 is an integer between 0 and 3; R⁸ represents a hydrogen atom, an alkyl group having 1 to 6 carbon atoms, an alkenyl group having 2 to 6 carbon atoms, a cyano group, a phenyl group, a carbamoyl group, an amino group, -(CH₂)ₖOR¹⁴, wherein k is an integer between 0 and 3, or -(CH₂)ⱼCO₂R¹⁵, wherein j is 0 or 1; R⁹ and R¹⁰ each independently represent a hydrogen atom, a halogen atom, an alkyl group having 1 to 6 carbon atoms, a cyano group, or a hydroxyl group, A³ and R⁹ may together form -(CH₂)₂-; R¹¹ and R¹³ each independently represent a hydrogen atom or an alkyl group having 1 to 6 carbon atoms; R¹² and R¹⁵ each independently represent a hydrogen atom, an alkyl group having 1 to 6 carbon atoms, or an aralkyl group having 7 to 12 carbon atoms; and R¹⁴ represents a hydrogen atom, an alkyl group having 1 to 6 carbon atoms, or an acyl group having 2 to 8 carbon atoms, or a pharmaceutically acceptable salt thereof.

2. The ureide derivative according to clauses 1, wherein A¹ represents formula (IIa), (IIb), or (IIc) and A² represents a hydrogen atom, or a pharmaceutically acceptable salt thereof.

3. The ureide derivative according to clause 1 or 2, wherein A¹ represents formula (IId), (IIe), or (IIf): 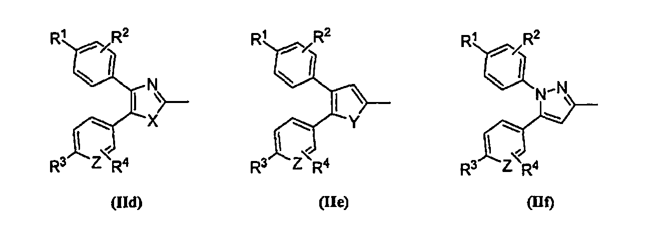 wherein X, Y, Z, R¹, R², R³, and R⁴ are as defined above, or a pharmaceutically acceptable salt thereof.

4. The ureide derivative according to any one of clauses 1 to 3, wherein A³ represents a hydrogen atom, a fluorine atom, a chlorine atom, a methyl group, an ethyl group, a cyano group, or a hydroxyl group, or a pharmaceutically acceptable salt thereof.

5. The ureide derivative according to any one of clauses 1 to 4, wherein X represents O or S and Y represents O, or a pharmaceutically acceptable salt thereof.

6. The ureide derivative according to any one of clauses 1 to 5, wherein W represents CR⁸, wherein R⁸ is as defined above, or a pharmaceutically acceptable salt thereof.

7. The ureide derivative according to any one of clauses 1 to 6, wherein R¹, R², R³, and R⁴ each independently represent a hydrogen atom, a fluorine atom, a chlorine atom, a methyl group, an ethyl group, an isopropyl group, a tert-butyl group, a difluoromethyl group, a trifluoromethyl group, a methoxy group, an ethoxy group, an isopropyloxy group, a hydroxyl group, or a 2-hydroxyethoxy group, or a pharmaceutically acceptable salt thereof.

8. The ureide derivative according to any one of clauses 1 to 7, wherein R⁵ represents a hydrogen atom, a methyl group, an ethyl group, an isopropyl group, a cyclohexyl group, a tert-butyl group, -CH₂CO₂CH₃, -CH₂CO₂CH₂CH₃, or -CH₂CO₂H, or a pharmaceutically acceptable salt thereof.

9. The ureide derivative according to any one of clauses 1 to 8, wherein R⁶ represents a hydrogen atom, a methyl group, an ethyl group, an isopropyl group, a tert-butyl group, a benzyl group, an acetyl group, a benzoyl group, -CO₂CH₃, -CH₂CO₂CH₃, -CH₂CO₂CH₂CH₃, or -CH₂CO₂H, or a pharmaceutically acceptable salt thereof.

10. The ureide derivative according to any one of clauses 1 to 9, wherein R⁸ represents a hydrogen atom, a methyl group, an ethyl group, a propyl group, 2-propenyl group, a cyano group, a phenyl group, a carbamoyl group, an amino group, a hydroxyl group, a hydroxymethyl group, an acetoxymethyl group, -CO₂CH₃, -CO₂CH₂CH₃, -CO₂CH₂Ph, - CO₂H, -CH₂CO₂CH₃, -CH₂CO₂CH₂CH₃, or -CH₂CO₂H, or a pharmaceutically acceptable salt thereof.

11. The ureide derivative according to any one of clauses 1 to 10, wherein R⁹ and R¹⁰ each independently represent a hydrogen atom, a fluorine atom, a chlorine atom, a methyl group, an ethyl group, a cyano group, or a hydroxyl group, and R⁹ and A³ may together form - (CH₂)₂-, or a pharmaceutically acceptable salt thereof.

12. The ureide derivative according to any one of clauses 1 to 10, wherein all of R⁹, R¹⁰, and A³ represent a hydrogen atom, or a pharmaceutically acceptable salt thereof.

13. A pharmaceutical comprising the ureide derivative according to any one of clauses 1 to 12 or a pharmaceutically acceptable salt thereof.

14. An analgesic comprising the ureide derivative according to any one of clauses 1 to 12 or a pharmaceutically acceptable salt thereof.

15. A therapeutic or preventive agent for neuropathic pain comprising the ureide derivative according to any one of clauses 1 to 12 or a pharmaceutically acceptable salt thereof.

16. A method of relieving pain comprising administering the ureide derivative according to any one of clauses 1 to 12 or a pharmaceutically acceptable salt thereof.

17. A method of treating or preventing neuropathic pain comprising administering the ureide derivative according to any one of clauses 1 to 12 or a pharmaceutically acceptable salt thereof.

18. Use of the ureide derivative according to any one of clauses 1 to 12 or a pharmaceutically acceptable salt thereof for the manufacture of an analgesic.

19. Use of the ureide derivative according to any one of clauses 1 to 12 or a pharmaceutically acceptable salt thereof for the manufacture of a therapeutic or preventive agent for neuropathic pain.
